# EUROPEAN PATENT APPLICATION

(11) **EP 2 460 805 A1**
(43) Date of publication of application: **06.06.2012**
(21) Application number: 10804475.1
(22) Date of filing: 29.07.2010
(51) Int. Cl.: C07D 487/04, A61K 31/519, A61P 11/06, A61P 17/00, A61P 29/00, A61P 35/00, A61P 35/02, A61P 37/06, A61P 37/08, A61P 43/00, C07D 498/04

(54) **FUSED IMIDAZOLE DERIVATIVE HAVING TTK INHIBITORY ACTION**

(30) Priority: 30.07.2009 JP 2009178452; 26.05.2010 JP 2010121045
(71) Applicant: Oncotherapy Science, Inc., Kawasaki-shi Kanagawa 213-0012 (JP)
(72) Inventor: KUSAKABE, Ken-ichi, Osaka-shi Osaka 553-0002 (JP); YOSHIDA, Hiroshi, Osaka-shi Osaka 553-0002 (JP); NOZU, Kohei, Sapporo-shi Hokkaido 001-0021 (JP); HASHIZUME, Hiroshi, Osaka-shi Osaka 553-0002 (JP); TADANO, Genta, Osaka-shi Osaka 553-0002 (JP); SATO, Jun, Osaka-shi Osaka 553-0002 (JP); TAMURA, Yuusuke, Osaka-shi Osaka 553-0002 (JP); MITSUOKA, Yasunori, Osaka-shi Osaka 553-0002 (JP)
(74) Representative: HOFFMANN EITLE
(86) International application number: PCT/JP2010/062747
(87) International publication number: WO 2011/013729

(57) **Abstract**

Provided are a compound represented by general formula (1) and having a TTK inhibitory action and a medicine containing the compound. In formula (1), (X, Y, V, W) is (-N=, =CR¹-, =N-, -CR⁷=), (-CR²=, =N-, =N-, -CR⁷=), etc.; A is an (un)substituted aromatic hydrocarbon ring, etc.; L is a single bond, -C(=O)-NR^{A}-, etc.; Z is a group represented by the formula -NR³R⁴ or a group represented by the formula -OR⁵; R¹ to R³, R⁶, and R⁷ each is a hydrogen atom, etc.; R⁴ and R⁵ each is an (un)substituted alkyl, etc.; and R⁸ is an (un)substituted cycloalkyl, etc.

## Description

### TECHNICAL FIELD

The present invention relates to a fused imidazole derivative having inhibitory/suppressing action for TTK (TTK protein kinase) activity. In another aspect, the present invention relates to a medicament containing the fused imidazole derivative.

### BACKGROUND ART

Protein kinase refers to an enzyme that adds a phosphate group (phosphorylates) to another protein molecule. The activity of the protein kinase involves transferring a phosphate group from ATP to the hydroxy group of an amino acid residue in a protein molecule by forming a covalent bond. Many protein kinases are classified in kinases (serine/threonine kinase) which react with the hydroxy group of serine or threonine of a protein molecule, a kinase (tyrosine kinase) which reacts with the hydroxy group of tyrosine, and a kinase (dual-specificity kinases) which react all of these three types. The activity of a protein kinase is accurately controlled. In some cases, a protein kinase itself is also controlled by phosphorylation. Such control is mediated by e.g., binding of another activation (or suppression) protein and a low molecular weight compound and change in localization of them within a cell. Malfunction of the kinase often causes diseases.

TTK protein kinase is a dual-specific kinase, which phosphorylates serine, threonine and tyrosine residues of a protein serving as a substrate (see, for example, Non Patent Literature 1). A kinase domain, which is required for exhibiting kinase activity, has been known (see, for example, Non Patent Literatures 1 and 2). As an endogenic substrate, e.g., Madl (see, for example, Non Patent Literature 3), Spcl10p (Nuflp) (see, for example, Non Patent Literature 4), CHK2 (see, for example, Non Patent Literature 5) and Borealin (see, for example, Non Patent Literature 6) are known. TTK expression correlates to cell growth and plays a role in regulating cell cycle. Furthermore, Patent Literature 1 refers to expression of TTK in malignant ovarian cancer and discloses a screening method including a step of measuring expression amount of TTK. Furthermore, Patent Literature 2 is a patent application concerning a method for identifying cancer cells by detecting TTK activity and concerning specifying a medicinal agent suppressing tumor growth, and discloses a method for measuring TTK activity using tau, cdc25 and partial peptides of these as a substrate. Furthermore, Patent Literature 3, as TTK activity screening method, discloses a method for measuring TTK activity by using a partial peptide of p38 MAPK as a substrate. Moreover, examples of a TTK inhibitory agent include those described in Patent Literatures 4 and 5.

Examples of the fused imidazole derivative include imidazopyrazine and imidazopyridazine derivatives. Examples of imidazopyrazine and imidazopyridazine derivatives include those described in Patent Literatures 6 to 16 and Non Patent Literature 7; however, none of them are known as a TTK inhibitory agent.

### PRIOR ART

### PATENT LITERATURE

PTL 1: International Publication No. WO01/94629
PTL 2: International Publication No. WO02/068444
PTL 3: Japanese Patent Laid-Open No. 2007-104911
PTL 4: International Publication No. WO2009/024824
PTL 5: International Publication No. WO2009/032694
PTL 6: International Publication No. WO2007/056468
PTL 7: International Publication No. WO2007/058873
PTL 8: International Publication No. WO2007/058942
PTL 9: International Publication No. WO2009/017701
PTL 10: International Publication No. WO2008/030795
PTL 11: U.S. Patent Application (Publication) No. 2004/0220189
PTL 12: U.S. Patent Application (Publication) No. 2005/0009832
PTL 13: U.S. Patent Application (Publication) No. 2008/0045536
PTL 14: International Publication No. WO2007/013673
PTL 15: International Publication No. WO2009/024585
PTL 16: International Publication No. WO2008/079460 NON PATENT LITERATURE

NPL 1: Mills et al., J. Biol. Chem. (1992) 267, 16000-16006
NPL 2: Lindberg et al., Oncogene (1993) 8, 351-359
NPL 3: Liu et al., Mol. Biol, Cell. (2003) 14, 1638-51
NPL 4: Friedman et al., J. Biol. Chem. (2001) 276, 17958-17967
NPL 5: Wei et al., J. Biol. Chem. (2005) 280, 7748-7757
NPL 6: Jelluma et al., Cell (2008) 25, 233-246
NPL 7: Goodacre et al., Bioorganic & Medicinal Chemistry Letters (2006) 16 (6), 1582-1585

### SUMMARY OF INVENTION

### TECHNICAL PROBLEM

An object of the present invention is to provide an effective inhibitory agent for TTK protein kinase and provide, by extension, an effective medicament.

### SOLUTION TO PROBLEM

The above object was attained by a compound of the present invention and by related inventions thereof (for example, a pharmaceutical composition, a TTK inhibitory agent).

The present applicant found a series of novel compounds that inhibit TTK kinase action and have specific properties, which are useful for preparing a pharmaceutical product for treating the aforementioned disease(s). Accordingly, the compound of the invention is useful for a disease conceivably effectively treated by inhibiting TTK kinase action.

Thus, the present invention provides, for example, the following items.

(1A) A compound represented by Formula (I'):

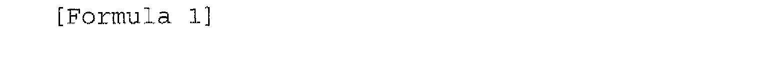

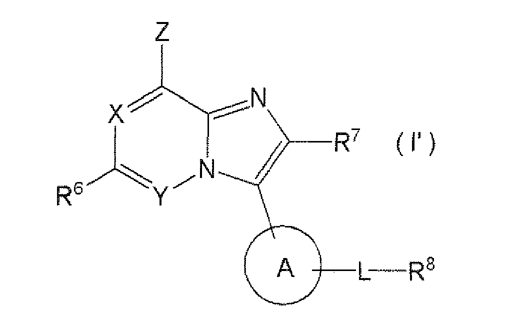

wherein
X and Y are any one of the following (X, Y) combinations:
(-N=, =CR¹-), (-CR²=, =N-) and (-N=, =N-),
R¹ and R² are each independently hydrogen, a halogen, a hydroxy, a cyano, a nitro, a carboxy, a substituted or unsubstituted alkyl, a substituted or unsubstituted alkenyl or a substituted or unsubstituted alkynyl,
Z is a group represented by Formula: -NR³R⁴ or a group represented by Formula: -OR⁵,
R³ is hydrogen or a substituted or unsubstituted alkyl,
R⁴ and R⁵ are each independently hydrogen, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl or a substituted or unsubstituted heterocyclyl,
R⁶ is hydrogen, a halogen, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted amino, a substituted or unsubstituted acyl, a substituted or unsubstituted alkoxy, a substituted or unsubstituted carbamoyl, a group represented by Formula: -SO₂-R', a group represented by Formula: -SO-R' or a group represented by Formula: -SR',
R' is hydrogen, a substituted or unsubstituted alkyl, a substituted or unsubstituted amino, a substituted or unsubstituted aryl, a substituted or unsubstituted cycloalkyl, a substituted or unsubstituted heteroaryl or a substituted or unsubstituted heterocyclyl,
R⁷ is hydrogen, a halogen, a hydroxy, a cyano, a nitro, a carboxy, a substituted or unsubstituted alkyl, a substituted or unsubstituted alkenyl or a substituted or unsubstituted alkynyl,
A is a substituted or unsubstituted aromatic hydrocarbon ring, a substituted or unsubstituted aromatic heterocyclic ring, a substituted or unsubstituted nonaromatic hydrocarbon ring or a substituted or unsubstituted nonaromatic heterocyclic ring,
L is a single bond, -C(=O)-NR^{A}-, -NR^{B}-C(=O)-, -S(O)ₙ-NR^{C}-, -NR^{D}-S(O)ₙ-, a substituted or unsubstituted alkylene, a substituted or unsubstituted alkenylene or a substituted or unsubstituted alkynylene,
R⁸ is hydrogen, a halogen, a hydroxy, a cyano, a carboxy, a substituted or unsubstituted alkoxy, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl or a. substituted or unsubstituted amino,
R^{A}, R^{B}, R^{C}, R^{D} is each independently hydrogen, a substituted or unsubstituted alkyl, or
R⁸ and R^{A} or R^{C} may be taken together with an adjacent nitrogen atom to form a substituted or unsubstituted nitrogen-containing heterocyclic ring,
n is an integer of 1 or 2,
when (X, Y) is (-N=, =CR¹-) and Z is a group represented by Formula: -NR³R⁴, L is -C(=O)-NR^{A}-;

with the proviso that when (X, Y) is (-N=, =CR¹-) and Z is a group represented by Formula: -OR⁵, R⁶ is not a halogen,
when (X, Y) is (-N=, =CR¹-), Z is a group represented by Formula: -NR³R⁴ and L is -C(=O)-NR^{A}-, R⁸ is not an alkyl substituted with an amino, a hydroxy, a pyridyl or a heterocyclyl, or hydrogen, and R⁸ and R^{A} do not form a substituted or unsubstituted nitrogen-containing heterocyclic ring together with an adjacent nitrogen atom;
with the proviso that the following compounds are excluded:

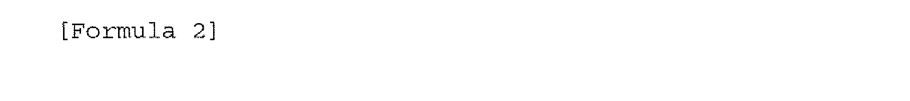

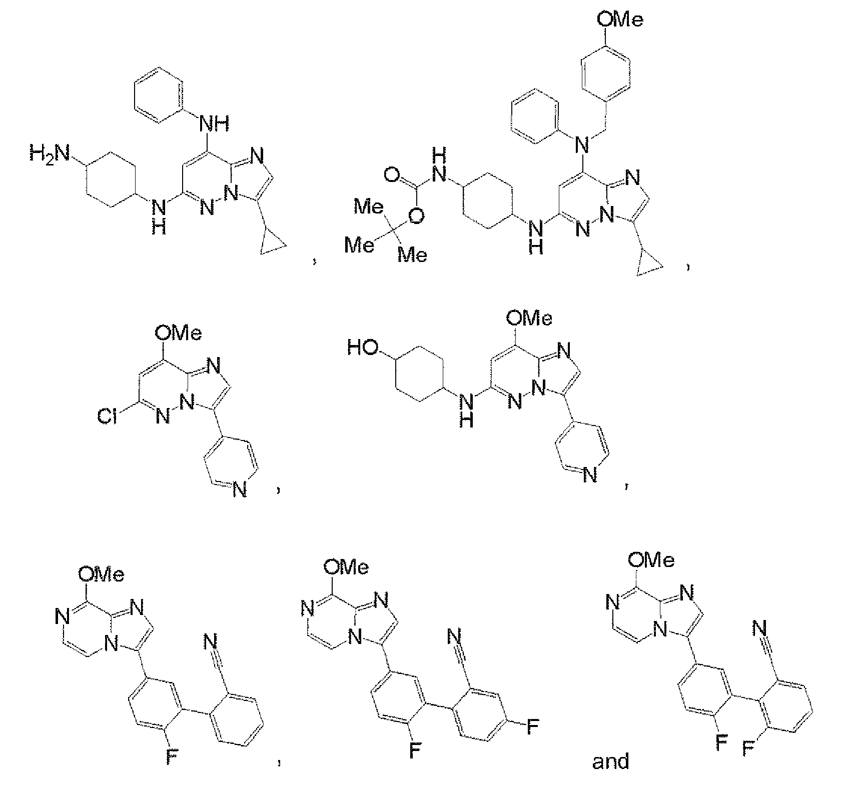

; a pharmaceutically acceptable salt or a solvate thereof.

(2A) The compound according to item (1A) in which (X, Y) is (-N=, =CR¹-) or (-CR²=, =N-) where R¹ and R² are the same as defined in item (1A); a pharmaceutically acceptable salt thereof or a solvate thereof.

(3A) The compound according to item (1A) or (2A) in which (X, Y) is (-N=, =CH-) or (-CH=, =N-); a pharmaceutically acceptable salt thereof or a solvate thereof.

(4A) The compound according to any one of items (1A) to (3A), in which Z is a group represented by Formula: -NR³R⁴ where R³ and R⁴ are the same as defined in item (1A); a pharmaceutically acceptable salt thereof or a solvate thereof.

(5A) The compound according to any one of items (1A) to (4A), in which Z is a group represented by Formula: -NHR⁴ where R⁴ is the same as defined in item (1A); a pharmaceutically acceptable salt thereof or a solvate thereof.

(6A) The compound according to any one of items (1A) to (5A), in which R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl; a pharmaceutically acceptable salt thereof or a solvate thereof.

(7A) The compound according to any one of items (1A) to (6A), in which Z is a group represented by Formula: -OR⁵ where R⁵ is the same as defined in item (1A); a pharmaceutically acceptable salt thereof or a solvate thereof.

(8A) The compound according to any one of items (1A) to (7A), in which R⁵ is a substituted or unsubstituted alkyl or a substituted or unsubstituted heterocyclylalkyl; a pharmaceutically acceptable salt thereof or a solvate thereof.

(9A) The compound according to any one of items (1A) to (8A), in which A is a substituted or unsubstituted aromatic hydrocarbon ring or a substituted or unsubstituted aromatic heterocyclic ring; a pharmaceutically acceptable salt thereof or a solvate thereof.

(10A) The compound according to any one of items (1A) to (9A), in which L is -C(=O)-NR^{A}-, -NR^{B}-C(=O)- or -S(O)ₙ-NR^{C}- where R^{A}, R^{B}, R^{C} and n are the same as defined in item (1A); a pharmaceutically acceptable salt thereof or a solvate thereof.

(11A) The compound according to any one of items (1A) to (10A), in which R⁸ is a substituted or unsubstituted alkyl, a substituted or unsubstituted cycloalkyl or a substituted or unsubstituted aryl; a pharmaceutically acceptable salt thereof or a solvate thereof.

(12A) The compound according to any one of items (1A) to (11A), in which L is -C(=O)-NH-, and R⁸ is a substituted or unsubstituted cycloalkyl; a pharmaceutically acceptable salt thereof or a solvate thereof.

(13A) The compound according to any one of items (1A) to (12A), in which R⁶ is hydrogen, a halogen, a substituted or unsubstituted alkyl, a substituted or unsubstituted alkenyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted alkoxy, a substituted or unsubstituted acyl or a substituted or unsubstituted amino; a pharmaceutically acceptable salt thereof or a solvate thereof.

(14A) The compound according to any one of items (1A) to (13A), in which R⁷ is hydrogen; a pharmaceutically acceptable salt thereof or a solvate thereof.

(15A) The compound according to any one of items (1A) to (14A), in which
(X, Y) is (-N=, =CR¹-) where R¹ is the same as defined in item (1A),
Z is a group represented by Formula: -NHR⁴,
R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is unsubstituted alkyl; A is a substituted or unsubstituted aromatic hydrocarbon ring,
L is -C(=O)-NH-, and
R⁸ is a substituted or unsubstituted cycloalkyl; a pharmaceutically acceptable salt thereof or a solvate thereof.

(16A) The compound according to any one of items (1A) to (15A), in which
(X, Y) is (-CR²=, =N-) where R² is the same as defined in item (1A), and
A is a substituted or unsubstituted aromatic hydrocarbon ring; a pharmaceutically acceptable salt thereof or a solvate thereof.

(17A) A pharmaceutical composition containing the compound according to any one of items (1A) to (16A), a pharmaceutically acceptable salt thereof or a solvate thereof.

(18A) The pharmaceutical composition according to item (17A), as a TTK inhibitory agent.

(19A) The pharmaceutical composition according to item (17A) or (18A), as a medicament for treating or preventing a disease, disorder or condition associated with TTK.

(20A) The pharmaceutical composition according to any one of items (17A) to (19A), for treating and/or preventing a cancer.

(21A) A method for preventing or treating a cancer, comprising administering the compound according to any one of items (1A) to (16A), a pharmaceutically acceptable salt thereof or a solvate thereof.

(22A) Use of the compound according to any one of items (1A) to (16A), a pharmaceutically acceptable salt thereof or a solvate thereof, in preparation of a medicament for treating and/or preventing a cancer.

(23A) The compound according to any one of items (1A) to (16A), a pharmaceutically acceptable salt thereof or a solvate thereof, for treating and/or preventing a cancer.

(24A) A TTK inhibitory agent containing the compound according to any one of items (1A) to (16A), a pharmaceutically acceptable salt thereof or a solvate thereof.

(25A) An anticancer agent containing the compound according to any one of items (1A) to (16A), a pharmaceutically acceptable salt thereof or a solvate thereof.

(26A) An immune system disease therapeutic agent containing the compound according to any one of items (1A) to (16A), a pharmaceutically acceptable salt thereof or a solvate thereof.

(27A) An immune-suppressive agent containing the compound according to any one of items (1A) to (16A), a pharmaceutically acceptable salt thereof or a solvate thereof.

(28A) An autoimmune disease therapeutic agent containing the compound according to any one of items (1A) to (16A), a pharmaceutically acceptable salt thereof or a solvate thereof.

(29A) A process, system, apparatus, kit, etc. for producing the compound according to any one of items (1A) to (16A), a pharmaceutically acceptable salt thereof or a solvate thereof.

(30A) A process, system, apparatus, kit, etc. for preparing a pharmaceutical composition containing the compound according to any one of items (1A) to (16A), a pharmaceutically acceptable salt thereof or a solvate thereof.

(31A) A process, system, apparatus, kit, etc. for using the compound according to any one of items (1A) to (16A), a pharmaceutically acceptable salt thereof or a solvate thereof.

Furthermore, the present invention provides, for example, the following items.

(1B) A compound represented by Formula (I):

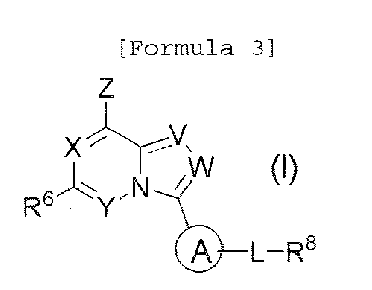

wherein
X, Y, V and W are any one of the following (X, Y, V, W) combinations:
(-N=, =CR¹-, =N-, -CR⁷=), (-CR²=, =N-, =N-, -CR⁷=), (-N=, =N-, =N-, -CR⁷=), (-N=, =CR¹-, =N-, -N=) and (-N=, =CR¹-, -O-, -N=),
R¹ and R² are each independently hydrogen, a halogen, a hydroxy, a cyano, a nitro, a carboxy, a substituted or unsubstituted alkyl, a substituted or unsubstituted alkenyl, or a substituted or unsubstituted alkynyl,
Z is a group represented by Formula: -NR³R⁴ or a group represented by Formula: -OR⁵,
R³ is hydrogen or a substituted or unsubstituted alkyl,
R⁴ and R⁵ are each independently hydrogen, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted alkoxy or a substituted or unsubstituted alkylsulfonyl,
R⁶ is hydrogen, a halogen, a hydroxy, a cyano, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted amino, a substituted or unsubstituted acyl, a substituted or unsubstituted alkoxy, a substituted or unsubstituted aryloxy, a substituted or unsubstituted heteroaryloxy, a substituted or unsubstituted cycloalkyloxy, a substituted or unsubstituted heterocyclyloxy, a substituted or unsubstituted carbamoyl, a group represented by Formula: -SO₂-R', a group represented by Formula: -SO-R', a group represented by Formula: -SR', a group represented by Formula: -O-N=C(R")₂ or a group represented by Formula: -O-N(R")₂ where two R" are each independently hydrogen, a substituted or unsubstituted alkyl, or two R" may be taken together with an adjacent carbon atom or nitrogen atom to form a substituted or unsubstituted nonaromatic hydrocarbon ring or nonaromatic heterocyclic ring,
R' is hydrogen, a substituted or unsubstituted alkyl, a substituted or unsubstituted amino, a substituted or unsubstituted aryl, a substituted or unsubstituted cycloalkyl, a substituted or unsubstituted heteroaryl or a substituted or unsubstituted heterocyclyl,
R⁷ is hydrogen, a halogen, a hydroxy, a cyano, a nitro, a carboxy, a substituted or unsubstituted alkyl, a substituted or unsubstituted alkenyl or a substituted or unsubstituted alkynyl,
A is a substituted or unsubstituted aromatic hydrocarbon ring, a substituted or unsubstituted aromatic heterocyclic ring, a substituted or unsubstituted nonaromatic hydrocarbon ring or a substituted or unsubstituted nonaromatic heterocyclic ring,
L is a single bond, -C(=O)-NR^{A}-, -NR^{B}-C(=O)-, -S(O)ₙ-NR^{C}-, -NR^{D}-S(O)ₙ-, a substituted or unsubstituted alkylene, a substituted or unsubstituted alkenylene or a substituted or unsubstituted alkynylene,
R⁸ is hydrogen, a halogen, a hydroxy, a cyano, a carboxy, a substituted or unsubstituted alkoxy, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl or a substituted or unsubstituted amino,
R^{A}, R^{B}, R^{C}, R^{D} are each independently hydrogen, a substituted or unsubstituted alkyl, or
R⁸ and R^{A}, or R⁸ and R^{C} may be taken together with an adjacent nitrogen atom to form a substituted or unsubstituted nitrogen-containing heterocyclic ring,
n is an integer of 1 or 2,
when (X, Y, V, W) is (-N=, =CR¹-, =N-, -CR⁷=) and Z is a group represented by Formula: -NR³R⁴ L is -C(=O)-NR^{A}-;

with the proviso that when (X, Y, V, W) is (-N=, =CR¹-, =N-, -CR⁷=) and Z is a group represented by Formula: - OR⁵, R⁶ is not halogen,
when (X, Y, V, W) is (-N=, =CR¹-, =N-, -CR⁷=), Z is a group represented by Formula: -NR³R⁴ and L is -C(=O)-NR^{A}-, R⁸ is not an alkyl substituted with an amino, a hydroxy, a pyridyl or a heterocyclyl, or hydrogen, and R⁸ and R^{A} are not taken together with an adjacent nitrogen atom to form a substituted or unsubstituted nitrogen-containing heterocyclic ring;
with the proviso that the following compounds are excluded:

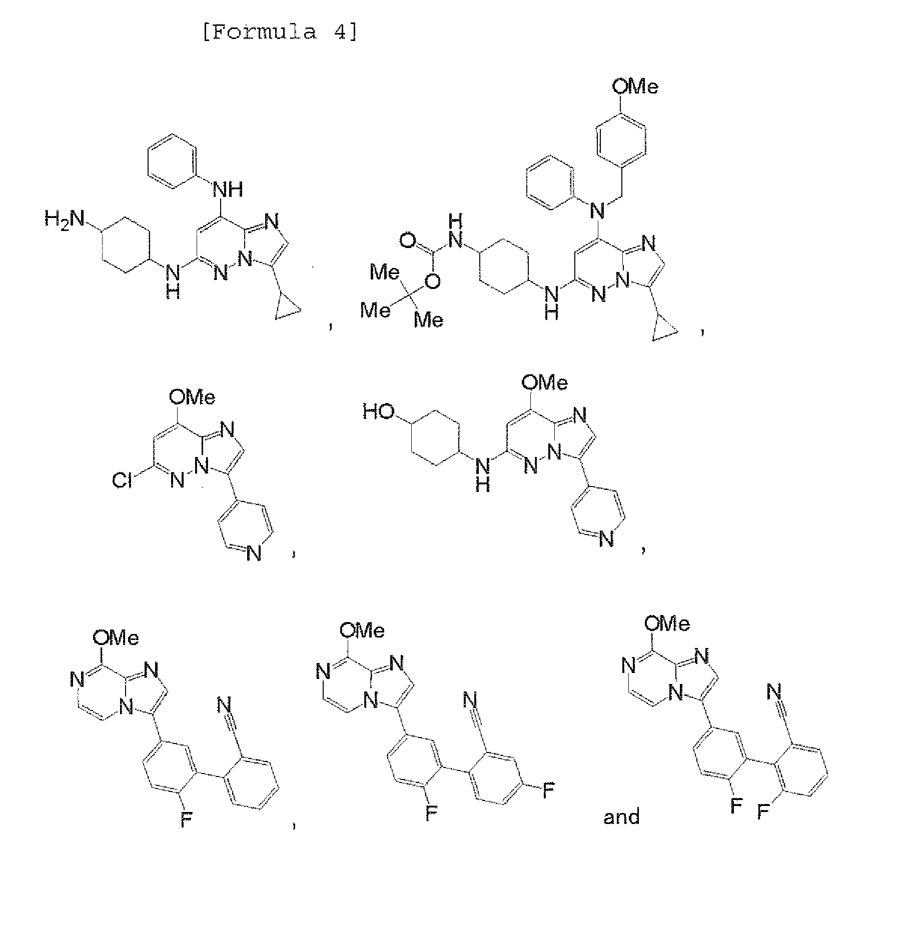

; a pharmaceutically acceptable salt thereof or a solvate thereof.

(2B) The compound according to item (1B), in which (X, Y, V, W) is (-N=, =CR¹-, =N-, -CR⁷=), (-CR²=, =N-, =N-, - CR⁷=), (-N=, =CR¹-, =N-, -N=) or (-N=, =CR¹-, -O-, -N=) where R¹, R² and R⁷ are the same as defined in item (1B); a pharmaceutically acceptable salt thereof or a solvate thereof.

(3B) The compound according to item (1B) or (2B), in which (X, Y, V, W) is (-N=. =CH-, =N-, -CR⁷=), (-CH=, -N-, =N-, -CR⁷=), (-N=, =CH-, =N-, -N=) or (-N=, =CH-, -O-, -N=) where R⁷ is the same as defined in item (1B); a pharmaceutically acceptable salt thereof or a solvate thereof.

(4B) The compound according to any one of items (1B) to (3B), in which Z is a group represented by Formula: -NR³R⁴ where R³ and R⁴ are the same as defined in item (1B); a pharmaceutically acceptable salt thereof or a solvate thereof.

(5B) The compound according to any one of items (1B) to (4B), in which Z is a group represented by Formula: -NHR⁴ where R⁴ is the same as defined in item (1B); a pharmaceutically acceptable salt thereof or a solvate thereof.

(6B) The compound according to any one of items (1B) to (5B), in which R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl; a pharmaceutically acceptable salt thereof or a solvate thereof.

(7B) The compound according to any one of items (1B) to (6B), in which Z is a group represented by Formula: -OR⁵ where R⁵ is the same as defined in item (1B); a pharmaceutically acceptable salt thereof or a solvate thereof.

(8B) The compound according to any one of items (1B) to (7B), in which R⁵ is a substituted or unsubstituted alkyl or a substituted or unsubstituted heterocyclylalkyl; a pharmaceutically acceptable salt thereof or a solvate thereof.

(9B) The compound according to any one of items (1B) to (8B), in which A is a substituted or unsubstituted aromatic hydrocarbon ring or a substituted or unsubstituted aromatic heterocyclic ring; a pharmaceutically acceptable salt thereof or a solvate thereof.

(10B) The compound according to any one of items (1B) to (9B), in which L is -C(=O)-NR^{A}-, -NR^{B}-C(=O)- or -S(O)ₙ-NR^{C}- where R^{A}, R^{B}, R^{C} and n are the same as defined in item (1B); a pharmaceutically acceptable salt thereof or a solvate thereof.

(11B) The compound according to any one of items (1B) to (10B), in which R⁸ is a substituted or unsubstituted alkyl, a substituted or unsubstituted cycloalkyl or a substituted or unsubstituted aryl; a pharmaceutically acceptable salt thereof or a solvate thereof.

(12B) The compound according to any one of items (1B) to (11B), in which L is -C(=O)-NH- and R⁸ is a substituted or unsubstituted cycloalkyl; a pharmaceutically acceptable salt thereof or a solvate thereof.

(13B) The compound according to any one of items (1B) to (12B), in which R⁶ is hydrogen, a halogen, a substituted or unsubstituted alkyl, a substituted or unsubstituted alkenyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted alkoxy, a substituted or unsubstituted aryloxy, a substituted or unsubstituted heteroaryloxy, a substituted or unsubstituted cycloalkyloxy, a substituted or unsubstituted heterocyclyloxy, a substituted or unsubstituted acyl or a substituted or unsubstituted amino; a pharmaceutically acceptable salt thereof or a solvate thereof.

(14B) The compound according to any one of items (1B) to (13B), where R⁷ is hydrogen; a pharmaceutically acceptable salt thereof or a solvate thereof.

(15B) The compound according to any one of items (1B) to (14B), in which
(X, Y, V, W) is (-N=, =CR¹-, =N-, -CR⁷=) where R¹ and R⁷ are the same as defined in item (1B),
Z is a group represented by Formula: -NHR⁴,
R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl; A is a substituted or unsubstituted aromatic hydrocarbon ring,
L is -C (=O) -NH-,
R⁸ is a substituted or unsubstituted cycloalkyl; a pharmaceutically acceptable salt thereof or a solvate thereof.

(16B) The compound according to any one of items (1B) to (15B), in which
(X, Y, V, W) is (-CR²=, =N-, =N-, -CR⁷=) where R² and R⁷ are the same as defined in item (1B), and
A is a substituted or unsubstituted aromatic hydrocarbon ring; a pharmaceutically acceptable salt thereof or a solvate thereof.

(17B) A pharmaceutical composition containing the compound according to any one of items (1B) to (16B), a pharmaceutically acceptable salt thereof or a solvate thereof.

(18B) The pharmaceutical composition according to item (17B), as a TTK inhibitory agent.

(19B) The pharmaceutical composition according to item (17B) or (18B), as a medicament for treating or preventing a disease, disorder or condition associated with TTK.

(20B) The pharmaceutical composition according to any one of items (17B) to (19B), for treating and/or preventing a cancer.

(21B) A method for preventing or treating a cancer, comprising administering the compound according to any one of items (1B) to (16B), a pharmaceutically acceptable salt thereof or a solvate thereof.

(22B) Use of the compound according to any one of items (1B) to (16B), a pharmaceutically acceptable salt thereof or a solvate thereof, in preparation of a medicament for treating and/or preventing a cancer.

(23B) The compound according to any one of items (1B) to (16B), a pharmaceutically acceptable salt thereof or a solvate thereof, for treating and/or preventing a cancer.

(24B) A TTK inhibitory agent containing the compound according to any one of items (1B) to (16B), a pharmaceutically acceptable salt thereof or a solvate thereof.

(25B) An anticancer agent containing the compound according to any one of items (1B) to (16B), a pharmaceutically acceptable salt thereof or a solvate thereof.

(26B) An immune system disease therapeutic agent containing the compound according to any one of items (1B) to (16B), a pharmaceutically acceptable salt thereof or a solvate thereof.

(27B) An immune-suppressive agent containing the compound according to any one of items (1B) to (16B), a pharmaceutically acceptable salt thereof or a solvate thereof.

(28B) An autoimmune disease therapeutic agent containing the compound according to any one of items (1B) to (16B), a pharmaceutically acceptable salt thereof or a solvate thereof .

(29B) A process, system, apparatus, kit, etc., for producing the compound according to any one of items (1B) to (16B), a pharmaceutically acceptable salt thereof or a solvate thereof.

(30B) A process, system, apparatus, kit, etc., for preparing a pharmaceutical composition containing the compound according to any one of items (1B) to (16B), a pharmaceutically acceptable salt thereof or a solvate thereof.

(31B) A method, system, apparatus, kit, etc., for using the compound according to any one of items (1B) to (16B), a pharmaceutically acceptable salt thereof or a solvate thereof.

Accordingly, these and other advantages of the present invention will be apparent from the following detailed description.

### ADVANTAGEOUS EFFECTS OF INVENTION

The present invention provides an effective inhibitory agent for TTK protein kinase and provides, by extension, an effective medicament for a disease, disorder or condition associated with TTK such as a cancer.

### DESCRIPTION OF EMBODIMENTS

The present invention will be described below with reference to the best mode. Throughout the specification, it should be understood that a concept expressed in a singular form include concept expressed in a plural form, unless otherwise noted. Accordingly, it should be understood that a word expressed in a singular form by use of a modifier (for example, articles such as "a", "an", "the" in English), unless otherwise noted, include a concept of the word expressed in a plural form. Furthermore, it should be understood that terms used herein, unless otherwise noted, mean those usually used in the art. Accordingly, unless otherwise defined, all specialized terms and scientific and technical terms used herein have the same meanings that those skilled in the art to which the present invention pertains generally understand. If there are some inconsistencies, priority is given to the meanings (concepts) of the specification (including definitions).

Now, terms used herein will be individually described below. The terms used herein each have unified meanings. If terms are used singly or in combination, they have the same meanings.

Abbreviations used herein will be described below.

### [Expression 1]

| Brevity code | Name |
|---|---|
| HATU | O-(7-azabenzotriazol-1-yl)-1,1,3,3-tetramethyluronium hexafluorophosphate |
| EDC | 1-Ethyl-3-(3-dimethylaminopropyl)carbodiimide hydrochloride |
| DIEA | *N,N*-diisopropylethylamine |
| TEA | Triethylamine |
| NMP | *N*-methylpyrrolidone |
| DCM | Dichloromethane |
| DMSO | Dimethylsulfoxide |
| THF | Tetrahydrofuran |
| DMF | *N,N*-dimethylformamide |
| DMA | *N,N*-dimethylacetamide |
| MeCN | Acetonitrile |
| TFA | Trifluoroacetic acid |
| Xantphos | 4,5-Bis(diphenylphosphino)-9,9-dimethylxanthene |
| RuPhos | 2-Dicyclohexylphosphino-2',6'-diisopropoxybiphenyl |
| X-Phos | 2-Dicyclohexylphosphino-2',4',6'-triisopropylbiphenyl |
| DPPF | 1,1'-Bis(diphenylphosphino)ferrocene |
| Pd(PPh₃)₄ | Palladium-tetrakis(triphenylphosphine) |
| Pd(OAc)₂ | Palladium acetate |
| Pd₂(dba)₃ | Tris(dibenzylideneacetone)bispalladium |
| PdCl₂(PPh₃)₂ | Dichlorobis(triphenylphosphine)palladium (II) |
| PdCl₂(dtbpf) | Dichloro[1,1,-bis(di-t-butylphosphino)ferrocene]palladium(II) |
| PdCl₂(dppf) | Dichloro[1,1'-bis(diphenylphosphino)ferrocene]palladium(II) |
| CH₂Cl₂ | -dichloromethane complex |
| DPPA | Diphenylphosphoryl azide |
| NBS | *N*-bromo-succinimide |
| NCS | *N*-chloro-succinimide |
| NIS | *N*-iodo succinimide |
| MeSNa | Sodium methylmercaptan |
| m-CPBA | m-Chloroperoxybenzoic acid |
| TFAA | Trifluoroacetic anhydride |
| Zn(CN)₂ | Zinc cyanide |
| TMSCl | Trimethylsilyl chloride |
| Me | Methyl |
| Et | Ethyl |
| Boc | t-Butoxycarbonyl |
| TMS | Trimethylsilyl |
| DMAP | *N,N*-4-dimethylaminopyridine |

When used herein, "halogen" refers to fluorine, chlorine, bromine and iodine.

When used herein, "alkyl" includes a linear or branched alkyl group having 1 to 10 carbon atoms, e.g., methyl, ethyl, n-propyl, isopropyl, n-butyl, isobutyl, sec-butyl, tert-butyl, n-pentyl, isopentyl, neopentyl, n-hexyl, isohexyl, n-heptyl, n-octyl, n-nonyl, n-decyl. Examples thereof include an alkyl having 1 to 6 or.1 to 4 carbon atoms, e.g., methyl, ethyl, n-propyl, isopropyl, n-butyl, isobutyl, sec-butyl, tert-butyl, n-pentyl, isopentyl, neopentyl, n-hexyl and isohexyl.

When used herein, "alkenyl" includes a linear or branched alkenyl having 2 to 8 carbon atoms, which is the aforementioned "alkyl" having one or more double bonds, e.g., vinyl, 1-propenyl, 2-propenyl, 1-butenyl, 2-butenyl, 3-butenyl, 1,3-butadienyl and 3-methyl-2-butenyl.

When used herein, "alkynyl" includes a linear or branched alkynyl having 2 to 8 carbon atoms, which is the aforementioned "alkyl" having one or more triple bonds, e.g., ethynyl, propynyl and butynyl. Furthermore, alkynyl, may have one or more double bonds.

When used herein, "cycloalkyl" includes, saturated cyclic hydrocarbon group having 3 to 15 carbon atoms, e.g., cyclopropyl, cyclobutyl, cyclopentyl, cyclohexyl, cycloheptyl, cyclooctyl, crosslinked cyclic hydrocarbon group, spirohydrocarbon group. Examples thereof include cyclopropyl, cyclobutyl, cyclopentyl, cyclohexyl and a crosslinked cyclic hydrocarbon group.

Note that when used herein, "crosslinked cyclic hydrocarbon group" include a group formed by removing a single hydrogen atom from an aliphatic ring having 5 to 12 carbon atoms and formed of two or more rings by sharing 2 or more atoms in common. Specific examples thereof include bicyclo[2.1.0]pentyl, bicyclo[2.2.1]heptyl, bicyclo[2.2.2]octyl and bicyclo[3.2.1]octyl, tricyclo[2.2.1.0]heptyl, bicyclo[3.3.1]nonane, 1-adamantyl and 2-adamantyl.

Furthermore, when used herein, "spiro hydrocarbon group" includes a group formed by removing a single hydrogen atom from a ring formed of two hydrocarbon rings sharing a single carbon atom in common. Specific examples thereof include spiro[3.4]octyl.

When used herein, "cycloalkenyl" includes an unsaturated cyclic aliphatic hydrocarbon group having 3 to 7 carbon atoms. Examples thereof include cyclopropenyl, cyclobutenyl, cyclopentenyl, cyclohexenyl and cycloheptenyl. Examples thereof include cyclopropenyl, cyclobutenyl, cyclopentenyl and cyclohexenyl. A crosslinked cyclic hydrocarbon group and a spiro hydrocarbon group having an unsaturated bond in the ring are included in cycloalkenyl.

When used herein, "acryl" includes a monocyclic ring or fused aromatic hydrocarbon ring. This may be fused with the above "cycloalkyl", the following "heteroaryl" and "heterocyclyl" at all possible positions. In the case an aryl has either one of a monocyclic ring and a fused ring, bonding can be made at all possible positions. Example thereof include phenyl, 1-naphthyl, 2-naphthyl, anthryl, tetrahydronaphthyl and 1,4-benzodioxanyl. Examples thereof include phenyl, 1-naphthyl and 2-naphthyl. For example, phenyl is mentioned.

When used herein, "aromatic hydrocarbon ring" includes six-membered aromatic ring containing only carbon atoms within the ring or a ring formed by fusing two or more of these rings. A monocyclic aromatic hydrocarbon ring includes a ring, which is derived from six-membered aromatic hydrocarbon ring and which may have a bond at any substitution-possible position. The fused aromatic hydrocarbon ring has a ring, which is formed by fusing a six-membered aromatic hydrocarbon ring with 1 to 4 six-membered aromatic hydrocarbon rings and which may have a bond at any substitution-possible position. For example, six-membered aromatic hydrocarbon ring is mentioned. Examples of the aromatic hydrocarbon ring include a benzene ring, a naphthalene ring, an anthracene ring, a tetrahydronaphthalene ring and a 1,4-benzodioxane ring. Examples thereof include a benzene ring and a naphthalene ring.

When used herein, "nonaromatic hydrocarbon ring" includes a three to eight-membered nonaromatic ring containing only carbon atoms within the ring or a ring formed by fusing two or more of these rings. Monocyclic nonaromatic hydrocarbon ring includes a group, which is derived from a three to eight-membered nonaromatic hydrocarbon ring and which may have a bond at any substitution-possible position. The fused nonaromatic hydrocarbon ring include a group, which is formed by fusing a five to eight-membered nonaromatic hydrocarbon ring with 1 to 4 five to eight-membered nonaromatic hydrocarbon rings and which may have a bond at any substitution-possible position. As long as a ring is nonaromatic, the ring may be saturated or unsaturated. For example, five to six-membered nonaromatic hydrocarbon ring is mentioned. Examples of the nonaromatic hydrocarbon ring include a cyclopropane ring, a cyclobutane ring, a cyclopentane ring, a cyclohexane ring, a cycloheptane ring, a cyclooctane ring, a cyclopropene ring, a cyclobutene ring, a cyclopentene ring, a cyclohexene ring and a cycloheptane ring. Examples thereof include a cyclopentane ring, a cyclohexane ring, a cyclopentene ring and a cyclohexene ring.

When used herein, "heteroaryl" includes a five to eight-membered aromatic hydrocarbon ring containing at least one atom arbitrarily selected from an oxygen atom, a sulfur atom and/or nitrogen atom within the ring. This may be fused with the above "cycloalkyl" and "aryl" and the following "heterocyclyl" or any one of other heteroaryls at all possible positions. When a heteroaryl is either one of a monocyclic ring and a fused ring, bonding can be made at all possible positions. Examples thereof include pyrrolyl (for example, 1-pyrrolyl, 2-pyrrolyl, 3-pyrrolyl), furyl (for example, 2-furyl, 3-furyl), thienyl (for example, 2-thienyl, 3-thienyl), imidazolyl.(for example, 2-imidazolyl, 4-imidazolyl), pyrazolyl (for example, 1-pyrazolyl, 3-pyrazolyl, 4-pyrazolyl), isothiazolyl (for example, 3-isothiazolyl), isoxazolyl (for example, 3-isoxazolyl), oxazolyl (for example, 2-oxazolyl, 4-oxazolyl, 5-oxazolyl), thiazolyl (for example, 2-thiazolyl, 4-thiazolyl, 5-thiazolyl), pyridyl (for example, 2-pyridyl, 3-pyridyl, 4-pyridyl), pyrazinyl (for example, 2-pyrazinyl), pyrimidinyl (for example, 2-pyrimidinyl, 4-pyrimidinyl), pyridazinyl (for example, 3-pyridazinyl), tetrazolyl (for example, 1H-tetrazolyl), oxadiazolyl (for example, 1,3,4-oxadiazolyl), thiadiazolyl (for example, 1,3,4-thiadiazolyl), indolizinyl (for example, 2-indolizinyl, 6-indolizinyl), iso indolyl (for example, 2-isoindolyl), indolyl (for example, 1-indolyl, 2-indolyl, 3-indolyl), indazolyl (for example, 3-indazolyl), purinyl (for example, 8-purinyl), quinolizinyl (for example, 2-quinolizinyl), isoquinolyl (for example, 3-isoquinolyl), quinolyl (for example, 2-quinolyl, 5-quinolyl), phthalazinyl (for example, 1-phthalazinyl), naphthyridinyl
(for example, 2-naphthyridinyl), quinazolinyl (for example, 2-quinazolinyl), cinnolinyl (for example, 3-cinnolinyl), pteridinyl (for example, 2-pteridinyl), carbazolyl (for example, 2-carbazolyl, 4-carbazolyl), phenanthridinyl (for example, 2-phenanthridinyl, 3-phenanthridinyl), acridinyl (for example, 1-acridinyl, 2-acridinyl), dibenzofuranyl (for example, 1-dibenzofuranyl, 2-dibenzofuranyl), benzimidazolyl (for example, 2-benzimidazolyl), benzoisoxazolyl (for example, 3-benzoisoxazolyl), benzoxazolyl (for example, 2-benzoxazolyl), benzoxadiazolyl (for example, 4-benzoxadiazolyl), benzisothiazolyl (for example, 3-benzisothiazolyl), benzothiazolyl (for example, 2-benzothiazolyl), benzofuryl (for example, 3-benzofuryl), benzothienyl (for example, 2-benzothienyl), dibenzothienyl (for example, 2-dibenzothienyl) and benzodioxolyl (for example, 1,3-benzodioxolyl).

When used herein, "heterocyclyl" includes a nonaromatic heterocyclic ring that may have 1 to 4 oxygen atoms, sulfur atoms, and/or nitrogen atoms within the ring and that may have a bond at any substitution-possible positions. Furthermore, such nonaromatic heterocyclic rings may be further crosslinked with an alkyl chain of 1 to 4 carbon atoms, and may be fused with a nonaromatic heterocyclic ring (five to six-membered ring is mentioned) and an aromatic hydrocarbon ring (for example, benzene ring). As long as a ring is nonaromatic, it may be saturated or unsaturated. For example, a five to eight-membered ring is mentioned. Example thereof include pyrrolinyl (for example, 1-pyrrolinyl, 2-pyrrolinyl, 3-pyrrolinyl), pyrrolidinyl (for example, 1-pyrrolidinyl, 2-pyrrolidinyl, 3-pyrrolidinyl), pyrrolidinone, imidazolinyl (for example, 1-imidazolinyl, 2-imidazolinyl, 4-imidazolinyl), imidazolidinyl (for example, 1-imidazolidinyl, 2-imidazolidinyl, 4-imidazolidinyl), imidazolidinone, pyrazolinyl (for example, 1-pyrazolinyl, 3-pyrazolinyl, 4-pyrazolinyl), pyrazolidinyl (for example, 1-pyrazolidinyl, 3-pyrazolidinyl, 4-pyrazolidinyl), piperidinone, piperidino, piperidinyl (for example, 2-piperidinyl, 3-piperidinyl, 4-piperidinyl), piperazinyl (for example, 1-piperazinyl, 2-piperazinyl), piperazinone, morpholinyl (for example, 2-morpholinyl, 3-morpholinyl), morpholino, tetrahydropyranyl and tetrahydrofuranyl.

When used herein, "aromatic heterocyclic ring" includes an aromatic five to eight-membered ring having at least one atom arbitrarily selected from an oxygen atom, a sulfur atom and/or a nitrogen atom within the ring or a ring formed by fusing two or more these rings. Monocyclic aromatic heterocyclic ring includes a ring, which is derived from a five to eight-membered aromatic hydrocarbon ring that may have 1 to 4 oxygen atoms, sulfur atoms and/or nitrogen atom within the ring. The monocyclic aromatic heterocyclic ring may have a bond at any substitution-possible position. A fused aromatic heterocyclic ring includes a ring formed by fusing a five to eight-membered aromatic hydrocarbon ring that may have 1 to 4 oxygen atoms, sulfur atoms and/or nitrogen atoms within the ring, with 1 to 4 six-membered aromatic hydrocarbon rings or other five to eight-membered aromatic heterocyclic rings. The fused aromatic heterocyclic ring may have a bond at any substitution-possible position. For example, a five to six-membered aromatic heterocyclic ring is mentioned. Examples of the aromatic heterocyclic ring include a pyrrole ring, a furan ring, a thiophene ring, an imidazole ring, a pyrazole ring, an isothiazole ring, an isoxazole ring, an oxazole ring, a thiazole ring, a pyridine ring, a pyrazine ring, a pyrimidine ring, a pyridazine ring, a tetrazole ring, an oxadiazole ring, a thiadiazole ring, an indolizine ring, an isoindole ring, an indole ring, an indazole ring, a purine ring, a quinolizine ring, an isoquinoline ring, a quinoline ring, a phthalazine ring, a naphthyridine ring, a quinazoline ring, a cinnoline ring, a pteridine ring, a carbazole ring, a phenanthridine ring, an acridine ring, a dibenzofuran ring, a benzimidazole ring, a benzisoxazole ring, a benzoxazole ring, a benzoxazole ring, a benzisothiazole ring, a benzothiazole ring, a benzofuran ring, a benzothiophene ring, a dibenzothiophene ring and a benzodioxole ring. Examples thereof include a thiophene ring, a pyridine ring, a furan ring, a thiazole ring, an oxazole ring and a pyrimidine ring.

When used herein, "nonaromatic heterocyclic ring" includes a nonaromatic five to eight-membered ring containing at least one atom arbitrarily selected from an oxygen atom, a sulfur atom and/or a nitrogen atom within the ring or a ring formed by fusing two or more these rings. Examples thereof include a pyrroline ring, a pyrrolidine ring, a pyrrolidinone ring, an imidazoline ring, an imidazolidine ring, a pyrazoline ring, a pyrazolidine ring, a piperidinone ring, a piperidine ring, a piperazine ring, a piperazinone ring, a morpholine ring, a tetrahydropyran ring, a tetrahydrofuran ring, a dihydropyran ring and a dihydrofuran ring. Examples thereof include a dihydropyran ring, a tetrahydropyran ring, a dihydrofuran ring and a tetrahydrofuran ring.

When used herein, "acyl" includes formyl, a substituted or unsubstituted alkylcarbonyl, a substituted or unsubstituted alkenylcarbonyl, a substituted or unsubstituted cycloalkylcarbonyl, a substituted or unsubstituted cycloalkenylcarbonyl, a substituted or unsubstituted arylcarbonyl, a substituted or unsubstituted heteroarylcarbonyl and a substituted or unsubstituted heterocyclylcarbonyl.

When used herein, "alkylene" includes a divalent group having 1 to 6 consecutive methylene groups. Specific examples include methylene, ethylene, trimethylene, tetramethylene, pentamethylene and hexamethylene.

When used herein, "alkenylene" includes a divalent group having 2 to 6 consecutive methylene groups and at least one of carbon-carbon bonds being a double bond.

When used herein, "alkynylene" includes a divalent group having 2 to 6 consecutive methylene groups and at least one of carbon-carbon bonds being a triple bond.

When used herein, examples of "alkoxy," include methyloxy, ethyloxy, n-propyloxy, isopropyloxy, n-butyloxy, isobutyloxy, sec-butyloxy, tert-butyloxy, n-pentyloxy, isopentyloxy, 2-pentyloxy, 3-pentyloxy, n-hexyloxy, isohexyloxy, 2-hexyloxy, 3-hexyloxy, n-heptyloxy and n-octyloxy. For example, a C1-C6 alkoxy is mentioned. Furthermore, a C1-C4 alkoxy is mentioned. Particularly, if the number of carbon atoms is specified, "alkoxy" having carbon atoms within the specified range of numbers is referred.

When used herein, "a substituted or unsubstituted nitrogen-containing heterocyclic ring formed by taking R⁸ and R^{A} or R^{C} together with an adjacent nitrogen atom" includes the following rings:

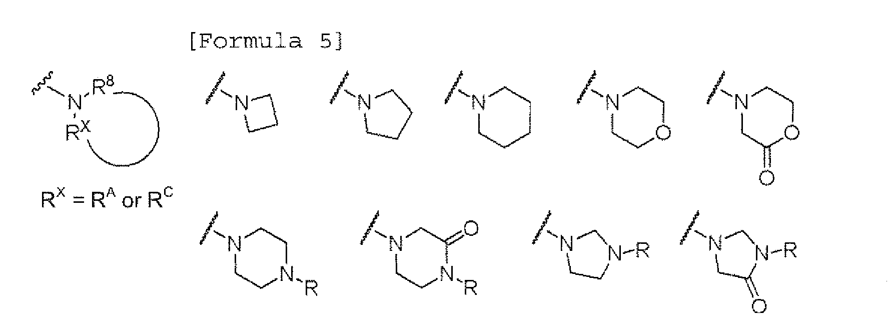

wherein R is hydrogen, a substituted or unsubstituted alkyl or a substituted or unsubstituted acyl.

When used herein, "a substituted or unsubstituted nitrogen-containing heterocyclic ring formed by taking R⁸ and R^{A} or R^{C} together with an adjacent nitrogen atom" includes the following rings:

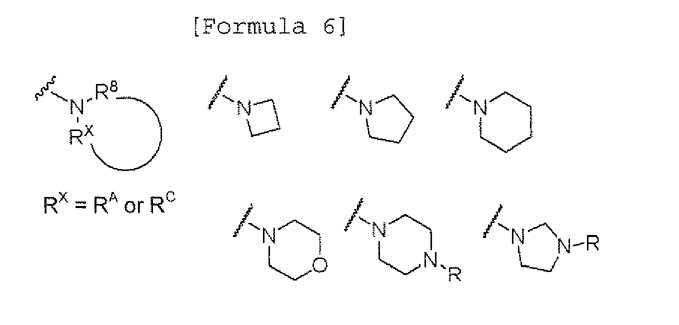

wherein R is hydrogen, a substituted or unsubstituted alkyl or a substituted or unsubstituted acyl.

When used herein, examples of a substituent in "a substituted or unsubstituted alkyl", "a substituted or unsubstituted alkenyl", "a substituted or unsubstituted alkynyl", "a substituted or unsubstituted cycloalkyl", "a substituted or unsubstituted cycloalkenyl", "a substituted or unsubstituted aryl", "a substituted or unsubstituted heteroaryl", "a substituted or unsubstituted heterocyclyl", "a substituted or unsubstituted alkoxy", "a substituted or unsubstituted acryl", "a substituted or unsubstituted alkylene", "a substituted or unsubstituted alkenylene", "a substituted or unsubstituted alkynylene", "a substituted or unsubstituted alkylsulfonyl", "a substituted or unsubstituted aryloxy", "a substituted or unsubstituted heteroaryloxy", "a substituted or unsubstituted cycloalkyloxy", "a substituted or unsubstituted heterocyclyloxy" or "substituted or unsubstituted nitrogen-containing heterocyclic ring formed by taking R⁸ and R^{A} or R^{C} together with an adjacent nitrogen atom" include hydroxy, carboxy, halogen, alkyl halide (e.g., CF₃, CH₂CF₃, CH₂CCl₃), nitro, nitroso, cyano, alkyl (e.g., methyl, ethyl, isopropyl, tert-butyl), alkenyl (e.g., vinyl), alkynyl (e.g., ethynyl), cycloalkyl (e.g., cyclopropyl, adamantyl), cycloalkylalkyl (e.g., cyclohexylmethyl, adamantylmethyl), cycloalkenyl (e.g., cyclopropenyl), aryl (e.g., phenyl, naphthyl), arylalkyl (e.g., benzyl, phenethyl), heteroaryl (e.g., pyridyl, furyl), heteroarylalkyl (e.g., pyridylmethyl), heterocyclyl (e.g., piperidyl), heterocyclylalkyl (e.g.,morpholylmethyl), alkoxy (e.g., methoxy, ethoxy, propoxy, butoxy), alkoxy halide (e.g., OCF₃), alkenyloxy (e.g., vinyloxy, allyloxy), aryloxy (e.g., phenyloxy), alkyloxycarbonyl (e.g., methoxycarbonyl, ethoxycarbonyl, tert-butoxycarbonyl), arylalkyloxy (e.g., benzyloxy), unsubstituted amino, substituted amino [e.g. alkylamino (e.g., methylamino, ethylamino, dimethylamino), acylamino (e.g., acetylamino, benzoylamino), arylalkylamino (e.g., benzylamino, tritylamino), hydroxyamino], alkylaminoalkyl (e.g., diethylaminomethyl), sulfamoyl, thio, oxo and carbamoyl. One to four substituents mentioned above may be used.

When used herein, examples of a substituent of "a substituted or unsubstituted amino" and "a substituted or unsubstituted carbamoyl" include alkyl, alkenyl, aryl, heteroaryl, cycloalkyl, heterocyclyl, alkylcarbonyl, cycloalkylcarbonyl, arylcarbonyl, heteroarylcarbonyl, heterocyclylcarbonyl, alkyloxycarbonyl, aryloxycarbonyl, heteroaryloxycarbonyl, heterocyclyloxycarbonyl, sulfamoyl, carbamoyl, alkylsulfonyl, cycloalkylsulfonyl, arylsulfonyl, heteroarylsulfonyl, heterocyclylsulfonyl, alkylsulfinyl, cycloalkylsulfinyl, arylsulfinyl, heteroarylsulfinyl, heterocyclylsulfinyl, hydroxy, mercapto, sulfino, sulfo and amino.

In the specification, A is a substituted or unsubstituted aromatic hydrocarbon ring, a substituted or unsubstituted aromatic heterocyclic ring, a substituted or unsubstituted nonaromatic hydrocarbon ring, or a substituted or unsubstituted nonaromatic heterocyclic ring. A includes the following examples:

a1: an aromatic hydrocarbon ring substituted with halogen, alkyl, alkoxy, amino, hydroxyl; or an unsubstituted aromatic hydrocarbon ring;

a2: an aromatic hydrocarbon ring substituted with halogen, C1-C6 alkyl, C1-C6 alkoxy, a substituted or unsubstituted amino, hydroxyl; or an unsubstituted aromatic hydrocarbon ring;

a3: an aromatic heterocyclic ring substituted with halogen, alkyl, alkoxy, amino; or an unsubstituted aromatic heterocyclic ring;

a4: an aromatic heterocyclic ring substituted with halogen, C1-C6 alkyl, C1-C6 alkoxy, a substituted or unsubstituted amino; or an unsubstituted aromatic heterocyclic ring;

a5: a pyridine substituted with halogen, alkyl, alkoxy, amino; or an unsubstituted pyridine; and

a6: a pyridine substituted with halogen, C1-C6 alkyl, C1-C6 alkoxy, a substituted or unsubstituted amino; or an unsubstituted pyridine.

The aforementioned a1 to a6 can be applied appropriately in combination with the following (A1), (A2-1) to (A2-10), (A3-1) to (A3-10).

When used herein, examples of a substituent in "a substituted or unsubstituted nitrogen-containing heterocyclic ring formed by taking R⁸ and R^{A} or R^{C} together with an adjacent nitrogen atom" include oxo, a substituted or unsubstituted alkyl and a substituted or unsubstituted acyl.

When used herein, examples of a substituent in "a substituted or unsubstituted nitrogen-containing heterocyclic ring formed by taking R⁸ and R^{A} or R^{C} together with an adjacent nitrogen atom" include oxo, a substituted or unsubstituted C1-C6 alkyl and a substituted or unsubstituted C1-C6 acyl.

When used herein, the alkyl moiety of each of "a substituted or unsubstituted heterocyclylalkyl", "a substituted or unsubstituted alkoxy", "a substituted or unsubstituted cycloalkylalkyl", "a substituted or unsubstituted arylalkyl", "a substituted or unsubstituted heteroarylalkyl" and "alkyl halide" means the aforementioned "alkyl".

When used herein, the alkoxy moiety of each of "a substituted or unsubstituted alkoxy" and "alkoxy halide" means the aforementioned "alkoxy,".

When used herein, the cycloalkyl moiety of "a substituted or unsubstituted cycloalkylalkyl" means the aforementioned "cycloalkyl".

When used herein, the aryl moiety of "a substituted or unsubstituted arylalkyl" means the aforementioned "aryl".

When used herein, the heteroaryl moiety of "a substituted or unsubstituted heteroarylalkyl" means the aforementioned "heteroaryl".

When used herein, the heterocyclyl moiety of "a substituted or unsubstituted heterocyclylalkyl" means the aforementioned "heterocyclyl".

As a pharmaceutically acceptable salt of a compound of the present invention, the following salts are mentioned.

Examples of a basic salt include an alkali metal salt such as a sodium salt or a potassium salt; an alkali earth metal salt such as a calcium salt or a magnesium salt; an ammonium salt; an aliphatic amine salt such as a trimethylamine salt, a triethylamine salt, a dicyclohexylamine salt, an ethanolamine salt, a diethanolamine salt, a triethanolamine salt, a procaine salt, a meglumine salt, a diethanolamine salt or an ethylenediamine salt; an aralkyl amine salt such as an? N,N-dibenzylethylene diamine salt or a benethamine salt; a heterocyclic aromatic amine salt such as a pyridine salt, a picoline salt, a quinoline salt or an isoquinoline; a quaternary ammonium salt such as tetramethylammonium salt, a tetraethylammonium salt, a benzyltrimethyl ammonium salt, a benzyltriethylammonium salt, a benzyltributyl ammonium salt, a methyltrioctylammonium salt or a tetrabutylammonium salt; and a basic amino acid salt such as an arginine salt or a lysine salt.

Examples of an acidic salt include an inorganic acid salt such as a hydrochloride, a sulfate, a nitrate, a phosphate, a carbonate, a hydrogen carbonate and a perchlorate; an organic acid salt such as an acetate, a propionate, a lactate, a maleate, fumarate, a tartrate, a malate, a citrate and an ascorbate; a sulfonate such as a methanesulfonate, an isethionate, a benzenesulfonate and a p-toluene sulfonate; and an acidic amino acid such as an aspartate and a glutamate.

When used herein, the solvate refers to a solvate of a compound of the present invention or a pharmaceutically acceptable salt thereof and includes an alcohol (e.g., ethanol) hydrate and a hydrate. Examples of the hydrate include a monohydrate and a dihydrate.

Furthermore, one or more hydrogen atoms, carbon atoms or other atoms of compounds of Formulas (I) and (I') can be substituted with isotopes of hydrogen atoms, carbon atoms or other atoms. Examples of compounds of Formulas (I) and (I') include all radiolabeled compounds represented by Formulas (I) and (I'). Such "radio-labeling" of the compounds of Formulas (I) and (I') and "radiolabeled compound" are each included in the present invention and useful as a tool for investigation and/or diagnosis in pharmacokinetic study of a metabolite and bonding assay. Also, they are useful as a pharmaceutical product.

Examples of the isotope to be integrated into each of the compounds represented by Formulas (I) and (I') of the present invention include those of hydrogen, carbon, nitrogen, oxygen, phosphorus, sulfur, fluorine, and chlorine, like ²H, ³H, ¹³C_{,} ¹⁴C, ¹⁵N, ¹⁸O, ¹⁷O, ³¹P, ³²P, ³⁵S, ¹⁸F, and ³⁶Cl. Radio-labeled compounds of the present invention can be prepared by a method known in the art. For example, each of the compounds represented by Formulas (I) and (I') and labeled with tritium can be prepared by introducing tritium into a predetermined compound of those represented by Formulas (I) and (I') by a catalytic dehalogenation reaction using tritium. This method may include a step of reacting a precursor, which is prepared by appropriately substituting any one of the compounds of Formulas (I) and (I') with a halogen, and tritium gas in the presence of an appropriate catalyst such as Pd/C and in the presence of or absence of a base. An appropriate method for preparing other tritium-labeled compound, a literature: Isotopes in the Physical and Biomedical Sciences, Vol. 1, Labeled Compounds (Part A), Chapter 6 (1987), can be referred to. 14C-label compound can be prepared by using a raw material containing 14C carbon.

### (Preferable fused imidazole derivative compound of the present invention)

In a compound contained in a pharmaceutical composition having the TTK inhibitory activity; a pharmaceutically acceptable salt thereof or a solvate or prodrug thereof (ester, amide, etc.), the aforementioned substituent can be arbitrarily selected from those described herein, for example, preferable substituents exemplified in the Solution to Problem can be arbitrarily used.

In consideration of a fused imidazole derivative of the present invention, the following factors can be considered.

Preferable embodiments of the present invention will be described in the following (A1) to (A3). The symbols are the same as defined in the above.

(A1) Individual substituents are the same as defined in the items (1A) or (1B) above, unless otherwise specified.

In the general formula [I']:

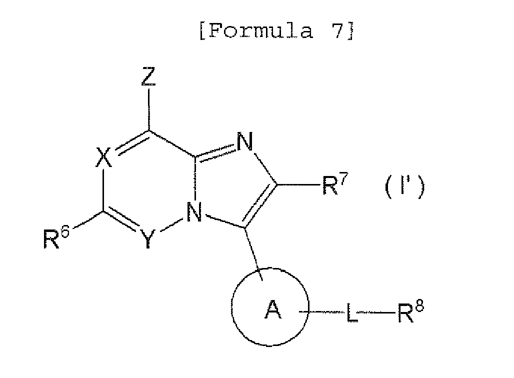

X and Y are any one of (X, Y) combinations including: (-N=, =CR¹-), (-CR²=, =N-) and (-N=, =N-).

For example, (X, Y) is (-N=, =CR¹-) or (-CR²=, =N-).

For example, (X, Y) is (-N=, =CH-) or (-CH=, =N-) .

R¹ and R² herein is each independently hydrogen, a halogen, a hydroxy, a cyano, a nitro, a carboxy, a substituted or unsubstituted alkyl, a substituted or unsubstituted alkenyl or a substituted or unsubstituted alkynyl.

Z is a group represented by Formula: -NR³R⁴ or a group represented by Formula: -OR⁵.

For example, Z is a group represented by Formula: - NR³R⁴_{.}

For example, Z is a group represented by Formula: - NHR⁴.

R³ herein is hydrogen or a substituted or unsubstituted alkyl.

R⁴ herein is hydrogen, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl or a substituted or unsubstituted heterocyclyl.

For example, R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

Furthermore, Z is a group represented by Formula: - OR⁵.

R⁵ herein is hydrogen, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl or a substituted or unsubstituted heterocyclyl.

For example, R⁵ is a substituted or unsubstituted alkyl or a substituted or unsubstituted heterocyclylalkyl.

R⁶ is hydrogen, a halogen, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted amino, a substituted or unsubstituted acyl, a substituted or unsubstituted alkoxy, a substituted or unsubstituted carbamoyl, a group represented by Formula: -SO₂-R', a group represented by Formula: -SO-R' or a group represented by Formula: -5R'.

R' herein is hydrogen, a substituted or unsubstituted alkyl, a substituted or unsubstituted amino, a substituted or unsubstituted aryl, a substituted or unsubstituted cycloalkyl, a substituted or unsubstituted heteroaryl or a substituted or unsubstituted heterocyclyl.

For example, R⁶ is hydrogen, a halogen, a substituted or unsubstituted alkyl, a substituted or unsubstituted alkenyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted alkoxy, a substituted or unsubstituted acyl or a substituted or unsubstituted amino.

R⁷ is hydrogen, a halogen, a hydroxy, a cyano, a nitro, a carboxy, a substituted or unsubstituted alkyl, a substituted or unsubstituted alkenyl or a substituted or unsubstituted alkynyl.

R⁷ is, for example, hydrogen.

A is a substituted or unsubstituted aromatic hydrocarbon ring, a substituted or unsubstituted aromatic heterocyclic ring, a substituted or unsubstituted nonaromatic hydrocarbon ring or a substituted or unsubstituted nonaromatic, heterocyclic ring.

For example, A is a substituted or unsubstituted aromatic hydrocarbon ring or a substituted or unsubstituted aromatic heterocyclic ring.

L is a single bond, -C(=O)-NR^{A}-, -NR^{B}-C(=O)-, -S(O)ₙ-NR^{C}-, -NR^{D}-S(O)ₙ-, a substituted or unsubstituted alkylene, a substituted or unsubstituted alkenylene or a substituted or unsubstituted alkynylene.

For example, L is -C(=O)-NR^{A}-, -NR^{B}-C(=O)- or -S(O)ₙ-NR^{C}-.

For example, L is -C(=O)-NH-.

R⁸ is hydrogen, a halogen, a hydroxy, a cyano, a carboxy, a substituted or unsubstituted alkoxy, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl or a substituted or unsubstituted amino.

For example, R⁸ is a substituted or unsubstituted alkyl, a substituted or unsubstituted cycloalkyl or a substituted or unsubstituted aryl.

For example, R⁸ is a substituted or unsubstituted cycloalkyl.

Furthermore, for example, L is -C(=O)-NH-, and R⁸ is a substituted or unsubstituted cycloalkyl.

R^{A}, R^{B}, R^{C}, R^{D} herein are each independently hydrogen or a substituted or unsubstituted alkyl.

Furthermore, R⁸ and R^{A} or R^{C} may be taken together with an adjacent nitrogen atom to form a substituted or unsubstituted nitrogen-containing hetero ring.

n is an integer of 1 or 2.

Furthermore, for example, when (X, Y) is (-N=, =CR¹-) and Z is a group represented by Formula: -NR³R⁴ and L is - C(=O)-NR^{A}-.

However, when (X, Y) is (-N=, =CR¹-) and Z is a group represented by Formula: -OR⁵, R⁶ is not halogen.

However, when (X, Y) is (-N=, =CR¹-), Z is a group represented by Formula: -NR³R⁴ and L is -C(=O)-NR^{A}-, R⁸ is not an alkyl substituted with an amino, a hydroxy, a pyridyl or a heterocyclyl and hydrogen, and R⁸ and R^{A} are not taken together with an adjacent nitrogen atom to form a substituted or unsubstituted nitrogen-containing hetero ring. Furthermore, the compounds shown below:

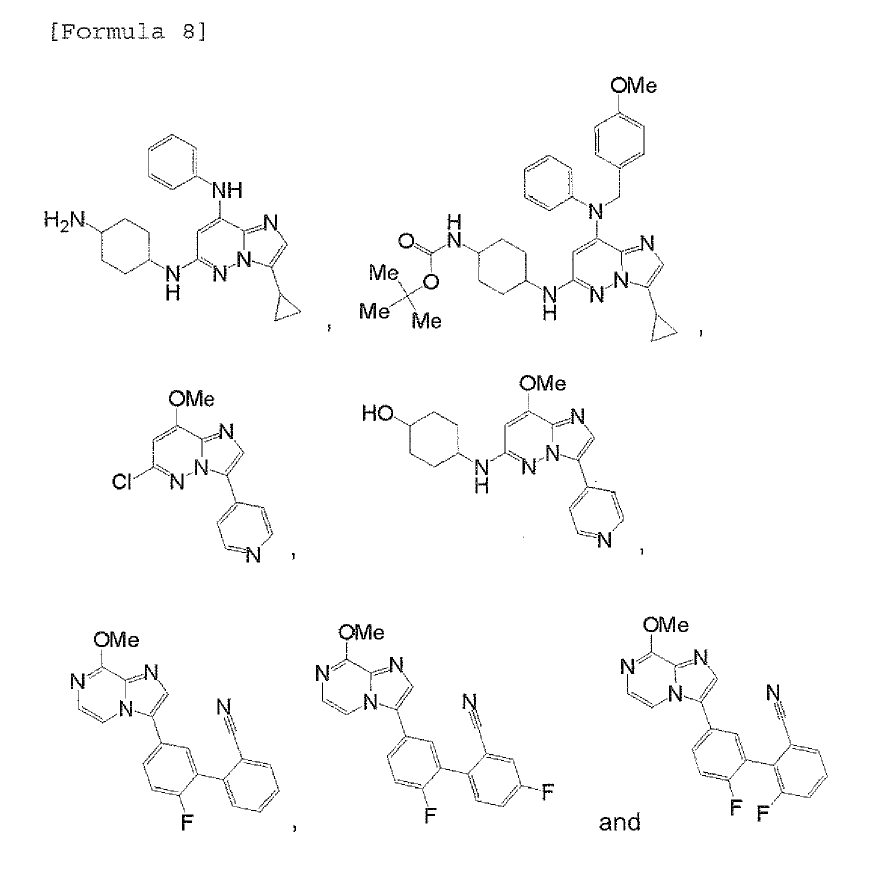

are excluded.

The following embodiments each may be one of the embodiments.

### (A2-1)

Individual substituents are the same as defined in item (1A) or (1B) above, unless otherwise specified.

In the general formula [I']:

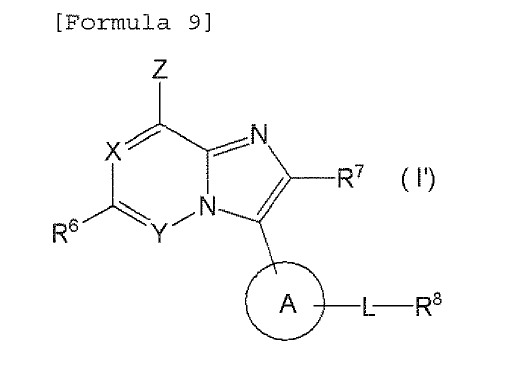

(X, Y) is (-N=, =CH-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted aromatic hydrocarbon ring.

L is -C(=O)-NH-.

R⁸ is a substituted or unsubstituted cycloalkyl.

R⁶ is a substituted or unsubstituted aryl, a substituted or unsubstituted heteroaryl, a substituted or unsubstituted amino, a substituted or unsubstituted alkoxy, a substituted or unsubstituted acyl or a substituted or unsubstituted alkyl.

R⁷ is hydrogen.

### (A2-2)

Individual substituents are the same as defined in item (1A) or (1B), unless otherwise specified.

In the general formula [I'],
(X, Y) is (-N=, =CH- .

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted aromatic hydrocarbon ring.

L is -C(=O)-NH-.

R⁸ is a substituted or unsubstituted cyclopropyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A2-3)

Individual substituents are the same as defined in item (1A) or (1B) above, unless otherwise specified.

In the general Formula [I']
(X, Y) is (-N=, =CH-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl, or R⁴ is an unsubstituted alkyl.

A is an aromatic hydrocarbon ring substituted with halogen, alkyl, alkoxy or amino; or A is an unsubstituted aromatic hydrocarbon ring.

L is -C(=O)-NH-.

R⁸ is a substituted or unsubstituted cycloalkyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A2-4)

Individual substituents are the same as defined in item (1A) or (1B) above, unless otherwise specified.

In the general formula [I']
(X, Y) is (-N=, =CH- .

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is an aromatic hydrocarbon ring substituted with a halogen, an alkyl, an alkoxy or an amino; or A is an unsubstituted aromatic hydrocarbon ring.

L is -C(=O)-NH-.

R⁸ is a substituted or unsubstituted cyclopropyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A2-5)

Individual substituents are the same as defined in item (1A) or (1B) above, unless otherwise specified.

In the general formula [I']
(X, Y) is (-N=, =CH-).

Z is a group represented by Formula: -NHR⁴

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is an aromatic hydrocarbon ring substituted with halogen, alkyl, alkoxy or amino; or A is an unsubstituted aromatic hydrocarbon ring.

L is -C(=O)-NH-.

R⁸ is a substituted or unsubstituted cyclopropyl.

R⁶ is an aryl substituted with cyano, amino or alkyl or an unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A2-6)

Individual substituents are the same as defined in item (1A) or (1B) above, unless otherwise specified.

In the general formula [I'],
(X, Y) is (-N=, =CH-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted aromatic heterocyclic ring.

L is -C(=O)-NH-.

R⁸ is a substituted or unsubstituted cycloalkyl.

R⁶ is a substituted or unsubstituted aryl, a substituted or unsubstituted heteroaryl, a substituted or unsubstituted amino, a substituted or unsubstituted alkyl, a substituted or unsubstituted
alkoxy or a substituted or unsubstituted acyl.

R⁷ is hydrogen.

### (A2-7)

Individual substituents are the same as defined in item (1A) or (1B) above, unless otherwise specified.

In the general formula [I'],
(X, Y) is (-N=, =CH-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted aromatic heterocyclic ring.

L is -C(=O)-NH-.

R⁸ is a substituted or unsubstituted cyclopropyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A2-8)

Individual substituents are the same as defined in item (1A) or (1B) above, unless otherwise specified.

In the general formula [I'],
(X, Y) is (-N=, =CH-) .

Z is a group represented by Formula: -NHR⁴.

R⁴ is an alkyl with substituted a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted pyridine.

L is -C(=O)-NH-.

R⁸ is a substituted or unsubstituted cycloalkyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A2-9)

Individual substituents are the same as defined in item (1A) or (1B) above, unless otherwise specified.

In the general formula [I'],
(X, Y) is (-N=, =CH-)

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted pyridine.

L is -C(=O)-NH-.

R⁸ is a substituted or unsubstituted cyclopropyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A2-10)

Individual substituents are the same as defined in item (1A) or (1B) above, unless otherwise specified.

In the general formula [I']
(X, Y) is (-N=, =CH-).

Z is a group represented by Formula: -NHR⁴

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted pyridine.

L is -C(=O)-NH-.

R⁸ is a substituted or unsubstituted cyclopropyl.

R⁶ is an aryl substituted with cyano, amino or alkyl, or an unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A3-1)

Individual substituents are the same as defined in item (1A) or (1B) above, unless otherwise specified.

In the general formula [I']:

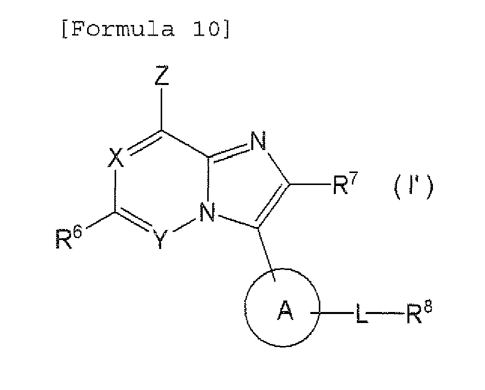

(X, Y) is (-CH=, =N- .

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted aromatic hydrocarbon ring.

L is -C=O)-NH-.

R⁸ is a substituted or unsubstituted cycloalkyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A3-2)

Individual substituents are the same as defined in item (1A) or (1B) above, unless otherwise specified.

In the general formula [I'],
(X, Y) is (-CH=, =N-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted aromatic hydrocarbon ring.

L is -C(=O)-NH-.

R⁸ is a substituted or unsubstituted cyclopropyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A3-3)

Individual substituents are the same as defined in item (1A) or (1B) above, unless otherwise specified.

In the general formula [I']
(X, Y) is (-CH=, =N-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is an aromatic hydrocarbon ring substituted with a halogen, an alkyl, an alkoxy or an amino; or A is an unsubstituted aromatic hydrocarbon ring.

L is -C(=O)-NH-.

R⁸ is a substituted or unsubstituted cycloalkyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A3-4)

Individual substituents are the same as defined in item (1) or (1B) above, unless otherwise specified.

In the general formula [I'],
(X, Y) is (-CH=, =N-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is an aromatic hydrocarbon ring substituted with a halogen, an alkyl, an alkoxy or an amino; or A is an unsubstituted aromatic hydrocarbon ring.

L is -C(=O)-NH-.

R⁸ is a substituted or unsubstituted cyclopropyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A3-5)

Individual substituents are the same as defined in item (1A) or (1B) above, unless otherwise specified.

In the general formula [I'],
(X, Y) is (-CH=, =N-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is an aromatic hydrocarbon ring substituted with a halogen, an alkyl, an alkoxy or an amino; or A is an unsubstituted aromatic hydrocarbon ring.

L is -C(=O)-NH-.

R⁸ is a substituted or unsubstituted cyclopropyl.

R⁶ is an aryl substituted with a cyano, an amino or an alkyl; or R⁶ is an unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A3-6)

Individual substituents are the same as defined in item (1A) or (1B) above, unless otherwise specified.

In the general formula [I'],
(X, Y) is (-CH=, =N-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted aromatic heterocyclic ring.

L is -C(=O)-NH-.

R⁸ is a substituted or unsubstituted cycloalkyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A3-7)

Individual substituents are the same as defined in item (1A) or (1B) above, unless otherwise specified.

In the general formula [I'],
(X, Y) is (-CH=, =N-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted aromatic heterocyclic ring.

L is -C(=O)-NH-.

R⁸ is a substituted or unsubstituted cyclopropyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A3-8)

Individual substituents are the same as defined in item (1A) or (1R) above, unless otherwise specified.

In the general formula [I'],
(X, Y) is (-CH=, =N-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted pyridine.

L is -C(=O)-NH-.

R⁸ is a substituted or unsubstituted cycloalkyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen.

### (A3-9)

Individual substituents are the same as defined in item (1A) or (1B) above, unless otherwise specified.

In the general formula [I'],
(X, Y) is (-CH=, =N-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted pyridine.

L is -C(=O)-NH-.

R⁸ is a substituted or unsubstituted cyclopropyl.

R⁶ is a substituted or unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen .

### (A3-10)

Individual substituents are the same as defined in item (1A) or (1B) above, unless otherwise specified.

In the general formula [I']
(X, Y) is (-CH=, =N-).

Z is a group represented by Formula: -NHR⁴.

R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl.

A is a substituted or unsubstituted pyridine.

L is -C(=O)-NH-.

R⁸ is a substituted or unsubstituted cyclopropyl.

R⁶ is an aryl substituted with a cyano, an amino or an alkyl; or R⁶ is an unsubstituted aryl, a substituted or unsubstituted heteroaryl, a substituted or unsubstituted amino, a substituted or unsubstituted alkyl, a substituted or unsubstituted alkoxy or a substituted or unsubstituted acyl.

R⁷ is hydrogen .

Preferable embodiments of the present invention will be described in the following (B1) to (B3). The symbols are the same as defined in the above.

### (B1)

Individual substituents are the same as defined in item (1A) or (1B) above, unless otherwise specified.

In the general formula [I]:

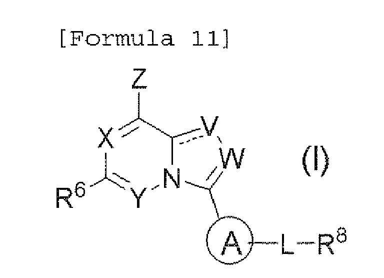

X, Y, V and W are any one of (X, Y, V, W) combinations including:
(-N=, =CR¹, =N-, -CR⁷=), (-CR²=, =N-, =N-, -CR⁷=), (-N=, =N-, =N-, -CR⁷=), (-N=, =CR¹-, =N-, -N=) and (-N=, =CR¹-, -O-, -N=) .

For example, (X, Y, V, W) combinations include (-N=, =CR¹-, =N-, -CR⁷=), (-CR²=, =N-, =N-, -CR⁷=), (-N=, =CR¹-, =N-, -N=) and (-N=, =CR¹-, -O-, -N=).

For example, (X, Y, V, W) combinations include (-N=, =CH-, =N-, -CR⁷=), (-CH=, =N-, =N-, -CR⁷=), (-N=, =CH-, =N-, - N=) and (-N=, =CH-, -O-, -N=).

R¹ and R² herein are each independently hydrogen, a halogen, a hydroxy, a cyano, a nitro, a carboxy, a substituted or unsubstituted alkyl, a substituted or unsubstituted alkenyl or a substituted or unsubstituted alkynyl.

Z is a group represented by Formula: -NR³R⁴ or Formula: -OR⁵.

R³ herein is hydrogen or a substituted or unsubstituted alkyl.

R⁴ and R⁵ herein are each independently hydrogen, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted alkoxy or a substituted or unsubstituted alkylsulfonyl.

For example, Z is a group represented by Formula: - NR³R⁴ where R³ and R⁴ are the same as defined in item (1A) or (1B) .

R⁴ is herein, for example, an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or an unsubstituted alkyl.

For example, Z is a group represented by Formula: - NHR⁴ where R⁴ is the same as defined in item (1A) or (1B).

For example, Z is a group represented by Formula: - OR⁵ where R⁵ is the same as defined in item (1A) or (1B).

R⁵ herein, for example, is a substituted or unsubstituted alkyl or a substituted or unsubstituted heterocyclylalkyl.

R⁶ is hydrogen, a halogen, a hydroxy, a cyano, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted amino, a substituted or unsubstituted acyl, a substituted or unsubstituted alkoxy, a substituted or unsubstituted aryloxy, a substituted or unsubstituted heteroaryloxy, a substituted or unsubstituted cycloalkyloxy, a substituted or unsubstituted heterocyclyloxy, a substituted or unsubstituted carbamoyl, a group represented by Formula: -SO₂-R', a group represented by Formula: -SO-R', a group represented by Formula: -SR', a group represented by Formula: -O-N=C(R")₂ or a group represented by Formula: -O-N(R")₂ where R" are each independently hydrogen, a substituted or unsubstituted alkyl, or two R" may be taken together with an adjacent carbon atom or nitrogen atom to form a substituted or unsubstituted nonaromatic hydrocarbon ring or nonaromatic heterocyclic ring.
R' herein is hydrogen, a substituted or unsubstituted alkyl, a substituted or unsubstituted amino, a substituted or unsubstituted aryl, a substituted or unsubstituted cycloalkyl, a substituted or unsubstituted heteroaryl or a substituted or unsubstituted heterocyclyl.

For example, R⁶. is hydrogen, a halogen, a substituted or unsubstituted alkyl, a substituted or unsubstituted alkenyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted alkoxy, a substituted or unsubstituted aryloxy, a substituted or unsubstituted heteroaryloxy, a substituted or unsubstituted cycloalkyloxy, a substituted or unsubstituted heterocyclyloxy, a substituted or unsubstituted acyl or a substituted or unsubstituted amino.

R⁷ is hydrogen, a halogen, a hydroxy, a cyano, a nitro, a carboxy, a substituted or unsubstituted alkyl, a substituted or unsubstituted alkenyl or a substituted or unsubstituted alkynyl.

For example, R⁷ is hydrogen.

A is a substituted or unsubstituted aromatic hydrocarbon ring, a substituted or unsubstituted aromatic heterocyclic ring, a substituted or unsubstituted nonaromatic, hydrocarbon ring or a substituted or unsubstituted nonaromatic heterocyclic ring.

For example, A is a substituted or unsubstituted aromatic hydrocarbon ring or a substituted or unsubstituted aromatic heterocyclic ring.

L is a single bond, -C(=O)-NR^{A}-, -NR^{B}-C(=O)-, -S(O)ₙ-NR^{C}-, -NR^{D}-S(O)ₙ-, a substituted or unsubstituted alkylene, a substituted or unsubstituted alkenylene or a substituted or unsubstituted alkynylene.

For example, L is -C(=O)-NR^{A}-, -NR^{B}-C(=O)- or -S(O)ₙ-NR^{C}-, where R^{A}, R^{B}, R^{C} and n are the same as define in item (1A) or (1B).

R⁸ is hydrogen, a halogen, a hydroxy, a cyano, a carboxy, a substituted or unsubstituted alkoxy, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl or a substituted or unsubstituted amino.

For example, R⁸ is a substituted or unsubstituted alkyl, a substituted or unsubstituted cycloalkyl or a substituted or unsubstituted aryl.

R^{A}, R^{B}, R^{C}, R^{D} herein are each independently hydrogen, a substituted or unsubstituted alkyl, or R⁸ and R^{A}, or R⁸ and R^{C} may be taken together with an adjacent nitrogen atom to form a substituted or unsubstituted nitrogen-containing heterocyclic ring.

For example, L is -C(=O)-NH- and R⁸ is a substituted or unsubstituted cycloalkyl.

n is an integer of 1 or 2.

When (X, Y, V, W) is (-N=, =CR¹-, =N-, -CR⁷=) and Z is a group represented by Formula: -NR³R⁴, L is -C(=O)-NR^{A}-.

However, when (X, Y, V, W) is (-N=, =CR¹-, =N-, - CR⁷=), and Z is a group represented by Formula: -OR⁵, R⁶ is not a halogen. In contrast, when (X, Y, V, W) is (-N=, =CR¹-, =N-, -CR⁷=), Z is a group represented by Formula: -NR³R⁴ and L is - C(=O)-NR^{A}-, R⁸ is not an alkyl substituted with an amino, a hydroxy, a pyridyl or a heterocyclyl, or R⁸ is not hydrogen, or R⁸ and R^{A} do not form a substituted or unsubstituted nitrogen-containing heterocyclic ring together with an adjacent nitrogen atom.

Furthermore, the compounds shown below:

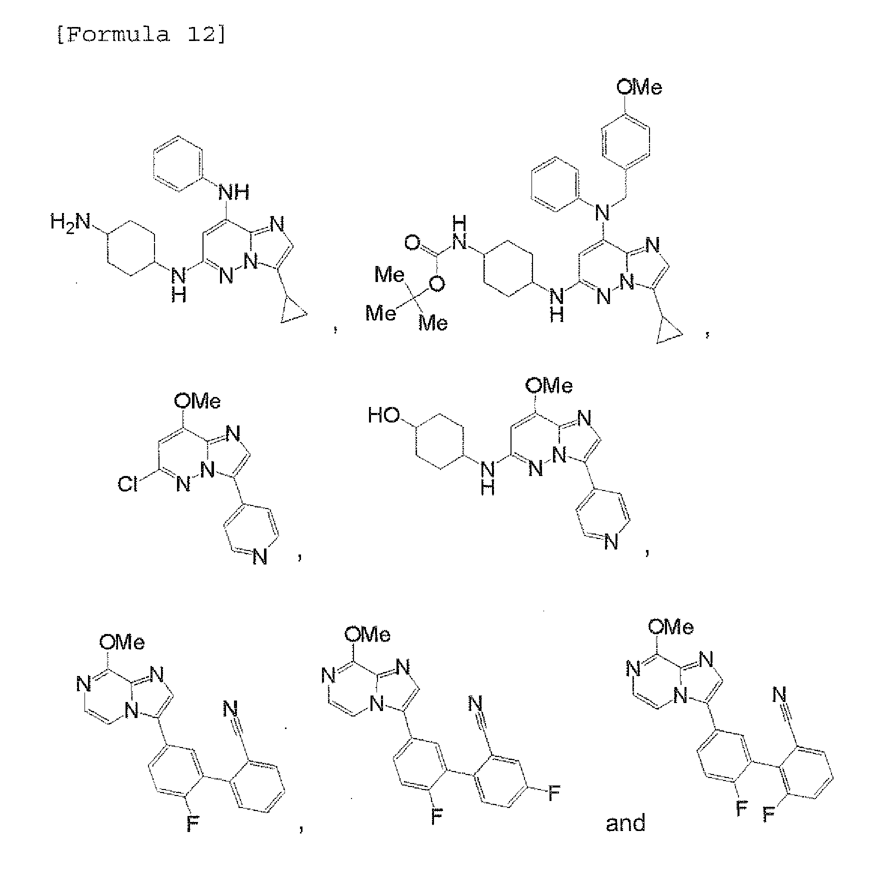

are excluded.

The following embodiments each may be one of the embodiments.

### (B2)

Individual substituents are the same as defined in item (1A) or (1B) above, unless otherwise specified.

In the general formula [I],
(X, Y, V, W) is (-N=, =CR¹-, =N-, -CR⁷=) where R¹ and R⁷ are the same as defined in item (1A) or (1B);
Z is a group represented by Formula: -NHR⁴;
R⁴ is an alkyl with substituted a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl; A is a substituted or unsubstituted aromatic hydrocarbon ring;
L is -C(=O)-NH-; and
R⁸ is a substituted or unsubstituted cycloalkyl.

### (B3)

Individual substituents are the same as defined in item (1A) or (1B) above, unless otherwise specified.

In the general formula [I],
(X, Y, V, W) is (-CR²=, =N-, =N-, -CR⁷=), where R² and R⁷ herein are the same as defined in item (1A) or (1B); and
A is a substituted or unsubstituted aromatic hydrocarbon ring.

In another embodiment, the present invention provides a pharmaceutical composition containing any one of the compounds described in the above; a pharmaceutically acceptable salt thereof or a solvate thereof, or a prodrug thereof (for example, an ester, an amide).

When used herein, "prodrug" and "prodrug compound" is a derivative of a compound of the present invention having a chemically or metabolically decomposable group, exhibiting a pharmaceutical activity by hydrolysis and solvolysis or decomposition under physiological conditions. Various forms of prodrugs are known in the art. Examples of such a prodrug derivative can be referred to the following literatures (a) to (f). Prodrugs of compounds represented by Formulas (I) and (I') are produced by a process for modifying a functional group present in each of the compounds represented by Formulas (I) and (I') such that the compound releases a parent compound thereof by cleavage in a living body. For example, a prodrug contains one of the compounds represented by Formulas (I) and (I') and having a hydroxy, amino or sulfhydryl group bonded to a group, which is to be decomposed in vivo such that a free hydroxy, sulfhydryl or amino group is regenerated. Examples of the prodrug are not limited to these; however, include an ester of a hydroxy functional group (for example, acetate, formate, and benzoate derivatives) of the compounds of Formulas (I) and (I') and carbamate (for example, N,N-dimethylaminocarbonyl).
(a) Design of Prodrugs, edited by H. Bundgaard, (Elsevier, 1985) and Methods in Enzymology, Vol. 42. p. 309-396, edited by K. Widder, et al. (Academic Press, 1985);
(b) A Textbook of Drug Design and Development, edited by Krogsgaard-Larsen;
(c) H. Bundgaard, Chapter 5 "Design and Application of Prodrugs", by H. Bundgaard p. 113-191 (1991);
(d) H. Bundgaard, Advanced Drug Delivery Reviews, 8, 1-38 (1992);
(e) H. Bundgaard, et al., Journal of Pharmaceutical Sciences, 77, 285 (1988); and
(f) N. Kakeya, et al., Chem Pharm Bull, 32, 692 (1984).

One of examples of the group (prodrug group) constituting a prodrug is a pharmaceutically acceptable ester that is cleaved in a human or an animal body to produce a parent acid, in short, an in-vivo cleavable ester group. For example, the prodrug group in concert with a carboxy group that the prodrug group is bonded to form a pharmaceutically acceptable ester and a pharmaceutically acceptable ester of a substituted or unsubstituted heterocyclic group, such as a C₁₋₆ alkyl ester or a C₁₋₆ cycloalkyl ester, for example, an ester of methyl, ethyl, propyl, isopropyl, n-butyl or cyclopentyl,; a C₁₋₆ alkoxymethyl ester, for example, methoxymethyl ester; a C₁₋₆ alkanoyloxymethyl ester, for example, pivaloyloxymethyl ester; a phthalidyl ester; a C₃₋₈ cycloalkoxycarbonyloxyC₁₋₆ alkyl ester, for example, 1-cyclohexylcarbonyloxyethyl ester; a 1,3-dioxolan-2-ylmethyl ester, for example, 5-methyl-1,3-dioxolan-2-ylmethyl ester; a C₁₋₆ alkoxycarbonyloxyethyl ester, for example, 1-methoxycarbonyloxyethyl ester; an aminocarbonylmethyl ester and a mono-or di-N-(C₁₋₆ alkyl) variety, for example, N,N-dimethylaminocarbonylmethyl ester and N-ethylaminocarbonylmethyl ester. In one of the embodiments, a prodrug is an ester with a C₁₋₄ alkyl group such as isopropyl or cyclopentyl or a group selected from a heterocyclic group that may have a substituent, such as N-methyltetrahydropyridyl.

A pharmaceutical composition of the present invention containing any one of the compounds described above is also characterized by being a TTK inhibitory agent. Accordingly, the present invention provides a pharmaceutical composition that produces a medicinal effect by administering the pharmaceutical composition to a patient requiring TTK inhibition.

In another embodiment, the present invention provides a medicament for treating or preventing a cancer or an immune disease and containing any one of the compounds described above.

### (Production process)

A general process for producing a compound of the present invention will be described below. Furthermore, extraction, purification, etc. may be performed in accordance with the treatments usually used in organic chemical experiments.

A process for producing a compound of the present invention will be described below.

Synthesis of a compound of the present invention can be carried out with reference to processes known in the art

As a raw-material compound, a commercially available compound or those described in Patent Literatures 3 to 16 and Non Patent Literature 7 can be used. Besides these, those described herein and those described in the literatures cited herein and other known compounds can be used.

Of the compounds of the present invention, there are compounds possibly having a tautomer, a regioisomer and an optical isomer. The present invention encompasses all possible isomers including these and mixtures of them.

To obtain a salt of a compound of the present invention, a compound of the present invention that is obtained in the form of salt may be just directly purified. Furthermore, when a compound of the present invention is obtained in free form, the compound may be dissolved or suspended in an appropriate organic solvent and an acid or a base is just added to form a salt by addition of an acid or a base in a customary process.

Furthermore, a compound of the present invention and a pharmaceutically acceptable salt thereof are sometimes present in the form of an adduct with water or a solvent (hydrate or solvate). These adducts are also encompassed in the present invention.

These derivatives are converted in vivo and activated and also herein referred to as, a "prodrug". It is understood that examples of the prodrug include not only the above salts and solvates but also esters (for example, alkyl ester) and amides.

As examples of a compound of the present invention, various compounds are described in Examples. Those skilled in the art can produce a compound that is not disclosed in the present invention with reference to these and put in use.

Furthermore, the present invention also relates to a system, apparatus and kit for producing a compound of the present invention. As the constitutional elements of such a system, apparatus and kit, known elements in the art can be used. It is understood that the elements can be appropriately designed by those skilled in the art.

### (General synthetic process)

General and typical synthetic processes for a compound of the present invention described in Examples are described in general synthetic processes 1 to 7. The compounds described in Examples were synthesized almost in accordance with these; however, the synthetic process is not particularly limited to these processes. The reaction solvents, bases, palladium catalysts and phosphine ligands available for producing the compounds will be described below. Of them, preferable ones are presented in general synthetic processes 1 to 7; however, reaction solvents, bases, palladium catalysts and phosphine ligands are not limited to them.
(1) Reaction solvent: N,N-dimethyl formamide (DMF), N-methyl-2-pyrrolidone (NMP), N,N-dimethyl acetamide (DMA), dimethyl sulfoxide, aromatic hydrocarbons (e.g., toluene, benzene, xylene), saturated hydrocarbons (e.g., cyclohexane, hexane), halogenated hydrocarbons (e.g., dichloromethane, chloroform, 1,2-dichloroethane), ethers (e.g., tetrahydrofuran, diethyl ether, dioxane, 1,2-dimethoxyethane), esters (e.g., methyl acetate, ethyl acetate), ketones (e.g., acetone, methyl ethyl ketone), nitriles (e.g., acetonitrile), alcohols (e.g., methanol, ethanol, t-butanol), solvent mixtures of water and these, etc.
(2) Base: metal hydrides (e.g., sodium hydride), metal hydroxides (e.g., sodium hydroxide, potassium hydroxide, lithium hydroxide, barium hydroxide), metal carbonates (e.g., sodium carbonate, potassium carbonate, calcium carbonate, cesium carbonate), metal alkoxides (e.g., sodium methoxide, sodium ethoxide, potassium t-butoxide), sodium hydrogen carbonate, metal sodium, organic amines (e.g., triethylamine, diisopropylethylamine, 1,8-diazabicyclo[5.4.0]undec-7-ene (DBU), pyridine, 4-dimethylaminopyridine, 2, 6-lutidine), alkyl lithium (n-butyl lithium (n-BuLi), sec-butyl lithium (sec-BuLi), tert-butyl lithium (tert-BuLi)). etc.
(3) Palladium catalyst used in Pd coupling: Pd(PPh₃)₄, PdCl₂(dppf), PdCl₂(PPh₃)₂, Pd(OAC)₂, Pd(dba)₂, Pd₂(dba)₃, PdCl₂, etc.
(4) Phosphine ligand: PPh₃; BINAP, Xantphos, S-Phos, X-Phos, DPPF, P (t-Bu)₃, tris(o-tolyl)phosphine, etc.

(General synthetic process 1: Synthetic process in the case where (X, Y) = (-N=, =CR¹-))

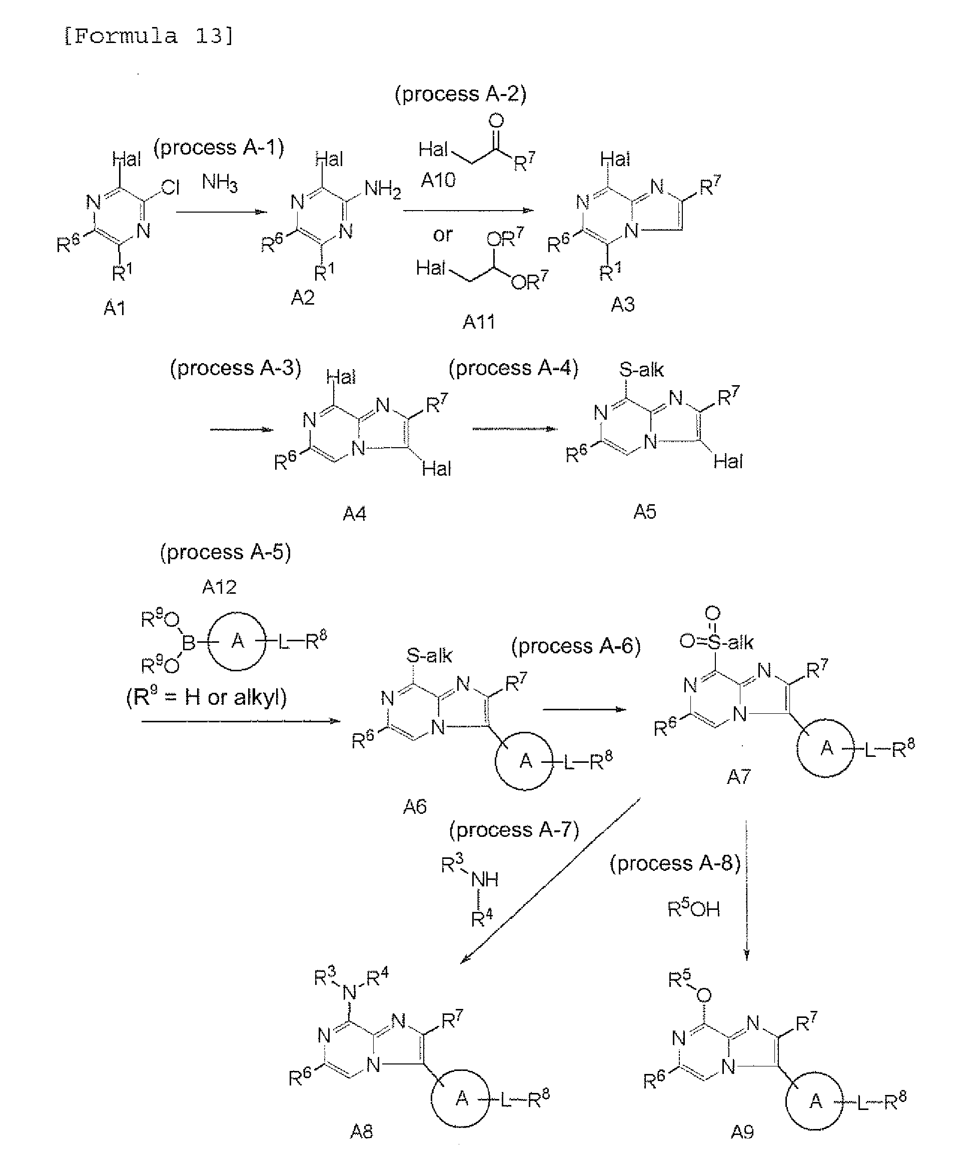

wherein symbols are the same as defined in above item (1A) or (1B); Hal represents halogen; and alk represents a C1-C3 alkyl.
In accordance with the process shown in the scheme, a compound represented by Formula A8 or Formula A9 can be produced from a compound represented by Formula A1- Details will be described below.

Process A-1:

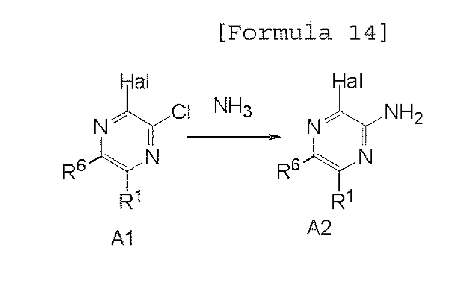

where symbols are the same as defined in above item (1A) or (1B), and Hal represents halogen.
A compound represented by Formula A2 can be produced by reacting an aqueous ammonia solution with a compound represented by Formula A1.

Desirably, the process is performed in a sealed tube at a temperature of about 100 to 150°C. The temperature may be further increased by use of a solvent mixture of a solvent such as dioxane, DMF and NMP and water depending on circumstances.

Process A-2:

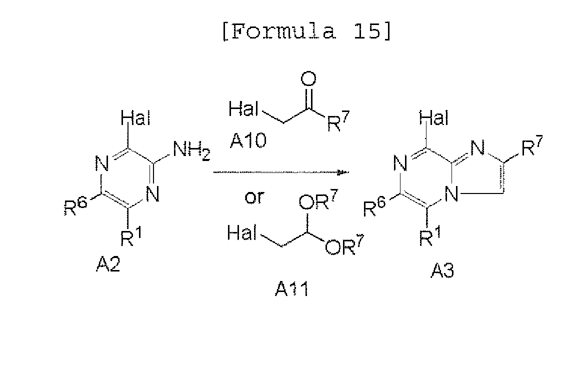

where symbols are the same as defined in above item (1A) or (1B) and Hal represents halogen.
A compound represented by Formula A3 can be produced by reacting, with a compound represented by Formula A2, a compound represented by Formula A10 or acetal shown in Formula A11. The reaction solvent is preferably water, solvents such as DMF, NMP, DMA or dioxane can be used as a mixture depending on circumstances. The reaction temperature is preferably about 100°C and may be increased to 150°C under sealed-tube conditions depending on circumstances.

Process A-3:

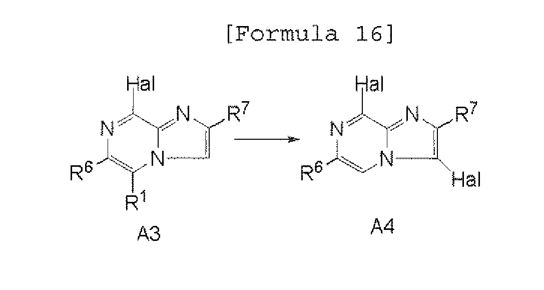

where symbols are the same as defined in above item (1A) or (1B) and Hal represents halogen.
A compound represented by Formula A4 can be produced by halogenating a compound represented by Formula A3. As the halogenation reagent, NBS, NIS, NCS or bromine can be used. As a reaction solvent, the solvents described in item (1) above are mentioned. An alcoholic solvent such as methanol and ethanol, a halide solvent such as DMF, NMP and dichloromethane, or acetic acid can be used. As the halogenation reagent, NBS, or NIS is desirably used. As a reaction solvent, an alcohol solvent, or DMF or NMP is desirably used. The reaction temperature may be about room temperature and may be increased to about 100°C depending on circumstances.

Process A-4:

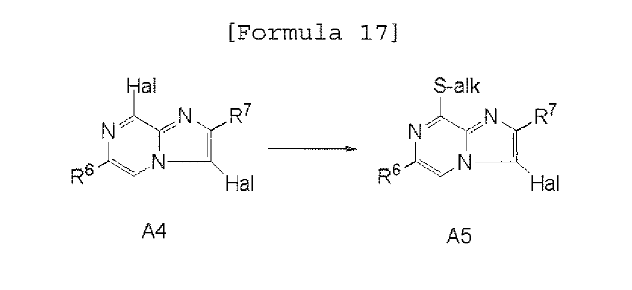

where symbols are the same as defined in above item (1A) or (1B), Hal represents halogen and alk represents a C1-C3 alkyl.
A compound represented by Formula A5 can be produced by reacting a sodium salt of an alkylthiol with a compound represented by Formula A4. As the alkyl group, a methyl group is preferable. As the reaction solvent, an alcohol solvent such as methanol and ethanol, dimethyl sulfoxide, NMP and DMF can be used. The reaction is desirably performed by increasing temperature to about 100°C or under reflux conditions.

Process A-5:

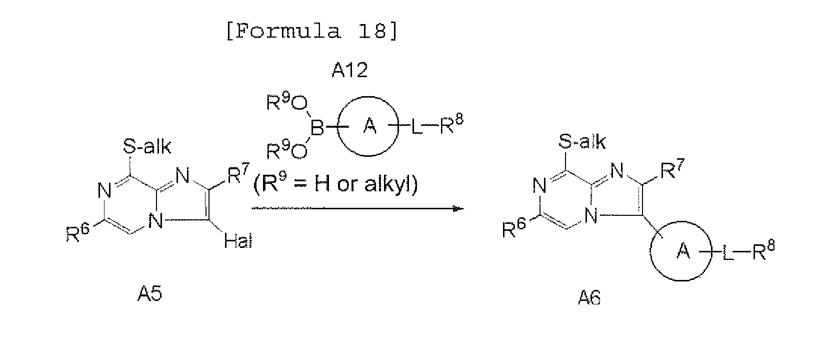

where symbols are the same as defined in above item (1A) or (1B), Hal represents halogen and alk represents a C1-C3 alkyl.
A compound represented by Formula A6 can be produced by the Suzuki coupling reaction between a compound represented by Formula A5 and a boronic acid or a boronic ester (i.e., R⁹=H or alkyl) represented by Formula A12. The Suzuki coupling reaction can be performed by using a known process, in which a palladium catalyst described in the above (3) and a phosphine ligand described in the above (4) depending on circumstances are used. As a reaction solvent, an ether solvent such as dioxane, THF and DME, an alcohol solvent such as ethanol and methanol, a solvent such as DMF, DMA and NMP, and solution mixtures of water and these solvents can be used. As the base, the bases described in the above (2), preferably a metal salt (e.g., sodium carbonate, calcium carbonate, cesium carbonate, sodium hydroxide, potassium fluoride, cesium fluoride, potassium acetate, sodium acetate) and an organic amine (e.g., triethylamine, diisopropylethylamine, DBU, 2,6-lutidine) can be used. The reaction can be performed by increasing temperature to about 60 to 100°C or under reflux conditions of the reaction solvent. If the reaction proceeds slowly, the temperature may be further increased by use of a microwave reaction apparatus.

Process A-6:

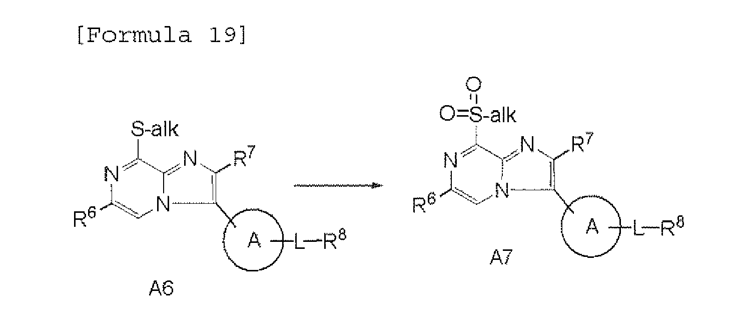

where symbols are the same as defined in above item (1A) or (1B), and alk represents a C1-C3 alkyl.
A compound represented by Formula A7 can be produced by oxidizing a compound represented by Formula A6 with m-CPBA. As the solvent, a halide solvent such as chloroform and dichloromethane is preferable. The reaction proceeds at room temperature.

Process A-7:

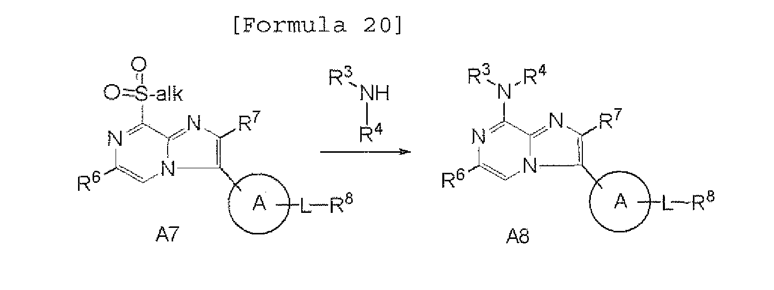

where symbols are the same as defined in above item (1A) or (1B), and alk represents a C1-C3 alkyl.
A compound represented by Formula A8 can be produced by reacting an amine represented by NHR³R⁴ with a compound represented by Formula A7. As a reaction solvent, an ether solvent such as dioxane, THF and DME, an alcohol solvent such as ethanol and methanol and a solvent such as DMF, DMA and NMP can be used. The reaction is preferably performed at about 100°C or under reflux conditions of the reaction solvent. If the reaction proceeds slowly, the temperature may be further increased by use of a microwave reaction apparatus.

Process A-8:

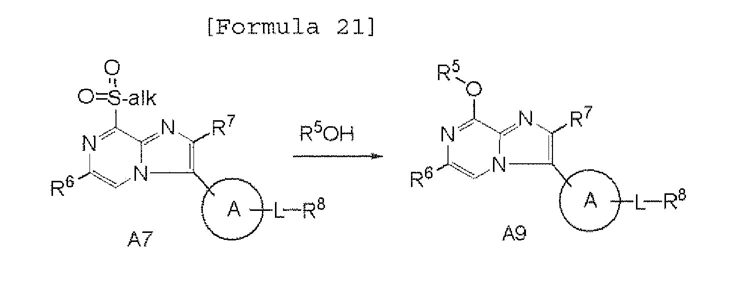

where symbols are the same as defined in above item (1A) or (1B) and alk represents a C1-C3 alkyl.
A compound represented by Formula A9 can be produced by reacting an alcohol represented by R⁵OH with a compound represented by Formula A7 in the presence of sodium hydride-As a reaction solvent., an ether solvent such as dioxane, THF and DME and a solvent such as DMF, DMA and NMP can be used. The reaction is preferably performed at about 100°C or under reflux conditions of the reaction solvent. If the reaction proceeds slowly, the temperature may be further increased by use of a microwave reaction apparatus.

(General synthetic process 2: synthetic process in the case where (X, Y) = (-CR² =, =N-))

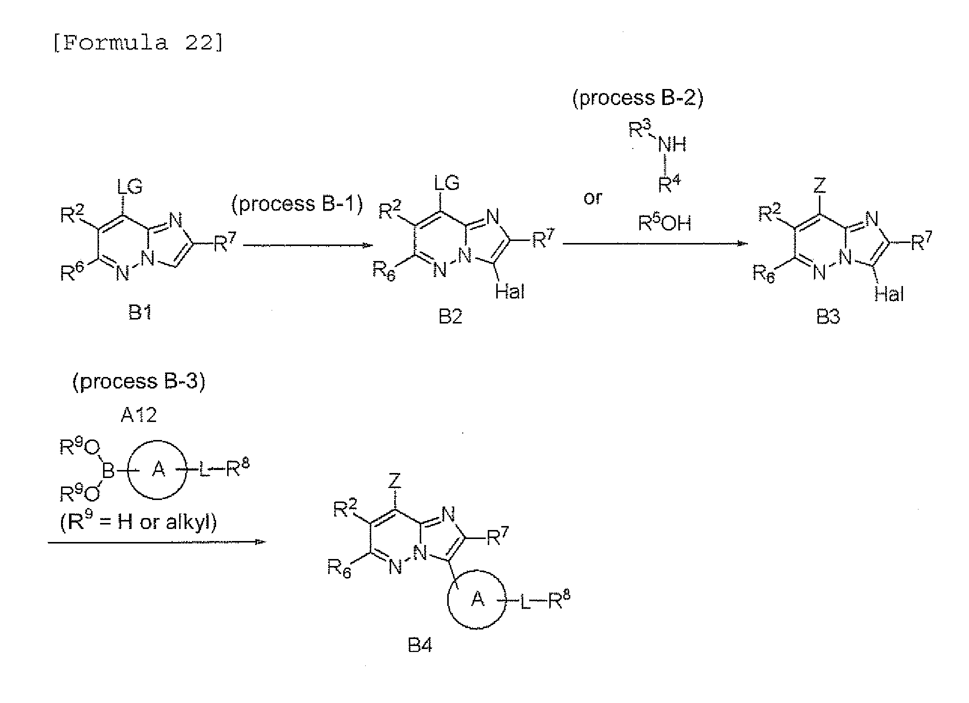

where symbols are the same as defined in above item (1A) or (1B), Hal represents halogen, LG represents a leaving group or halogen and Z represents NR³R⁴ or OR⁵ In accordance with the scheme, a compound represented by Formula B4 can be synthesized from a compound represented by Formula B1. The details will be described below.

### Process B-1:

A compound represented by Formula B2 can be produced by halogenating a compound represented by Formula B1 in the same conditions as in Process A-3. As the halogenation reagent, preferably, NBS or NIS is used and desirably an alcohol solvent is used. The reaction temperature may be about room temperature; however, increased to about 100°C depending upon circumstances.

### Process B-2:

A compound represented by Formula B3 can be produced by reacting an amine represented by NHR³R⁴ or R⁵OH with a compound represented by Formula B2 in the same conditions as in Process A-7 or A-8

### Process B-3:

A compound represented by Formula B3 can be produced by the Suzuki coupling reaction between the compound represented by Formula B3 and a boronic acid or a boronic ester (i.e., R⁹=H or alkyl) represented by Formula A12 in the same conditions as in Process A-5.

(General synthetic process 3: Process for modifying R⁶ moiety of Formulas (I) and (I'))

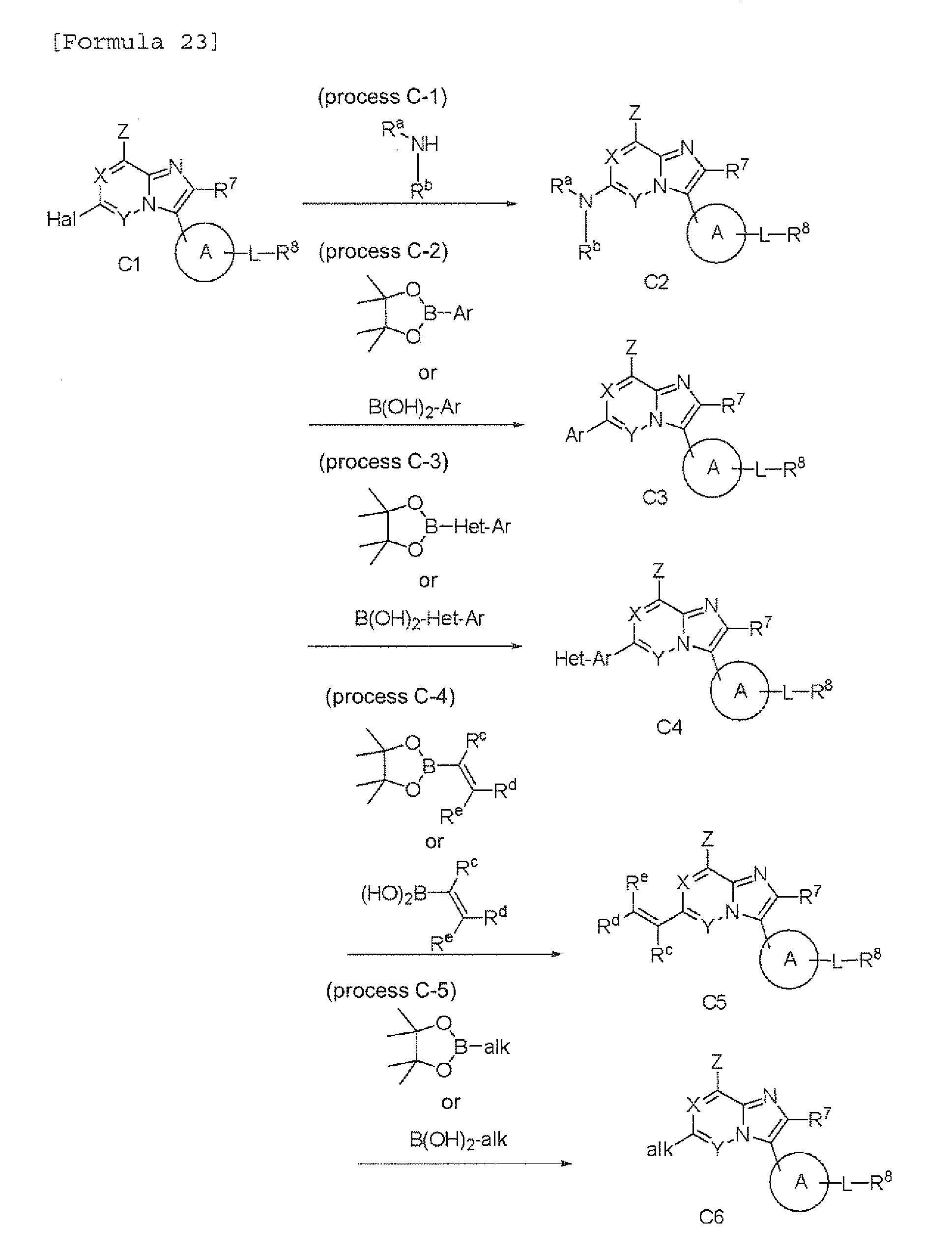

where symbols are the same as defined in above item (1A) or (1B), Hal represents halogen, Z represents NR³R⁴ or OR⁵ and alk represents a C1-C3 alkyl.
The R⁶ moiety of compounds represented by Formulas (I) and (I') is modified with various groups in accordance with the aforementioned Process C-1 to C-5.

### Process C-1:

A compound represented by Formula C2 can be produced in the same conditions as in Process A-7 by reacting an amine represented by NHR^{a}R^{b} with a compound represented by Formula C1, where R^{a} and R^{b} are each independently an alkyl that is optionally substituted or R^{a} and R^{b} may be taken together with an adjacent nitrogen atom to form a substituted or unsubstituted nitrogen-containing hetero ring.

### Processes C-2 to C-5:

Compounds represented by Formulas C3 to C6 can be produced each by the Suzuki coupling reaction between a compound represented by Formula C1 and the aforementioned boronic acid or boronic ester in the same conditions as in Process A-5. In the formula, Ar is aryl that is optionally substituted, Het-Ar is a heteroaryl that is optionally substituted and R^{c}, R^{d} and R^{e} is H or an aryl.

(General synthetic process 4: Process for modifying L-R⁸ moiety of Formulas (I) and (I'))

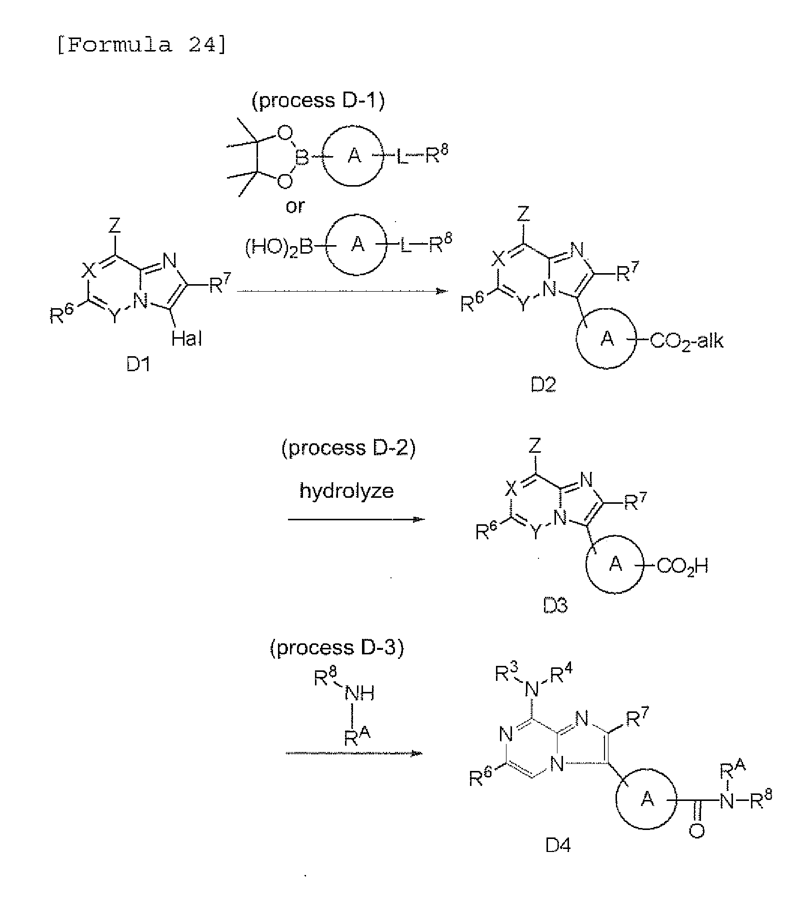

where symbols are the same as defined in above item (1A) or (1B), Hal represents halogen, Z represents NR³R⁴ or OR⁵ and alk represents a C1-C3 alkyl.
The L-R⁸ moiety of compounds represented by Formulas (I) and (I') can be converted into various substituents in accordance with the aforementioned scheme.

### Process D-1:

A compound represented by Formula D2 can be produced by the Suzuki coupling reaction between a compound represented by Formula D1 and a boronic acid or a boronic ester represented by the aforementioned Formula in the same conditions as in Process A-5.

If the compound represented by Formula D2 where L-R⁸ is represented by CO₂-alk, the compound can be converted into a compound represented by Formula D3 by the following process D-2.

### Process D-2:

A compound represented by Formula D3 can be produced by hydrolyzing a compound represented by Formula D2. As a reaction solvent, an ether solvent such as dioxane, THF and DME, an alcohol solvent such as ethanol and methanol, and a solvent mixture of a solvent such as DMF, DMA, DMSO and NMP and water can be used. As a base, sodium hydroxide and lithium hydroxide can be used. The reaction temperature is desirably room temperature; however, if the reaction proceeds slowly, the reaction temperature may be further increased.

### Process D-3:

A compound represented by Formula D4 can be produced by reacting an amine represented by NHR⁸R^{A} with a compound represented by Formula D3 in the same conditions as in Process A-7.

(General synthetic process 5: Process for synthesizing a boronic ester)

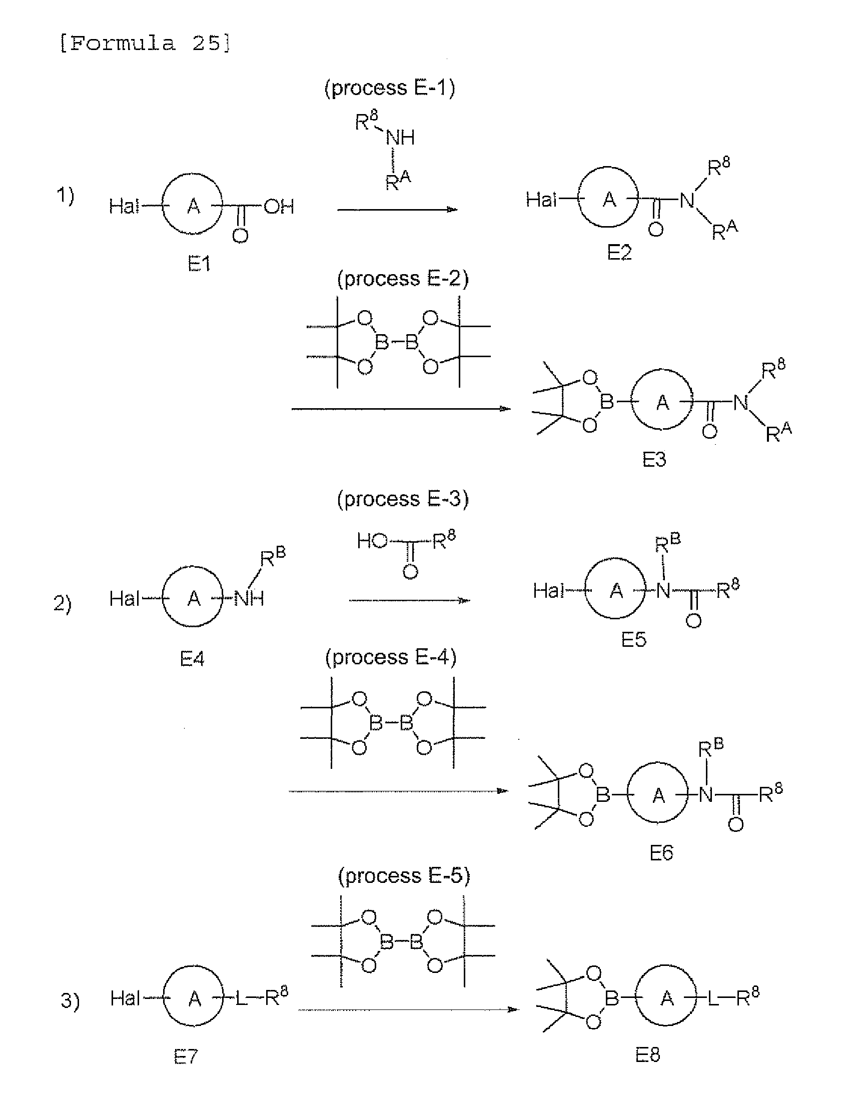

where symbols are the same as defined in above item (1A) or (1B) and Hal represents halogen
In accordance with schemes 1) to 3), a boronic ester can be produced for synthesizing the L-R⁸ moiety of Formulas (I) and (I').

### Process E-1:

A compound represented by Formula E2 can be synthesized by fusing a compound represented by Formula E1 with a compound represented by Formula NHR^{A}R⁸. As a reaction solvent, DMF, NMP, DMA, dimethyl sulfoxide, dichloromethane, tetrahydrofuran, dioxane, acetonitrile, etc. can be used. As a condensing agent, dicyclohexyl carbodiimide (DCC), benzotriazol-1-yloxy-trisdimethylaminophosphate (BOP), hexafluorophosphoric acid (benzotriazol-1-yloxy)tripyrrolidinophosphonium (PyBOP), hexafluorophosphoric acid bromotrispyrrolidino phosphonium (PyBrop), HATU, DPPA, 1-ethyl-3-(3-dimethylaminopropyl)carbodiimide hydrochloride (WSC), 4-(4,6-dimethoxy-1,3,5-triazin-2-yl)-4-methylmorpholinium chloride (DMT-MM), etc. can be used. Furthermore, these reagents can be used in combination with, for example, 1-hydroxysuccineimide (HOSu), 1-hydroxybenzotriazole (HOBt) and 1-hydroxy-7-azabenzotriazole (HOAt) can be used. Depending on circumstances, the reaction is preferably performed in the presence of an organic amine such as triethylamine and DIEA. The reaction temperature and the reaction time are not particularly limited; however, the reaction is usually performed at room temperature. If the reaction proceeds slowly, the reaction is sometimes facilitated by increasing the temperature.

### Process E-2:

A compound represented by Formula E3 can be produced by the Suzuki coupling reaction between a compound represented by Formula E2 and a boronic ester represented by the aforementioned Formula in the same conditions as in Process A-5.

### Process E-3:

A compound represented by Formula E5 can be synthesized by fusing a compound represented by Formula E4 with a carboxylic acid represented by Formula HO(C=O)R⁸ in the same conditions as in Process E-1.

### Process E-4:

A compound represented by Formula E6 can be produced by the Suzuki coupling reaction between a compound represented by Formula E5 and a boronic ester represented by the aforementioned Formula in the same conditions as in Process A-5.

### Process E-5:

A compound represented by Formula E8 can be produced by the Suzuki coupling reaction between a compound represented by Formula E7 and a boronic ester represented by the aforementioned Formula in the same conditions as in Process A-5.

(General synthetic process 6: Another synthesis process in the case where (X, Y) = (-N=, =CR¹-))

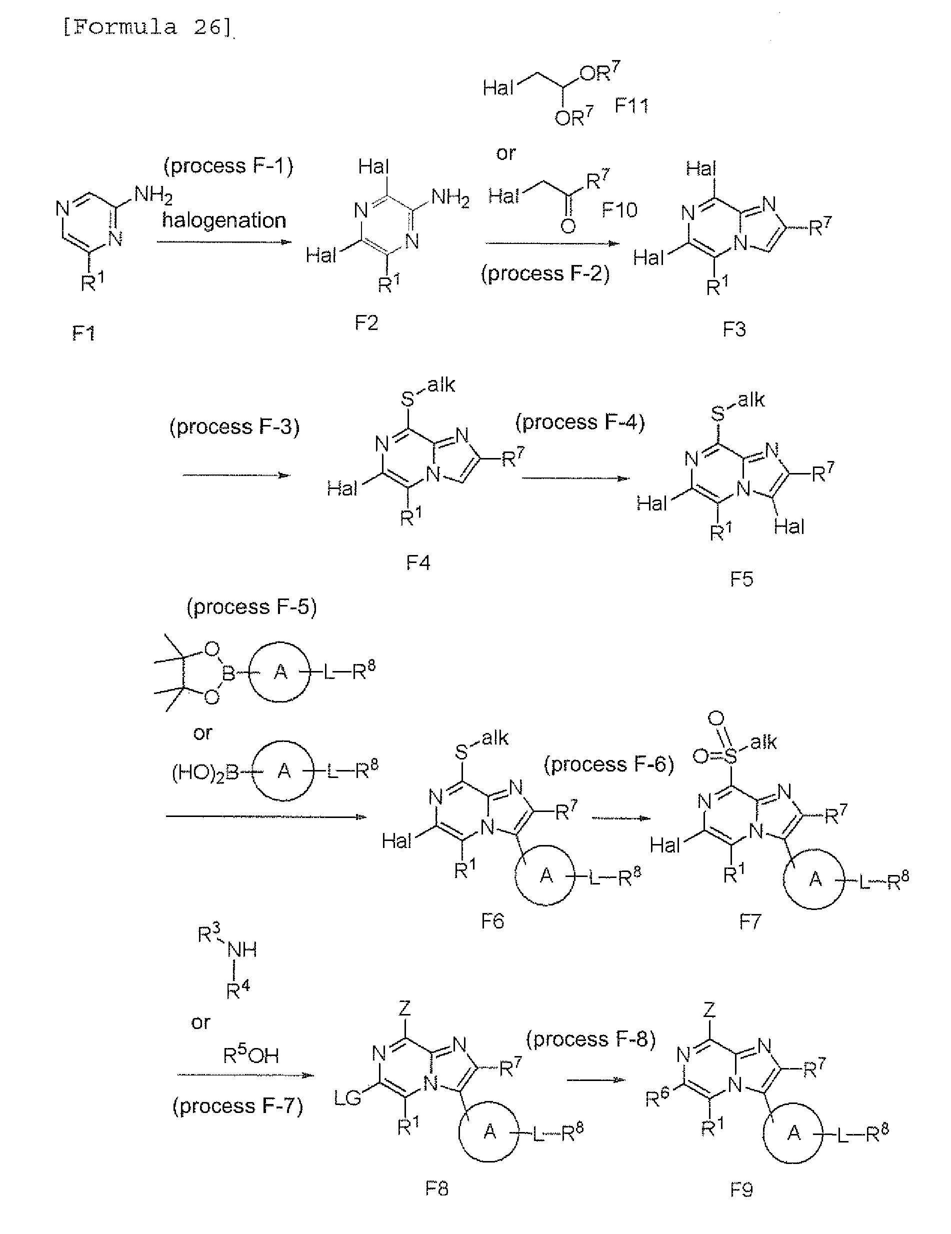

where symbols are the same as defined in above item (1A) or (1B), Hal represents halogen, LG represents a leaving group or a halogen, Z represents NR³R⁴ or OR⁵ and alk represents a C1-C3 alkyl.
In accordance with the process of the scheme, a compound represented by Formula F9 can be produced from a compound represented by Formula F1. The details will be described below.

### Process F-1:

A compound represented by Formula F2 can be produced by halogenating a compound represented by Formula F1 in the same conditions as in Process A-3.

### Process F-2:

A compound represented by Formula F3 can be produced by reacting a compound represented by Formula F10 or acetal represented by Formula F11 with a compound represented by Formula F2 in the same conditions as in Process A-2.

### Process F-3:

A compound represented by Formula F4 can be produced by reacting a sodium salt of an alkylthiol with a compound represented by Formula F3 in the same conditions as in Process A-4.

### Process F-4:

A compound represented by Formula F5 can be produced by halogenating a compound represented by Formula F4 in the same manner as in Process A-3.

### Process F-5:

A compound represented by Formula F6 can be produced by the Suzuki coupling reaction between a compound represented by Formula F5 and a boronic ester or a boronic acid represented by the aforementioned Formula in the same conditions as in Process A-5.

### Process F-6:

A compound represented by Formula F7 can be produced by oxidizing a compound represented by Formula F6 with m-CPBA in the same manner as in Process A-6.

### Process F-7:

A compound represented by Formula F8 can be produced by reacting an amine represented by NHR³R⁴ or R⁵OH with a compound represented by Formula F7 in the same conditions as in Process A-7 or A-8.

### Process F-8:

A compound represented by Formula F9 can be produced from a compound represented by Formula F8 in the process described in the above general synthetic process 3.

(General synthetic process 7: Another process for modifying R⁶ moiety of Formulas (I) and (I'))

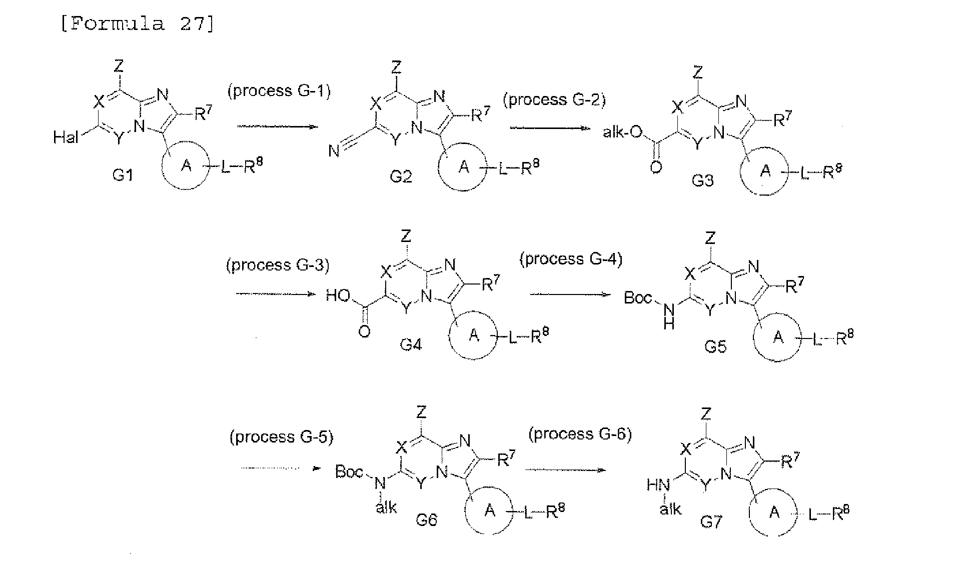

where symbols are the same as defined in above item (1A) or (1B), Hal represents a halogen, Z represents NR³R⁴ or OR⁵ and alk represents a C1-C3 alkyl.
The R⁶ moiety of the compounds of Formulas (I) and (I') can be converted into various substituents in accordance with the scheme.

### Process G-1:

A compound represented by Formula G2 can be produced by using a compound represented by Formula G1, Zn (CN)₂, a palladium catalyst described in the above (3), and, if necessary, a phosphine ligand described in the above (4). As a reaction solvent, an ether solvent such as dioxane, THF and DME, an alcohol solvent such as ethanol and methanol, a solvent such as DMF, DMA and NMP, and a solvent mixture of these and water can be used. The reaction is performed by increasing the temperature to about 60 to 100°C or under reflux conditions of the reaction solvent.

### Process G-2:

A compound represented by Formula G3 can be produced by using a compound represented by Formula G2, trimethylsilyl chloride, and an alcohol solvent (alk-OH). As the alcohol solvent, ethanol and methanol are mentioned. The reaction is performed by increasing the temperature to about 60 to 100°C or under reflux conditions of the reaction solvent.

### Process G-3:

A compound represented by Formula G4 can be produced by hydrolyzing a compound represented by Formula G3 in the same conditions as in Process D-2.

### Process G-4:

A compound represented by Formula G5 can be produced by reacting a compound represented by Formula G4 with an azide compound in the presence of an alcohol solvent. As the azide compound, DPPA is preferred. Furthermore, if t-BuOH is used as the alcohol solvent, the corresponding compound can be obtained. The reaction can be performed by increasing temperature to about 60 to 100°C or under reflux conditions of the reaction solvent.

### Process G-5:

A compound represented by Formula G6 can be produced by reacting a compound represented by Formula G5 with an alkyl halide in the presence of a base. As the base, those described in the above (2), preferably sodium hydride can be used. The reaction temperature and reaction time are not particularly limited; however, the reaction is usually performed at room temperature. If the reaction proceeds slowly, the reaction is sometimes facilitated by increasing the temperature.

### Process G-6

A compound represented by Formula G7 can be produced by deprotecting a compound represented by Formula G6. Deprotection can be performed by using, for example, TFA, at room temperature.

(General synthetic process 8)

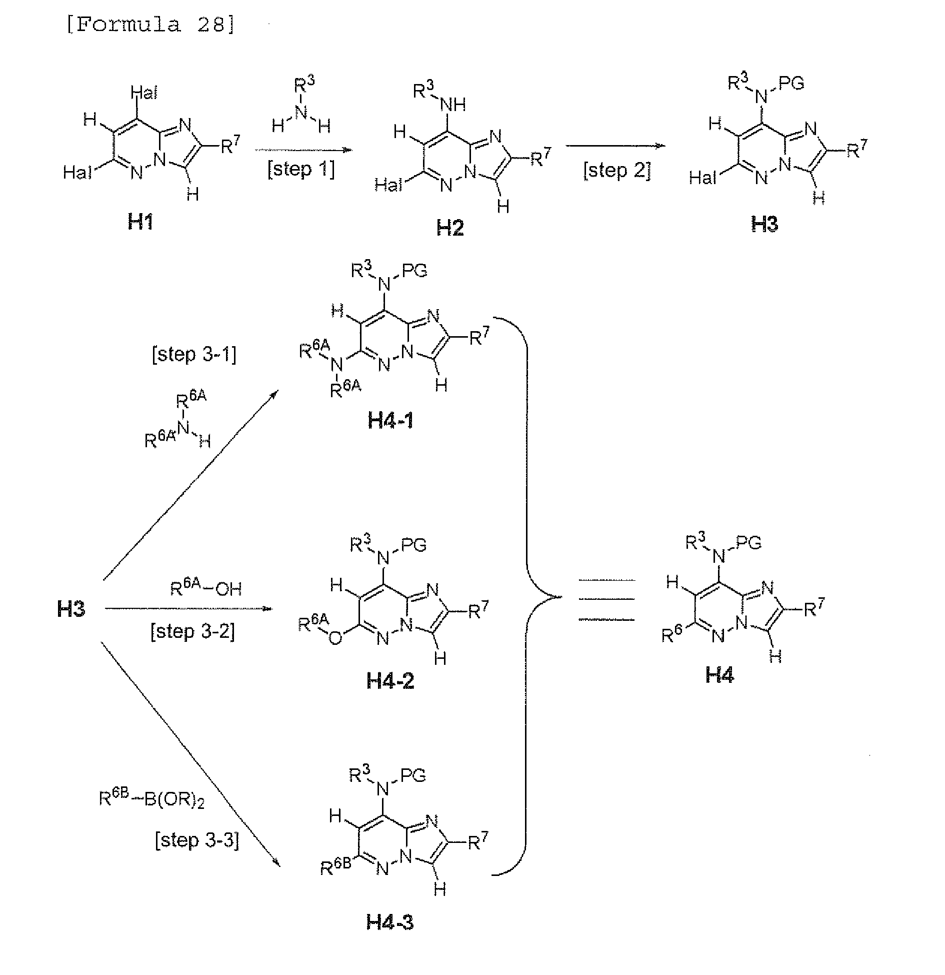

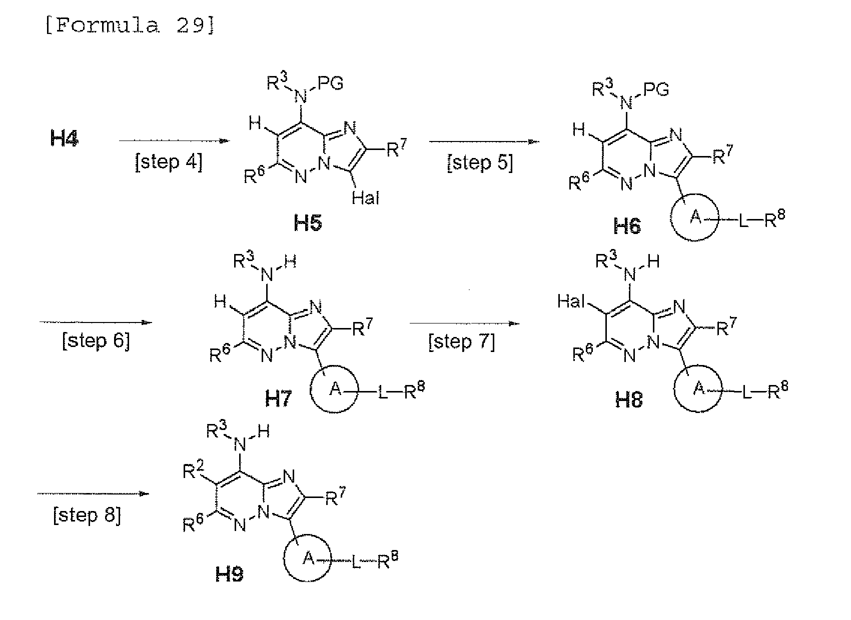

In accordance with the process shown in the scheme, a compound (H9) can be synthesized from a compound (H1) The symbols shown in each Formula are the same as defined in the above.

### Step 1:

A compound (H2) can be produced by the addition reaction between a compound (H1) and an amine (R³-NH₂). The reaction can be performed in an alcohol solvent such as ethanol or a solvent such as NMP and DMF, in the presence of a tertiary amine such as DIEA or an inorganic base such as potassium carbonate. The reaction temperature is preferably 50 to 100°C. If the reaction proceeds slowly, the temperature can be further increased by use of a microwave reaction apparatus.

### Step 2:

This step is a step of protecting an amino group of a compound (H2). As the protective group, a carbamate based protective group is preferred. In particular, a Boc group is preferred. The reaction can be performed using an excessive amount of BOC₂O and in the presence of a catalytic amount of DMAP in a solvent such as THF, at a reaction temperature of room temperature to about 50°C.

### Step 3:

This step is a step of synthesizing a compound represented by Formula (H4) by introducing a substituent R⁶ by a substitution reaction between a compound (H3) and a nucleophile or by a cross coupling reaction using a palladium catalyst, etc. Note that a compound represented by Formula (H4) is identical with each of compounds represented by Formula (H4-1), Formula (H4-2) and Formula (H4-3). Two R^{6A} indicated in the formula (R^{6A}R^{6A}NH) of amine may be each independently substituted with a different substituent.

Step 3-1 is a step of producing a compound (H4-1) by the Buchwald reaction of a compound (H3) and an amine (R^{6A}R^{6A}NH). This reaction is a process known to those skilled in the art. For example, as a palladium catalyst, e.g., Pd(OAc)₂ or Pd₂(dba)₃ is used. Xantphos or RuPhos is mentioned as the ligand, dioxane and toluene as the reaction solvent, and potassium carbonate, cesium carbonate and sodium-t-butoxide as the base. The reaction temperature is preferably 50°C to about solvent reflux temperature. If the reaction proceeds slowly, the temperature can be further increased by use of a microwave reaction apparatus. In this step, in particular, if a substituent represented by R^{6A} is bulky, it is preferable that RuPhos is used as a ligand and sodium-t-butoxide is used as a base. Furthermore, if a hydrochloride of an amine is used, a dioxane solvent is preferably used.

Step 3-2 is a step of producing a compound (H4-2) by reacting a compound (H3) with an alcohol or a phenol derivative (R^{6A}-OH). This reaction can be performed at room temperature to a temperature of about 100°C by adding a compound (H3), after a compound represented by R^{6A}-OH is treated with a base such as sodium hydride in a solvent such as THF. If the reaction proceeds slowly, the temperature can be further increased by use of a microwave reaction apparatus. Furthermore, if R^{6A} is a bulky aliphatic alcohol, the reaction can be sometimes facilitated by using a palladium catalyst. Pd₂(dba)₃ can be used as the palladium catalyst, X-Phos as the ligand, toluene or dioxane as the solvent.

Step 3-3 is a step of producing a compound (H4-3) by the Suzuki reaction between a compound (H3) and organic boronic acid or boronic ester (R^{6A}-B (OR)₂). R represents hydrogen or an alkyl group, each may form a ring. The Suzuki reaction is a process known to those skilled in the art and conditions disclosed in literatures can be employed. PdCl₂(dppf)-CH₂Cl₂ or PdCl₂(dtbpf) can be used as the palladium catalyst, an aqueous potassium carbonate solution or an aqueous sodium carbonate solution as the base, and THF, dioxane, DMF, NMP, etc., as the solvent. The reaction temperature is preferably room temperature to about a solvent reflux temperature. If the reaction proceeds slowly, the temperature can be further increased by use of a microwave reaction apparatus.

### Step 4:

This step is a step of producing a compound (H5) and can be performed in the same manner as in Process A-3 for producing a compound (A4).

### Step 5:

This step is a step of producing a compound (H6) and can be performed in the same manner as in Process A-5 for producing a compound (A6).

### Step 6:

This Step is a step of producing a compound (H7) by deprotecting the protective group represented by PG of a compound (H6). If the protective group is a Boc group (PG = Boc), deprotection can be performed by treatment with 30 to 50% TFA/dichloromethane.

### Step 7:

This step is a step of producing a compound (H8) by introducing a halogen into a compound (H7). The halogenation reaction can be performed in reaction conditions known to those skilled in the art. For example, a fluorine group can be introduced by use of an electrophilic fluorination reagent such as N-fluoro-2,6-dichloropyridinium triflate, a chloro group by use of NCS, a bromo group by use of NBS or bromine, and an iodine group by use of NIS or iodine.

### Step 8:

This step is a step of producing a compound (H9) by substituting a halogen of a compound (H8) with a substituent R² by use of a cross-coupling reaction, etc. By the methods of Step 3-1, Step 3-2, and Step 3-3, an amino group, an alkoxy group, a phenoxy group, an aryl group, a heteroaryl group, etc. can be introduced. Furthermore, a cyano group can be introduced in the same manner as in a production process, Process G-1 for a compound (G2).

(General synthetic process 9)

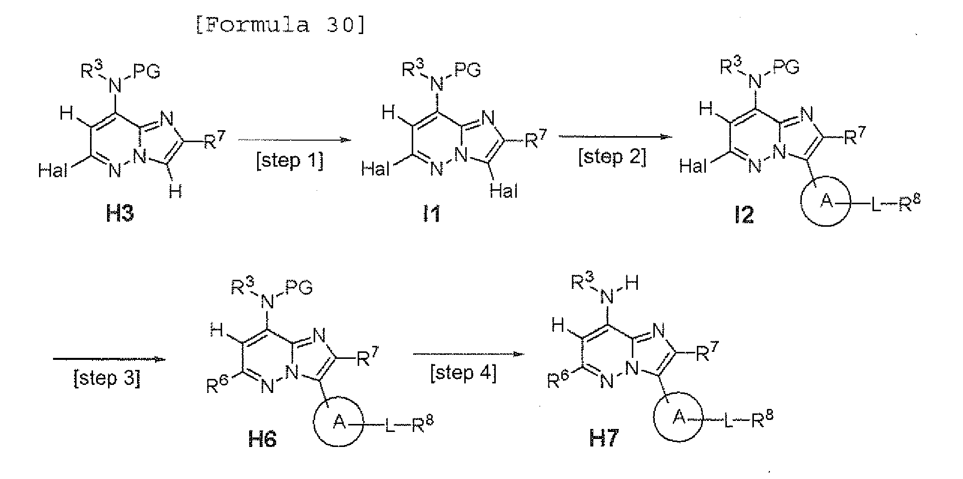

### Step 1:

This step is a step of producing a compound (I1) by introducing a halogen group into a compound (H3) and can be performed in the same manner as in Process A-3 for producing a compound (A4).

### Step 2:

This step is a step of producing a compound (I2) and can be performed in the same manner as in Process A-6 for producing a compound (A6).

### Step 3:

This step is a step of producing a compound (H6) and can be performed in the same manner as in Step 3-1, Step 3-2, and Step 3-3 of general synthetic process 8.

### Step 4:

This step is a step of producing a compound (H7) and can be performed in the same manner as in Step 6 of general synthetic process 8.

(General synthetic process 10)

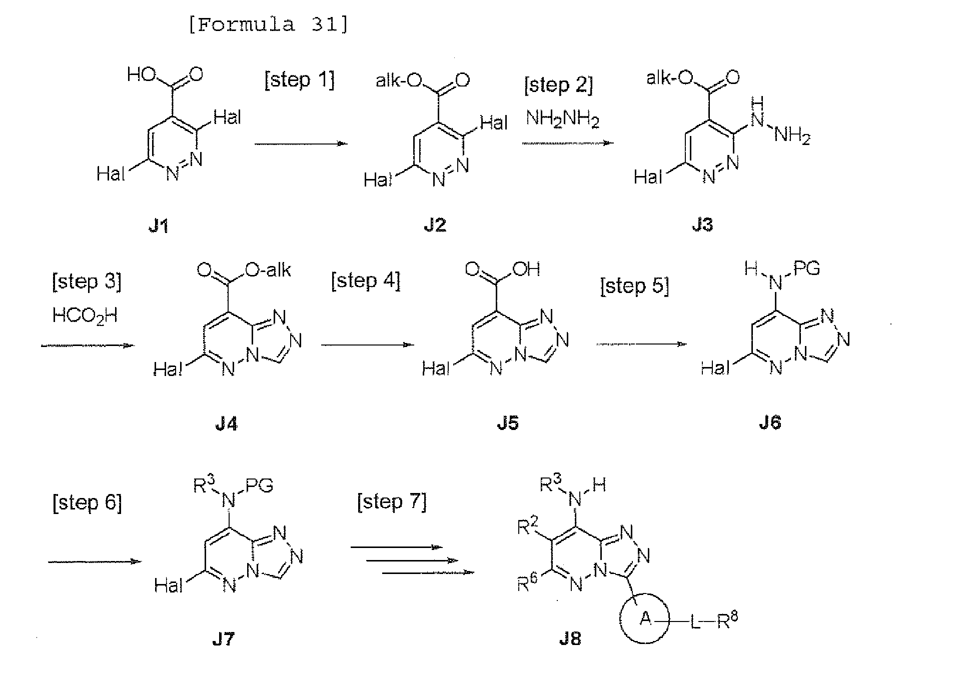

In accordance with the process shown in the above scheme, a compound (J8) can be synthesized from a compound (J1). The symbols shown in each Formula are the same as defined in the above.

### Step 1:

A compound (J2) can be produced by the reaction between a compound (J1) and an alcohol (alk-OH). The reaction can be performed in a solvent such as THF, DMF and NMP in the presence of alcohol (alk-OH), a condensing agent such as EDC and HATU and a catalytic amount of DMAP, at room temperature.

### Step 2:

A compound (J3) can be produced by the reaction between a compound (J2) and a hydrazine hydrate (NH₂NH₂). The reaction can be performed in a solvent such as THF at a reaction temperature of room temperature to 50°C.

### Step 3:

This step is a step of producing a compound (J4) by the reaction between a compound (J3) and formic acid. The reaction can be performed in a solvent such as toluene at a reaction temperature of 50 to 100°C.

### Step 4:

This step is a step of producing a compound (J5) by hydrolyzing a compound (J4) and can be performed in the same manner as in Process D-2

### Step 5:

This step is a step of producing a compound (J6) and can be performed in the same manner as in Process G-4

### Step 6:

This step is a step of producing a compound (J7) by fusing a compound (J6) and an alcohol in accordance with the Mitsunobu reaction. The Mitsunobu reaction can be performed by a method known to those skilled in the art.

### Step 7:

This step is a step of producing a compound (J8) and can be performed in the same manner as in Steps 3-1 to 3-3, Steps 4 to 8 of general synthetic process 8.

(General synthetic process 11)

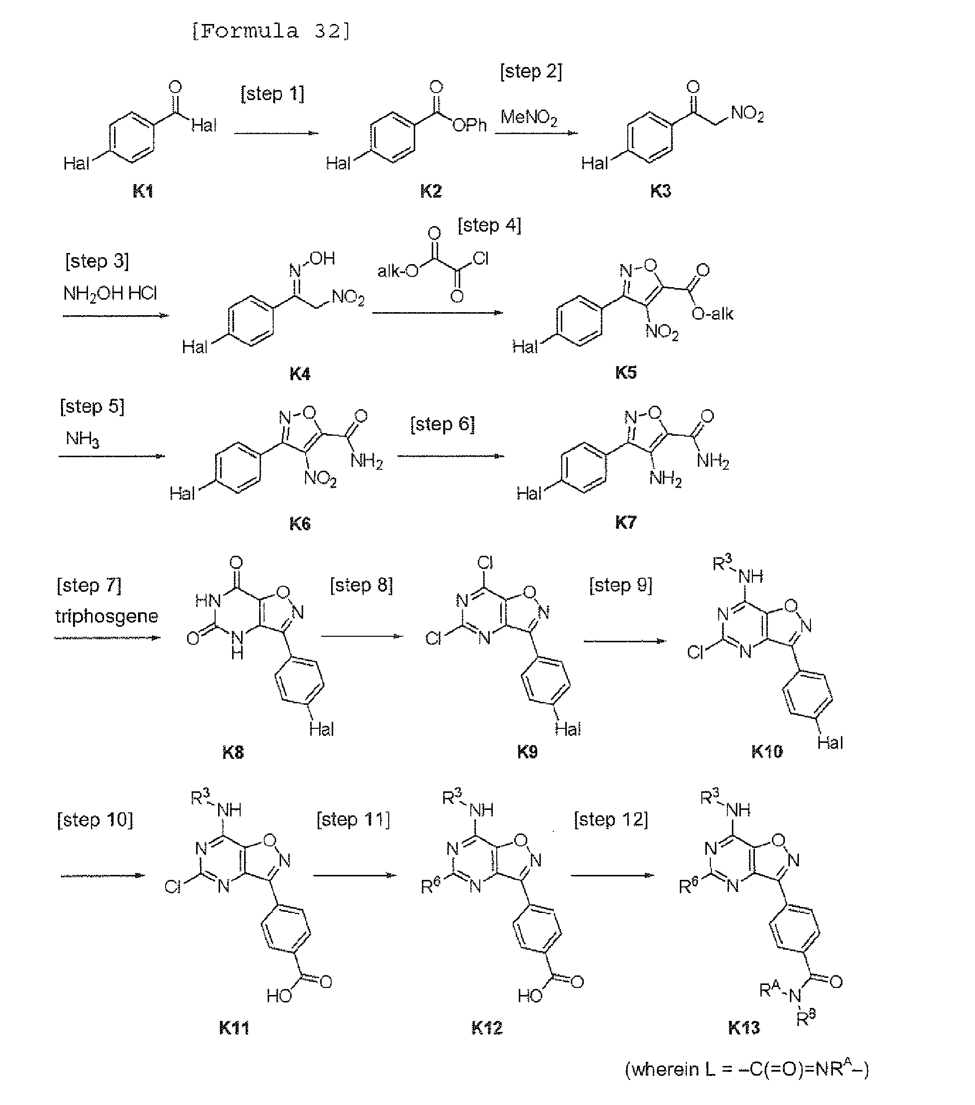

In accordance with the process shown in the above scheme, a compound (K13) can be synthesized from a compound (K1). The symbols shown in each Formula are the same as defined in the above.

### Step 1:

A compound (K2) can be produced by the reaction between a compound (K1) and phenol. The reaction can be performed in a solvent such as dichloromethane in the presence of a tertiary amine such as triethylamine. The reaction temperature is preferably 0 to 50°C.

### Step 2:

A compound (K3) can be produced by the reaction between a compound (K2) and nitromethane. The reaction can be performed in a solvent such as DMSO, in the presence of a base such as potassium-t-butoxide. The reaction temperature is preferably 0 to 50°C.

### Step 3:

A compound (K4) can be produced by the reaction between a compound (K3) and hydroxylamine hydrochloride. The reaction can be performed in a solvent such as ethanol. The reaction temperature is preferably room temperature to 100°C.

### Step 4:

A compound (K5) can be produced by the reaction between a compound (K4) and alkyl 2-chloro-2-oxoacetate (for example, ethyl 2-chloro-2-oxoacetate). The reaction can be performed in a solvent such as THF in the presence of a base such as DIEA. The reaction temperature is preferably 0 to 50°C.

### Step 5:

A compound (K6) can be produced by the reaction between a compound (K5) and an ammonia·methanol solution. The reaction temperature is preferably 0 to 50°C.

### Step 6:

A compound (K7) can be produced by reducing a compound (K6). The reaction can be performed in a solvent mixture such as THF/methanol/water, in the presence of a reducing agent such as iron powder. The reaction temperature is preferably 0 to 100°C.

### Step 7:

A compound (K8) can be produced by the reaction between a compound (K7) and triphosgene. The reaction can be performed in, a solvent such as dioxane at a reaction temperature of 0 to 100°C.

### Step 8:

A compound (K9) can be produced by the reaction between a compound (K8) and a reagent such as phenylphosphonic dichloride and oxyphosphorus chloride. The reaction temperature is preferably about 100 to 200°C. If the reaction proceeds slowly, the temperature can be further increased by use of a microwave reaction apparatus.

### Step 9:

This step is a step of producing a compound (K10) and performed in the same manner as in Step 1 of general synthetic process 8.

### Step 10:

A compound (K11) can be produced by converting a halogen of a compound (K10) to a carboxylic acid. The reaction can be performed by reacting the compound (K10) and an n-butyl lithium/hexane solution and further adding CO₂ (dry ice). The reaction temperature is preferably -78°C.

### Step 11:

This step is a step of producing a compound (K12) and can be performed in the same manner as in Steps 3-1 to 3-3 of general synthetic process 8. To introduce an amino group, an amine corresponding to the compound (K11) can be reacted in a solvent such as NMP at a temperature of 100 to 200°C.

### Step 12:

This step is a step of producing a compound (K13) by reacting a compound (K12) and an amine (R^{A}R⁸NH) and can be performed in the same manner as in Process E-1.

Production of the present invention can be performed by appropriately modifying preferable embodiments as mentioned above, combining them or adding conventional technique.

A compound of the present invention can be protected by using a protective group. For example, a compound of the present invention can be produced by protecting an appropriate substituent by a method known in the art, typically at a halogen (I, Br, Cl, F, etc.), a lower (typically represents C1-C6 herein but is not limited thereto) alkoxy, a lower alkylthio, a lower alkylsulfonyloxy (represents aryl sulfonyloxy etc..). Examples of such a protective group include protective groups of ethoxycarbonyl, t-butoxycarbonyl, acetyl and benzyl described Protective Groups in Organic Synthesis, written by T. W. Green, John Wiley & Sons Inc. (1981), etc. A protective group can be introduced or removed by a method customarily used in organic synthetic chemistry [see, for example, Protective Groups in Organic Synthesis, written by T. W. Greene, John Wiley & Sons Inc. (1981)] or in accordance with these customary methods. Furthermore, a functional group contained in a substituent can be converted by not only the production process but also a known method [for example, Comprehensive Organic Transformations, written by R. C. Larock (1989)]. The compounds of the present invention include a compound, which serves as a synthetic intermediate to further obtain a novel derivative. In the aforementioned production processes, intermediates and target compounds can be isolated and purified by subjecting them to a purification method customarily used in organic synthetic chemistry, e.g., neutralization., filtration, extraction, washing, drying, concentration., recrystallization, various chromatographic methods. Furthermore, an intermediate can be subjected to the following reaction without particularly being purified.

### (TTK protein kinase)

"TTK protein kinase" is an enzyme described and defined in Patent Literature 3, and is exemplified by a polypeptide having an amino acid sequence registered, for example, as Genbank NM_003318 or an amino acid sequence where one or more amino acids have been deleted, added, inserted or replaced in the above amino acid sequence, and having kinase activity. As a preferable amino acid sequence herein, any one of the amino acid sequences enumerated in Patent Literature 3 can be employed. "TTK protein kinase activity" and "TTK activity" refer to phosphorylation of threonine and/or serine and/or tyrosine. For example, in p38 MAPK (see Patent Literature 3), phosphorylation of the 180th threonine and/or the 182th tyrosine is mentioned. TTK activity can be measured by use of a measurement method used in a screening method of Patent Literature 3. Whether or not a polypeptide has kinase activity can be checked by bringing, for example, a polypeptide, a substrate for measuring TTK activity and a phosphate-group donor into contact with each other, measuring TTK activity and comparing the TTK activity with that of wild-type TTK protein kinase measured in the same conditions. A substrate for TTK activity measurement and a phosphoric acid donor known in the art can be used. Alternatively, a novel substrate for TTK activity measurement described in the present invention may be used. As to the TTK activity, Non Patent Literature 1 can serve as a reference. As measurement of TTK activity, the TTK activity measurement described in, for example, Patent Literature 3 can be mentioned. Any polypeptide can serve as TTK protein kinase as long as the polypeptide has TTK protein kinase activity. To describe more specifically, any polypeptide is acceptable as long as it contains the amino acid sequence, which is known as a kinase activity domain and described in Patent Literature 3 and as long as the amino acid sequence has a kinase activity even if one or more amino acids are deleted, added, inserted or replaced therein. The whole length of the polypeptide, addition of a modification group, and modification of an amino acid residue, etc. may be appropriately selected, if necessary. The sequence thereof is not limited solely to that described in the specification. Therefore, if "TTK" is simply referred to in the specification, unless otherwise noted, it can be interpreted that it has the same meaning as the "TTK protein kinases". Note that TTK has the following alternative names and can be referred to by these names.
TTK → TTK PROTEIN KINASE
hMPS1 → human MONOPOLARSPINDLE 1
PYT→ PHOSPHOTYROSINE-PICKED THREONINE KINASE
MPS1L1 → MONOPOLARSPINDLE 1-LIKE 1
ESK → ESK; MOUSE HOMOLOG OF ESK (= EC STY Kinase).

A compound or a salt thereof obtained by the screening method of the present invention can exert a therapeutic or prophylactic action to a disease which is developed in association with elevation of TTK activity. For example, according to the screening method of the present invention, it is possible to screen a candidate compound for a therapeutic or prophylactic agent effective for e.g., a cancer and immune disease developed in association with elevation of TTK activity.

Examples of "cancer" as mentioned above include various malignant neoplasms such as solid cancer, angiotna, blood vessel endothelioma, sarcoma, Kaposi's sarcoma and hematopoietic tumor, also include large intestine cancer and liver cancer, and further include metastasis of these cancers. In the present invention, a series of novel compounds inhibiting TTK kinase action and having specific properties particularly useful for preparing a pharmaceutical product for treating the aforementioned diseases were successfully found. Particularly, the compounds of the invention are useful for treating proliferative diseases such as cancer which has been known to have activated TTK kinase action and developed in the form of either a solid tumor or a blood tumor, in particular, diseases such as colon/rectal cancer, breast cancer, lung cancer, prostate cancer, pancreatic cancer or bladder cancer and renal cancer, as well as leukemia and lymphoma.

Examples of "immune disease" include atopy, asthma, rheumatism, collagen disease and allergy.

Furthermore, by using a compound of the present invention or a salt thereof, a pharmaceutical composition for use in treating and preventing a disease in which TTK protein kinase is involved, for example, cancer and an immune disease in which TTK protein kinase is involved.

The pharmaceutical composition is characterized by containing a compound of the present invention or a salt thereof as an active ingredient. Accordingly, the pharmaceutical composition exerts an excellent effect on a disease developed in association with TTK protein kinase by suppressing the activity of the enzyme. For example, a pharmaceutical composition of the present invention exerts an excellent effect on cancer and an immune disease, particularly cancer and an immune disease developed in association with TTK protein kinase by suppressing the activity of the enzyme. When the pharmaceutical composition is used for treating or preventing cancer, the composition can be used simultaneously with a conventional cancer therapy, for example, radiotherapy, chemotherapy, in particular, application of a DNA degrading agent, which renders tumor cells to be responsive in advance, or can be used even before application of such a therapy.

The content of the compound or a salt thereof in the pharmaceutical composition can be appropriately controlled depending upon the target disease for therapy, and the age and weight of a patient. Any content thereof is acceptable as long as it is a therapeutically effective amount. If the compound is a low molecular weight compound or a polymer compound, the content is, for example, 0.0001 to 1000 mg and preferably 0.001 to 100 mg. If the compound is a polypeptide or a derivative thereof, the content is, for example, 0.0001 to 1000 mg and preferably 0.001 to 100 mg. If the compound is a nucleic acid or a derivative thereof, it is desired that the content is, for example, 0.00001 to 100 mg and preferably 0.0001 to 10 mg.

The pharmaceutical composition may further contain various types of auxiliary agents capable of stably keeping the above compound or a salt thereof. Specific examples include a pharmaceutically acceptable auxiliary agent, excipient, binding agent, stabilizer, buffer, solubilization agent and isotonic agent having a property of suppressing decomposition of an active ingredient until the active ingredient reaches a delivery target site.

A mode of administration of the pharmaceutical composition is appropriately selected depending upon the type of active ingredient; the administration target such as an individual, an organ, a local site and tissue; the age and weight of a target individual to be administered. Examples of the mode of administration include subcutaneous injection, intramuscular injection, intravenous injection and local administration.

Furthermore, the dose of the pharmaceutical composition is appropriately selected depending upon the type of active ingredient; administration target such as an individual, an organ, a local site and tissue; the age and weight of a target individual to be administered. The dose is not particularly limited. If the active ingredient is a low molecular compound or a polymer compound, the amount of active ingredient per dose is, for example, 0.0001 to 1000 mg/kg weight and preferably 0.001 to 100 mg/kg weight. In the case of a polypeptide or a derivative thereof, the amount of active ingredient is, for example, 0.0001 to 1000 mg/kg weight and preferably 0.001 to 100 mg/kg weight. In the case of a nucleic acid or a derivative thereof, the amount of active ingredient is, for example, 0.00001 to 100 mg/kg weight and preferably 0.0001 to 10mg/kg weight. Administration may be performed multiple times, for example, 1 to 3 times per day.

### (Medicament)

A compound of the present invention or a pharmaceutically acceptable salt thereof can be administered by itself, and preferably administered usually as various types of pharmaceutical preparations. Furthermore, these pharmaceutical preparations are used in animals and humans.

As an administration route, the most effective route for treatment is preferably used. For example, an oral route and a parenteral route such as rectal, intraoral, subcutaneous, intramuscular and intravenous routes can be mentioned.

Examples of dosage form include an encapsulated formulation, a tablet, a granule, a powdered medicine, a syrup, an emulsion, a suppository and an injection agent. A liquid preparation adequate for oral administration such as an emulsion and a syrup can be produced by using water, a saccharide such as sucrose, sorbit and fructose, glycol such as polyethylene glycol and propylene glycol, oil such as sesame oil, olive oil and soybean oil, an antiseptic agent such as p-hydroxybenzoic acid ester, and flavor such as strawberry flavor and peppermint. Furthermore, e.g., an encapsulated formulation, a tablet, a powder and a grain can be produced by using an excipient such as lactose, glucose, sucrose and mannite, a disintegrator such as starch and sodium alginate, a lubricant such as magnesium stearate and talc, a binder such as polyvinyl alcohol, hydroxypropylcellulose and gelatin, a surfactant such as fatty acid ester and a plasticizer such as glycerin.

A preparation appropriate for parenteral administration is a sterilized aqueous preparation containing an active compound and preferably being isotonic to blood of a recipient. For example, in the case of an injection, a solution for injection is prepared by using a carrier composed of saline water and a glucose solution or a mixture of saline water and a glucose solution.

A local preparation is prepared by dissolving or suspending an active compound in one or more mediums such as mineral oil, petroleum oil, polyhydric alcohol or another base that is used for a local pharmaceutical preparation.

A preparation for enteral administration is prepared by using a conventional carrier such as cacao butter, hydrogenated fat and hydrogenated aliphatic carboxylic acid and provided as a suppository.

In the present invention, also in a parenteral agent, one or more auxiliary components selected from a glycol, an oil, a flavor, an antiseptic agent (including an anti-oxidizing agent), an excipient, a disintegrator, a lubricant, a binding agent, a surfactant, a plasticizer etc. as exemplified in the case of an oral agent can be added.

Effective dose and administration frequency of a compound of the present invention or a pharmaceutically acceptable salt vary depending upon the dosage form, the age and weight of a patient, symptom or severity of a disease to be treated. Usually, the dose per day is 0.01 to 1000 mg/person and preferably 5 to 500 mg/person. The dose is administered at a frequency of one per day or in portions, multiple times.

A compound of the present invention is preferably as follows. In the case of screening a compound having TTK kinase activity suppressing action by using p38 MAPK peptide, the compound of the present invention is a compound having TTK IC₅₀ (which is suppression activity of a test substance based on a fluorescent value in the absence of a test substance) of 1 µM or less, preferably 0.1 µM or less, and more preferably 0.01 µM or less. Alternatively, in the case of screening (A549assay) a compound inhibiting cancer cell growth, a compound of the present invention is a compound having an IC₅₀ value within the range of 10 nM to 10 µM, preferably less than 10 µM and more preferably less than 1 µM.

As to further information on producing a preparation, Comprehensive Medicinal Chemistry (Corwin Hansch; Chairman of Editorial Board), Pergamon Press 1990, Vol. 5, Chapter 25.2 can be referred to.

The present invention further relates to a system, apparatus and kit for producing a pharmaceutical composition of the present invention. As the constitutional elements of such a system, apparatus and kit, those known in the art can be used. Thus, it is understood that the constitutional elements can be appropriately designed by those skilled in the art.

The present invention further relates to a system, apparatus, and kit using a compound of the present invention and a pharmaceutically acceptable salt thereof or solvates thereof. As the constitutional elements of such a system, apparatus and kit, those known in the art can be used. Thus, it is understood that the constitutional elements can be appropriately designed by those skilled in the art.

A compound of the present invention is a compound having usefulness as a medicament. Examples of usefulness as a medicament include excellent metabolic stability, less induction of drug-metabolizing enzyme, less inhibition of other drug metabolizing enzymes for metabolizing other medicinal agents, high oral absorption of a compound, low clearance and sufficiently long half-life for expressing a medicinal effect.

Cited literatures such as scientific literatures, patents and publications of Japanese Patent Application cited in the specification are incorporated herein by reference as if each were specifically described in their entireties.

### EXAMPLES

The present invention will be more specifically described by way of Examples, below; however, the technical range of the present invention is not limited by these Examples, etc.

The apparatuses, measurement conditions and so forth shown below were employed.

LC/MS analysis was made by use of the system of Waters (ZQ2000 mass detector; 1525 HPLC pump; 2996 photodiode array detector; 2777 autosampler). In the analysis, a reverse-phase C18 column (Waters, X-Bridge C18, 4.6 × 50 mm, 5 uM) was used and water/acetonitrile (0.1% formic acid) was used as an elution solvent. The elution conditions are: a flow rate of 3 mL/min, 10-100% acetonitrile (3 minutes, linear gradient) and 100% acetonitrile (1 minute). LC/MS t_{R} described herein indicates retention time (minutes) of a target compound in LC/MS analysis, peak detection was made by use of UV at 254 nm.

Reverse-phase preparatory liquid chromatography was performed by use of the system of Waters (ZQ 2000 Mass detector; 2525 HPLC pump; 2996 photodiode array detector; 2777 autosampler). A reverse-phase C18 column (Waters, X-Bridge, 19 × 50 mm, 5 uM) was used and water/acetonitrile (0.1% formic acid) was used as an elution solvent. Elution conditions are: a flow rate of 25 mL/min, 10-100% acetonitrile (5 minutes, linear gradient), and 100% acetonitrile (2 minutes).

As silica gel chromatography, the systems of Yamazen (YFLC-Wprep2XY), Moritex (Purif-α2) and Isco Inc. (Combi Flash Companion) were used. As a column, a Hi-Flash column (S to 5L) of Yamazen was used. As an elution solvent, hexane/ethyl acetate or chloroform/methanol was used.

1H NMR spectrum was measured by use of Varian Gemini-300 (300 MHz) and Bruker AV-400 (400 MHz). Chemical shift was described by δ value (ppm) based on TMS (tetramethylsilane) as the internal standard. In analysis results, brevity codes: s; singlet, d: doublet, t: triplet, q: quartet, m: multiplet, br: broad were used.

As a microwave reaction apparatus, initiator 8 and initiator 60 of Biotage were used.

### (Evaluation method)

Screening of a compound having a TTK kinase activity suppressing action using p38 MAPK peptide
A test substance 1.0 µL (solvent: 10% (v/v) DMSO), TTK solution 5 µL (composition: 4 µg/ml TTK, 25 mM Tris-HCl, pH7.5, 5 mM β-glycerophosphate, 2 mM DTT, 0.1 mM Na₃VO₄, 5 mM MgCl₂, 0.1% (w/v)BSA) and a substrate solution 5 µL (composition: 60 µM p38 MAPK peptide, 60 µM ATP, 25 mM Tris-HCl, pH7.5, 5 mM β-glycerophosphate, 2 mM DTT, 0.1 mM Na₃VO₄, 5 mM MgCl₂, 0.1% (w/v)BSA) were mixed in the wells of a 384-well microtiter plate (manufactured by Corning Incorporated) made of polypropylene and loaded in a constant temperature and humidity room. After the reaction mixture was allowed to stand still overnight at a temperature of 25°C and a humidity of 95%, 50 µL of a reaction terminating solution (composition: 25 mM Tris-HCl, pH7.5, 100 mM EDTA, 0.01% (v/v) TritonX-100, 0.1% (w/v)BSA) was added and mixed.

From the resultant mixture, an aliquot of 1.7 µL was taken out and mixed with the reaction terminating solution (60 µL) in the wells of a 384-well microtiter plate (manufactured by Corning Incorporated) made of polypropylene. Thereafter, 40 µL of the mixture was transferred to a 384-well black NeutrAvidin plate (PIERCE), sealed and incubated under room temperature for 30 minutes. After the plate was washed with 100 µL of TTBS (composition: 10 mM Tris, 40 mM Tris-HCl, 150 mM NaCl₂, 0.05% (v/v)Tween 20) three times, 40 µL of a primary antibody solution (Anti-phosphop38 antibody-28B10 (Cell Signaling, #9216)) was added and incubated under room temperature for one hour. Similarly, the plate was washed with TTBS (100 µL) three times, 40 µL of a secondary antibody solution (Eu-N1 labeled Anti-mouse IgG (Perkin Elmer, #AD0124)) was added and incubated under room temperature for 30 minutes. After the plate was washed with TTBS (100 µL) five times, 40 µL of an enhance solution (Perkin Elmer) was added and incubated under room temperature for 5 minutes. The fluorescence value at 615 nm was measured by ARVO (Perkin Elmer). Based on the fluorescence value in the absence of a test substance, the suppression activity of a test substance was measured. A compound exhibiting TTK kinase activity suppressing action was obtained as a candidate compound of a TTK activity suppressing agent.

Screening of cancer cell growth inhibiting compound A cell suspension solution (RERF-LC-AI, A549: 1 × 10⁴/ml, MRC5: 3 × 10⁴/ml and 1 × 1.0⁵/ml) adjusted to an appropriate concentration with D-MEM (hereinafter, referred to as a culture solution and manufactured by Nacalai Tesque Inc.) supplemented with 10% FBS (HyClone) was added to the wells of a 96-well plate (hereinafter, well plate) at a rate of 100 µL/well and incubated in a CO₂ incubator at 37°C for a day. Two µL of a 10 mM compound (100% DMSO solution) exhibiting TTK kinase activity suppressing action in a 96-well assay block was added to a culture solution (998 µl) to prepare a 20 µM solution and serially subjected to a doubling-dilution 10 times. Next, cells were added to each well of the well plate prepared and the above compound dilution solution was added at a rate of 100 µL/well to obtain 200 µL/well. Thereafter, the cells were incubated in the CO₂ incubator at 37°C for further three days. To each well of the well plate, 10 µL of a WST-8 kit (Kishida Chemical Co., Ltd.) solution for counting the number of cells was added and a color reaction was performed in the CO₂ incubator for 1 to 4 hours. After the absorbance at 450 nm (reference wavelength: 620 nm) was measured by a microplate reader, suppressing action of the test substance was calculated based on the absorbance value in the absence of a test substance. In this manner, a compound exhibiting cell growth inhibitory action in cancer cells was selected.

(Example 1)

### Example 1-1

### N-cyclopropyl-4-(8-((tetrahydro-2H-pyran-4-yl)methylamino)imidazo[1,2-a]pyrazin-3-yl)benzamide

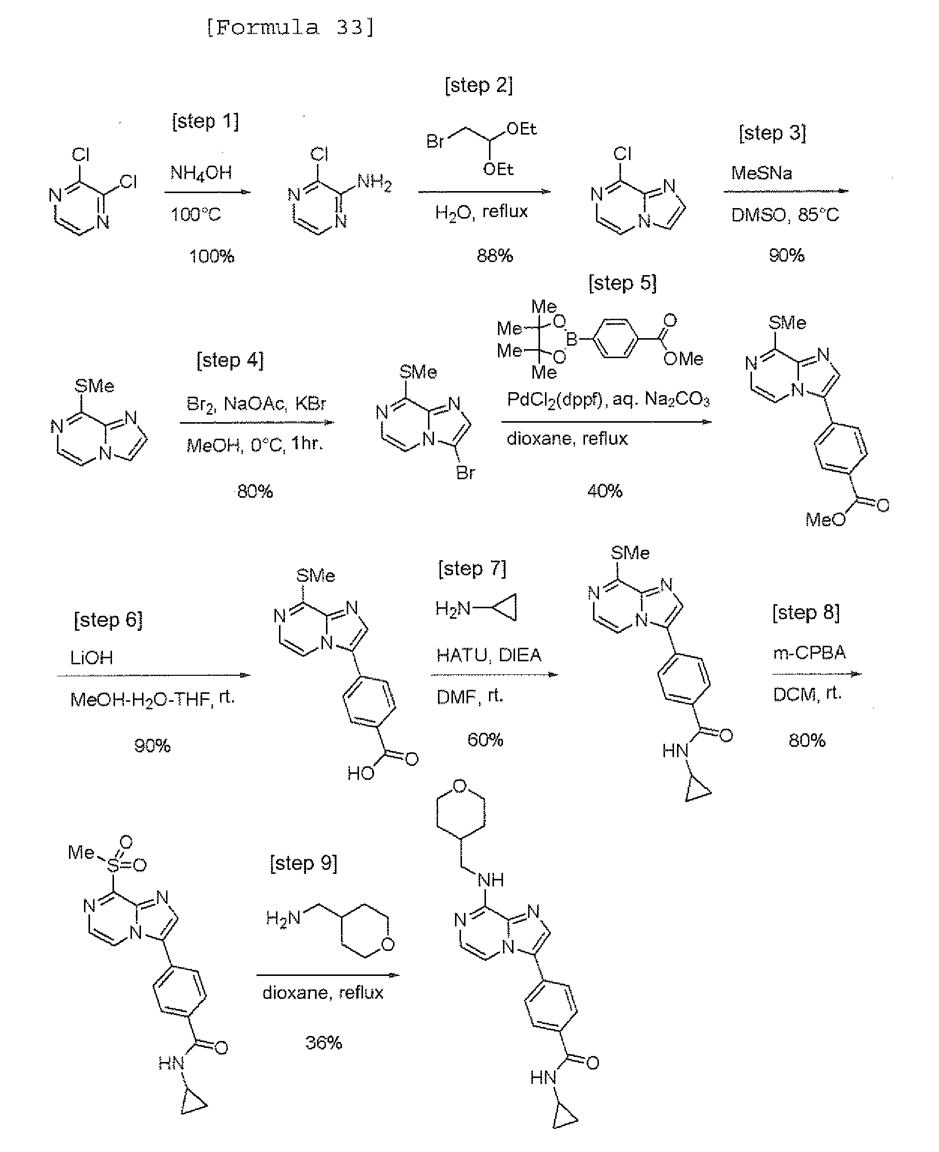

### Step 1: 3-Chloropyrazine-2-amine

2,3-Dichloropyrazine (5.0 g, 33.6 mmol) and 28% ammonia water (20 mL) were placed in a tube, sealed and stirred at 100°C for 18 hours. The reaction solution was diluted with water, the resultant solid substance was obtained by filtration to obtain 3-chloropyrazine-2-amine (3.46 g, 100%) as a white solid substance.
MS (ESI) m/z = 130 (M+H)⁺.

### Step 2: 8-Chloroimidazo[1,2-a]pyrazine

To a 48% aqueous hydrogen bromide solution (0.893 mL) and water (9 mL), 2-bromo-1,1-diethoxyethane (5.98 mL, 38.5 mmol) was added, and stirred for one hour while heating under reflux. To the reaction solution, water and ethyl ether were added to separate phases. The water phase was extracted with ethyl ether and organic phases were combined, dried over magnesium sulfate, filtrated and concentrated under reduced pressure. To the resultant residue and a dimethoxyethane (22 mL) solution of 3-chloropyrazine-2-amine (2.0 g, 15.4), a 48% aqueous hydrogen bromide solution (0.3 mL) was added, stirred for 3 hours while heating under reflux. The resultant solid substance was obtained by filtration and washed with ether to obtain 8-chloroimidazo[1,2-a]pyrazine (2.07 g, 88%) as a black solid substance.
MS (ESI) m/z = 154 (M+H)⁺.

### Step 3: 8-(Methylthio)imidazo[1,2-a]pyrazine

To a dimethyl sulfoxide (5 mL) solution of 8-chloroimidazo[1,2-a]pyrazine (1.15 g, 5.12 mmol), sodium methyl mercaptan (100 mg, 1.55 mmol) was added and stirred at 85°C for one hour. To the reaction solution, water and dichloromethane were added to separate phases. The water phase was extracted with dichloromethane and organic phases were combined, dried over magnesium sulfate, filtrated and concentrated under reduced pressure to obtain 8-(methylthio)imidazo[1,2-a]pyrazine (192 mg, 90%) as a solid substance.
MS (ESI) m/z = 166 (M+H)⁺.

### Step 4: 3-Bromo-8-(methylthio)imidazo[1,2-a]pyrazine

To 8-(methylthio)imidazo[1,2-a]pyrazine (1.15 g, 5.12 mmol), potassium bromide (119 mg, 1.00 mmol) and a methanol (5 mL) solution of sodium acetate (798 mg, 3.00 mmol), bromine (20 mL) was added at 0°C and stirred at the same temperature for one hour. To the reaction solution, water and dichloromethane were added to separate phases. The water phase was extracted with dichloromethane and organic phases were combined, washed with saturated sodium bicarbonate water and saturated saline, and dried over magnesium sulfate. The organic phase was filtrated and then concentrated under reduced pressure. The resultant residue was purified by medium-pressure silica gel chromatography (hexane: ethyl acetate = 2: 1) to obtain the titled compound (197 mg, 80%) as a white solid substance.
1H-NMR (400 MHz, DMSO-d₆) δ 2.61 (s, 3H), 7.85 (s, 1H), 7.91 (d, 1H), 8.17 (d, 1H).
MS (EST) m/z = 247 (M+H)⁺.

### Step 5: Methyl 4-(8-(methylthio)imidazo[1,2-a]pyrazin-3-yl)benzoate

To a dioxane (100 mL) solution of methyl 4-(4,4,5,5-tetramethyl-1,3,2-dioxaborolan-2-yl)benzoate (5.24 g, 20 mmol), 3-bromo-8-(methylthio)imidazo[1,2-a]pyrazine (119 mg, 1.00 mmol) and sodium carbonate (4.24 g, 40 mmol), PdCl₂(dppf) (1.60 g, 2.0 mmol) was added and stirred for 2 hours while heating under reflux. To the reaction solution, water and ethyl acetate were added to separate phases. The water phase was extracted with ethyl acetate and organic phases were combined, washed with saturated sodium bicarbonate water and saturated saline, and dried over magnesium sulfate. The organic phase was filtrated and then concentrated under reduced pressure. The resultant residue was purified by medium-pressure silica gel chromatography (hexane: ethyl acetate = 4: 1) to obtain the titled compound (2.39 g, 40%) as a white solid substance.
MS (ESI) m/z = 300 (M+H)⁺,

### Step 6: 4-(8-(Methylthio)imidazo[1,2-a]pyrazin-3-yl)benzoic acid

To a THF-methanol (1:1, 160 mL) solution of methyl 4-(8-(methylthio)imidazo[1,2-a]pyrazin-3-y1)benzoate (10.0 g, 33.4 mmol), an aqueous solution (80 mL) of LiOH (2.80 g, 66.8 mmol) was added and stirred at room temperature for 2 hours. To the reaction solution, 1N HCl was added to adjust to pH = 4 and thereafter the organic solvent was distilled away. To the resultant residue, water and ethyl acetate were added to dilute, the water phase was extracted with ethyl acetate. Organic phases were combined, washed with saturated sodium bicarbonate water and saturated saline, and dried over magnesium sulfate. The organic phase was filtrated and then concentrated under reduced pressure to obtain the titled compound (8.60 g, 90%) as a white solid substance.
MS (ESI) m/z = 286 (M+H)⁺.

### Step 7: N-Cyclopropyl-4-(8-(methylthio)imidazo[1,2-a]pyrazin-3-yl)benzamide

To a DMF (50 mL) solution of 4-(8-(methylthio)imidazo[1,2-a]pyrazin-3-yl)benzoic acid (8.00 g, 28.1 mmol), cyclopropaneamine (3.20 g, 56.2 mmol) and DIEA (10.9 g, 84.0 mmol), HATU (16.0 g, 42.2 mmol) was added and stirred at room temperature for 17 hours. To the reaction solution, water and ethyl acetate were added to separate phases. The water phase was extracted with ethyl acetate and organic phases were combined, washed with saturated sodium bicarbonate water and saturated saline, and dried over magnesium sulfate. The organic phase was filtrated and then concentrated under reduced pressure. The resultant residue was purified by medium-pressure silica gel chromatography (hexane: ethyl acetate = 2: 1) to obtain the titled compound (5.40 g, 60%) as a white solid substance.
MS (ESI) m/z = 325 (M+H)⁺.

### Step 8: N-Cyclopropyl-4-(8-(methylsulfonyl)imidazo[1,2-a]pyrazin-3-yl)benzamide

To a dichloromethane (150 mL) solution of N-cyclopropyl-4-(8-(methylthio)imidazo[1,2-a]pyrazin-3-yl)benzamide (7.50 g, 23.2 mmol), m-CPBA (12.0 g, 69.6 mmol) was added and stirred at room temperature for 17 hours. To the reaction solution, water was added to separate phases and then the water phase was extracted with ethyl acetate. Organic phases were combined, washed with saturated sodium bicarbonate water and saturated saline, and dried over magnesium sulfate. The organic phase was filtrated and then concentrated under reduced pressure. The resultant residue was purified by medium-pressure silica gel chromatography (hexane: ethyl acetate = 3: 1) to obtain the titled compound (7.00 g, 80%) as a white solid substance.
MS (ESI) m/z = 357 (M+H)⁺.

### Step 9: Titled compound

To a dioxane (1 mL) solution of N-cyclopropyl-4-(8-(methylsulfonyl)imidazo[1,2-a]pyrazin-3-yl)benzamide (150 mg, 0.42 mmol), (tetrahydro-2H-pyran-4-yl)methaneamine (96.7 mg, 0.84 mmol) was added and stirred for 12 hours while heating under reflux. To the reaction solution, water was added to separate phases and then, the water phase was extracted with ethyl acetate. Organic phases were combined, washed with water and saturated saline, and dried over magnesium sulfate. The organic phase was filtrated and then concentrated under reduced pressure. The resultant residue was purified by medium-pressure silica gel chromatography (chloroform: methanol = 20: 1) to obtain the titled compound (59 mg, 0.15 mmol, 36%) as a yellow solid substance.
1H-NMR (400 MHz, DMSO-d₆) δ 0.60 (m, 2H), 0.71 (m, 2H), 1.25 (m, 2H), 1.63 (m, 2H), 1.98 (m, 1H), 2.88 (m, 1H), 3.25 (m, 2H), 3.38 (m, 2H), 3.84 (m, 2H), 7.36 (d, 1H), 7.65 (t, 1H), 7.73 (m, 3H), 7.80 (s, 1H), 7.98 (d, 2H), 8.55 (d, 1H).
MS (ESI) m/z = 392 (M+H)⁺.
LC/MS t_{R} = 0.90 min

Example 1-3

### N-Cyclopropyl-4-(8-((tetrahydro-2H-pyran-4-yl)methoxy)imidazo[1,2-a]pyrazin-3-yl)benzamide

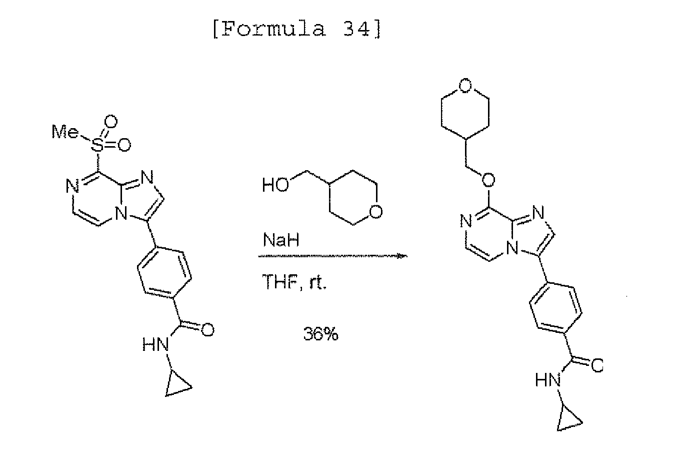

To a THF (0.54 mL) solution of (tetrahydro-2H-pyran-4-yl)methanol (5.87 mg, 0.051 mmol) and sodium hydride (60% wt, 4 mg), N-cyclopropyl-4-(8-(methylsulfonyl)imidazo[1,2-a]pyrazin-3-yl)benzamide (18 mg, 0.051 mmol) was added and stirred at room temperature for 5 minutes. To the reaction solution, water was added to separate phases and then the water phase was extracted with ethyl acetate. Organic phases were combined, washed with a saturated aqueous ammonium chloride solution and saturated saline, and dried over magnesium sulfate. After the organic phase was filtrated and concentrated under reduced pressure, the resultant residue was purified by reverse-phase preparatory liquid chromatography (C18 column; water/acetonitrile/0.1% formic acid; 10-100% acetonitrile gradient) to obtain the titled compound (11.4 mg, 0.029 mmol, 36%).
MS (ESI) m/z = 393 (M+H)⁺.
LC/MS t_{R} = 1.40 min.

Example 1-4

### N-Cyclopropyl-4-(8-isobutoxyimidazo[1,2-a]pyrazin-3-yl)benzamide

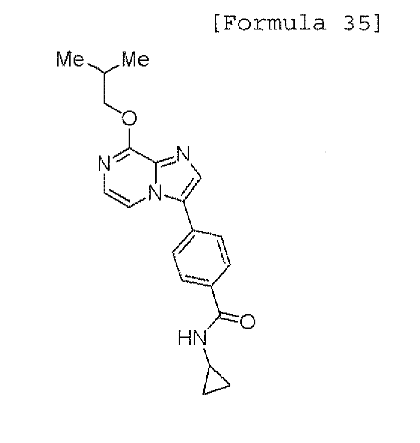

The titled compound was synthesized in accordance with the process of Example 1-3
MS (ESI) m/z = 351 (M+H)⁺.
LC/MS t_{R} = 1.72 min.

[Table 1-1A]

**Table 1-1**

| | | |
|---|---|---|
| 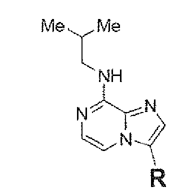 | | |

| Compounds described in Table 1-1 were synthesized in accordance with the processes of Examples mentioned above. | | |
|---|---|---|
| Example No. | R | property data |
| Example 1-6 | 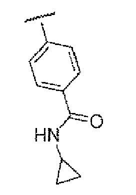 | 1H-NMR (400 MHz, DMSO-d₆) δ 0.60 (m, 2H), 0.71 (m, 2H), 0.84 (d, 6H), 2.03 (m, 1H), 2.88 (m, 1H), 3.31 (m, 2H), 7.34 (d, 1H), 7.60 (t, 1H), 7.75 (m, 4H), 7.98 (d, 2H), 8.55 (d, 1H). MS (ESI) m/z = 350 (M+H)⁺, |
| Example 1-7 | 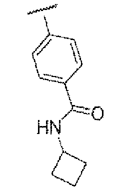 | MS (ESI) m/z = 364 (M+H)⁺. |
| Example 1-8 | 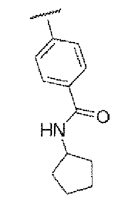 | MS (ESI) m/z = 378 (M+H)⁺. |
| Example 1-9 | 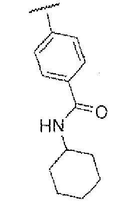 | MS (ESI) m/z = 392 (M+H)⁺. |

**[Table 1-1B]**

| Example No. | R | property data |
|---|---|---|
| Example 1-10 | 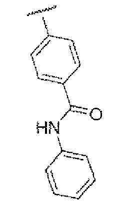 | MS (ESI) m/z = 386 (M+H)⁺. |
| Example 1-11 | 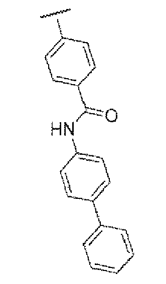 | MS (ESI) m/z = 462 (M+H)⁺. |
| Example 1-12 | 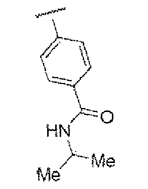 | MS (ESI) m/z = 352 (M+H)⁺. |
| Example 1-13 | 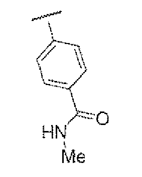 | MS (ESI) m/z = 324 (M+H)⁺, |
| Example 1-14 | 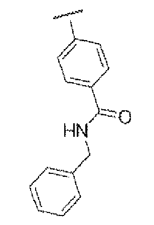 | MS (ESI) m/z = 400 (M+H)⁺, |

**[Table 1-1C]**

| Example No. | R | property data |
|---|---|---|
| Example 1-15 | 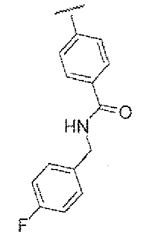 | MS (ESI) m/z =418 (M+H)⁺. |
| Example 1-16 | 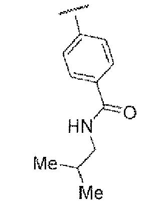 | MS (ESI) m/z = 366 (M+H)⁺. |
| Example 1-17 | 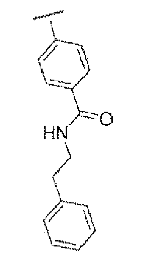 | MS (ESI) m/z = 414 (M+H)⁺. |
| Example 1-18 | 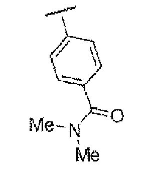 | MS (ESI) m/z = 338 (M+H)⁺. |
| Example 1-19 | 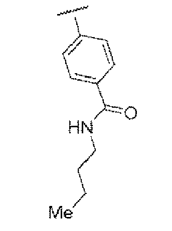 | MS (ESI) m/z = 366 (M+H)⁺. |

**[Table 1-1D]**

| Example No. | R | property data |
|---|---|---|
| Example 1-20 | 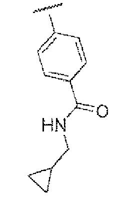 | MS (ESI) m/z = 364 (M+H)⁺. |
| Example 1-25 | 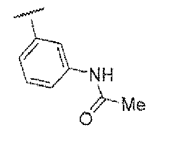 | MS (ESI) m/z = 324 (M+H)⁺. |
| Example 1-31 | 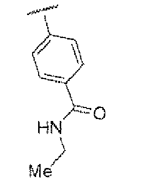 | MS (ESI) m/z = 338 (M+H)⁺, |
| Example 1-32 | 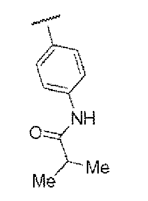 | MS (ESI) m/z = 352 (M+H)⁺. |
| Example 1-33 | 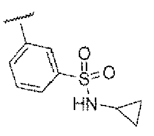 | MS (ESI) m/z = 386 (M+H)⁺. |
| Example 1-34 | 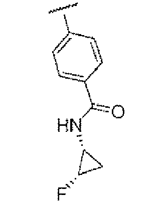 | MS (ESI) m/z = 368 (M+H)⁺. LC/MS t_{R} = 1.02 min. |

**[Table 1-1E]**

| Examples No. | R | property data |
|---|---|---|
| Example 1-35 | 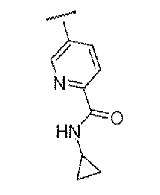 | MS (ESI) m/z = 351 (M+H)⁺. LC/MS t_{R}= 1.10 min. |

[Table 1-2A]

**Table 1-2**

| | | |
|---|---|---|
| 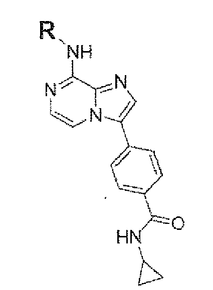 | | |

| Compounds described in Table 1-2 were synthesized in accordance with the processes of Examples, mentioned above. | | |
|---|---|---|
| Example No. | R | property data |
| Example 1-36 | 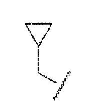 | 1H-NMR (400 MHz, DMSO-d₆) δ 0.26 (m, 2H), 0.43 (m, 2H), 0.61 (m, 2H), 0.72 (m, 2H), 1.17 (m, 1H), 2.89 (m, 1H), 3.36 (t, 2H), 7.35 (d, 1H), 7.59 (t, 1H), 7.77 (m, 4H), 7.98 (d, 2H), 8.55 (d, 1H), MS (ESI) m/z = 348 (M+H)⁺. |
| Example 1-37 | 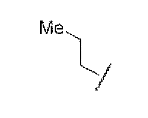 | 1H-NMR (400 MHz, DMSO-d₆) δ 0.60 (m, 2H), 0.73 (m, 2H), 0.95 (m, 3H), 1.68 (m, 2H), 2.89 (m, 1H), 3.49 (d, 2H), 7.33-8.04 (m, 7H), 8.61 (d, 1H), 9.65 (d, 1H). MS (ESI) m/z = 336 (M+H)⁺. |
| Example 1-38 | 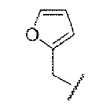 | 1H-NMR (400 MHz, DMSO-d₆) δ 0.60 (m, 2H), 0.72 (m, 2H), 2.88 (m, 1H), 4.68 (m, 2H), 6.25 (d, 1H), 6.37 (m, 1H), 7.38 (d, 1H), 7.55 (d, 1H), 7.75 (d, 2H), 7.83 (d, 2H), 7.99 (m, 3H), 8.56 (d, 1H). MS (ESI) m/z = 374 (M+H)⁺. |

**[Table 1-2B]**

| Example No. | R | property data |
|---|---|---|
| Example 1-39 | 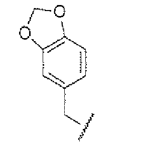 | 1H-NMR (400 MHz, DMSO-d₆) δ 0.60 (m, 2H), 0.71 (m, 2H), 2.88 (m, 1H), 5.96 (s, 2H), 6.84 (m, 2H), 6.95 (s, 1H), 7.35 (d, 1H), 7.78 (m, 4H), 7.97 (d, 2H), 8.11 (t, 1H), 8.55 (d, 1H). |
| | | MS (ESI) m/z = 428 (M+H)⁺. |
| Example 1-40 | 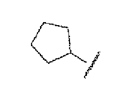 | 1H-NMR (400 MHz, DMSO-d₆) δ 0.60 (m, 2H), 0.72 (m, 2H), 1.56 (m, 2H), 1.65 (m, 2H), 1.72 (m, 2H), 1.97 (m, 2H), 2.88 (m, 1H), 4.46 (m, 1H), 7.33 (d, 1H), 7.37 (d, 1H), 7.77 (m, 4H), 7.99 (d, 2H), 8.56 (d, 1H). |
| | | MS (ESI) m/z = 362 (M+H)⁺, |
| Example 1-41 | 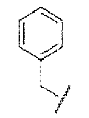 | 1H-NMR (400 MHz, DMSO-d₆) δ 0.59 (m, 2H), 0.71 (m, 2H), 2.87 (m, 1H), 4.76 (d, 2H), 7.24-8.01 (m, 12H), 8.57 (t, 1H), 9.21 (s, 1H). |
| | | MS (ESI) m/z = 384 (M+H)⁺. |
| Example 1-42 | 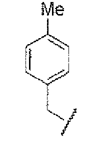 | 1H-NMR (400 MHz, DMSO-d₆) δ 0.60 (m, 2H), 0.71 (m, 2H), 2.25 (s, 3H), 2.88 (m, 1H), 4.65 (d, 2H), 7.09-8.13 (m, 13H), 8.56 (d, 1H), |
| | | MS (ESI) m/z = 398 (M-H)⁺. |
| Example 1-43 | 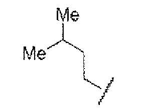 | 1H-NMR (400 MHz, DMSO-d₆) δ 0.61 (m, 2H), 0.73 (m, 2H), 0.93 (d, 6H), 1.58 (m, 2H), 1.68 (m, 1H), 2.89 (m, 1H), 3.54 (m, 2H), 7.33-8.03 (m, 7H), 8.60 (d, 1H), 9.46 (s, 1H). |
| | | MS (ESI) m/z = 364 (M+H)⁺. |
| Example 1-44 | 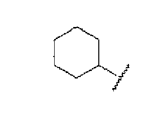 | 1H-NMR (400 MHz, DMSO-d₆) δ 0.61 (m, 2H), 0.72 (m, 2H), 1.37 (m, 4H), 1.69 9m, 4H), 1.92 (d, 2H), 2.88 (m, 1H), 4.03 (d, 1H), 7.18 9d, 1H), 7.35 (d, 1H), 7.76 (m, 4H), 7.98 (d, 2H), 8.55 (d, 1H). |
| | | MS (ESI) m/z = 376 (M+H)⁺. |
| Example 1-45 | 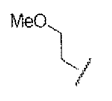 | 1H-NMR (400 MHz, DMSO-d₆) δ 0.60 (m, 2H), 0.71 (m, 2H), 2.88(m, 1H), 3.29 (s, 3H), 3.56(t, 2H), 3.83 (m, 2H), 7.37 (d, 1H), 7.43 (t, 1H), 7.78 (m, 4H), 7.99 (d, 2H), 8.56 (d, 1H). |
| | | MS (ESI) m/z = 352 (M+H)⁺. |

**[Table 1-2C]**

| Example No. | R | property data |
|---|---|---|
| Example 1-46 | 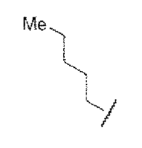 | 1H-NMR (400 MHz, DMSO-d₆) δ 0.68 (m, 2H), 0.71 (m, 2H), 0.87 (m, 4H), 1.30 (m, 4H), 1.63 (t, 2H), 2.88 (m, 1H), 3.46 (m, 2H), 7.35 (d, 1H), 7.55 (t, 1H), 7.75 (m, 4H), 7.97 (d, 2H), 8.04 (d, 1H). |
| | | MS (ESI) m/z = 364 (M+H)⁺. |
| Example 1-47 | 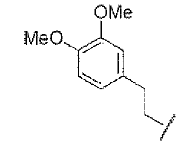 | 1H-NMR (400 MHz, DMSO-d₆) δ 0.60 (m, 2H), 0.72 (m, 2H), 2.89 (t, 3H), 3.73 (m, 8H), 6.78 (d, 1H), 6.86 (m, 2H), 7.39 (d, 1H), 7.54 (t, 1H), 7.77 (m, 4H), 7.98 (d, 2H), 8.55 (d, 1H). |
| | | MS (ESI) m/z = 458 (M+H)⁺, |
| Example 1-48 | 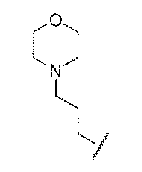 | 1H-NMR (400 MHz, DMSO-d₆) δ 0.60 (m, 2H), 0.71 (m, 2H), 1.79 (m, 2H), 2.40 (m, 6H), 2.88 (m, 1H), 3.53(m, 2H), 3.63 (m, 4H), 7.36 (m, 1H), 7.77 (m, 5H), 7.98 (d, 2H), 8.55 (d, 1H). |
| | | MS (ESI) m/z = 421 (M+H)⁺, |
| Example 1-49 | 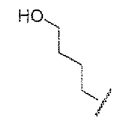 | 1H-NMR (400 MHz, DMSO-d₆) δ 0.61 (m, 2H), 0.72 (m, 2H), 1.50 (m, 2H), 1.67 (m, 2H), 2.89 (m, 1H), 3.51 (m, 5H), 7.35 (m, 1H), 7.74-8.00 (m, 7H), 8.57 (d, 1H). |
| | | MS (ESI) m/z = 366 (M+H)⁺. |
| Example 1-50 | 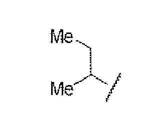 | 1H-NMR (400 MHz, DMSO-d₆) δ 0.54 (m, 2H), 0.65 (m, 2H), 0.83 (m, 3H), 1.14 (m, 3H), 1.50 (m, 1H) 1.61 (m, 1H) 2.82 (m, 1H), 4.14 (m, 1H), 7.08 (d, 1H), 7.28 (d, 1H), 7.67-7.93 (m, 6H), 8.49 (d, 1H). |
| | | MS (ESI) m/z = 350 (M+H)⁺. |
| Example 1-51 | 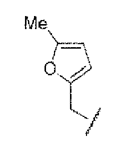 | 1H-NMR (400 MHz, DMSO-d₆) δ 0.60 (m, 2H), 0.72 (m, 2H), 2.22 (m, 3H), 2.88 (d, 1H), 4.62 (d, 2H), 5.96(m, 1H), 6.11 (d, 1H), 7.38 (d, 1H), 7.75 (d, 2H), 7.82 (d, 2H), 7.89 (m, 1H), 7.98 (d, 2H), 8.55 (d, 1H). |
| | | MS (ESI) m/z = 388 (M+H)⁺. |

**[Table 1-2D]**

| Example No. | R | property data |
|---|---|---|
| Example 1-52 | 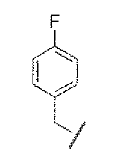 | 1H-NMR (400 MHz, DMSO-d₆) δ 0.60 (m, 2H), 0.71 (m, 2H), 2.88(m, 1H), 4.67 (d, 2H), 7.12 (t, 2H), 7.33 (d, 1H), 7.40 (m, 2H), 7.75 (d, 2H), 7.79 (d, 1H), 7.83 (s, 1H), 7.98 (d, 2H), 8.22 (t, 1H), 8.55 (d, 1H). |
| | | MS (ESI) m/z = 402 (M+H)⁺. |
| Example 1-53 | 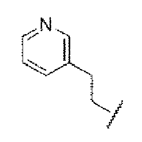 | 1H-NMR (400 MHz, DMSO-d₆) δ 0.60 (m, 2H), 0.71 (m, 2H), 2.89 (s, 1H), 2.99 (t, 2H), 3.75 (m, 2H), 7.32 (t, 1H), 7.37 (d, 1H), 7.74 (m, 5H), 7.98 (d, 2H), 8.41 (d, 1H), 8.47 (s, 1H), 8.56 (d, 1H). |
| | | MS (ESI) m/z = 399 (M+H)⁺. |
| Example 1-54 | 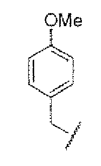 | 1H-NMR (400 MHz, DMSO-d₆) δ 0.61 (m, 2H), 0.71 (m, 2H), 2.88 (m, 1H), 3.71 (s, 3H), 4.61 (d, 2H), 6.86 (d, 2H), 7.30 (d, 2H), 7.34 (d, 1H), 7.76 (m, 3H), 7.82 (s, 1H), 7.98 (d, 2H), 8.10 (t, 1H), 8.55 (d, 1H). |
| | | MS (ESI) m/z = 414 (M+H)⁺. |
| Example 1-55 | 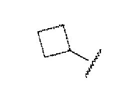 | 1H-NMR (400 MHz, DMSO-d₆) δ 0.61 (m, 2H), 0.71 (m, 2H), 1.68 (m, 2H), 2.17 (m, 2H), 2.27 (m, 2H), 2.88 (m, 1H), 4.65 (m, 1H), 7.35 (d, 1H), 7.77 (m, 5H0. 7.98 (d, 2H), 8.56 (d, 1H). |
| | | MS (ESI) m/z = 348 (M+H)⁺. |
| Example 1-56 | 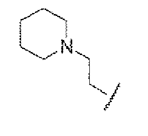 | 1H-NMR (400 MHz, DMSO-d₆) δ 0.61 (m, 2H), 0.73 (m, 2H), 1.76 (m, 6H), 2.89 (m, 1H), 3.12 (m, 2H), 3.38 (m, 2H), 3.60 (m, 2H), 3.89 (m, 2H), 7.41 (m, 1H), 7.75-8.21 (m, 7H), 8.58 (d, 1H). |
| | | MS (ESI) m/z = 405 (M+H)⁺. |
| Example 1-57 | 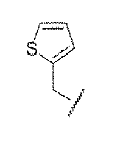 | 1H-NMR (400 MHz, DMSO-d₊) δ 0.60 (m, 2H), 0.72 (m, 2H), 2.88 (m, 1H), 4.85 (d, 2H), 6.94 (m, 1H), 7.05 (t, 1H), 7.33 (m, 1H), 7.40 (d, 1H), 7.75 (d, 2H), 7.83 (t, 2H), 7.98 (d, 2H), 8.21 (t, 1H), 8.55 (d, 1H). |
| | | MS (ESI) m/z = 390 (M+H)⁺. |

**[Table 1-2E]**

| Example No. | R | property data |
|---|---|---|
| Example 1-58 | 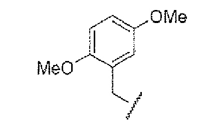 | 1H-NMR (400 MHz, DMSO-d₆) δ 0.60 (m, 2H), 0.71 (m, 2H), 2.87 (m, 1H), 3.64 (s, 3H), 3.78 (s, 3H), 4.68 (d, 2H), 6.78-8.01 (m, 11H), 8.58 (d, 1H). |
| | | MS (ESI) m/z = 444 (M+H)⁺. |
| Example 1-59 | 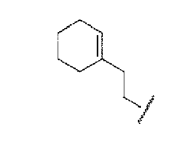 | 1H-NMR (400 MHz, DMSO-d₆) δ 0.60 (m, 2H), 0.71 (m, 2H), 1.49 (m, 2H), 1.58 (m, 2H), 1.94 (m, 4H), 2.26 (t, 2H), 2.88 (m, 1H), 3.55 (m, 2H), 5.44 (s, 1H), 7.37 (m, 2H), 7.76 (m, 4H), 7.98 (d, 2H), 8.55 (d, 1H). |
| | | MS (ESI) m/z = 402 (M+H)⁺. |
| Example 1-60 | 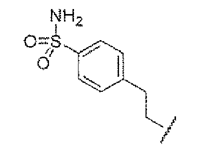 | 1H-NMR (400 MHz, DMSO-d₆) δ 0.61 (m, 2H), 0.72 (m, 2H), 2.88 (m, 1H), 3.05(m, 2H), 3.75 (m, 2H), 7.29 (s, 2H), 7.39 (d, 1H), 7.45 (d, 2H), 7.66 (t, 1H), 7.76 (m, 4H), 7.79 (d, 2H), 7.98 (d, 2H), 8.56 (d, 1H). |
| | | MS (ESI) m/z = 477 (M+H)⁺. |
| Example 1-61 | 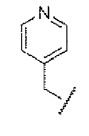 | 1H-NMR (400 MHz, DMSO-d₆) δ 0.61 (m, 2H), 0.72 (m, 2H), 2.90 (m, 1H), 4.98 (m, 2H), 7.31 (d, 1H), 7.77-8.85 (m, 12H), |
| | | MS (ESI) m/z = 385 (M+H)⁺. |
| Example 1-62 | 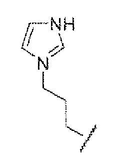 | 1H-NMR (400 MHz, DMSO-d₆) δ 0.61 (m, 2H), 0.71 (m, 2H), 2.07 (m, 2H), 2.89 (m, 1H), 3.47 (m, 2H), 4.06 (t, 2H), 6.89 (s, 1H), 7.23 (s, 1H), 7.36 (d, 1H), 7.68 (s, 1H), 7.78 (m, 5H), 7.99 (d, 2H), 8.55 (d, 1H). |
| | | MS (ESI) m/z = 402 (M+H)⁺. |
| Example 1-63 | 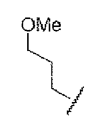 | 1H-NMR (400 MHz, DMSO-d₆) δ 0.61 (m, 2H), 0.72 (m, 2H), 1.92 (m, 2H), 2.90 (m, 1H), 3.05 (s, 2H), 3.44 (m, 2H), 3.59 (m, 2H),7.33 (d, 1H), 7.75-8.60 (m, 7H), 9.45 (s, 1H). |
| | | MS (ESI) m/z = 366 (M+H)'. |
| Example 1-64 | 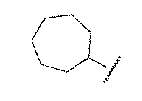 | 1H-NMR (400 MHz, DMSO-d₆) δ 0.60 (m, 2H), 0.73 (m, 2H), 1.50-2.01 (m, 12H), 2.85 (m, 1H), 4.12 (m, 2H), 7.33 (d, 1H), 7.75-8.03 (m, 6H), 8.61 (d, 1H) 9.12 (s, 1H). |
| | | MS (ESI) m/z = 390 (M+H)⁺. |

**[Table 1-2F]**

| Example No. | R | property data |
|---|---|---|
| Example 1-65 | 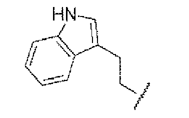 | 1H-MMR (400 MHz, DMSO-d₆) δ 0.60 (m, 2H), 0.74 (m, 2H), 2.89 (m, 1H), 3.15 (m, 2H), 3.85 (s, 1H), 6.98-8.61 (m, 13H), 9.65 (s, 1H) 10.9 (s, 1H). |
| | | MS (ESI) m/z = 437 (M+H)⁴. |
| Example 1-66 | 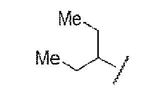 | 1H-NMR (400 MHz, DMSO-d₆) δ 0.60 (m, 2H), 0.73 (m, 2H), 0.88 (t, 6H), 1.61 (t, 4H), 2.89 (m, 1H), 4.12 (m, 1H), 7.10 (d, 1H), 7.33 (d, 1H), 7.73-8.00 (m, 6H), 8.55 (d, 1H). |
| | | MS (ESI) m/z = 364 (M+H)⁺. |
| Example 1-67 | 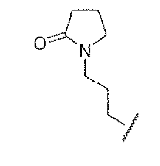 | 1H-NMR (400 MHz, DMSO-d₆) δ 0.61 (m, 2H), 0.72 (m, 2H), 1.80 (m, 2H), 1.92 (m, 2H), 2.22 (m, 2H), 2.89 (m, 1H), 3.26 (t, 2H), 3.34 (m, 2H), 3.45 (m, 2H), 7.36 (d, 1H), 7.58 (t, 1H), 7.77 (m, 4H), 7.98 (d, 2H), 8.55 (d, 1N). |
| | | MS (ESI) m/z = 419 (M+H)⁺. |
| Example 1-68 | 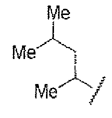 | 1H-NMR (400 MHz, DMSO-d₆) δ 0.60 (m, 2H), 0.72 (m, 2H), 0.892 (m, 6H), 1.18 (d, 3H), 1.31 (m, 1H), 1.68 (m, 2H), 2.89 (m, 2H), 4.45 (m, 1H), 7.20-8.11 (m, 8H), 8.56 (m, 1H). |
| | | MS (ESI) m/z = 378 (M+H)⁺. |
| Example 1-69 | 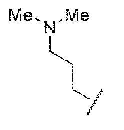 | 1H-NMR (400 MHz, DMSO-d₆) δ 0.60 (m, 2H), 0.71 (m, 2H), 1.79 (m, 2H), 2.23 (m, 6H), 2.40 (t, 2H), 2.68 (m, 1H), 3.51 (m, 2H), 7.35 (d, 1H), 7.76(m, 5H), 7.98 (d, 2H), 8.55 (d, 1H). |
| | | MS (ESI) m/z = 379 (M+H)⁺. |
| Example 1-70 | 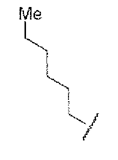 | 1H-NMR (400 MHz, DMSO-d₆) δ 0.60 (m, 2H), 0.71 (m, 2H), 0.87 (m, 3H), 1.30 (m, 6H), 1.61 (m, 2H), 2.88 (m, 1H), 3.46 (m, 2H), 7.35 (d, 1H), 7.56(t, 1H), 7.78 (m, 4H), 7.98 (d, 2H), 8.55 (d, 1H), |
| | | MS (ESI) m/z = 378 (M+H)⁺. |
| Example 1-71 | 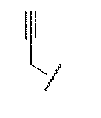 | 1H-NMR (400 MHz, DMSO-d₆) δ 0.61 (m, 2H), 0.71 (m, 2H), 2.88 (m, 1H), 3.02 (t, 1H), 4.23 (m, 2H), 7.42 (d, 1H), 7.76 (d, 2H), 7.86 (t, 2H), 7.98 (m, 3H), 8.56 (d, 1H). |
| | | MS (ESI) m/z = 332 (M+H)⁺. |

**[Table 1-2G]**

| Example No. | R | property data |
|---|---|---|
| Example 1-72 | 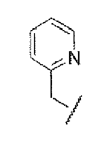 | 1H-NMR (400 MHz, DMSO-d₆) δ 0.69 (m, 2H), 0.85 (m, 2H), 2.90 (m, 1H), 4.90 (d, 2H), 7.35 (d, 2H), 7.49 (d, 1H), 7.80 (m, 5H), 8.00 (t, 2H), 8.55 (d, 1H). |
| | | MS (ESI) m/z = 385 (M+H)⁺. |
| Example 1-73 | 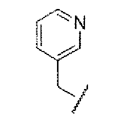 | 1 H-NMR (400 MHz, DMSO-d₆) δ 0.60(m, 2H), 0.71 (m, 2H), 2.88(m, 1H), 4.33 (d, 1H), 4.71 (d, 2H), 7.33 (m, 2H), 7.78 (m, 5H), 7.98 (d, 2H), 8.29 (t, 1H), 8.57(t, 2H). |
| | | MS (ESI) m/z = 385 (M+H)⁺. |
| Example 1-74 | 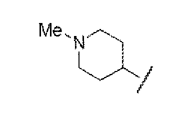 | 1H-NMR (400 MHz, DMSO-d₆) δ 0.61 (m, 2H), 0.71 (m, 2H), 1.74 (m, 2H), 1.88 (t, 2H), 2.06 (t, 2H), 2.22 (s, 3H), 2.86 (m, 3H), 4.03 (m, 1H), 7.32 (d, 1H), 7.35 (d, 1H), 7.77 (m, 4H), 7.39 (d, 2H), 8.56 (d, 1H). |
| | | MS (ESI) m/z = 391 (M+H)⁺. |
| Example 1-75 | 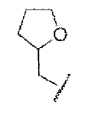 | 1 H-NMR (400 MHz, DMSO-d₆) δ 0.69 (m, 2H), 0.86 (m, 2H), 0.87 (s, 2H), 1.31 (m, 1H), 2.00 (m, 2H), 2.17 (m, 1H), 2.90 (m, 1H), 3.82 (m, 3H), 3.93 (m, 1H), 4.25 (m, 1H), 7.26 (d, 1H), 7.78 (d, 2H), 7.94 (t, 2H), 8.04 (d, 2H). |
| | | MS (ESI) m/z = 378 (M+H)⁺. |
| Example 1-76 | 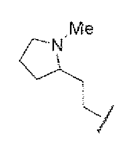 | 1H-NMR (400 MHz, DMSO-d₆) δ 0.61 (m, 2H), 0.72 (m, 2H), 1.57 (m, 1H), 1.72 (m, 3H), 2.08 (m, 2H), 2.42 (s, 3H), 2.88 (m, 1H), 3.39 (m, 3H), 3.55 (m, 2H), 7.37 (d, 1H), 7.77 (m, 5H), 7.99 (d, 2H), 8.58 (d, 1H). |
| | | MS (ESI) m/z = 405 (M+H)⁺. |
| Example 1-77 | 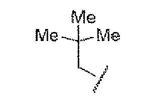 | 1H-NMR (400 MHz, DMSO-d₅) δ 0.60 (m, 2H), 0.71 (m, 2H), 0.95 (s, 9H), 2.89 (m, 1H), 3.40 (d, 2H), 7.24 (t, 1H), 7.35 (d, 1H), 7.76 (t, 3H), 7.81 (s, 1H), 7.99 (d, 2H), 8.56 (d, 1H). |
| | | MS (ESI) m/z = 364 (M+H)⁺. |
| Example 1-78 | 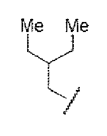 | 1H-NMR (400 MHz, DMSO-d₆) δ 0.60 (m, 2H), 0.71 (m, 2H), 0.87 (m, 6N),1.34 (m, 4H), 1.71 (m, 1H), 2.88(m, 1H), 3.41 (t, 2H), 7.35 (d, 1H), 7.45 (t, 1H), 7.75 (t, 4H), 7.98 (d, 2H), 8.55 (d, 1H). |
| | | MS (ESI) m/z = 378 (M+H)⁺. |

**[Table 1-2H]**

| Example No. | R | property data |
|---|---|---|
| Example 1-79 | 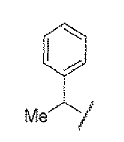 | 1H-NMR (400 MHz, DMSO-d₆) δ 0.60 (m, 2H), 0.71 (m, 2H), 1.57 (d, 3H), 2.88 (m, 1H), 5.42 (m, 1H), 7.20 (m, 1H), 7.29 (m, 3H), 7.45 (m, 2H), 7.76 (m, 3H), 7.84 (s, 1H), 7.91 (d, 1H), 7.97 (d, 2H), 8.55 (d, 1H). |
| | | MS (ESI) m/z = 398 (M+H)⁺. |
| Example 1-80 | 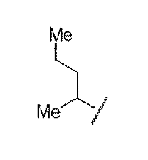 | 1H-NMR (400 MHz, DMSO-d₆) δ 0.61 (m, 2H), 0.72 (m, 2H), 0.87 (m, 3H), 1.22 (m, 3H), 1.34 (m, 2H), 1.51 (m, 1H), 1.67 (m,1H), 2.89 (m, 1H), 4.32 (m, 1H), 7.18 (d, 1H), 7.35 (d, 1H), 7.75 (m, 3H), 7.79 (s, 1H), 7.98 (d, 2H), 8.56 (d, 1H). |
| | | MS (ESI) m/z = 364 (M+H)⁺. |
| Example 1-81 | 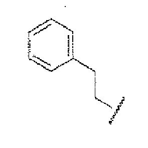 | 1H-NMR (400 MHz, DMSO-d₆) δ 0.60 (m, 2H), 0.71 (m, 2H), 2.89 (m, 1H), 2.96 (m, 2H), 3.72 (m, 2H), 7.20 (m, 1H), 7.28 (m, 4H), 7.39 (m, 1H), 7.59 (t, 1H), 7.78 (m, 4H), 7.98 (d, 2H), 8.55 (d, 1H). |
| | | MS (ESI) m/z = 398 (M+H)⁺. |
| Example 1-82 | 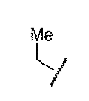 | 1H-NMR (400 MHz, DMSO-d₆) δ 0.60 (m, 2H), 0.72 (m, 2H), 1.21 (m, 3H), 2.89 (m, 1H), 3.51 (m, 2H), 7.36 (d, 1H), 7.76 (m, 3H), 7.80 (s, 1H), 7.98 (d, 2H), 8.55 (d, 1H). |
| | | MS (ESI) m/z = 322 (M+H)⁺. |
| Example 1-83 | 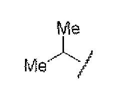 | 1H-NMR (400 MHz, DMSO-d₆) δ 0.60 (m, 2H), 0.71 (m, 2H), 1.17 (m, 6H), 4.35(m, 1H), 7.21 (d, 1H),7.36 (d, 1H), 7.59 (t, 4H), 7.98 (d, 2H), 8.55 (d, 1H). |
| | | MS (ESI) m/z = 336 (M+H)⁺. |
| Example 1-84 | 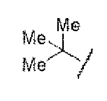 | 1H-NMR (400 MHz, DMSO-d₆) δ 0.68 (m, 2H), 0.851 (m, 2H), 1.59 (s, 9H), 2.90 (m, 1H), 7.41 (d, 1H), 7.67 (s, 1H), 7.73 (m, 3H), 7.98 (d, 2H). |
| | | MS (ESI) m/z = 350 (M+H)⁺. |
| Example 1-85 | 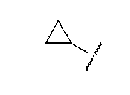 | 1H-NMR (400 MHz, DMSO-d₆) δ 0.71 (m, 8H), 2.90 (m, 2H), 7.40 (d, 1H), 7.78 (m, 5H), 7.98 (d, 2H), 8.55 (d, 1H). |
| | | MS (ESI) m/z = 334 (M+H)⁺. |

**[Table 1-2I]**

| Example No. | R | property data |
|---|---|---|
| Example 1-86 | 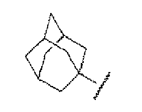 | 1H-NMR (400 MHz, DMSO-d₆) δ 0.66 (m, 2H), 0.91 (m, 2H), 1.73 (m, 8H), 2.20 (s, 7H), 2.93 (m, 1H), 7.36 (s 1H), 7.56 (m, 4H), 7.87 (d, 2H). |
| | | MS (ESI) m/z = 428 (M+H)⁺. |
| Example 1-87 | 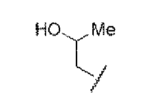 | 1H-NMR (400 MHz, DMSO-d₆) δ 0.60 (m, 2H), 0.71 (m, 2H), 1.11 (d, 3H), 2.88 (m, 1H), 3.38 (m, 1H), 3.50 (m, 1H), 3.93 (m, 1H), 4.91 (d, 1H), 7.29 (t, 1H), 7.35 (d, 1H), 7.78 (m, 4H), 7.99 (d, 2H), 8.55 (d, 1H). |
| | | MS (ESI) m/z = 352 (M+H)⁺. |
| Example 1-88 | 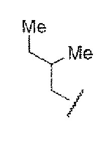 | 1H-NMR (400 MHz, DMSO-d₆) δ 0.60 (m, 2H), 0.72 (m, 2H), 0.87 (m, 6H), 1.14 (m, 1H), 1.45 (m, 1H), 1.83 (m, 1H), 2.89 (m, 1H), 3.30 (m, 1H), 3.40 (m, 1H), 7.35 (d, 1H), 7.54 (t, 1H), 7.77 (m, 4H), 7.99 (d, 2H), 8.55 (d, 1H). |
| | | MS (ESI) m/z = 364 (M+H)⁺. |
| Example 1-89 | 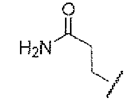 | 1H-NMR (400 MHz, DMSO-d₆) δ 0.68 (m, 2H), 0.83 (m, 2H), 2.65 (t, 2H), 2.89 (m, 1H), 3.84 (t, 2H), 7.36 (d, 1H), 7.72 (m, 4H), 7.97 (d, 2H). |
| | | MS (ESI) m/z = 365 (M+H)⁺. |
| Example 1-90 | 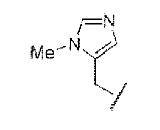 | 1H-NMR (400 MHz, DMSO-d₆) δ 0.59 (m, 2H), 0.70 (m, 2H), 2.89 (m, 1H), 3.66 (s, 2H), 4.64 (d, 2H), 6.83 (d, 1H), 7.38-7.99 (m, 9H), 8.58 (d, 1H). |
| | | MS (ESI) m/z = 388 (M+H)⁺. |
| Example 1-91 | 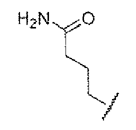 | 1 H-NMR (400 MHz, DMSO-d₆) δ 0.59 (m, 2H), 0.70 (m, 2H), 2.89 (m, 1H), 1.83 (m, 2H), 2.12 (m, 2H), 2.89 (m, 1H), 3.47 (m, 1H), 6.76 (s, 1H), 7.34 (d, 2H), 7.63-7.98 (m, 7H), 8.56 (d, 1H). |
| | | MS (ESI) m/z = 379 (M+H)⁺. |
| Example 1-92 | 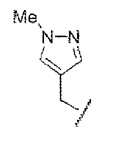 | 1H-NMR (400 MHz, DMSO-d₆) δ 0.60 (m, 2H), 0.72 (m, 2H), 2.89 (m, 1H), 3.76 (s, 3H), 4.49 (d, 2H), 7.38-8.00 (m, 10H), 8.56 (d, 1H). |
| | | MS (ESI) m/z = 388 (M+H)⁺. |
| Example 1-93 | 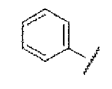 | MS (ESI) m/z = 370 (M+H)⁺. |
| | | LC/MS t_{R} 1.72 min. |

**[Table 1-2J]**

| Example No. | R | property data |
|---|---|---|
| Example 1-94 | 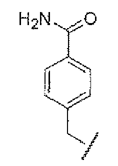 | MS (ESI) m/z = 427 (M+H)⁺. |
| | | LC/MS t_{R} = 0.99 min. |
| Example 1-95 | 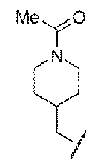 | MS (ESI) m/z = 433 (M+H)⁺. |
| | | LC/MS t_{R} = 0.88 min. |
| Example 1-96 | 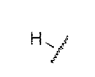 | MS (ESI) m/z = 294 (M+H)⁺. |
| | | LC/MS t_{R} = 0.86 min. |

Example 1-97

### 4-(6-Bromo-8-(isobutylamino)imidazo[1,2-a]pyrazin-3-yl)-N-cyclopropylbenzamide

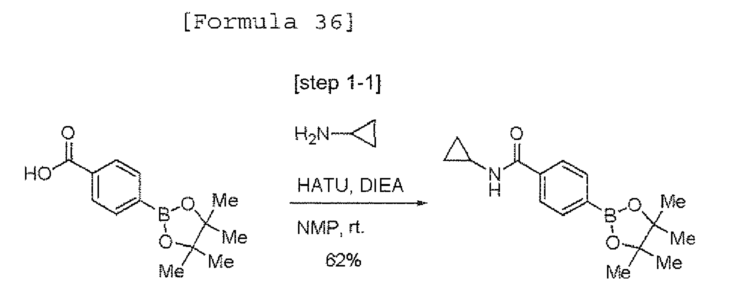

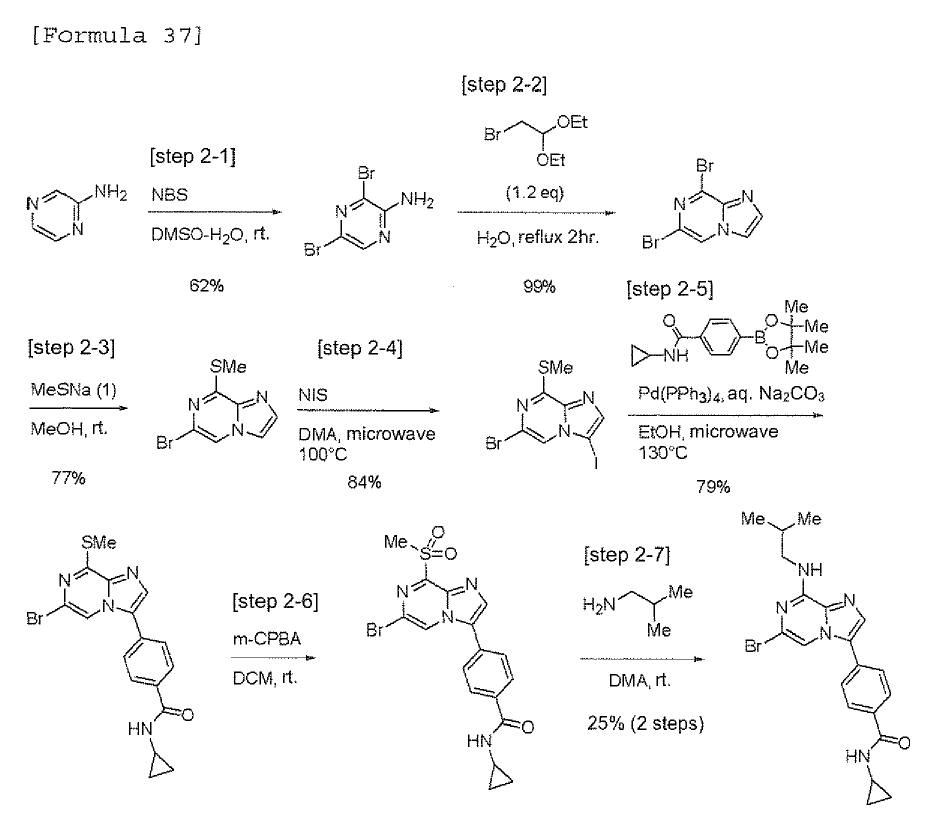

### Step 1-1: 4-(Cyclopropylcarbamoyl)phenylboronic acid pinacol ester

To a DMF (96 mL) solution of 4-carboxyphenylboronic acid pinacol ester (19.2 g, 77 mmol), cyclopropaneamine (8.84 g, 10.7 mL, 155 mmol) and triethylamine (11.8 g, 116 mmol), HATU (32.4 g, 85 mmol) was added and stirred at room temperature for one hour. To the reaction solution under ice cooling, water (96 mL) was added dropwise and the resultant solid substance was obtained by filtration and washed with water to obtain the titled compound (13.7 g, 47.6 mmol, 62%) as a white solid substance.
1H-NMR (300 MHz, DMSO-d₆) δ 0.54-0.60 (m, 2H), 0.63-0.71 (m, 2H), 1.29 (s, 12H), 2.80-2.88 (m, 1H), 7.71 (d, J = 7.7 Hz, 2H), 7.81 (d, J = 7.7 Hz, 2H), 8.48 (d, J = 4.2 Hz, 1H).
MS (ESI) m/z = 288 (M+H)⁺.

### Step 2-1: 3,5-Dibromopyrazine-2-amine

To a DMSO-water (4.61 kg-114 g) solution of pyrazine-2-amine (456 g, 4.79 mol), NBS (1.79 kg, 10.1 mol) was added under ice cooling and stirred at the same temperature for 5 hours. To the reaction solution under ice cooling, ice water was added and diluted with ethyl acetate and then the water phase was extracted with ethyl acetate. Organic phases were combined, washed with water, and dried over magnesium sulfate. The organic phase was filtrated and then concentrated under reduced pressure to obtain the titled compound (830 g, 3.28 mmol, 69%) as a brown solid substance.
MS (ESI) m/z = 252 (M+H)⁺.

### Step 2-2: 6,8-Dibromoimidazo[1,2-a]pyrazine

To an aqueous (10 kg) solution of 3,5-dibromopyrazine-2-amine (1018 g, 4.03 mol), 2-bromo-1,1-dimethoxyethane (1.79 kg, 4.14 mol) was added at room temperature and stirred for 2 hours while heating under reflux. To the reaction solution, water (15.3 kg) and sodium hydrogen carbonate (744 g) were added and further stirred for 15 minutes. The resultant solid substance was obtained by filtration to obtain the titled compound (1106 g, 3.99 mmol, 99%) as a brown solid substance. 1H-NMR (400 MHz, DMSO-d₆) δ 7.90 (s, 1H), 8.23 (s, 1H), 9.02 (s, 1H) .

### Step 2-3: 6-Bromo-8-(methylthio) imidazo[1,2-a]pyrazine

An aqueous (10 kg) solution of 3,5-dibromopyrazine-2-amine (1018 g, 4.03 mol), NBS (1.79 kg, 10.1 mol) was added under ice cooling and stirred at the same temperature for 5 hours. Under ice cooling, ice water was added to the reaction solution, which was diluted with ethyl acetate and then the water phase was extracted with ethyl acetate. Organic phases were combined, washed with water, and dried over magnesium sulfate. The organic phase was filtrated and then concentrated under reduced pressure to obtain the titled compound (830 g, 3.28 mmol, 69%) as a brown solid substance. 1E-NMR (400 MHz, CDCl₃) δ 2.68 (s, 1H), 7.58 (s, 1H), 7.67 (s, 1H), 7.97 (s, 1H).

### Step 2-4: 6-Bromo-3-iodo-8-(methylthio)imidazo[1,2-a]pyrazine

20 mL-microwave reaction container was charged with a DMA (10 mL) solution of 6-bromo-8-(methylthio)imidazo[1,2-a]pyrazine (2.93 g, 12.0 mmol). To this, NIS (4.46 g, 19.8 mmol) was added, capped, and stirred by use of a Biotage Optimizer reaction apparatus at 100°C for 10 minutes. To the reaction solution, water and ethyl acetate were added and separate phases. Thereafter the water phase was extracted with ethyl acetate and organic phases were combined, washed with water and saturated saline, and dried over magnesium sulfate. The organic phase was filtrated and then concentrated under reduced pressure to obtain the titled compound (3.72 g, 10.1 mmol, 84%).
1H-NMR (300 MHz, CDCl₃) δ 2.72 (s, 3H), 7.74 (s, 1H), 7.96 (s, 1H).

### Step 2-5: 4-(6-Bromo-8-(methylthio)imidazo[1,2-a]pyrazin-3-yl)-N-cyclopropylbenzamide

To an ethanol (10 mL) solution of 6-bromo-3-iodo-8-(methylthio)imidazo[1,2-a]pyrazine (1.07 g, 2.89 mmol), 4-(cyclopropylcarbamoyl)phenyl boronic acid pinacol ester (830 mg, 2.89 mmol) and a 2M aqueous sodium hydrogen carbonate solution (4.34 mL) in a 20 mL-microwave reaction container, Pd(PPh₃)₄ (167 mg, 0.145 mmol) was added, capped, and stirred by use of a Biotage Optimizer reaction apparatus at 130°C for 5 minutes. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 10-50% ethyl acetate gradient) to obtain the titled compound (920 mg, 2.28 mmol, 79%).
1H-NMR (300 MHz, CDCl₃) δ 0.63-0.66 (m, 2H), 0.88-0.92 (m, 2H), 2.68 (s, 3H), 2.92-2.94 (m, 1H), 6.30 (s, 1H), 7.58 (d, J = 8.4 Hz, 2H), 7.74 (s, 1H), 7.90 (d, J = 8.4 Hz, 2H), 8.04 (s, 1H).

### Step 2-6: 4-(6-Bromo-8-(methylsulfonyl)imidazo[1,2-a]pyrazin-3-yl)-N-cyclopropylbenzamide

To a chloroform (20 mL) solution of 4-(6-bromo-8-(methylthio)imidazo[1,2-a]pyrazin-3-yl)-N-cyclopropylbenzamide (1.0 g, 2.48 mmol), m-CPBA (1.71 g, 9.91 mmol) was added at room temperature and stirred overnight. To the reaction solution, an aqueous sodium carbonate solution was added and diluted with chloroform. Thereafter, the water phase was extracted with chloroform and organic phases were combined, washed with water, and dried over magnesium sulfate. After the organic phase was filtrated and concentrated under reduced pressure to obtain the titled compound (852 mg, 1.96 mmol, 79%).
1H-NMR (300 MHz, CDCl₃) δ 0.63-0.68 (m, 2H), 0.88-0.92 (m, 2H), 2.91-2.96 (m, 1H), 6.37 (s, 1H), 7.60 (d, J = 6.6 Hz, 2H), 7.96 (d, J = 6.6 Hz, 2H), 8.08 (s, 1H), 8.52 (s, 1H).

### Step 2-7: 4-(6-Bromo-8-(isobutylamino)imidazo[1,2-a]pyrazin-3-yl)-N-cyclopropylbenzamide

A 5 mL-microwave reaction container was charged with a DMA (3 mL) solution of 4-(6-bromo-8-(methylsulfonyl)imidazo[1,2-a]pyrazin-3-yl)-N-cyclopropylbenzamide (830 mg, 1.91 mmol). To this, isobutylamine (559 mg, 0.764 mmol) was added, capped and stirred by use of a Biotage Optimizer reaction apparatus at 130°C for 5 minutes. To the reaction solution, water and ethyl acetate were added and separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 10-70% ethyl acetate gradient) to obtain the titled compound (212 mg, 0.495 mmol, 26%).
1H-NMR (400 MHz, DMSO-d₆) δ0.65-0.69 (m, 2H), 0.88-0.95 (m, 2H), 1.04 (d, 6H, J = 6.9 Hz,), 1.96-2.05 (m, 1H), 2.93-2.97 (m, 1H), 3.46 (dd, 2H, J = 6.0 Hz, 6.9 Hz), 6.17 (t, 1H, J = 6.0 Hz), 6.31 (brs, 1H), 7.56-7.60 (m, 3H), 7.65 (s, 1H), 7.88-7.91 (m, 2H).
MS (ESI) m/z = 428 (M+H)⁺.
LC/MS t_{R} = 2.07 min.

Example 1-98

### 4-(6-(4-Cyanophenyl)-8-(isobutylamino)imidazo[1,2-a]pyrazin-3-yl)-N-cyclopropylbenzamide

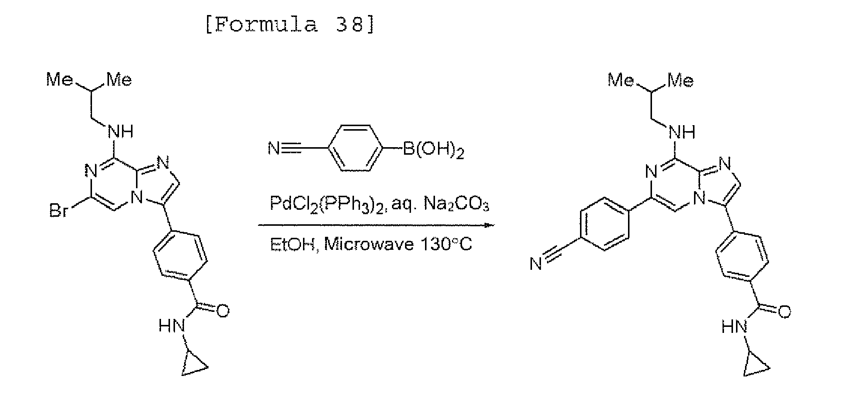

To an ethanol (2.5 mL) solution of 4-(6-bromo-8-(isobutylamino)imidazo[1,2-a]pyrazin-3-yl)-N-cyclopropylbenzamide (257 mg, 0.60 mmol), 4-cyanophenylboronic acid (106 mg, 0.72 mmol) and a 2M aqueous sodium hydrogen carbonate solution (0.60 mL) in a 5 mL-microwave reaction container, PdCl₂(PPh₃)₂ (21 mg) was added, capped, and stirred by use of a Biotage Optimizer reaction apparatus at 130°C for 10 minutes. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 10-70% ethyl acetate gradient) to obtain the titled compound (77 mg, 0.171 mmol, 29%).
1H-NMR (400 MHz, DMSO-d₆) δ 0.58-0.63 (m, 2H), 0.70-0.76 (m, 2H), 0.97 (d, 6H, J = 6.9 Hz), 2.09-2.18 (m, 1H), 2.87-2.93 (m, 1H), 3.44 (t, 2H, J = 6.4 Hz), 7.82-7.91 (m, 5H), 8.02 (d, 2H, J = 8.6 Hz), 8.22 (d, 2H, J = 8.6 Hz), 8.31 (s, 1H), 8.56 (d, 1H, J = 4.4 Hz).
MS (ESI) m/z = 451 (M+H)⁺.
LC/MS t_{R} = 2.29 min.

[Table 1-3A]

**Table 1-3**

| | | |
|---|---|---|
| 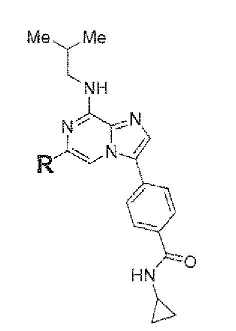 | | |

| Compounds described in Table 1-3 were synthesized in accordance with the processes of Examples mentioned above. | | |
|---|---|---|
| Example No. | R | property data |
| Example 1-99 | 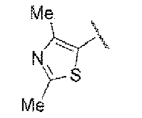 | MS (ESI) m/z = 461 (M+H)⁺. |
| | | LC/MS t_{R} = 2.13 min, |
| Example 1-100 | 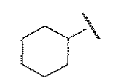 | MS (ESI) m/z = 432 (M+H)⁺. |
| | | LC/MS t_{R} = 2.19 min. |
| Example 1-101 | 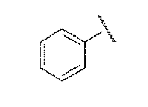 | 1H-NMR (400 MHz, DMSO-d₆) δ 0.58-0.63 (m, 2H), 0.70-0.76 (m, 2H), 0.97 (d, 6H, J = 6.6 Hz), 2.09-2.18 (m, 1H), 2.86-2.93 (m, 1H), 3.44 (dd, 2H, J = 6.0 Hz, 6.9 Hz), 7.33-7.38 (m, 1H), 7.42-7,47 (m, 2H), 7.74-7.78 (m, 1H), 7.81-7.85 (m. 3H), 7.98-8.03 (m, 4H), 8.14 (s, 1H), 8.56 (d, 1H, J = 4.4 Hz,). |
| | | MS (ESI) m/z = 426 (M+H)⁺. |
| | | LC/MS t_{R} = 2.49 min. |
| Example 1-102 | 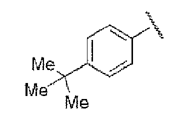 | MS (ESI) m/z = 482 (M+H)⁺. |
| | | LC/MS t_{R} = 2.94 min. |

**[Table 1-3B]**

| Example No. | R | property data |
|---|---|---|
| Example 1-103 | 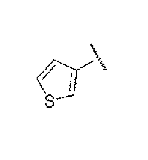 | 1H-NMR (400 MHz, DMSO-d₆) δ 0.58-0.63 (m, 2H), 0,70-0.76 (m, 2H), 0.96 (d. 6H, J = 6.6 Hz), 2.10-2.17 (m, 1H), 2.87-2.93 (m, 1H), 3.42 (t, 2H. J = 6.5 Hz), 7.58-7.61 (m. 1H), 7.64-7.67 (m, 1H), 7.69-7.73 (m, 1H), 7,79-7.82 (m, 3H), 7.99-8.03 (m, 3H), 8.10 (s, 1H), 8.56 (d, 1H, J = 4.2 Hz). |
| | | MS (ESI) m/z = 432 (M+H)⁺. |
| | | LO/MS t_{R} = 2.41 min. |
| Example 1-104 | 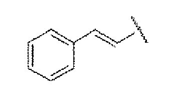 | MS (ESI) m/z = 452 (M+H)⁺. |
| | | LC/MS t_{R} = 2.59 min. |
| Example 1-150 | 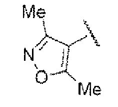 | MS (ESI) m/z = 445 (M+H)⁺. |
| | | LC/MS t_{R}=2.11 min. |
| Example 1-106 | 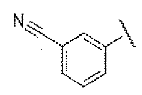 | MS (ESI) m/z = 451 (M+H)⁺. |
| | | LC/MS t_{R} = 2.40 min. |
| Example 1-107 | 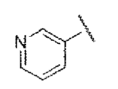 | MS (ESI) m/z = 427 (M+H)⁺. |
| | | LC/MS t_{R} = 1.50 min. |
| Example 1-108 | 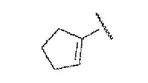 | MS (ESI) m/z = 416 (M+H)⁺. |
| | | LC/MS t_{R} = 2.48 min. |
| Example 1-109 | 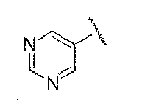 | MS (ESI) m/z = 428 (M+H)⁺. |
| | | LC/MS t_{R} = 1.89 min. |
| Example 1-110 | 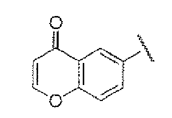 | MS (ESI) m/z = 494 (M+H)⁺. |
| | | LC/MS t_{R} = 2.15 min. |

**[Table 1-3C]**

| Example No. | R | property data |
|---|---|---|
| Example 1-111 | 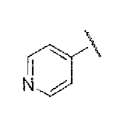 | 1H-NMR (300 MHz, DMSO-d₆) δ 0.58-0.63 (m, 2H), 0.70-0.76 (m, 2H), 0.98 (d, 6H, J = 6.6 Hz), 2,09-2.18 (m, 1H), 2.86-2.93 (m, 1H), 3.45 (t, 2H, J = 6.5 Hz), 7.83-7.92 (m, 4H), 7.98-8.04 (m, 4H), 8.36 (s, 1H), 8.57 (d, 1H, J = 4.4 Hz), 8.61-8.64 (m, 1H), |
| | | MS (ESI) m/z = 427 (M+H)⁺. |
| | | LC/MS t_{R} = 1.37 min. |
| Example 1-112 | 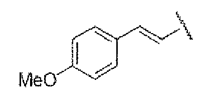 | MS (ESI) m/z = 482 (M+H)⁺. |
| | | LC/MS t_{R} = 2.48 min. |
| Example 1-113 | 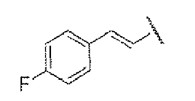 | MS (ESI) m/z = 470 (M+H)⁺. |
| | | LC/MS t_{R} = 2.60 min. |
| Example 1-114 | 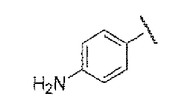 | MS (ESI) m/z = 441 (M+H)⁺. |
| | | LC/MS t_{R} = 1.64 min. |
| Example 1-115 | 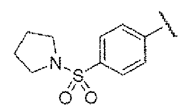 | MS (ESI) m/z = 559 (M+H)⁺. |
| | | LC/MS t_{R} = 2.40 min. |
| Example 1-116 | 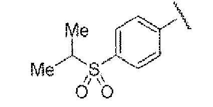 | MS (ESI) m/z = 532 (M+H)⁺. |
| | | LC/MS t_{R} = 2.32 min. |
| Example 1-117 | 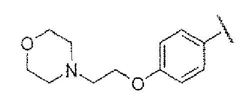 | MS (ESI) m/z = 555 (M+H)⁺. |
| | | LC/MS t_{R} = 1.39 min. |
| Example 1-118 | 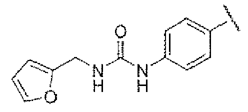 | MS (ESI) m/z = 564 (M+H)⁺, |
| | | LC/MS t_{R} = 2.05 min. |

**[Table 1-3D]**

| Example No. | R | property data |
|---|---|---|
| Example 1-119 | 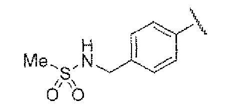 | MS (ESI) m/z = 533 (M+H)⁺. |
| | | LC/MS t_{R} = 1.94 min. |
| Example 1-120 | 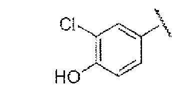 | MS (ESI) m/z = 476 (M+H)⁺. |
| | | LC/MS t_{R} = 2.13 min. |
| Example 1-121 | 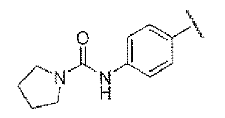 | MS (ESI) m/z = 538 (M+H)⁺. |
| | | LC/MS t_{R} = 2.01 min. |
| Example 1-122 | 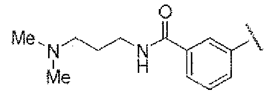 | MS (ESI) m/z = 554 (M+H)⁺. |
| | | LC/MS t_{R} = 1.37 min. |
| Example 1-123 | 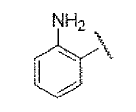 | MS (ESI) m/z = 441 (M+H)⁺. |
| | | LC/MS t_{R}= 2,11 min. |
| Example 1-124 | 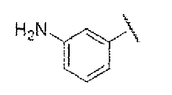 | MS (ESI) m/z = 441 (M+H)⁺. |
| | | LC/MS t_{R} = 1.92 min. |
| Example 1-125 | 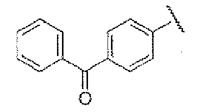 | MS (ESI) m/z = 530 (M+H)⁺. |
| | | LC/MS t_{R} = 2.24 min. |
| Example 1-126 | 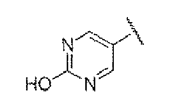 | MS (ESI) m/z = 444 (M+H)⁺. |
| | | LC/MS t_{R} = 1.61 min. |
| Example 1-127 | 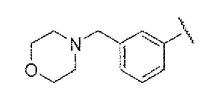 | MS (ESI) m/z = 525 (M+H)⁺. |
| | | LC/MS t_{R} = 1.50 min. |

**[Table 1-3E]**

| Example No. | R | property data |
|---|---|---|
| Example 1-128 | 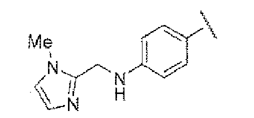 | MS (ESI) m/z = 535 (M+H)⁺. |
| | | LC/MS t_{R} = 1.44 min. |
| Example 1-129 | 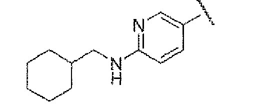 | MS (ESI) m/z = 538 (M+H)⁺. |
| | | LC/MS t_{R}= 1.83 min. |
| Example 1-130 | 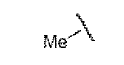 | MS (ESI) m/z = 364 (M+H)⁺. |
| | | LC/MS t_{R} = 1.34 min. |
| Example 1-131 | 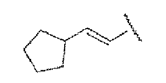 | MS (ESI) m/z = 444 (M+H)⁺. |
| | | LC/MS t_{R} = 2.80 min. |
| Example 1-132 | 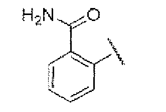 | MS (ESI) m/z = 469 (M+H)⁺. |
| | | LC/MS t_{R} = 1.70 min. |
| Example 1-133 | 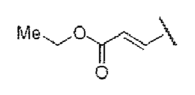 | MS (ESI) m/z = 448 (M+H)⁺. |
| | | LC/MS t_{R} = 2.48 min. |
| Example 1-134 | 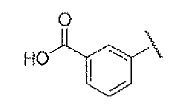 | MS (ESI) m/z = 470 (M+H)⁺. |
| | | LC/MS t_{R} = 2.08 min. |
| Example 1-135 | 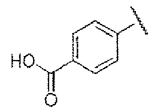 | MS (ESI) m/z = 470 (M+H)⁺. |
| | | LC/MS t_{R} = 2.08 min, |
| Example 1-136 | 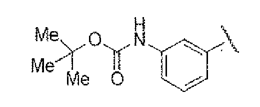 | MS (ESI) m/z = 541 (M+H)⁺. |
| | | LC/MS t_{R} = 2.60 min. |

**[Table 1-3F]**

| Example No. | R | property data |
|---|---|---|
| Example 1-137 | 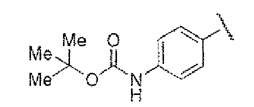 | MS (ESI) m/z = 541 (M+H)⁺. |
| | | LC/MS t_{R} = 2.59 min. |
| Example 1-138 | 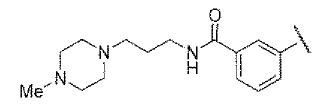 | MS (ESI) m/z = 609 (M+H)⁺. |
| | | LC/MS t_{R} = 1.68 min. |
| Example 1-139 | 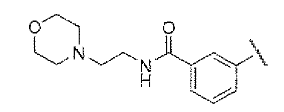 | MS (ESI) m/z = 582 (M+H)⁺. |
| | | LC/MS t_{R} = 1.77 min. |
| Example 1-140 | 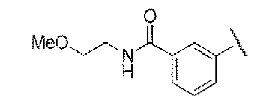 | MS (ESI) m/z = 527 (M+H)⁺, |
| | | LC/MS t_{R} = 1.85 min. |
| Example 1-141 | 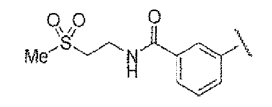 | MS (ESI) m/z = 575 (M+H)⁺. |
| | | LC/MS t_{R} = 1.74 min. |
| Example 1-142 | 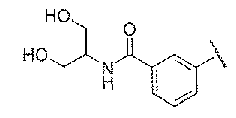 | MS (ESI) m/z = 543 (M+H)⁺, |
| | | LC/MS t_{R} = 1.53 min. |
| Example 1-143 | 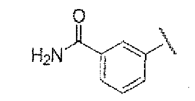 | MS (ESI) m/z = 469 (M+H)⁺. |
| | | LC/MS t_{R} 1.70 min, |
| Example 1-144 | 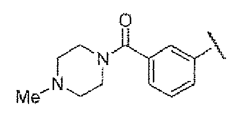 | MS (ESI) m/z = 552 (M+H)⁺. |
| | | LC/MS t_{R} = 1.78 min, |
| Example 1-145 | 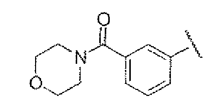 | MS (ESI) m/z = 539 (M+H)⁺. |
| | | LC/MS t_{R} = 1.85 min. |

**[Table 1-3G]**

| Example No. | R | property data |
|---|---|---|
| Example 1-146 | 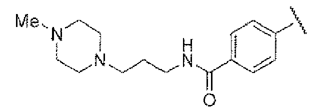 | MS (ESI) m/z = 609 (M+H)⁺. |
| | | LC/MS t_{R} = 1.64 min. |
| Example 1-147 | 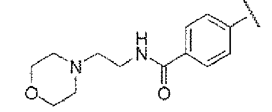 | MS (ESI) m/z = 582 (M+H)⁺. |
| | | LC/MS t_{R} = 1.72 min. |
| Example 1-148 | 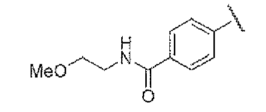 | MS (ESI) m/z = 527 (M+H)⁺. |
| | | LC/MS t_{R}= 1,81 min. |
| Example 1-149 | 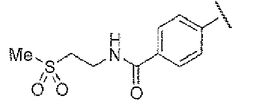 | MS (ESI) m/z = 575 (M+H)⁺. |
| | | LC/MS t_{R} = 1.68 min. |
| Example 1-150 | 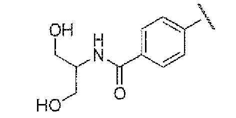 | MS (ESI) m/z = 543 (M+H)⁺. |
| | | LC/MS t_{R} = 1.48 min. |
| Example 1-151 | 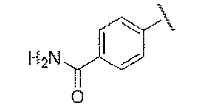 | MS (ESI) m/z = 469 (M+H)⁺. |
| | | LC/MS t_{R} = 1,64 min. |
| Example 1-152 | 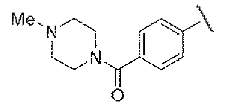 | MS (ESI) m/z = 552 (M+H)⁺. |
| | | LC/MS t_{R} = 1.76 min. |
| Example 1-153 | 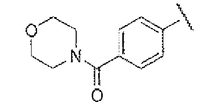 | MS (ESI) m/z = 539 (M+H)⁺. |
| | | LC/MS t_{R} = 1.83 min. |
| Example 1-154 | 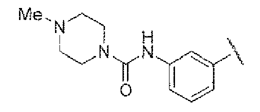 | MS (ESI) m/z = 567 (M+H)⁺. |
| | | LC/MS t_{R} = 1.22 min. |

**[Table 1-3H]**

| Example No. | R | property data |
|---|---|---|
| Example 1-155 | 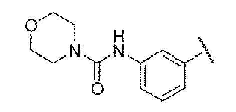 | MS (ESI) m/z = 554 (M+H)⁺. |
| | | LC/MS t_{R} = 1.89 min. |
| Example 1-156 | 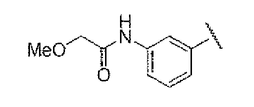 | MS (ESI) m/z = 513 (M+H)⁺. |
| | | LC/MS t_{R} = 2.02 min. |
| Example 1-157 | 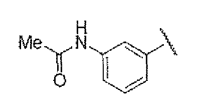 | MS (ESI) m/z = 483 (M+H)⁺. |
| | | LC/MS t_{R} = 1.88 min, |
| Example 1-158 | 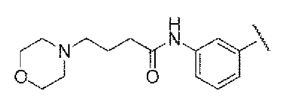 | MS (ESI) m/z = 596 (M+H)⁺. |
| | | LC/MS t_{R} = 1.27 min. |
| Example 1-159 | 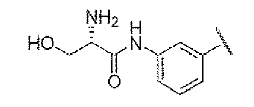 | MS (ESI) m/z = 528 (M+H)⁺. |
| | | LC/MS t_{R} = 1.18 min. |
| Example 1-160 | 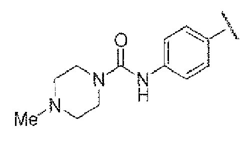 | MS (ESI) m/z = 567 (M+H)⁺. |
| | | LC/MS t_{R} = 1.17 min. |
| Example 1-161 | 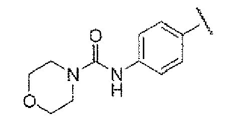 | MS (ESI) m/z = 554 (M+H)⁺. |
| | | LC/MS t_{R} = 1.84 min. |
| Example 1-162 | 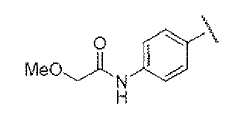 | MS (ESI) m/z = 513 (M+H)⁺. |
| | | LC/MS t_{R} = 2.01 min. |
| Example 1-163 | 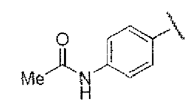 | MS (ESI) m/z = 483 (M+H)⁺. |
| | | LC/MS t_{R} = 1.83 min. |

**[Table 1-3I]**

| Example No. | R | property data |
|---|---|---|
| Example 1-164 | 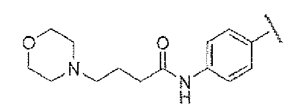 | MS (ESI) m/z = 596 (M+H)⁺. |
| | | LC/MS t_{R}= 1,24 min. |
| Example 1-165 | 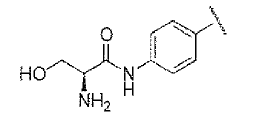 | MS (ESI) m/z = 528 (M+H)⁺. |
| | | LC/MS t_{R} = 1.11 min, |
| Example 1-166 | 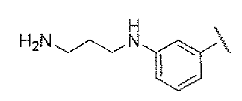 | MS (ESI) m/z = 498 (M+H)⁺. |
| | | LC/MS t_{R} = 1.19 min. |
| Example 1-167 | 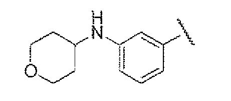 | MS (ESI) m/z = 525 (M+H)⁺. |
| | | LC/MS t_{R} = 2.08 min, |
| Example 1-168 | 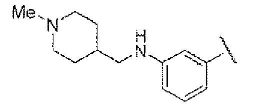 | MS (ESI) m/z = 552 (M+H)⁺. |
| | | LC/MS t_{R} = 1.28 min. |
| Example 1-169 | 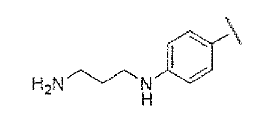 | MS (ESI) m/z = 498 (M+H)⁺, |
| | | LC/MS t_{R} = 1.14 min. |
| Example 1-170 | 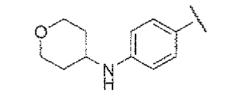 | MS (ESI) m/z = 525 (M+H)⁺. |
| | | LC/MS t_{R} = 1.99 min. |
| Example 1-171 | 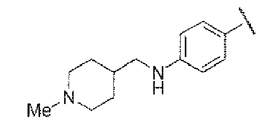 | MS (ESI) m/z = 552 (M+H)⁺. |
| | | LC/MS t_{R} = 1.26 min. |
| Example 1-172 | 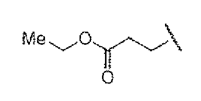 | MS (ESI) m/z = 450 (M+H)⁺. |
| | | LC/MS t_{R} = 1.70 min. |

**[Table 1-3J]**

| Example No. | R | property data |
|---|---|---|
| Example 1-173 | 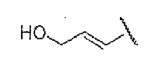 | MS (ESI) m/z = 406 (M+H)⁺. |
| | | LC/MS t_{R} = 1.51 min. |
| Example 1-174 | 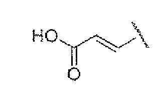 | MS (ESI) m/z = 420 (M+H)⁺. |
| | | LC/MS t_{R} = 1.79 min. |
| Example 1-175 | 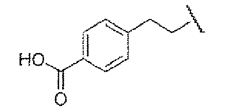 | MS (ESI) m/z = 498 (M+H)⁺. |

[Table 1-4A]

**Table 1-4**

| | | |
|---|---|---|
| 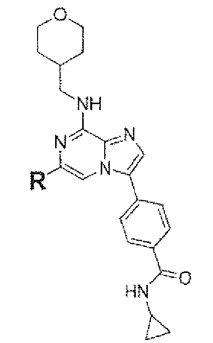 | | |

| Compounds described in Table 1-4 were synthesized in accordance with the processes of Examples mentioned above. | | |
|---|---|---|
| Example No. | R | property data |
| Example 1-176 | 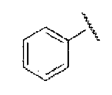 | MS (ESI) m/z = 468 (M+H)⁺. |
| | | LC/MS t_{R} = 2.06 min. |
| Example 1-177 | 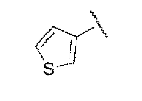 | MS (ESI) m/z = 474 (M+H)⁺. |
| | | LC/MS t_{R} = 2.00 min. |

**[Table 1-4B]**

| Example No. | R | property data |
|---|---|---|
| Example 1-178 | 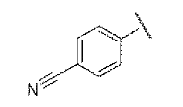 | MS (ESI) m/z = 493 (M+H)⁺. |
| | | LC/MS t_{R}= 1.94 min. |
| Example 1-179 | 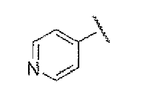 | MS (ESI) m/z = 469 (M+H)⁺. |
| | | LC/MS t_{R} 1.07 min. |
| Example 1-180 | 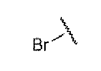 | MS (ESI) m/z = 470 (M+H)⁺. |
| | | LC/MS t_{R} = 1.66 min. |

[Table 1-5A]

**Table 1-5**

| | | |
|---|---|---|
| 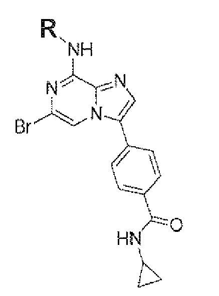 | | |

| Compounds described in Table 1-5 were synthesized in accordance with the processes of Examples, mentioned above. | | |
|---|---|---|
| Example No. | R | property data |
| Example 1-181 | 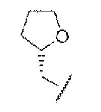 | MS (ESI) m/z = 456 (M+H)⁺. |
| | | LC/MS t_{R} = 2.95 min. |
| Example 1-182 | 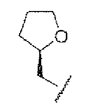 | MS (ESI) m/z = 456 (M+H)⁺. |
| | | LC/MS t_{R} = 2,93 min. |

**[Table 1-5B]**

| Example No. | R | property data |
|---|---|---|
| Example 1-183 | 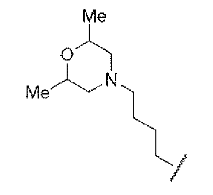 | MS (ESI) m/z = 541 (M+H)⁺. |
| | | LC/MS t_{R} = 1.85 min. |
| Example 1-184 | 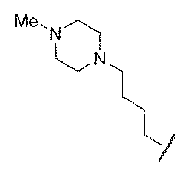 | MS (ESI) m/z = 526 (M+H)⁺. |
| | | LC/MS t_{R} = 1.68 min. |
| Example 1-185 | 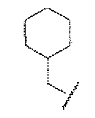 | MS (ESI) m/z = 468 (M+H)⁺. |
| | | LC/MS t_{R} = 3.54 min. |
| Example 1-186 | 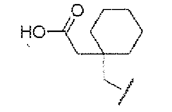 | MS (ESI) m/z = 526 (M+H)⁺. |
| | | LC/MS t_{R} = 3.25 min. |
| Example 1-187 | 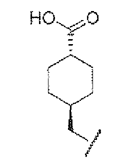 | MS (ESI) m/z = 512 (M+H)⁺. |
| | | LC/MS t_{R} = 3.02 min. |
| Example 1-188 | 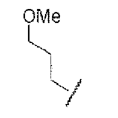 | MS (ESI) m/z = 444 (M+H)⁺. |
| | | LC/MS t_{R} = 2.87 min, |
| Example 1-189 | 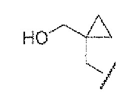 | MS (ESI) m/z = 456 (M+H)⁺. |
| | | LC/MS t_{R} = 2.80 min. |
| Example 1-190 | 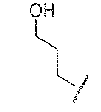 | MS (ESI) m/z = 430 (M+H)⁺. |
| | | LC/MS t_{R} = 2.56 min. |

**[Table 1-5C]**

| Example No. | R | property data |
|---|---|---|
| Example 1-191 | 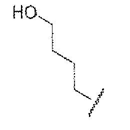 | MS (ESI) m/z = 444 (M+H)⁺. |
| | | LC/MS t_{R}= 2,69 min. |
| Example 1-192 | 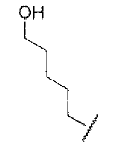 | MS (ESI) m/z = 458 (M+H)⁺. |
| | | LC/MS t_{R} = 2.77 min. |
| Example 1-193 | 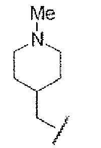 | MS (ESI) m/z = 483 (M+H)⁺. |
| | | LC/MS t_{R} = 1.71 min. |
| Example 1-194 | 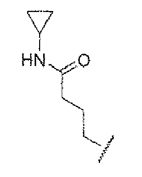 | MS (ESI) m/z = 467 (M+H)⁺. |
| | | LC/MS t_{R} = 2.68 min. |
| Example 1-195 | 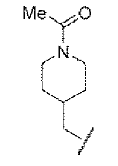 | MS (ESI) m/z =. 511 (M+H)⁺. |
| | | LC/MS t_{R} = 2.75 min. |
| Example 1-196 | 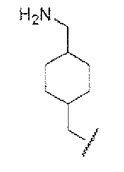 | MS (ESI) m/z = 497 (M+H)⁺. |
| | | LC/MS t_{R} = 2.02 min. |
| Example 1-197 | 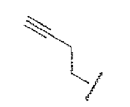 | MS (ESI) m/z = 424 (M+H)⁺. |
| | | LC/MS t_{R} = 2.89 min. |

**[Table 1-5D]**

| Example No. | R | property data |
|---|---|---|
| Example 1-198 | 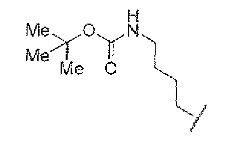 | MS (ESI) m/z = 543 (M+H)⁺. |
| | | LC/MS t_{R} = 3.12 min. |
| Example 1-199 | 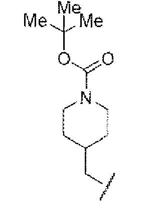 | MS (ESI) m/z = 569 (M+H)⁺. |
| | | LC/MS t_{R} = 3.31 min. |
| Example 1-200 | 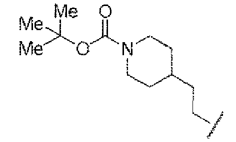 | MS (ESI) m/z = 583 (M+H)⁺. |
| | | LC/MS t_{R} = 3.40 min. |
| Example 1-201 | 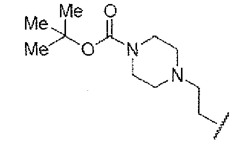 | MS (ESI) m/z = 584 (M+H)⁺. |
| | | LC/MS t_{R} = 2.08 min. |
| Example 1-202 | 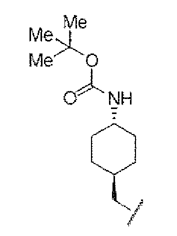 | MS (ESI) m/z = 583 (M+H)⁺. |
| | | LC/MS t_{R} = 3.29 min. |
| Example 1-203 | 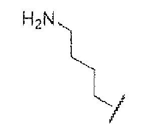 | MS (ESI) m/z = 443 (M+H)⁺. |
| | | LC/MS t_{R} = 1.70 min. |

**[Table 1-5E]**

| Example No. | R | property data |
|---|---|---|
| Example 1-204 | 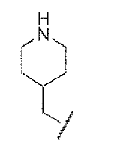 | MS (ESI) m/z = 469 (M+H)⁺. |
| | | LC/MS t_{R} = 1,77 min. |
| Example 1-205 | 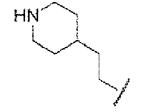 | MS (ESI) m/z = 483 (M+H)⁺. |
| | | LC/MS t_{R} = 1,82 min. |
| Example 1-206 | 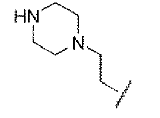 | MS (ESI) m/z = 484 (M+H)⁺. |
| | | LC/MS t_{R} = 1.56 min. |
| Example 1-207 | 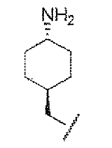 | MS (ESI) m/z = 483 (M+H)⁺. |
| | | LC/MS t_{R} = 1.90 min. |
| Example 1-208 | 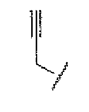 | MS (ESI) m/z = 410 (M+H)⁺. |
| | | LC/MS t_{R} = 2,78 min. |
| Example 1-209 | 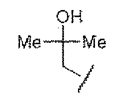 | MS (ESI) m/z = 444 (M+H)⁺. |
| | | LC/MS t_{R} = 2.77 min. |
| Example 1-210 | 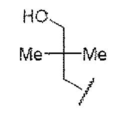 | MS (ESI) m/z = 458 (M+H)⁺. |
| | | LC/MS t_{R} = 2,90 min. |
| Example 1-211 | 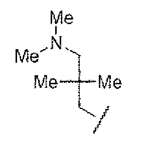 | MS (ESI) m/z = 485 (M+H)⁺. |
| | | LC/MS t_{R} = 1.73 min. |

[Table 1-6A]

**Table 1-6**

| | | |
|---|---|---|
| 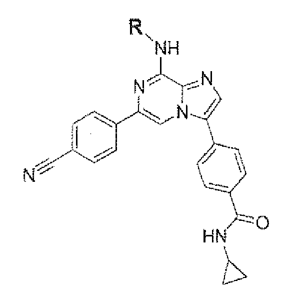 | | |

| Compounds described in Table 1-6 were synthesized in accordance with the processes of Examples mentioned above. | | |
|---|---|---|
| Example No. | R | property data |
| Example 1-212 | 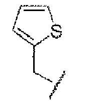 | MS (ESI) m/z = 491 (M+H)⁺. |
| | | LC/MS t_{R} = 3.25 min. |
| Example 1-213 | 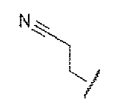 | MS (ESI) m/z = 448 (M+H)⁺. |
| | | LC/MS t_{R} = 2.76 min. |
| Example 1-214 | 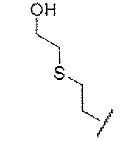 | MS (ESI) m/z = 499 (M+H)⁺. |
| | | LC/MS t_{R} = 2.89 min. |
| Example 1-215 | 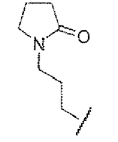 | MS (ESI) m/z = 520 (M+H)⁺. |
| | | LC/MS t_{R} = 2.83 min. |
| Example 1-216 | 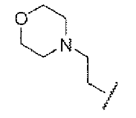 | MS (ESI) m/z = 508 (M+H)⁺. |
| | | LC/MS t_{R} = 1,86 min. |

**[Table 1-6B]**

| Example No. | R | property data |
|---|---|---|
| Example 1-217 | 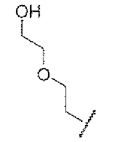 | MS (ESI) m/z = 483 (M+H)⁺. |
| | | LC/MS t_{R} = 2.83 min. |
| Example 1-218 | 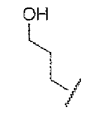 | MS (ESI) m/z = 453 (M+H)⁺. |
| | | LC/MS t_{R} = 2.76 min. |
| Example 1-219 | 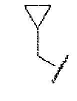 | MS (ESI) m/z = 449 (M+H)⁺. |
| | | LC/MS t_{R} = 3.25 min. |
| Example 1-220 | 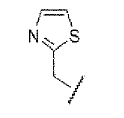 | MS (ESI) m/z = 492 (M+H)⁺. |
| | | LC/MS t_{R} = 2.88 min. |
| Example 1-221 | 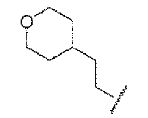 | MS (ESI) m/z = 507 (M+H)⁺. |
| | | LC/MS t_{R} = 3.16 min. |
| Example 1-222 | 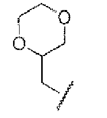 | MS (ESI) m/z = 495 (M+H)⁺. |
| | | LC/MS t_{R} = 2.89 min. |
| Example 1-223 | 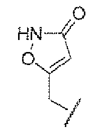 | MS (ESI) m/z =. 492 (M+H)⁺. |
| | | LC/MS t_{R} = 2.78 min. |
| Example 1-224 | 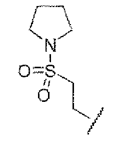 | MS (ESI) m/z = 556 (M+H)⁺. |
| | | LC/MS t_{R} = 2.86 min. |
| Example 1-225 | 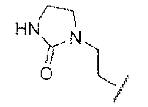 | MS (ESI) m/z = 507 (M+H)⁺. |
| | | LC/MS t_{R} = 2.70 min. |

**[Table 1-6C]**

| Example No. | R | property data |
|---|---|---|
| Example 1-226 | 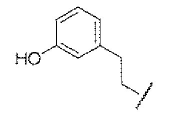 | MS (ESI) m/z = 515 (M+H)⁺. |
| | | LC/MS t_{R} = 3.11 min. |
| Example 1-227 | 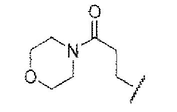 | MS (ESI) m/z = 536 (M+H)⁺. |
| | | LC/MS t_{R} = 2.75 min. |
| Example 1-228 | 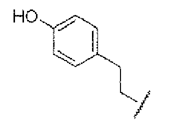 | MS (ESI) m/z =515 (M+H)⁺. |
| | | LC/MS t_{R} = 3.05 min. |
| Example 1-229 | 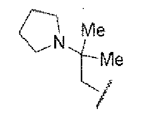 | MS (ESI) m/z = 521 (M+H)⁺. |
| | | LC/MS t_{R} = 1.94 min. |
| Example 1-230 | 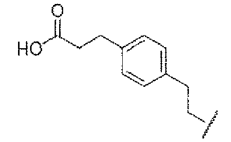 | MS (ESI) m/z = 628 (M+H)⁺. |
| | | LC/MS t_{R} = 3.38 min. |
| Example 1-231 | 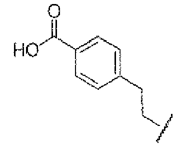 | MS (ESI) m/z = 599 (M+H)⁺. |
| | | LC/MS t_{R} = 3.51 min. |
| Example 1-232 | 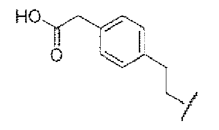 | MS (ESI) m/z = 614 (M+H)⁺. |
| | | LC/MS t_{R} = 3.51 min. |
| Example 1-233 | 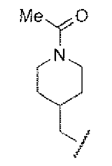 | MS (ESI) m/z = 534 (M+H)⁺. |
| | | LC/MS t_{R} = 2.89 min. |

**[Table 1-6D]**

| Example No. | R | property data |
|---|---|---|
| Example 1-234 | 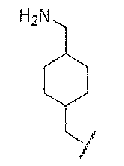 | MS (ESI) m/z = 521 (M+H)⁺. |
| | | LC/MS t_{R} = 2.22 min. |
| Example 1-235 | 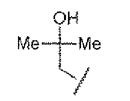 | MS (ESI) m/z = 467 (M+H)⁺. |
| | | LC/MS t_{R} = 2.88 min. |
| Example 1-236 | 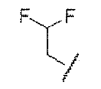 | MS (ESI) m/z = 459 (M+H)⁺. |
| | | LC/MS t_{R} = 2.99 min. |
| Example 1-237 | 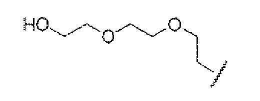 | MS (ESI) m/z = 527 (M+H)⁺. |
| | | LC/MS t_{R} = 2.79 min. |
| Example 1-238 | 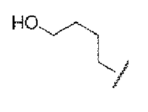 | MS (ESI) m/z = 467 (M+H)⁺. |
| | | LC/MS t_{R} = 2.90 min. |
| Example 1-239 | 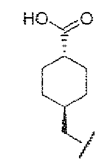 | MS (ESI) m/z = 535 (M+H)⁺. |
| | | LC/MS t_{R} 3.20 min. |

### Example 1-240

tert-Butyl cyclohexylmethyl(3-(4-(cyclopropylcarbonyl)phenyl)-8-((tetrahydro-2H-pyran-4-yl)methylamino)imidazo[1,2-a]pyrazin-6-yl)carbamate

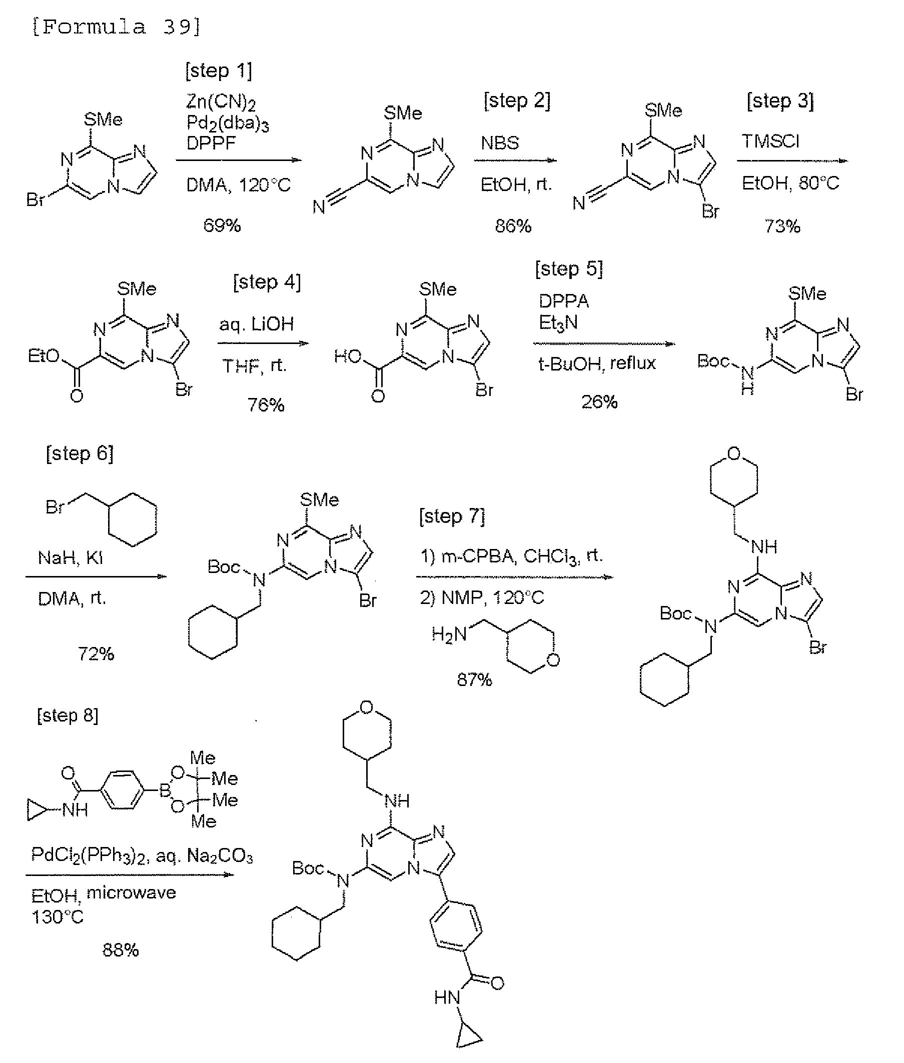

### Step 1: 8-(Methylthio)imidazo[1,2-a]pyrazine-6-carbonitrile

To a DMA (100 mL) solution of 6-bromo-8-(methylthio)imidazo[1,2-a]pyrazine (5.00 g, 20.5 mmol), Zn(CN)₂ (5.05 g, 43.0 mmol) and DPPF (2.27 g, 4.10 mmol), Pd₂(dba)₃ (1.88 g, 0.205 mmol) was added and stirred at 120°C for 5 hours. To the reaction solution, ethyl acetate was added to dilute. Thereafter, the precipitated insoluble matter was removed by Celite filtration. The resultant residue was concentrated under reduced pressure, purified by medium-pressure silica gel chromatography (hexane/ethyl acetate; 10-100% ethyl acetate gradient) to obtain the titled compound (2.70 g, 14.2 mmol, 69%).
1H-NMR (300 MHz, CDCl₃) δ 2.67 (s, 3H), 7.68 (d, J = 1.2 Hz, 1H), 7.77 (d, J = 1.2 Hz, 1H), 8.29 (s, 1H).

### Step 2: 3-Bromo-8-(methylthio)imidazo[1,2-a]pyrazine-6-carbonitrile

To an ethanol (80 mL) solution of 8-(methylthio)imidazo[1,2-a]pyrazine-6-carbonitrile (2.66 g, 14.0 mol), NBS (2.74 g, 15.4 mmol) was added and stirred at room temperature for 4 hours. To the reaction solution, sodium bicarbonate water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant solid substance was solidified with hexane/ethyl acetate to obtain the titled compound (3.25 g, 12.1 mmol, 86%).
1H-NMR (300 MHz, DMSO-d₆) δ 2.65 (s, 3H), 8.01 (s, 1H), 9.18 (s, 1H).

### Step 3: Ethyl 3-bromo-8-(methylthio)imidazo[1,2-a]pyrazine-6-carboxylate

To an ethanol (80 mL) solution of 3-bromo-8-(methylthio)imidazo[1,2-a]pyrazine-6-carbonitrile (1.61 g, 5.98 mmol), trimethylsilyl chloride (7.7 mL, 60 mmol) was added and stirred at 80°C for 36 hours. The reaction solution was diluted with water and concentrated and then the resultant solid substance was purified by medium-pressure silica gel chromatography (hexane/ethyl acetate; 10-100% ethyl acetate gradient) to obtain the titled compound (1.38 g, 4.36 mmol, 73%).
1H-NMR (300 MHz, CDCl₃) δ 1.44 (t, J = 7.1 Hz, 3H), 2.75 (s, 3H), 4.46 (q, J = 7.1 Hz, 2H), 7.70 (s, 1H), 8.60 (s, 1H).

### Step 4: 3-Bromo-8-(methylthio)imidazo[1,2-a]pyrazine-6-carboxylic acid

To a THF solution (2 mL) of ethyl 3-bromo-8-(methylthio)imidazo[1,2-a]pyrazine-5-carboxylate (63 mg, 0.20 mmol), a 2.5N aqueous lithium hydroxide solution (1.0 mL) was added and stirred at room temperature for 20 minutes. The reaction solution was washed with ether and then a 10% aqueous citric acid solution and ethyl acetate were added to separate phases. The water phase was extracted with ethyl acetate and organic phases were combined, washed with water and saturated saline, and dried over magnesium sulfate. The organic phase was filtrated and then concentrated under reduced pressure. The resultant residue was purified by reverse-phase preparatory liquid chromatography (C18 column; water/acetonitrile/0.1% formic acid; 10-100% acetonitrile gradient) to obtain the titled compound (44 mg, 0.15 mmol, 76%).
1H-NMR (300 MHz, DMSO-d₆) δ 2.66 (s, 3H), 7.93 (s, 1H), 8.54 (s, 1H) .

### Step 5: tert-Butyl 3-bromo-8-(methylthio)imidazo[1,2-a]pyrazin-6-ylcarbamate

To a tert-butanol (7.0 mL) solution of 3-bromo-8-(methylthio)imidazo[1,2-a]pyrazine-6-carboxylic acid (29 mg, 0.10 mmol) and triethylamine (42 µL, 0.30 mmol), DPPA (27 µL, 0.12 mmol) was added and then stirred for 4 hours while heating under reflux. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 10-100% ethyl acetate gradient) to obtain the titled compound (9.3 mg, 0.026 mmol, 26%).
1H-NMR (300 MHz, CDCl₃) δ 1.54 (s, 9H), 2.58 (s, 3H), 6.97 (s, 1H), 7.57 (s, 1H), 8.44 (s, 1H) .

### Step 6: tert-Butyl 3-bromo-8-(methylthio) imidazo [1,2-a] pyrazin-6-yl(cyclohexylmethyl)carbamate

To a DMA (7.0 mL) solution of tert-butyl 3-promo-8-(methylthio)imidazo[1,2-a]pyrazin-6-ylcarbamate (360 mg, 1.00 mmol), sodium hydride (60% wt, 80 mg, 2.0 mmol) and potassium iodide (0.83 g, 5.0 mmol), (bromomethyl)cyclohexane (0.70 mL, 5.0 mmol) was added and then stirred at room temperature for 6 hours. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 10-100% ethyl acetate gradient) to obtain the titled compound (340 mg, 0.747 mmol, 72% .
1H-NMR (300 MHz, CDCl₃) δ 0.92-0.99 (m, 2H), 1.09-1.19 (m, 3H), 1.50 (s, 9H), 1.62-1.70 (m, 6H), 2.62 (s, 3H), 3.83 (d, J = 7.2 Hz, 2H), 7.61 (s, 1H), 8.12 (s, 1H) .
MS (ESI) m/z = 455 (M+H)⁺.

### Step 7: tert-Butyl 3-bromo-8-((tetrahydro-2H-pyran-4-yl)methylamino)imidazo[1,2-a]pyrazin-6-yl(cyclohexylmethyl)carbamate

To a chloroform (9.6 mL) solution of tert-butyl 3-bromo-8-(methylthio)imidazo[1,2-a]pyrazin-6-yl(cyclohexylmethyl)carbamate (320 mg, 0.700 mmol), m-CPBA (388 mg) was added at room temperature and stirred for 6 hours. To the reaction solution, an aqueous sodium carbonate solution was added and diluted with chloroform. Thereafter, the water phase was extracted with ethyl acetate and organic phases were combined, washed with water and saturated saline, and dried over magnesium sulfate. The organic phase was filtrated and then concentrated under reduced pressure to obtain a residue.

To an NMP (4.5 mL) solution of the resultant residue in a 5 mL-microwave reaction container, (tetrahydro-2H-pyran-4-yl)methylamine (281 mg, 2.44 mmol) was added, capped and stirred by use of a Biotage Optimizer reaction apparatus at 120 °C for 5 minutes. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by reverse-phase preparatory liquid chromatography (C18 column; water/acetonitrile /0.1% formic acid; 10-100% acetonitrile gradient) to obtain the titled compound (280 mg, 0.535 mmol, 87%).
MS (ESI) m/z = 522 (M+H)⁺.

### Step 8: Titled compound

To an ethanol (2.7 mL) solution of tert-butyl 3-bromo-8-((tetrahydro-2H-pyran-4-yl)methylamino)imidazo[1,2-a]pyrazin-6-yl(cyclohexylmethyl)carbamate (265 mg, 0.51 mmol), 4-(cyclopropylcarbamoyl)phenylboronic acid pinacol ester (168 mg, 0.585 mmol) and a 2M aqueous sodium hydrogen carbonate solution (0.75 mL) in a 5 mL-microwave reaction container, PdCl₂(PPh₃)₂ (11 mg) was added, capped and stirred by use of a Biotage Optimizer reaction apparatus at 130°C for 10 minutes. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by reverse-phase preparatory liquid chromatography (C18 column; water/acetonitrile/0.1% formic acid; 10-100% acetonitrile gradient) to obtain the titled compound (268 mg, 0.444 mmol, 88%).
1H-NMR (300 MHz, DMSO-d₆) δ 0.58-0.63 (m, 2H), 0.70-0.76 (m, 2H), 0.88-1.34 (m, 8H), 1.40 (s, 9H), 1.56-1.68 (m, 7H), 1.98 (m, 1H), 2.85-2.91 (m, 1H), 3.23 (t, J = 10.8 Hz, 2H), 3.83-3.87 (m, 2H), 7.69 (d, J = 8.1 Hz, 2H), 7.79 (s, 1H), 7.85 (s, 1H), 7.90 (t, J = 6.0 Hz, 1H), 7.99 (d, J = 8.1Hz, 2H), 8.55 (d, J = 4.2 Hz, 1H).

Example 1-241

### 4-(6-(Cyclohexylmethylamino)-8-((tetrahydro-2H-pyran-4-yl)methylamino)imidazo[1,2-a]pyrazin-3-yl)-N-cyclopropylbenzamide

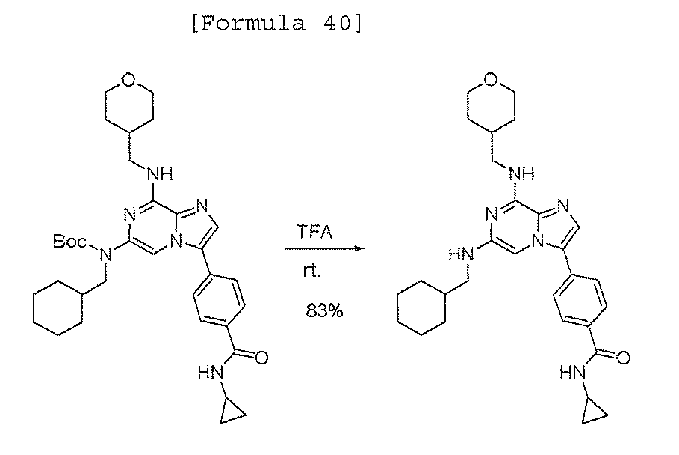

To tert-butyl cyclohexylmethyl(3-(4-(cyclopropylcarbonyl)phenyl)-8-((tetrahydro-2H-pyran-4-yl)methylamino)imidazo[1,2-a]pyrazin-6-yl)carbamate (17.7 mg, 0.0294 mmol), TFA (2.4 mL) was added and stirred for 15 minutes. To the reaction solution, sodium bicarbonate water and ethyl acetate were added and separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by reverse-phase preparatory liquid chromatography (C18 column; water/acetonitrile/0.1% formic acid; 10-100% acetonitrile gradient) to obtain the titled compound (12.2 mg, 0.0243 mmol, 83% .
MS (ESI) m/z = 503 (M+H)⁺.
LC/MS t_{R} = 2.06 min.

### Example 1-242

3-(4-(Cyclopropylcarbamoyl)phenyl)-8-((tetrahydro-2H-pyran-4-yl)methylamino)imidazo[1,2-a]pyrazine-6-carboxamide

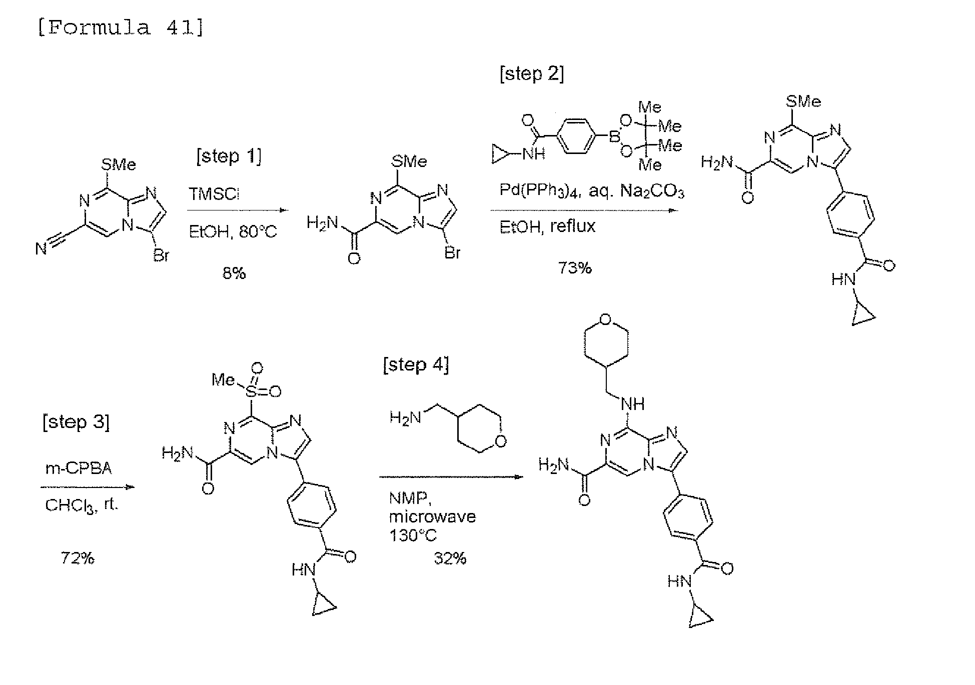

### Step 1: 3-Bromo-8-(methylthio)imidazo[1,2-a]pyrazine-6-carboxamide

To an ethanol (80 mL) solution of 3-bromo-8-(methylthio)imidazo[1,2-a]pyrazine-6-carbonitrile (1.61 g, 5.98 mmol), trimethylsilyl chloride (7.7 mL, 60 mmol) was added and then stirred at 80°C for 36 hours. The reaction solution was diluted with water and concentrated, the resultant solid substance was purified by medium-pressure silica gel chromatography (hexane/ethyl acetate; 10-100% ethyl acetate gradient) to obtain the titled compound (130 mg, 0.452 mmol, 8%).
MS (ESI) m/z = 287 (M+H)⁺.

### Step 2: 3-(4-(Cyclopropylcarbamoyl)phenyl)-8-(methylthio)imidazo[1,2-a]pyrazine-6-carboxamide

To an ethanol (2.6 mL) solution of 3-bromo-8-(methylthio)imidazo[1,2-a]pyrazine-6-carboxamide (130 mg, 0.45 mmol), 4-(cyclopropylcarbamoyl)phenylboronic acid pinacol ester (150 mg, 0.52 mmol) and a 2M aqueous sodium hydrogen carbonate solution (0.675 mL) in a 5 mL-microwave reaction container, Pd(PPh₃)₄ (16 mg, 0.0139 mmol) was added, capped, and stirred by use of a Biotage Optimizer reaction apparatus at 120 °C for 20 minutes. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by reverse-phase preparatory liquid chromatography (C18 column; water/acetonitrile /0.1% formic acid; 10-100% acetonitrile gradient) to obtain the titled compound (120 mg, 0.327 mmol, 73%)
1H-NMR (300 MHz, DMSO-d₆) δ 0.56-0.62 (m, 2H), 0.69-0.75 (m, 2H), 2.72 (s, 3H), 2.85-2.91 (m, 1H), 7.80 (d, J = 8.2 Hz, 2H), 7.85 (s, 1H), 8.01 (d, J = 8.2 Hz, 3H), 8.03 (s, 1H), 8.09 (s, 1H), 8.57 (d, J = 4.0 Hz, 1H), 8.65 (s, 1H).
MS (ESI) m/z = 368 (M+H)⁺.

### Step 3: 3-(4-(cyclopropylcarbamoyl)phenyl)-8-(methylsulfonyl)imidazo[1,2-a]pyrazine-6-carboxamide

To a chloroform (2.5 mL) solution of 3-(4-(cyclopropylcarbamoyl)phenyl)-8-(methylthio)imidazo[1,2-a]pyrazine-6-carboxamide (125 mg, 0.340 mmol), m-CPBA (235 mg, 1.02 mmol) was added at room temperature and stirred for 3 hours. To the reaction solution, an aqueous sodium carbonate solution was added and diluted with chloroform and then a precipitated solid substance was obtained by filtration and washed with chloroform to obtain the titled compound (98.4 mg, 0.246 mmol, 72%) as a light yellow solid substance. 1H-NMR (300 MHz, DMSO-d₆) δ 0.57-0.62 (m, 2H), 0.67-0.76 (m, 2H), 2.84-2.93 (m, 1H), 3.70 (s, 3H), 7.87 (d, J = 8.4 Hz, 2H), 8.00 (s, 1H), 8.04 (d, J = 8.4 Hz, 3H), 8.14 (s, 1H), 8.36 (s, 1H), 8.61 (d, J = 4.3 Hz, 1H), 9.07 (s, 1H).

### Step 4: Titled compound

To an NMP (0.93 mL) solution of 3-(4-(cyclopropylcarbamoyl)phenyl)-8-(methylsulfonyl)imidazo[1,2-a]pyrazine-6-carboxamide (93.0 mg, 0.233 mmol) in a 5 mL-microwave reaction container, (tetrahydro-2H-pyran-4-yl)methylamine (134 mg, 1.16 mmol) was added, capped, and stirred by use of a Biotage Optimizer reaction apparatus at 130 °C for 5 minutes. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by reverse-phase preparatory liquid chromatography (C18 column; water/acetonitrile/0.1% formic acid; 10-100% acetonitrile gradient) to obtain the titled compound (32.1 mg, 0.074 mmol, 32%) as a light yellow amorphous substance.
1H-NMR (300 MHz, DMSO-d₆) δ 0.55-0.62 (m, 2H), 0.66-0.73 (m, 2H), 1.24-1.36 (m, 2H), 1.63 (d, J = 11. 1 Hz, 2H), 1.91-2.01 (m, 1H), 2.81-2.90 (m, 1H), 3.21-3.29 (m, 2H), 3.48-3.52 (m, 2H), 3.81-3.86 (m, 2H), 7.58 (s, 1H), 7.73-7.77 (m, 1H), 7.83 (s, 1H), 7.90 (d, J = 8.4 Hz, 2H), 8.01 (d, J = 8.4 Hz, 2H), 8.33 (s, 1H), 8.46 (d, J = 4.3 Hz, 1H), 8.51 (s, 1H).
MS (ESI) m/z = 435 (M+H)⁺.
LC/MS t_{R} = 2.50 min.

[Table 1-7A]

**Table 1-7**

| | | |
|---|---|---|
| 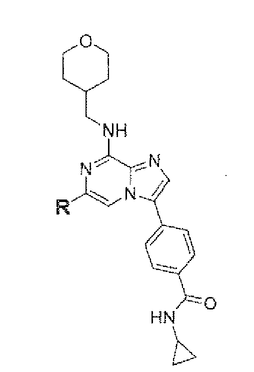 | | |

| Compounds described in Table 1-7 were synthesized in accordance with the processes of Examples mentioned above. | | |
|---|---|---|
| Example No. | R | property data |
| Example 1-243 | 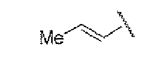 | MS (ESI) m/z = 432 (M+H)⁺. |
| | | LC/MS t_{R} = is min. |
| Example 1-244 | 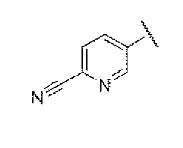 | 1H-NMR (300 MHz, DMSO-d₆) δ 0.56-0.62 (m, 2H), 0.68-0.75 (m, 2H), 1.22-1.35 (m, 2H), 1.60-1.69 (m, 2H), 2.02 (s, 1H), 2.88 (s, 1H), 3.20-3.30 (m, 3H), 3.50 (br s, 2H), 3.80-3.88 (m, 2H), 7.80-7.85 (m, 3H), 7.94-8.03 (m, 3H), 8.07 (d, J = 7,8 Hz, 1H), 6.43 (s, 1H), 8.55 (s, 1H), 8.59 (d, J = 6.3 Hz, 1 H), 9.37 (s, 1H), |
| | | MS (ESI) m/z = 494 (M+H)⁺. |
| | | LC/MS t_{R}= 1.70 min. |
| Example 1-245 | 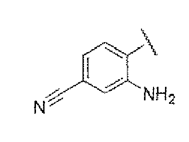 | 1H-NMR (300 MHz, DMSO-d₆) δ 0.58 (br s, 2H), 0.70-0.71 (m, 2H), 1.16-1.30 (m, 2H), 1.63 (d, J = 12.1 Hz, 2H), 1.95 (s, 1H), 2.88 (s, 1H), 3.23-3.26 (m, 2H), 3.39 (br s, 2H), 3.80-3.88 (m, 2H), 6.34 (s, 2H), 6.95 (d, J = 7.3 Hz, 1H), 7.06 (s, 1H), 7.53 (d, J = 7,6 Hz, 1H), 7.77-7.84 (m, 3H), 7.91 (s, 1H), 7.93-8,00 (m, 3H), 8.53 (s, 1H), |
| | | MS (ESI) m/z = 508 (M+H)⁺. |
| | | LC/MS t_{R} = 1.69 min. |

**[Table 1-7B]**

| Example No. | R | property data |
|---|---|---|
| Example 1-246 | 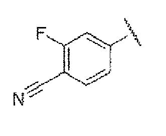 | 1H-NMR (300 MHz, DMSO-d₆) δ 0.60 (br s, 2H), 0.71-0.72 (m, 2H), 1.26-1.29 (m, 2H), 1.63 (d, J = 12.1 Hz, 2H), 1.99 (br s, 1H), 2.88 (br s, 1H), 3.24-3.26 (m, 2H), 3.47 (br s, 2H), 3.82-3.85 (m, 2H), 7.79 (br s, 3H), 7.84-7.93 (m, 2H), 8.00 (br s, 3H), 8.07 (d, J = 11.1 Hz, 1H), 8.32 (s, 1H), 8.55 (s, 1H). |
| | | MS (ESI) m/z =511 (M+H)⁺. |
| | | LC/MS t_{R} = 1.96 min. |
| Example 1-247 | 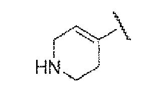 | MS (ESI) m/z = 473 (M+H)⁺. |
| | | LC/MS t_{R} 0.95 min. |
| Example 1-248 | 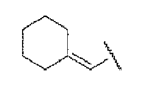 | MS (ESI) m/z = 486 (M+H)⁺. |
| | | LC/MS t_{R} = 1.89 min. |
| Example 1-249 | 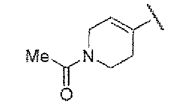 | MS (ESI) m/z = 515 (M+H)⁺. |
| | | LC/MS t_{R} = 1.38 min. |
| Example 1-250 | 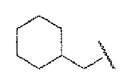 | MS (ESI) m/z = 488 (M+H)⁺. |
| | | LC/MS t_{R} = 1.74 min. |

### Example 1-251

N-cyclopropyl-4-(6-(piperidine-1-carbonyl)-8-((tetrahydro-2H-pyran-4-yl)methylamino)imidazo[1,2-a]pyrazin-3-yl)benzamide

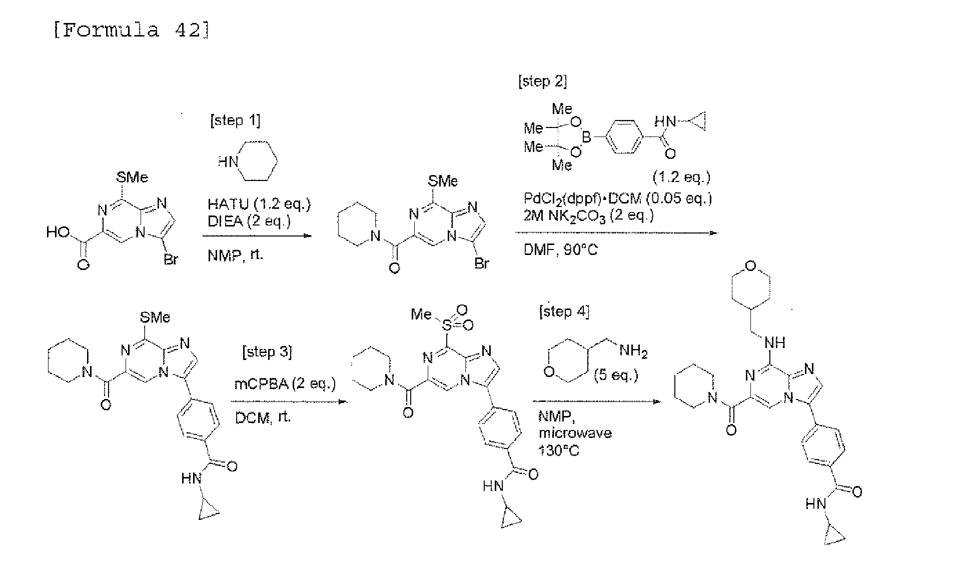

### Step 1: (3-bromo-8-(methylthio)imidazo[1,2-a]pyrazin-6-yl) (piperidin-1-yl)methanone

To an NMP (5.0 mL) solution of 3-bromo-8-(methylthio)imidazo[1,2-a]pyrazine-6-carboxylic acid (543 mg, 1.89 mmol), piperidine (0.279 mL, 2.83 mmol) and DIEA (0.658 mL, 3.77 mmol), HATU (860 mg, 2.26 mmol) was added and stirred at room temperature overnight. To the reaction solution, water and ethyl acetate were added to separate phases. The water phase was extracted with ethyl acetate and organic phases were combined, washed with saturated sodium bicarbonate water and saturated saline, and dried over magnesium sulfate. The organic phase was filtrated and then concentrated under reduced pressure. The resultant residue was purified by medium-pressure silica gel chromatography (hexane/ethyl acetate; 20-50% ethyl acetate gradient) to obtain the titled compound (562 mg, 1.58 mmol, 84%) as a white solid substance.
1H-NMR (400 MHz, DMSO-d₆) δ 1.50-1.67 (br m, 6H), 2.60 (s, 3H), 3.45-3.64 (m, 4H), 7.87 (s, 1H), 8.27 (s, 1H) .
MS (ESI) m/z = 355 (M+H)⁺.
LC/MS t_{R} = 1.64 min.

### Step 2: N-Cyclopropyl-4-(8-(methylthio)-6-(piperidine-1-carbonyl)imidazo[1,2-a]pyrazin-3-yl)benzamide

To a DMF (3.0 mL) solution of (3-bromo-8-(methylthio)imidazo[1,2-a]pyrazin-6-yl)(piperidin-1-yl)methanone (250 mg, 0.704 mmol), 4-(cyclopropylcarbamoyl)phenylboronic acid pinacol ester (242 mg, 0.844 mmol) and a 2M aqueous sodium hydrogen carbonate solution (0.704 mL), PdCl₂(dppf)/CH₂Cl₂ (28.7 mg, 0.035 mmol) was added and stirred at 90°C for 8 hours. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 50-100% ethyl acetate gradient) to obtain the titled compound (185 mg, 0.425 mmol, 60%) as a yellow solid substance. 1H-NMR (400 MHz, DMSO-d₆) δ 0.59 (br s, 2H), 0.67-0.74 (m, 2H), 1.52-1.67 (m, 6H), 2.61 (s, 3H), 2.87 (s, 1H), 3.57 (s, 4H), 7.77 (d, J = 8.1 Hz, 2H), 7.98-8.01 (m, 3H), 8.43 (s, 1H), 8.56 (br s, 1H).
MS (ESI) m/z = 436 (M+H)⁺.
LC/MS t_{R} = 1.56 min.

### Step 3: N-Cyclopropyl-4-(8-(methylsulfonyl)-6-(piperidine-1-carbonyl)imidazo[1,2-a]pyrazin-3-yl)benzamide

To a chloroform (3.0 mL) solution of N-cyclopropyl-4-(8-(methylthio)-6-(piperidine-1-carbonyl)imidazo[1,2-a]pyrazin-3-yl)benzamide (171 mg, 0.393 mmol), m-CPBA (203 mg, 1.18 mmol) was added at room temperature and stirred for 4 hours. To the reaction solution, saturated sodium bicarbonate water and ethyl acetate were added and separate phases. Thereafter, the water phase was extracted with ethyl acetate and organic phases were combined and washed with saturated sodium bicarbonate water and saturated saline, and dried over magnesium sulfate. The organic phase was filtrated and then concentrated under reduced pressure. The resultant residue was purified by medium-pressure silica gel chromatography (hexane/ethyl acetate; 50-100% ethyl acetate gradient) to obtain the titled compound (208 mg, 0.444 mmol, >100%) as a brown solid substance.
1H-NMR (400 MHz, DMSO-d₆) δ 0.60 (br s, 2H), 0.71-0.72 (m, 2H), 1.55-1.58 (br m, 7H), 2.72 (s, 1H), 2.88 (s, 2H), 3.55 (s, 3H), 3.63 (br s, 4H), 7.87 (d, J = 8.1 Hz, 2H), 8.03 (d, J = 8.1 Hz, 2H), 8.37 (s, 1H), 8.59 (s, 1H), 8.97 (s, 1H),
MS (ESI) m/z = 468 (M+H)⁺.
LC/MS t_{R} = 1.30 min.

### Step 4: Titled compound

To an NMP (3.0 mL) solution of N-cyclopropyl-4- (8-(methylsulfonyl)-6-(piperidine-1-carbonyl)imidazo[1,2-a]pyrazin-3-yl)benzamide (183 mg, 0.391 mmol) in a 5 mL-microwave reaction container, (tetrahydro-2H-pyran-4-yl)methylamine (0.239 mL, 1.96 mmol) was added, capped, and stirred by use of a Biotage Optimizer reaction apparatus at 130°C for 5 minutes. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was solidified with ethyl acetate/ether to obtain the titled compound (127 mg, 0.252 mmol, 64%).
1H-NMR (300 MHz, DMSO-d₆) δ 0.58 (br s, 2H), 0.68-0.70 (m, 2H), 1.20-1.23 (m, 2H), 1.49-1.66 (m, 11H), 1.97 (s, 1H), 2.86 (s, 1H), 3.22 (t, J = 11.5 Hz, 2H), 3.56 (s, 5H), 3.78-3.88 (m, 2H), 7.78 (s, 1H), 7.90 (d, J = 7.8 Hz, 2H), 8.01 (br s, 4H), 8.44 (s, 2H).
MS (ESI) m/z = 503 (M+H)⁺.
LC/MS t_{R} = 1.54 min.

### Example 1-252

N-Cyclohexyl-3-(4-(cyclopropylcarbamoyl)phenyl)-8-((tetrahydro-2H-pyran-4-yl)methylamino)imidazo[1,2-a]pyrazine-6-carboxamide

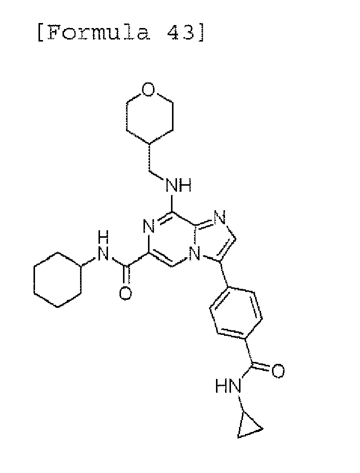

The titled compound was synthesized in accordance with the process of Example 1-251 (127 mg, 0.252 mmol).
1H-NMR (300 MHz, DMSO-d₆) δ 0.58 (br s, 2H), 0.66-0.74 (m, 2H), 1.16-1.73 (m, 12H), 1.81 (br s, 2H), 2.00 (br s, 1H), 2.85 (br s, 1H), 3.25 (t, J = 11.6 Hz, 2H), 3.46 (br s, 2H), 3.75-3.90 (m, 3H), 7.90-7.91 (m, 3H), 7.96-8.01 (m, 3H), 8.36 (s, 1H), 8.46 (s, 1H), 8.51 (s, 1H).
MS (ESI) m/z = 517 (M+H)⁺.
LC/MS t_{R} = 1.89 min.

(Example 2)

### Example 2-1

### 4-(6-Chloro-8-(isobutylamino)imidazo[1,2-b]pyridazin-3-yl)-N-cyclopropylbenzamide

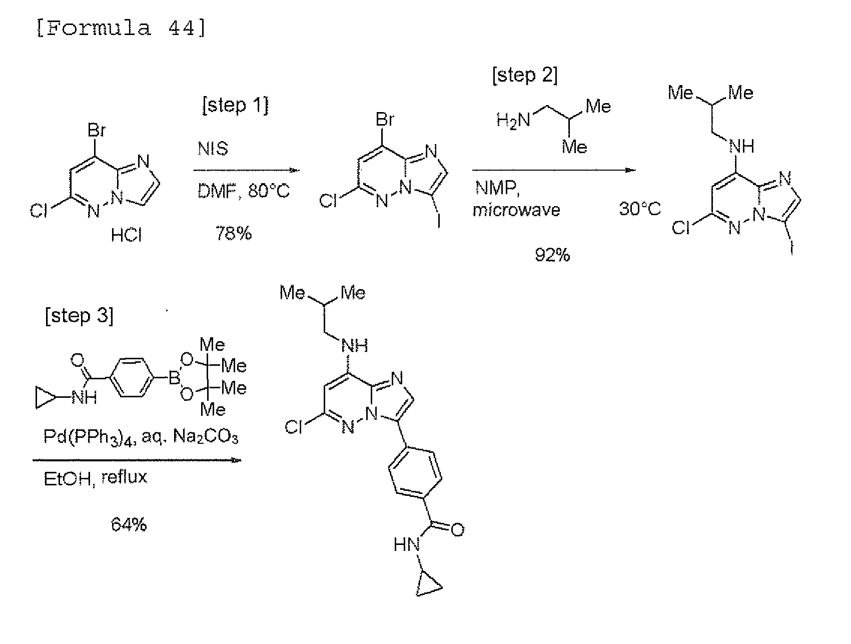

### Step 1: 8-Bromo-6-chloro-3-iodoimidazo[1,2-b]pyridazine

To a DMF (100 mL) solution of 8-bromo-6-chloroimidazo[1,2-b]pyridazine hydrochloride (10 g, 37.1 mol), NIS (9.58 g, 42.6 mmol) was added and stirred at 80°C for one hour. The reaction solution was poured in ice water to quench. The resultant solid substance precipitated was obtained by filtration. The resultant solid substance was purified by medium-pressure silica gel chromatography (hexane/ethyl acetate; 5-40% ethyl acetate gradient) to obtain the titled compound (9.11 g, 25.4 mmol, 68%) as a yellow solid substance. 1H-NMR (300 MHz, DMSO-d₆) δ 7.98 (s, 1H), 7.98 (s, 1H).

### Step 2: 6-Chloro-3-iodo-N-isobutylimidazo[1,2-b]pyridazine-8-amine

To an NMP (2.4 mL) solution of 8-bromo-6-chloro-3-iodoimidazo[1,2-b]pyridazine (600 mg, 1.67 mmol) in a 5 mL-microwave reaction container, isobutylamine (490 mg, 6.70 mmol) was added, capped, and stirred by use of a Biotage Optimizer reaction apparatus at 130°C for 10 minutes. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 10-60% ethyl acetate gradient) to obtain the titled compound (542 mg, 1.55 mmol, 92%) as a light yellow solid substance.
1H-NMR (300 MHz, DMSO-d₆) δ 0.88 (s, 3H), 0.90 (s, 3H), 1.94-1.96 (m, 1H), 3.11 (br s, 1H), 6.29 (s, 1H), 7.61 (s, 1H), 8.01-8.03 (br m, 1H).

### Step 3: Titled compound

To an ethanol (7.5 mL) solution of 6-chloro-3-iodo-N-isobutylimidazo[1,2-b]pyridazine-8-amine (500 mg, 1.43 mmol), 4-(cyclopropylcarbamoyl)phenylboronic acid pinacol ester (573 mg, 2.00 mmol) and a 2M aqueous sodium carbonate solution (1.78 mL), PdCl₂(PPh₃)₂ (100 mg, 0.143 mmol) was added and stirred for 20 hours while heating under reflux. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure to obtain the titled compound (352 mg, 0.917 mmol, 64%) as a light yellow solid substance.
1H-NMR (300 MHz, DMSO-d₆) δ 0.58-0.74 (m, 4H), 0.93 (d, 6H, J = 6.6 Hz), 2.00 (m, 1H), 2.85 (m, 1H), 3.16 (m, 2H), 6.34 (s, 1H), 7.91 (m, 2H), 8.06 (m, 1H), 8.08 (s, 1H), 8.15 (m, 2H), 8.48 (d, 1H, J = 3.9 Hz).
MS (ESI) m/z = 384 (M+H)⁺.
LC/MS t_{R} = 2.28 min.

Example 2-2

### 4-(6-(Cyclohexylamino)-8-(isobutylamino)imidazo[1,2-blpyridazin-3-yl)-N-cyclopropylbenzamide

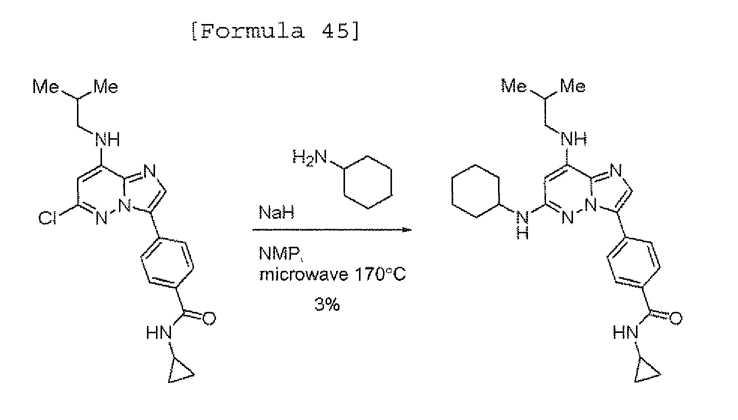

To a THF (30 mL) solution of 4-(6-chloro-8-(isobutylamino)imidazo[1,2-b]pyridazin-3-yl)-N-cyclopropylbenzamide (2.0 g, 5.21 mmol) and sodium hydride (60% wt, 1.0 g, 26.1 mmol), cyclohexylamine (1.29 g, 13.0 mmol) was added and stirred at room temperature for 30 minutes. The reaction solution was portioned and transferred to five 20 mL-microwave reaction containers and stirred by use of a Biotage Optimizer reaction apparatus at 170 °C for 40 minutes. To each of the reaction solutions, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by reverse-phase preparatory liquid chromatography (C18 column; water/acetonitrile /0.1% formic acid; 10-100% acetonitrile gradient) to obtain the titled compound (60.3 mg, 0.135 mmol, 3%) as a light yellow solid substance.
1H-NMR (300 MHz, DMSO-d₆) δ 0.56-0.74 (m, 4H), 0.93 (d, 2H, J = 6.6 Hz), 1.12-1.46 (m, 5H), 1.62-1.80 (m, 3H), 1.94-2.10 (m, 3H), 2.85 (m, 1H), 2.99 (m, 2H), 3.61 (m, 1H), 5.58 (s, 1H), 6.31 (d, 1H, J = 6.9 Hz), 6.89 (t, 1H, J = 5.7 Hz), 7.83 (s, 1H), 7.86 (m, 2H), 8.32 (m, 2H), 8.44 (d, 1H, J = 3.9 Hz)
MS (ESI) m/z = 447 (M+H)⁺.
LC/MS t_{R} = 1.98 min.

Example 2-3

### 4-(6-Chloro-8-((tetrahydro-2H-pyran-4-yl)methylamino)imidazo[1,2-b]pyridazin-3-yl)-N-cyclopropylbenzamide

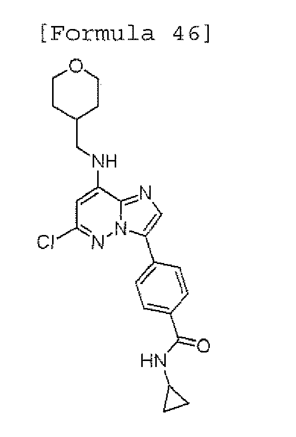

The titled compound was synthesized in accordance with the process of Example 2-1.
1H-NMR (300 MHz, DMSO-d₆) δ 0.58-0.74 (m, 4H), 1.20-1.33 (m, 2H), 1.60-1.65 (m, 2H), 1.95 (m, 1H), 2.86 (m, 1H), 3.20-3.33 (m, 4H), 3.85 (m, 2H), 6.40 (s, 1H), 7.91 (m, 2H), 8.07 (s, 1H), 8.09 (m, 1H), 8.15 (m, 2H), 8.48 (d, 1H, J = 3.9 Hz).
MS (ESI) m/z = 426 (M+H)⁺.
LC/MS t_{R} = 1.90 min.

Example 2-4

### 4-(6-(4-Carbamoylphenyl)-8-((tetrahydro-2H-pyran-4-yl)methylamino)imidazo[1,2-b]pyridazin-3-yl)-N-cyclopropylbenzamide

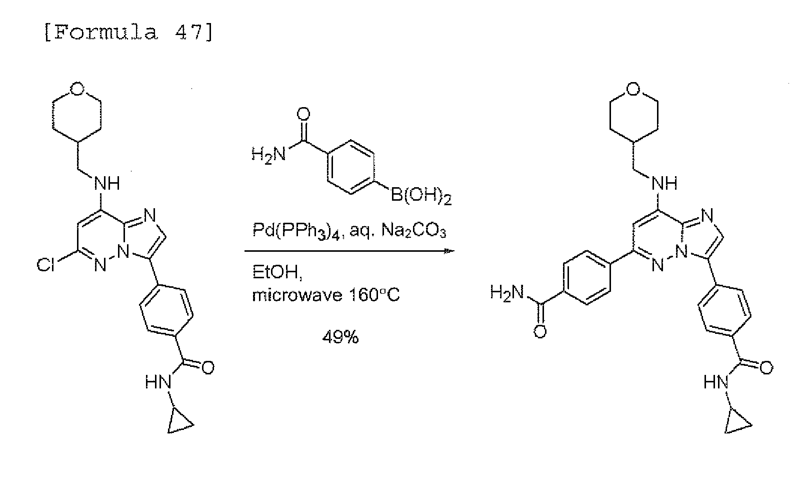

To an ethanol (0.30 mL) solution of 4-(6-chloro-8-((tetrahydro-2H-pyran-4-yl)methylamino)imidazo[1,2-b]pyridazin-3-yl)-N-cyclopropylbenzamide (130 mg, 0.305 mmol), 4-carbamoylphenylboronic acid (76 mg, 0.458 mmol) and a 2M aqueous sodium carbonate solution (0.458 mL) in a 5 mL-microwave reaction container, PdCl₂(dppf) (24.9 mg, 0.031 mmol) was added, capped, and stirred by use of a Biotage Optimizer reaction apparatus at 160°C for 10 minutes. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was solidified with chloroform to obtain the titled compound (76.4 mg, 0.150 mmol, 49%) as a light yellow solid substance. 1H-NMR (300 MHz, DMSO-d₆) δ 0.56-0.82 (m, 2H), 0.67-0.74 (m, 2H), 1.23-1.37 (m, 2H), 1.65-1.69 (m, 2H), 1.94-2.06 (m, 1H), 2.84-2.90 (m, 1H), 3.23-3.26 (m, 2H), 3.37-3.41 (m, 2H), 3.85 (dd, J = 11.1, 2.8 Hz, 2H), 6.78 (s, 1H), 7.48 (s, 1H), 7.66-7.70 (m, 1H), 7.94 (d, J = 8.5 Hz, 2H), 8.01 (d, J = 8.5 Hz, 2H), 8.08 (s, 1H), 8.11 (s, 1H), 8.15 (d, J = 8.5 Hz, 3H), 8.33 (d, J = 8.5 Hz, 2H), 8.47 (d, J = 4.3 Hz, 1H).
MS (ESI) m/z = 511 (M+H)⁺.
LC/MS t_{R} = 2.80 min.

Example 2-5

### 4-(6-(4-Cyanophenyl)-8-((tetrahydro-2H-pyran-4-yl)methylamino)imidazo[1,2-blpyridazin-3-yl)-N-cyclopropylbenzamide

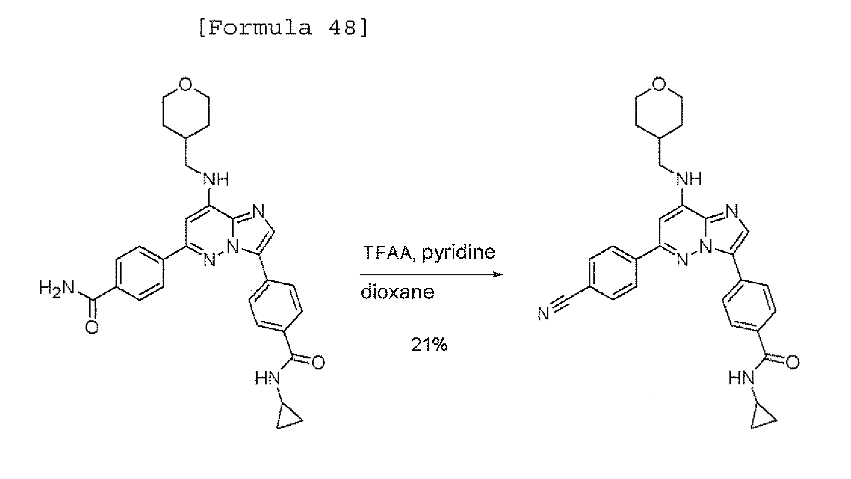

To a dioxane (0.64 mL) solution of 4-(6-(4-carbamoylphenyl)-8-((tetrahydro-2H-pyran-4-yl)methylamino)imidazo[1,2-b]pyridazin-3-yl)-N-cyclopropylbenzamide (64 mg, 0.125 mmol) and pyridine (70.4 mg, 0.890 mmol), TFAA (113 mg, 0.539 mmol) was added at 0°C and stirred at room temperature overnight. To the reaction solution, an aqueous potassium carbonate solution and ethyl acetate were added and separate phases. Thereafter, the water phase was extracted with ethyl acetate and organic phases were combined, washed with water and saturated saline, and dried over magnesium sulfate. The resultant solid substance was purified by medium-pressure silica gel chromatography (hexane/ethyl acetate; 10-100% ethyl acetate gradient) to obtain the titled compound (13.1 mg, 0.027 mmol, 21%) as a light yellow solid substance.
1H-NMR (300 MHz, DMSO-d₆) δ 0.56-0.63 (m, 2H), 0.67-0.74 (m, 2H), 1.22-1.36 (m, 2H), 1.66 (d, J = 11.9 Hz, 2H), 1.94-2.05 (m, 1H), 2.83-2.91 (m, 1H), 3.22-3.39 (m, 4H), 3.85 (d, J = 9.9 Hz, 2H), 6.82 (s, 1H), 7.75-7.80 (m, 1H), 7.95 (d, J = 8.4 Hz, 2H), 8.01 (d, J = 8.4 Hz, 2H), 8.12 (s, 1H), 8.26-8.31 (m, 4H), 8.48 (d, J = 4.1 Hz, 1H).

Example 2-6

### 4, 4'- (8- ((Tetrahydro-2H-pyran-4-yl)methylamino)imidazo[1,2-b]pyridazine-3,6-diyl)bis(N-cyclopropylbenzamide)

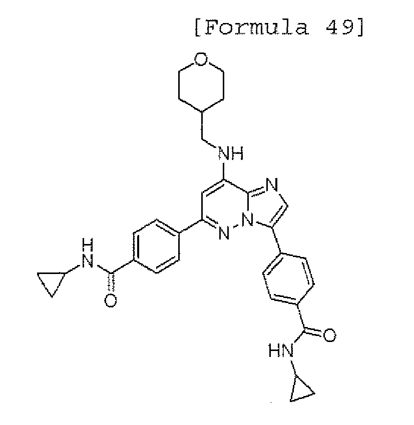

The titled compound was synthesized in accordance with the process of Example 2-4. MS (ESI) m/z = 551 (M+H)⁺. LC/MS t_{R} = 2.96 min.

[Table 2-1]

**Table 2-1**

| | | |
|---|---|---|
| 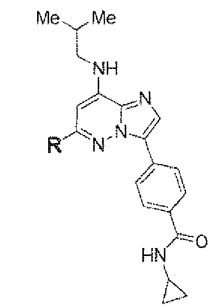 | | |

| Compounds described in Table 2-1 were synthesized in accordance with the process of Example 2-4. | | |
|---|---|---|
| Example No. | R | property data |
| Example 2-7 | 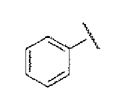 | 1H-NMR (400 MHz, DMSO-d₆) δ 0.56-0.76 (m, 4H), 0.97 (d, 6H, 6.6 Hz), 2.07 (m, 1H), 2.87 (m, 1H), 3.29 (dd, 2H, J=6.6, 6.3 Hz), 6.68 (s, 1H), 7.50-7.64 (m, 4H), 7.95 (m, 2H), 8.07 (m, 2H), 8.11 (s, 1H), 8.36 (m, 2H), 8.48 (d, 1H, J = 4.2 Hz). |
| | | MS (ESI) m/z = 426 (M+H)⁺. |
| | | LC/MS t_{R} = 2.50 min. |
| Example 2-8 | 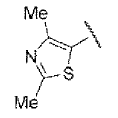 | MS (ESI) m/z = 461 (M+H)⁺. |
| | | LC/MS t_{R} = 2.28 min. |

[Table 2-2A]

**Table 2-2**

| | | |
|---|---|---|
| 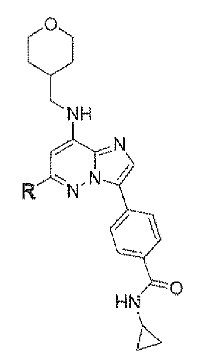 | | |

| Compounds described in Table 2-2 were synthesized in accordance with the process of Examples 2-5, 2-87 (where an adjacent position to N is a primary or secondary carbon) or Example 2-88 (where an adjacent position to N is a tertiary carbon), | | |
|---|---|---|
| Example No. | R | property data |
| Example 2-9 | 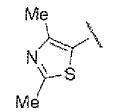 | MS (EST) m/z = 503 (M+H)⁺. |
| | | LC/MS t_{R} = 1.92 min, |
| Example 2-10 | 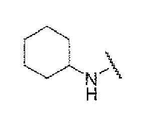 | 1H-NMR (300 MHz, DMSO-d₆) δ 0.56-0.75 (m, 4H), 1.13-1.48 (m, 8H), 1.57-1.80 (m, 2H), 1.72-1.84 (m, 2H), 1.94 (m, 1H), 2.02-2.12 (m, 2H), 2.85 (m, 1H), 3.08 (t, J = 6.6 Hz, 2H), 3.22-3.34 (m, 2H), 3.59 (m, 1H), 3.81-3.90 (m, 2H), 5.61 (s, 1H), 6.30 (d, J = 6.9 Hz, 1H), 6.94 (t, J = 6.3 Hz, 1H), 7.82 (s, 1H), 7.86 (do J = 8.4Hz, 2H), 8.32 (d, J = 8.4 Hz, 2H), 8.44 (d, J = 3.9 Hz, 1H). |
| | | MS (ESI) m/z = 489 (M+H)⁺. |
| | | LC/MS t_{R} = 1.59 min. |
| Example 2-11 | 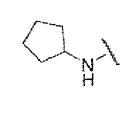 | 1H-NMR (300 MHz, DMSO-d₆) δ 0.56-0.74 (m, 4H), 1.15-1.30 (m, 2H), 1.48-1.78 (m, 8H), 1.86-2.08 (m, 3H), 2.87 (m, 1H), 3.08 (t, J = 6.6 Hz, 2H), 3.22-3.34 (m, 2H), 3.81-3.90 (m, 2H), 4.06 (m, 1H), 5.60 (s, 1H), 6.41(d, J = 5.7 Hz, 1H), 6.94 (t, J = 5.7 Hz, 1H), 7.81 (s, 1H), 7.87 (d, J = 8.4 Hz, 2H), 8.32 (d, J = 8.4 Hz, 2H), 8.41 (d, J = 4.5 Hz, 1H). |
| | | MS (ESI) m/z = 475 (M+H)⁺. |
| | | LC/MS t_{R} = 1.51 min. |

**[Table 2-2B]**

| Example No. | R | property data |
|---|---|---|
| Example 2-12 | 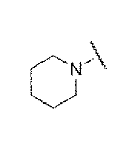 | 1H-NMR (300 MHz, DMSO-d₆) δ 0.55-0.74 (m, 4H), 1.17-1.34 (m, 2H), 1.53-1.70 (m, 8H), 1.94 (m, 1H), 2.88 (m, 1H), 3.22 (t, J = 6.6 Hz, 2H), 3.25-3.34 (m, 2H), 3.42-3.52 (m, 4H), 4.80-3.90 (m, 2H), 5.98 (s, 1H), 7.01 (t, J = 6.3 Hz. 1H), 7.88 (s, 1H), 7.88 (d, J = 8.4 Hz. 2H), 8.26 (d, J = 8.7 Hz, 2H), 8.43 (d, J = 4.2 Hz, 1H). |
| | | MS (ESI) m/z = 475 (M+H)⁺. |
| | | LC/MS t_{R} = 1.62 min. |
| Example 2-13 | 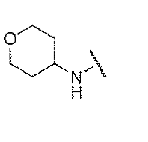 | 1H-NMR (300 MHz, DMSO-d₆) δ 0.56-0.75 (m, 4H), 1.14-1.31 (m, 2H), 1.4a-1.56 (m, 2H), 1.56-1.67 (m, 2H), 1.95 (m, 1H), 1.98-2.07 (m, 2H), 2.86 (m, 1H), 3.09 (t, J = 6.3 Hz, 2H), 3.22-3.32 (m, 2H), 3.42-3.54 (m, 2H), 3.76-3.97 (m, 5H), 5.60 (s, 1H), 6.44 (d, J = 6.9 Hz, 1H), 7.00 (t, J = 6.3 Hz, 1H), 7.83 (s, 1H), 7.87 (d, J = 8.4 Hz, 2H), 8.29 (d, J = 8.4 Hz, 2H), 8.43 (d, J = 3.9 Hz, 1H). |
| | | MS (ESI) m/z = 491 (M+H)⁺. |
| | | LC/MS t_{R} = 1.18 min. |
| Example 2-14 | 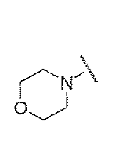 | 1H-NMR (300 MHz. DMSO-d₆) δ 0.56-0.74 (m, 4H), 1.16-1.34 (m, 2H), 1.58-1.68 (m, 2H), 1.95 (m, 1H), 2.87 (m, 1H), 3.23 (t, J = 6.3 Hz, 2H), 3.26-3.32 (m, 2H), 3.39-3.48 (m, 4H), 3.72-3.80 (m, 4H), 3.81-3.90 (m, 2H), 6.01 (s, 1H), 7.14 (t, J = 6.0 Hz, 1H), 7.89 (d, J = 8.1 Hz, 2H), 7.91 (s, 1H), 8.24 (d, J = 8.4 Hz, 2H), 8.43 (d, J = 4.2 Hz.1H). |
| | | MS (ESI) m/z = 477 (M+H)⁺. |
| | | LC/MS t_{R} = 1.27 min. |
| Example 2-15 | 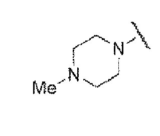 | 1H-NMR (400 MHz. DMSO-d₆) δ 0.56-0.76 (m, 4H), 0.97 (d, 6H, 6.6 Hz). 2.07 (m, 1H), 2.87 (m, 1H), 3.29 (dd, 2H, J=6.6. 6.3 Hz), 6.68 (s, 1H), 7.50-7.64 (m, 4H), 7.95 (m, 2H), 8.07 (m, 2H), 8.11 (s, 1H), 8.36 (m, 2H), 8.48 (d, 1H, J = 4.2 Hz) |
| | | MS (ESI) m/z = 490 (M+H)⁺. |
| | | LC/MS t_{R} = 0.89 min. |

**[Table 2-2C]**

| Example No. | R | property data |
|---|---|---|
| Example 2-16 | 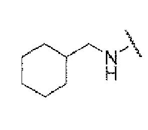 | 1H-NMR (300 MHz. DMSO-d₆) δ 0.54-0.74 (m, 4H), 0.30-1.07 (m, 2H), 1.12-1.32 (m, 4H), 1.57-1.88 (m, 7H), 1.94 (m, 1H), 1.94 (m, 1H), 2.85 (m, 1H), 3.04-3.14 (m, 4H), 3.80-3.92 (m, 2H), 5.63 (s, 1H), 6.44 (t, J = 5.4 Hz, 1H), 6.90 (t, J = 6.0 Hz, 1H), 7.81 (s, 1H), 7.85 (d, J = 8.4 Hz, 2H), 8.30 (d, J = 8.4 Hz, 2H), 8.43 (d, J = 4.2 Hz, 1H). |
| | | MS (ESI) m/z = 503 (M+H)⁺. |
| | | LC/MS t_{R} = 1.96 min. |
| Example 2-17 | 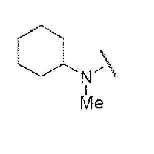 | 1H-NMR (300 MHz, DMSO-d₆) δ 0.56-0.75 (m, 4H), 0.90-1.07 (m, 2H), 1.10-2.06 (m, 14H), 2.85 (m, 1H), 2.91 (s, 3H), 3.16-3.30 (m, 4H), 3.80-3.90 (m, 2H), 4.13 (m, 1H), 5.79 (s, 1H), 7.00 (t, J = 6.3 Hz, 1H), 7.87 (d, J = 8.4 Hz. 2H), 7.88 (s, 1H), 7.85 (d, J = 8.4 Hz. 2H), 8.31 (d, J = 8.4 Hz, 2H), 8.44 (d, J = 3.9 Hz, 1H). |
| | | MS (ESI) m/z = 503 (M+H)⁺. |
| | | LC/MS t_{R} = 1.77 min. |
| Example 2-18 | 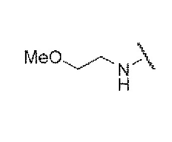 | 1H-NMR (300 MHz, DMSO-d₆) δ 0.54-0.74 (m, 4H), 1.14-1.32 (m, 2H), 1.56-1.67 (m, 2H), 1.94 (m, 1H), 2.86 (m, 1H), 3.09 (t, J = 6.0 Hz, 2H), 3.22-3.30 (m, 2H), 3.31 (s, 3H), 3.37-3.46 (m, 2H), 3.56 (t, J = 6.0 Hz, 2H), 3.82-3.92 (m, 2H), 5.68 (s, 1H), 6.43 (t, J = 5.4 Hz, 1H), 6.97 (t, J = 6.0 Hz, 1H), 7.81 (s, 1H), 7.87 (d, J = 8.4 Hz, 2H), 8.28 (d, J = 8.4 Hz, 2H), 8.41 (d, J = 4.2 Hz, 1H). |
| | | MS (ESI) m/z = 465 (M+H)⁺. |
| | | LC/MS t_{R} = 1.1 min. |
| Example 2-19 | 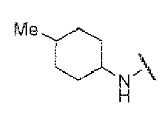 | 1H-NMR (300 MHz, DMSO-d₆) δ 0.55-0.75 (m, 4H), 0.93 and 0.95 (each s, total 3H), 1.00-2.06 (m, 13H), 2.14 (m, 1H), 2.86 (m, 1H), 3.04-3.14 (m, 2H), 3.20-3.34 (m, 2H), 3.53 (m, 1H), 3.82-3.92 (m, 2H), 5.74 and 5.59 (each s, total 1H), 6.26 (d, J = 6.9 Hz, 1H), 6.97 (d, J = 7.8 Hz, 1H), 7.78-7.90 (m, 3H), 8.26-8.35 (m, 2H), 8.40 and 8.45 (each d, J = 4.4 Hz, total 1H). |
| | | MS (ESI) m/z = 503 (M+H)⁺. |
| | | LC/MS t_{R} = 1.77 min. |

**[Table 2-2D]**

| Example No. | R | property data |
|---|---|---|
| Example 2-20 | 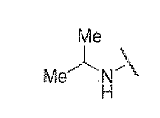 | 1H-NMR (300 MHz, DMSO-d₆) δ 0.55-0.75 (m, 4H), 1.12-1.33 (m, 2H), 1.22 (d, J = 6.3 Hz, 6H), 1.58-1.68 (m, 2H), 1.95 (m, 1H), 2.86 (m, 1H), 3.09 (t, J = 5.4 Hz, 2H), 3.20-3.32 (m, 2H), 3.81-3.90 (m, 2H), 3.93 (m, 1H), 5.59 (s, 1H), 6.24 (d, J = 7.2 Hz, 1H), 6.30 (t, J = 6.0 Hz, 1H), 7.81 (s, 1H), 7.87 (d, J = 8.4 Hz, 2H), 8.29 (d, J = 8.4 Hz, 2H), 8.40 (d, J = 4.5 Hz, 1H). |
| | | MS (ESI) m/z = 449 (M+H)⁺. |
| | | LC/MS t_{R} = 1.39 min. |
| Example 2-21 | 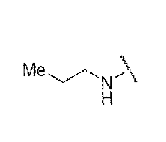 | 1H-NMR (300 MHz, DMSO-d₆) δ 0.55-0.75 (m, 4H), 0.98 (t, J = 7.5 Hz, 3H), 1.15-1.32 (m, 2H), 1.56-1.72 (m, 4H), 1.95 (m, 1H), 2.86 (m, 1H), 3.09 (t, J = 6.6 Hz, 2H), 3.15-3.30 (m, 4H), 3.81-3.90 (m, 2H), 5.61 (s, 1H), 6.41 (t, J = 5.4 Hz, 1H), 6.93 (t, J = 6.0 Hz, 1H), 7.81 (s, 1H), 7.87 (d, J = 8.4 Hz, 2H), 8.30 (d, J = 8.4 Hz, 2H), 8.42 (d, J = 3.9 Hz, 1H), |
| | | MS (ESI) m/z = 449 (M+H)⁺. |
| | | LC/MS t_{R} = 1.38 min. |
| Example 2-22 | 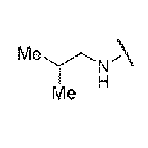 | 1H-NMR (300 MHz, DMSO-d₆) δ 0.55-0.75 (m, 4H), 0.97 (d, J = 6.9 Hz, 6H), 1.15-1.32 (m, 2H), 1.58-1.68 (m, 2H), 1.86-2.06 (m, 2H), 2.86 (m, 1H), 3.02-3.13 (m, 4H), 3.22-3.30 (m, 2H), 3.81-3.90 (m, 2H), 5.64 (s, 1H), 6.48 (t, J = 5.4 Hz, 1H), 6.92 (t, J = 6.0 Hz, 1H), 7.81 (s, 1H), 7.86 (d, J = 8.4 Hz, 2H), 8.30 (d, J = 8.4 Hz, 2H), 8.42 (d, J = 3.9 Hz, 1H). |
| | | MS (ESI) m/z = 463 (M+H)⁺, |
| | | LC/MS t_{R} = 1.49 min, |
| Example 2-23 | 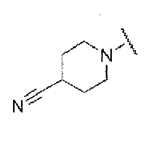 | 1H-NMR (300 MHz, DMSO-d₆) δ 0.55-0.75 (m, 4H), 1.17-1.35 (m, 2H), 1.58-1.69 (m, 2H), 1.75-2.08 (m, 5H), 2.86 (m, 1H), 3.12 (m, 1H), 3.18-3.40 (m, 6H), 3.68-3.80 (m, 2H), 3.81-3.90 (m, 2H), 6.02 (s, 1H), 7.09 (t, J = 5.7 Hz, 1H), 7.89 (s, 1H), 7.89 (d, J = 8.4 Hz, 2H), 8.23 (d, J = 8.4 Hz, 2H), 8.42 (d, J = 3.9 Hz, 1H), |
| | | MS (ESI) m/z = 500 (M+H)⁺, |
| | | LC/MS t_{R} = 1.38 min. |

**[Table 2-2E]**

| Example No. | R | property data |
|---|---|---|
| Example 2-24 | 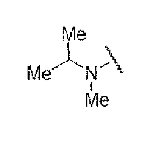 | 1H-NMR (300 MHz, DMSO-d₆) δ 0.55-0.75 (m, 4H), 1.12-1.35 (m, 2H), 1.19 (d, J = 6.6 Hz, 6H), 1.58-1.70 (m, 2H), 1.96 (m, 1H), 2.86 (m, 1H), 2.86 (s, 3H), 3.20-3.30 (m, 4H), 3.82-3.92 (m, 2H), 4.56 (m, 1H), 5.84 (s, 1H), 6.97 (t, J = 6.0 Hz, 1H), 7.87 (s, 1H), 7.88 (d, J = 8.4 Hz, 2H), 8.28 (d, J = 8.4 Hz, 2H), 8.42 (d, J = 4.2 Hz, 1H). |
| | | MS (ESI) m/z = 463 (M+H)⁺. |
| | | LO/MS t_{R} = 1.50 min, |
| Example 2-25 | 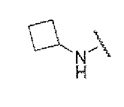 | 1H-NMR (300 MHz, DMSO-d₆) δ 0.56-0.75 (m, 4H), 1.14-1.32 (m, 2H), 1.57-1.67 (m, 2H), 1.68-1.84 (m, 2H), 1.85-2.02 (m, 3H), 2.30-2.46 (m, 2H), 2.87 (m, 1H), 3.09 (t, J = 6.6 Hz, 2H), 3.22-3.32 (m, 2H), 3.80-3.91 (m, 2H), 4.18 (m, 1H), 5.56 (s, 1H), 6.67 (d, J = 6.6 Hz, 1H), 6.97 (t, J = 6.0 Hz, 1H), 7.81 (s, 1H), 7.89 (d, J = 8.4 Hz, 2H), 8.30 (d, J = 8.4 Hz, 2H), 8.42 (d, J = 3.9 Hz, 1H), |
| | | MS (ESI) m/z = 461 (M+H)⁺. |
| | | LC/MS t_{R} = 1.43 min, |
| Example 2-26 | 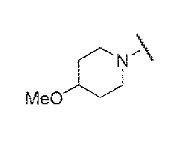 | 1H-NMR (300 MHz, DMSO-d₆) δ 0.56-0.74 (m, 4H), 1.16-1.35 (m, 2H), 1.45-1.57 (m, 2H), 1.58-1.68 (m, 2H), 1.86-2.02 (m, 3H), 2.86 (m, 1H), 3.11-3.30 (m, 6H), 3.28 (s, 3H), 3.42 (m, 1H), 3.78-3.91 (m, 4H), 6.01 (s, 1H), 7.03 (t, J = 6.6 Hz, 1H), 7.88 (s, 1H), 7.89 (d, J = 8.4 Hz, 2H), 8.24 (d, J = 8.4 Hz, 2H), 8.42 (d, J = 4.2 Hz, 1H). |
| | | MS (ESI) m/z = 505 (M+H)⁺. |
| | | LC/MS t_{R} = 1.39 min. |
| Example 2-27 | 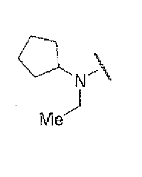 | 1H-NMR (300 MHz, DMSO-d₆) δ 0.55-0.75 (m, 4H), 1.20 (t, J = 6.9 Hz, 3H), 1.16-1.36 (m, 2H), 1.51-1.82 (m, 8H), 1.86-2.04 (m, 3H), 2.85 (m, 1H), 3.16-3.34 (m, 4H), 3.43 (q, J = 6.9 Hz, 2H), 3.80-3.90 (m, 2H), 4.51 (m, 1H), 5.77 (s, 1H), 6.98 (t, J = 6.0 Hz, 1H), 7.87 (s, 1H), 7.87 (d, J = 8.7 Hz, 2H), 8.29 (d, J = 8.7 Hz, 2H), 8.43 (d, J = 4.2 Hz, 1H). |
| | | MS (ESI) m/z = 503 CM+H)⁺. |
| | | LC/MS t_{R} = 1.75 min, |

**[Table 2-2F]**

| Example No. | R | property data |
|---|---|---|
| Example 2-28 | 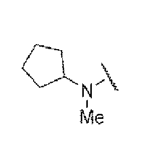 | 1H-NMR (300 MHz, DMSO-d₆) δ 0.56-0.74 (m, 4H), 1.18-1.36 (m, 2H), 1.53-1.96 (m, 11H), 2.83 (m, 1H), 2.90 (s, 3H), 3.18-3.34 (m, 4H), 3.80-3.90 (m, 2H), 4.68 (m, 1H), 5.86 (s, 1H), 6.97 (t, J = 6.0 Hz, 1H), 7.87 (s, 1H), 7.87 (d, J = 8.4 Hz, 2H), 8.29 (d, J = 8.4 Hz, 2H), 8.42 (d, J = 4.2 Hz, 1H), |
| | | MS (ESI) m/z = 489 (M+H)⁺. |
| | | LC/MS t_{R} = 1.89 min, |
| Example 2-29 | 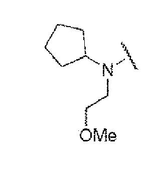 | 1H-NMR (300 MHz, DMSO-d₆) δ 0.54-0.74 (m, 4H), 1.16-1.34 (m, 2H), 1.50-1.80 (m, 8H), 1.86-2.04 (m, 3H), 2.85 (m, 1H), 3.16-3.32 (m, 4H), 3.30 (s, 3H), 3.54 (brs, 4H), 3.80-3.90 (m, 2H), 4.49 (m, 1H), 5.90 (s, 1H), 7.05 (t, J = 6.0 Hz, 1H), 7.86 (d, J = 8.7 Hz, 2H), 7.88 (s, 1H), 8.27 (d, J = 8.7 Hz, 2H), 8.44 (d, J = 4.2 Hz, 1H). |
| | | MS (ESI) m/z = 533 (M+H)⁺. |
| | | LC/MS t_{R} = 1.70 min, |
| Example 2-30 | 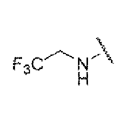 | 1H-NMR (300 MHz, DMSO-d₆) δ 0.54-0.74 (m, 4H), 1.15-1.32 (m, 2H), 1.56-1.68 (m, 2H), 1.95 (m, 1H), 2.86 (m, 1H), 3.12 (t, J = 6.6 Hz, 2H), 3.22-3.34 (m, 2H), 3.80-3.91 (m, 2H), 4.05-4.20 (m, 2H), 5.71 (s, 1H), 7.03 (t, J = 6.0 Hz, 1H), 7.22 (t, J = 6.0 Hz, 1H), 7.85 (s, 1H), 7.88 (d, J = 8.4 Hz, 2H), 8.23 (d, J = 8.4 Hz, 2H), 8.45 (d, J = 4.2 Hz,1H). |
| | | MS (ESI) m/z = 489 (M+H)⁺. |
| | | LC/MS t_{R} = 1.38 min. |
| Example 2-31 | 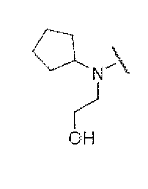 | 1H-NMR (300 MHz, DMSO-d₆) δ 0.55-0.75 (m, 4H), 1.18-1.33 (m, 2H), 1.50-1.80 (m, 8H), 1.85-2.00 (m, 3H), 2.15 (m, 1H), 2.33 (m, 1H), 2.85 (m, 1H), 3.35-3.65 (m, 6H), 3.80-4.24 (m, 2H), 4.43-4.58 (m, 2H), 4.79 (t, J = 5.1 Hz, 1H), 5.62 (s, 1H), 7.87 (d, J = 8.7 Hz, 2H), 7.89 (s, 1H), 8.28 (d, J = 8.7 Hz, 2H), 8.45 (d, J = 3.9 Hz, 1H). |
| | | MS (ESI) m/z = 519 (M+H)⁺. |
| | | LC/MS t_{R} = 1.32 min. |

**[Table 2-2G]**

| Example No. | R | property data |
|---|---|---|
| Example 2-32 | 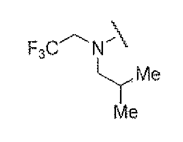 | 1H-NMR (300 MHz, DMSO-d₆) δ 0.55-0.75 (m, 4H), 0.92 (d, J = 6.6 Hz, 6H), 1.16-1.34 (m, 2H), 1.56-1.68 (m, 2H), 1.93 (m, 1H), 2.18 (m, 1H), 2.85 (m, 1H), 3.17-3.32 (m, 4H), 3.42 (d, J = 6.9 Hz, 2H), 3.78-3.90 (m, 2H), 4.47 (q. J = 9.0 Hz, 2H), 5.91 (s, 1H), 7.24 (t, J = 6.0 Hz, 1H), 7.88 (d, J = 8.4 Hz, 2H), 7.90 (s, 1H), 8.22 (d, J = 8.4 Hz, 2H), 8.46 (d, J = 3.9 Hz, 1H). |
| | | MS (ESI) m/z = 545 (M+H)⁺. |
| | | LC/MS t_{R} = 1.88 min, |
| Example 2-33 | 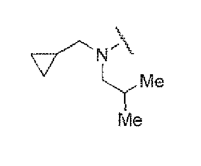 | 1H-NMR (300 MHz, DMSO-d₆) δ 0.28-0.35 (m, 2H), 0.44-0.52 (m, 2H), 0.55-0.75 (m, 4H), 0.94 (d, J = 6.6 Hz, 6H), 1.08-1.35 (m, 3H), 1.57-1.69 (m, 2H), 1.94 (m, 1H), 2.13 (m, 1H), 2.85 (m, 1H), 3.17-3.38 (m, 6H), 3.42 (d, J = 6.0 Hz, 2H), 3.80-3.90 (m, 2H), 5.77 (s, 1H), 7.04 (t, J = 6.3 Hz, 1H), 7.86 (d, J = 8.7 Hz, 2H), 7.88 (s, 1H), 8.31 (d, J = 8.7 Hz, 2H), 8.45 (d, J = 3.9 Hz, 1H). |
| | | MS (ESI) m/z = 517 (M+H)⁺. |
| | | LC/MS t_{R} = 1.86 min, |
| Example 2-34 | 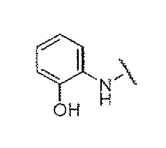 | 1H-MMR (300 MHz, DMSQ-d₆) δ 0.55-0.76 (m, 4H), 1.16-1.36 (m, 2H), 1.58-1.72 (m, 2H), 1.99 (m, 1H), 2.85 (m, 1H), 3.10-3.20 (m, 2H), 3.23-3.34 (m, 2H), 3.82-3.93 (m, 2H), 6.30 (s, 1H), 6.79-6.92 (m, 3H), 7.21 (t, J = 6.0 Hz, 1H), 7.84-7.94 (m, 4H), 8.16 (m, 1H), 8.23 (d, J = 8.4 Hz, 2H), 8.48 (d, J = 3.9 Hz, 1H), 9.79 (s, 1H). |
| | | MS (ESI) m/z = 499 (M+H)⁺. |
| | | LC/MS t_{R} = 1.38 min. |
| Example 2-35 | 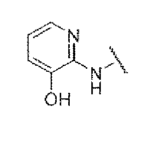 | 1H-NMR (300 MHz, DMSO-d₆) δ 0.53-0.74 (m, 4H), 1.16-1.34 (m, 2H), 1.58-1.72 (m, 2H), 1.96 (m, 1H), 2.82 (m, 1H), 3.18-3.32 (m, 4H), 3.82-3.92 (m, 2H), 6.13 (s, 1H), 7.35 (d, J = 7.5 Hz, 1H), 7.45 (m, 1H), 7.62 (d, J = 8.7 Hz, 2H), 7.64-7.72 (m, 2H), 7.86 (d, J = 8.7 Hz, 2H), 7.94 (s, 1H), 7.97 (m, 1H), 8.41 (d, J = 3.9 Hz, 1H), 12.75 (brs, 1H), |
| | | MS (ESI) m/z = 500 (M+H)⁺. |
| | | LC/MS t_{R} = 1.05 min, |
| Example 2-36 | 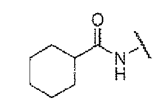 | 1H-NMR (300 MHz, DMSO-d₆) δ |
| | | MS (ESI) m/z = (M+H)⁺. |
| | | LC/MS t_{R} = min. |

**[Table 2-2H]**

| Example No. | R | property data |
|---|---|---|
| Example 2-37 | 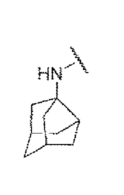 | 1H-NMR (300 MHz, DMSO-d₆) δ 0.59-0.69 (m, 2H), 0.82-0.92 (m, 2H), 1.28-1.47 (m, 2H), 1.49-1.79 (m, 6H), 1.81-1.97 (m, 1H), 1.97-2.18 (m, 6H), 2.24-2.34 (m, 2H), 2.41-2.52 (m, 1H), 2.84-2.97 (m, 1H), 3.05-3.18 (m, 2H), 3.29-3.42 (m, 2H), 3.99 (dd, J = 11.54, 4.39 Hz, 2H), 4.35-4.44 (m, 1H), 5.34-5.41 (m, 1H), 5.48-5.59 (m, 1H), 6.19-6.30 (m, 1H), 7.54-7.62 (m, 1H), 7.77 (d, J = 8.52 Hz, 2H), 8.06 (d, J = 8.52 Hz, 2H). |
| | | MS (ESI) m/z = 527 (M+H)⁺. |
| | | LC/MS t_{R} = 1.90 min, |
| Example 2-38 | 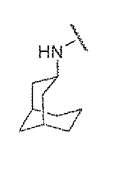 | 1H-MMR (300 MHz, DMSO-d₆) δ 0.53-0.63 (m, 2H), 0.65-0.76 (m, 2H), 1.13-1.32 (m, 2H), 1.36-1.68 (m, 5H), 1.68-1.83 (m, 4H), 1.86-2.06 (m, 4H), 2.09-2.24 (m, 2H), 2.77-2.89 (m, 1H), 3.03-3.13 (m, 2H), 3.20-3.29 (m, 2H), 3.80-3.93 (m, 2H), 4.49-4.72 (m, 1H), 5.59(s, 1H), 6.1 (d, J = 7.69 Hz, 1H), 6.88-7.00 (m, 1H), 7.81 (d, J = 8.79 Hz, 2H), 7.87 (s, 1H), 8.37 (d, J = 8.24 Hz, 2H), 8.45 (d, J = 3.85 Hz, 1H), |
| | | MS (ESI) m/z = 529 (M+H)⁺. |
| | | LC/MS t_{R} = 2.01 min, |
| Example 2-39 | 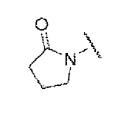 | 1H-NMR (400 MHz, CDCl₃) δ 0.62-0.67 (m, 2H), 0.86-0.93 (m, 2H), 1.39-1.50 (m, 2H), 1.73-1.81 (m, 2H), 1.94-2.04 (m, 1H), 2.14-2.23 (m, 2H), 2.69 (t, J = 8.1 Hz, 2 H), 2.90-2.98 (m, 1H), 3.31 (t, J = 6.1 Hz, 2H), 3.40 (dt, J = 2.0, 11.7 Hz, 2H), 4.01 (dd, J = 3.0, 11.7 Hz, 2H), 4.12 (t, J = 7.1 Hz, 2H), 5.83 (t, J = 5.6 Hz, 1H), 6.25 (s, 1H), 7.51 (s, 1H), 7.79 (s, 1H), 7.83 (d, J = 8.6 Hz, 2H), 8.13 (d, J = 8.6 Hz, 2H), |
| | | MS (ESI) m/z = 475 (M+H)⁺. |
| | | LC/MS t_{R} = 1.57 min, |
| Example 2-40 | 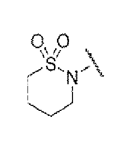 | 1H-NMR (300 MHz, DMSO-d₆) δ 0.62-0.68 (m, 2H), 0.88-0.94 (m, 2H), 1.37-1.49 (m, 2H), 1.75 (d, J = 13.2 Hz, 2H), 1.92-2.07 (m, 3H), 2.32-2.40 (m, 2H), 2.90-2.98 (m, 1H), 3.22-3.30 (m, 4H), 3.40 (t, J = 11.7 Hz, 2H), 3.98-4.10 (m, 4H), 5.92 (t, J = 5.6 Hz, 1H), 6.24 (s, 1H), 6.26 (s, 1H), 7.79 (s, 1H), 7.83 (d, J = 8.1 Hz, 2H), 8.06 (d, J = 8.1 Hz, 2H). |
| | | MS (ESI) m/z = 525 (M+H)⁺. |
| | | LC/MS t_{R} = 1.69 min, |

**[Table 2-2I]**

| Example No. | R | property data |
|---|---|---|
| Example 2-41 | 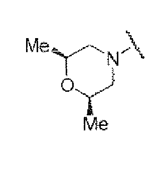 | 1H-NMR (400 MHz, DMSO-d₆) δ 0.60 (s, 2H), 0.71 (d, J = 5.6 Hz, 2H), 1.19 (d, J = 5.6 Hz, 6H), 1.26-1.29 (m, 2H), 1.65 (d, J = 12.0 Hz, 2H), 1.95 (s, 1H), 2.48 (t, J = 11.2 Hz, 3H), 2.87 (s, 1H), 3.24-3.30 (m, 2H), 3.70 (s, 2H), 3.86 (d, J = 9.6 Hz, 2H), 4.00 (d, J = 12.0 Hz, 2H), 6.02 (s, 1H), 7.03 (s, 1H), 7.91 (s, 3H), 8.25 (d, J = 8.0 Hz, 2H), 8.48 (s, 1H). |
| | | MS (E5I) m/z = 505 (M+H)⁺. |
| | | LC/MS t_{R} = 1.52 min. |
| Example 2-42 | 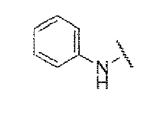 | 1H-NMR (400 MHz, DMSO-d₆) δ 0.65 (s, 2H), 0.71 (s, 2H), 1.27 (s, 2H), 1.68 (s, 2H), 1.99 (s, 1H), 2.89 (s, 1H), 3.17 (s, 2H), 3.29 (s, 1H), 3.85 (s, 2H), 6,12 (s, 1H), 6.93 (s, 1H), 7.33 (s, 2H), 7.42 (s, 1H), 7.71 (s, 2H), 7.98 (s, 3H), 8.22 (s, 2H), 8.64 (s, 1H), 9.35 (s, 1H). |
| | | MS (ESI) m/z = 483 (M+H)⁺. |
| | | LC/MS t_{R} = 1.54 min. |
| Example 2-43 | 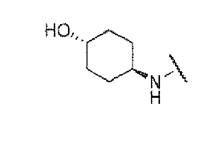 | 1H-NMR (400 MHz, DMSO-d₆) δ 0.60 (s, 2H), 0.71 (d, J = 5,6 Hz, 2H), 1.22-1.36 (m, 6H), 1.63 (d, J = 12.8 Hz, 2H), 1.91 (d, J = 9.6 Hz. 3H), 2.09 (d, J = 9.6 Hz, 2H), 2.86 (s, 1H), 3.08 (s, 2H), 3.18 (d, J = 5.2 Hz, 1H), 3.27 (t, J = 11.2 Hz. 1H), 3.86 (d, J = 10.0 Hz, 2H), 4.15 (s, 1H), 4.67 (s, 1H), 5.59 (s, 1H), 6,29 (d, J = 8.4 Hz. 1H), 6.90 (s, 1H), 7.84-7.88 (m, 3H), 8.32 (d, J = 8.4 Hz, 2H), 8.49 (s, 1H). |
| | | MS (ESI) m/z = 505 (M+H)⁺. |
| | | LC/MS t_{R} = 1.09 min. |
| Example 2-44 | 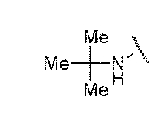 | 1H-NMR (400 MHz, DMSO-d₆) δ 0.59 (s, 2H), 0.70 (d, J = 5.8 Hz, 2H), 1.24 (s, 2H), 1.43 (s, 9H), 1.63 (d, J = 13.2 Hz, 2H), 1.91 (s, 1H), 2.88 (s, 1H), 3.07 (s, 2H), 3.27 (t, J = 12.0 Hz, 2H), 3.86 (d, J = 10.0 Hz, 2H), 5.67 (s, 1H), 6.07 (s, 1H), 6.84 (s, 1H), 7.78 (s, 1H), 7.89 (d, J = 80 Hz, 2H), 8.25 (d, J = 7.6 Hz, 2H), 8.46 (s, 1H). |
| | | MS (ESI) m/z = 463 (M+H)⁺. |
| | | LC/MS t_{R} = 1.48 min. |

**[Table 2-2J]**

| Example No. | R | property data |
|---|---|---|
| Example 2-46 | 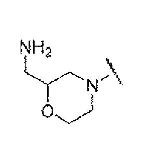 | 1H-NMR (400 MHz, DMSO-d₆) δ 0.57-0.63 (2H, m), 0.69-0.76 (2H, m), 1.23-1.35 (2H, m). 1.67 (2H, d, J = 12.4 Hz), 1.96 (1H, s), 2.62 (2H, d, J = 10.0 Hz), 2.69 (1H_{,} d, J = 5.6 Hz). 2.84-2.97 (2H, m), 3.23-3.33 (4H, m), 3.87 (2H, d, J = 8.0 Hz), 3.96-4.05 (3H, m), 6.02 (1H, s), 7.07 (1H, t, J = 6.0 Hz), 7.91 (3H, d, J = 8.0 Hz), 8.26 (2H, d, J = 8.4 Hz), 8.48 (1H, d, J = 4.0 Hz). |
| | | MS (ESI) m/z = 506 (M+H)⁺. |
| | | LC/MS t_{R} = 0.85 min. |
| Example 2-47 | 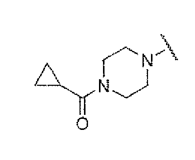 | 1H-NMR (400 MHz, DMSO-d₆) δ 0.59 (2H, s), 0.70-0.76 (6H, m), 1.15-1.33 (2H, m), 1.65 (2H, d, J = 12.0 Hz), 1.91-2.08 (2H, m), 2.87 (1H, s), 3.26 (2H, d, J = 10.4 Hz), 3.50 (2H, d, J = 20.8 Hz,), 3.64 (1H, s), 3.84 (3H, s), 6.05 (1H, s), 7.14 (1H, t, J = 5.2 Hz), 7.90-7.91 (3H, m), 8.26 (2H, d, J = 6.0 Hz), 8.44 (1H, s). |
| | | MS (ESI) m/z = 544 (M+H)⁺. |
| | | LC/MS t_{R} = 1.37 min. |
| Example 2-48 | 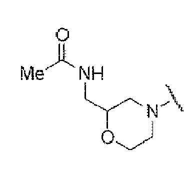 | 1H-NMR (400 MHz, DMSO-d₆) δ 0.55-0.60 (2H, m), 0.64-0.73 (2H, m), 1.14-1.32 (2H, m). 1.63 (2H, d, J = 12,0 Hz), 1.84 (3H, s), 1.93 (1H, br s), 2.61 (1H, t, J = 11.6 Nz), 2.85-2.94 (2H, m), 3.58-3.65 (2H, m), 3.82-4.00 (5H, m), 5.99 (1H, s), 7.13 (1H, t, J = 6.0 Hz), 7.89 (3H, d, J = 7.2 Hz), 8.02 (1H, t, J = 6.0 Hz), 8.23 (2H, d, J = 8.4 Hz), 8.42 (1H, d, J = 4.0 Hz), |
| | | MS (ESI) m/z = 548 (M+H)⁺. |
| | | LC/MS t_{R} = 1.21 min. |
| Example 2-49 | 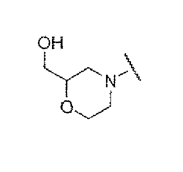 | 1H-NMR (400 MHz. DMSO-d₆) δ 0.55-0.60 (2H, m), 0.64-0.72 (2H, m), 1.25 (2H, dq, J = 4.2, 12.0 Hz), 1.63 (2H, d, J = 12.0 Hz), 1.94 (1H, br s), 2.65 (1H, t, J = 11.2 Hz), 2.81-2.95 (2H, m), 3.22-3.26 (1H, m), 3.48-3.65 (4H, m), 3.82-4.06 (5H, m), 4.83 (1H, s), 5.98 (1H, s), 7.11 (1H, s), 7.88 (3H, d, J = 10.8 Hz), 8.24 (2H, d, J = 8.0 Hz), 8.43 (1H, d, J = 4.4 Hz). |
| | | MS (ESI) m/z = 507 (M+H)⁺. |
| | | LC/MS t_{R} = 1.10 min. |

**[Table 2-2K]**

| Example No. | R | property data |
|---|---|---|
| Example 2-50 | 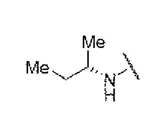 | 1H-NMR (400 MHz, DMSO-d₆) δ 0.58 (2H, s), 0,69 (2H, d, J = 6.8 Hz), 0.94 (4H, t, J = 8.0 Hz), 1.17-1.23 (6H, m), 1.43-1.52 (1H, m), 1.61-1.68 (3H, m), 1.93 (1H, br s), 2.86 (1H, br s), 3.07 (2H, t, J = 6,0 Hz), 3.70-3.78 (1H, m), 3.85 (2H, d, J = 8.4 Hz), 5. 61 (1H, s), 6.22 (1H, d, J = 7.2 Hz), 6.91 (1H, s), 7.80 (1H, s), 7.86 (2H, d, J = 8.8 Hz), 8.29 (2H, d, J = 8.0 Hz), 8.42 (1H, s). |
| | | MS (ESI) m/z = 463 (M+H)⁺. |
| | | LC/MS t_{R} = 1.49 min. |
| Example 2-51 | 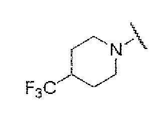 | 1H-NMR (400 MHz, DMSO-d₆) δ 0,59 (2H, s), 0.69 (2H, d, J = 4.4 Hz), 1.25 (2H, q, J = 8,8 Hz), 1,52-1.65 (4H, m), 1.90 (2H, d, J = 12.0 Hz), 2.90 (3H, t, J = 12.4 Hz), 3.84 (2H, d, J = 8.8 Hz), 4.25 (2H, d, J = 12.0 Hz), 6.02 (1H, s), 7.09 (1H, s), 7.87 (2H, s), 7.89 (1H, s), 8.23 (2H, d, J = 8.0 Hz), 8.43 (1H, d, J = 4.0 Hz). |
| | | MS (ESI) m/z = 543 (M+H)⁺. |
| | | LC/MS t_{R} = 1.78 min, |
| Example 2-52 | 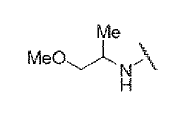 | 1H-NMR (300 MHz, DMSO-d₆) δ 0.59 (2H, s), 0.69 (2H, d, J = 7.2 Hz), 1.16-1.23 (6H, m), 1.62 (2H, d, J = 12.4 Hz), 1.92 (1H, s), 2.86 (1H, s), 3.07 (2H, t, J = 6,4 Hz), 3.50 (1H, dd, J = 3.2. 10.0 Hz), 3.85 (2H, d, J = 7.2 Hz), 4.03 (1H, t, J = 6.2 Hz), 5.64 (1H, s), 6.27 (1H, d, J = 6.8 Hz), 6.97 (1H, s), 7.81-7.87 (3H, m), 8.27 (2H, d, J = 6.8 Hz), 8.42 (1H, s). |
| | | MS (ESI) m/z = 479 (M+H)⁺. |
| | | LC/MS t_{R} = 1.27 min. |
| Example 2-53 | 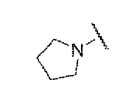 | 1H-NMR (400 MHz, DMSO-d₆) δ 0.60 (2H, s), 0.70 (2H, s), 1.26 (3H, d, J = 9,6 Hz), 1.64 (2H, d, J = 10.8 Hz), 1.96 (7H, s), 2.88 (1H, s), 3.20-3.30 (3H, m), 3.84 (3H, s), 5.62 (1H, s), 6.99 (1H, s), 7.88 (3H, s), 8.34 (2H, s), 8.42 (1H, s). |
| | | MS (ESI) m/z = 461 (M+H)⁺. |
| | | LC/MS t_{R} = 1.43 min. |

**[Table 2-2L]**

| Example No. | R | property data |
|---|---|---|
| Example 2-54 | 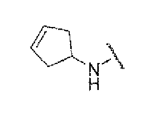 | 1H-NMR (400 MHz, DMSO-d₆) δ 0.59 (2H, s), 0.70 (2H, s), 1.23 (5H, s), 1.63 (3H, d, J = 12.0 Hz), 1.94 (2H, s), 2.32 (2H, d, J = 17.6 Hz), 2.79-2.85 (3H, m), 3.09 (2H, s), 3.86 (3H, d, J = 10.0 Hz), 4.40 (1H, s), 5.62 (1H, s), 5.78 (2H, s), 6.60 (1H, s), 6.96 (1H, s), 7.83 (1H, s), 7.89 (2H, d, J = 8.4 Hz), 8.34 (2H, d, J = 8.4 Hz), 8.43 (1H, s). |
| | | MS (ESI) m/z = 473 (M+H)⁺. |
| | | LC/MS t_{R} = 2.68 min. |
| Example 2-55 | 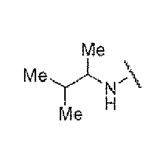 | 1H-NMR (400 MHz. DMSO-d₆) δ 0.59 (2H, s), 0.70 (2H, s), 0.91 (2H, d, J = 6.4 Hz), 0.99 (2H, d, J = 6.4 Hz), 1.11 (3H, d, J = 6.4 Hz), 1.25 (3H, br s), 1.64 (2H, d, J = 12.8 Hz), 1.96 (2H, s), 2.87 (1H, s), 3.09 (2H, s), 3.77 (1H, s), 3,87 (2H, d, J = 10.4 Hz), 5.67 (1H, s), 6.24 (1H, s), 6.89 (1H, s). 7.82 (1H, s), 7.87 (2H, d, J = 8.0 Hz), 8.31 (2H, d, J = 8.0 Hz), 8.44 (1H, s). |
| | | MS (ESI) m/z =477 (M+H)⁺. |
| | | LC/MS t_{R} = 2.75 min. |
| Example 2-56 | 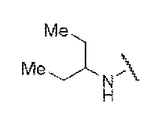 | 1H-NMR (400 MHz, DMSO-d₆) δ 0.60 (2H, s), 0.71 (2H, s). 0.94 (6H, t, J = 6.4 Hz), 1.25 (3H, br s), 1.54-1.66 (6H, m), 1.95 (1H, s), 2.87 (1H, s). 3.09 (2H, s), 3.63 (1H, s), 3.87 (2H, d, J = 10.0 Hz). 5.67 (1H, s), 6.18 (1H, d, J = 6.4 Hz), 6.89 (1H, s), 7.82 (1H, s), 7.88 (2H, d, J = 8.0 Hz), 8.31 (2H, d, J = 8.0 Hz), 8.44 (1H, s). |
| | | MS (ESI) m/z = 477 (M+H)⁺. |
| | | LC/MS t_{R} = 2.72 min. |
| Example 2-57 | 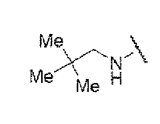 | 1H-NMR (400 MHz, DMSO-d₆) δ 0.60 (2H, s). 0.71 (2H, s), 0.85 (1H, s), 0.98 (9H, s), 1.23 (4H, br s), 1.64 (2H, d, J = 12.4 Hz), 1.95 (1H, s), 2.87 (1H, s), 3.12 (4H, d, J = 16.8 Hz), 3.86 (2H, d, J = 9.2 Hz). 5.77 (1H, s). 6.32 (1H, s). 6.88 (1H, s), 7.82 (1H, s), 7.89 (2H, d, J = 8.0 Hz), 8.32 (2H, d, J = 8.0 Hz), 8.46 (1H, s). |
| | | MS (ESI) m/z = 477 (M+H)⁺. |
| | | LC/MS t_{R} = 2.81 min. |

**[Table 2-2M]**

| Example No. | R | property data |
|---|---|---|
| Example 2-58 | 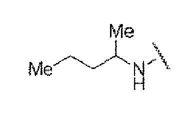 | 1H-NMR (400 MHz, DMSO-d₆) δ 0.59 (2H, s), 0.70 (2H, s), 0.91 (3H, s), 1.18-1.25 (6H, m), 1.42 (3H, s), 1.64 (2H, d, J = 10.0 Hz). 1.94 (1H, s), 2.87 (1H, s), 3.09 (2H, s), 3.85 (3H, s), 5.61 (1H, s), 6.22 (1H, s), 6.91 (1H, s), 7.82 (1H, s), 7.87 (2H, d, J = 8.0 Hz), 8.30 (2H, d, J = 8.0 Hz), 8.44 (1H, s). |
| | | MS (ESI) m/z =477 (M+H)⁺. |
| | | LC/MS t_{R} = 2.78 min. |
| Example 2-59 | 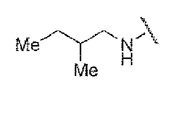 | 1H-NMR (400 MHz, DMSO-d₆) δ 0.59 (2H, s). 0.70 (2H, s), 0.91 (3H, s), 1.18-1.25 (6H, m), 1.42 (3H, s). 1.64 (2H, d, J = 10.0 Hz), 1.94 (1H, s). 2.87 (1H, s), 3.09 (2H, s), 3.85 (3H, s). 5.61 (1H, s), 6.22 (1H, s). 6.91 (1H, s), 7.82 (1H, s), 7.87 (2H, d, J = 8.0 Hz), 8.30 (2H, d, J = 8.0 Hz), 8.44 (1H, s). |
| | | MS (ESI) m/z = 477 (M+H)⁺. |
| | | LC/MS t_{R} = 2.81 min. |
| Example 2-60 | 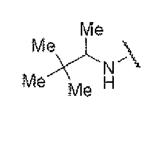 | 1H-NMR (400 MHz, DMSO-d₆) δ 0.60 (2H, s). 0.70 (2H, s), 0.98 (9H, s), 1,12 (3H, d, J = 5.2 Hz), 1.25 (2H, br s), 1.65 (2H, d, J = 12.0 Hz), 1.96 (1H, s), 2.87 (1H, s), 3.09 (2H, s), 3.86 (3H, s), 5.76 (1H, s), 6.05 (1H, d, J = 8.4 Hz), 6.87 (1H, s), 7.81 (1H, s), 7.89 (2H, d, J = 8.4 Hz). 8.31 (2H, d, J = 8.4 Hz). 8.45 (1H, s). |
| | | MS (ESI) m/z = 491 (M+H)⁺. |
| | | LC/MS t_{R} = 2.85 min. |
| Example 2-61 | 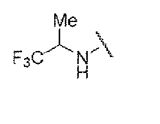 | 1H-NMR (400 MHz, DMSO-d₆) δ 0.60 (2H, s), 0.71 (2H, s), 1.25 (2H, s), 1.37 (3H, d, J = 6.4 Hz), 1.64 (2H, d, J = 12.4 Hz), 1.96 (1H, s), 2.87 (1H, s). 3.12 (2H, s).3.87 (2H, d, J = 9.2 Hz), 4.75 (1H, s), 5.70 (1H, s), 6.90 (1H, d, J = 7.6 Hz). 7.20 (1H, s). 7.85 (1H, s), 7.90 (1H, d, J = 8.0 Hz). 8.23 (2H, d, J = 8.0 Hz). 8.46 (1H, s). |
| | | MS (ESI) m/z = 503 (M+H)⁺. |
| | | LC/MS t_{R} = 1.51 min. |
| Example 2-62 | 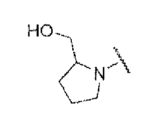 | 1H-NMR (400 MHz, DMSO-d₆) δ 0.60 (2H, s), 0.70 (2H, s), 1.26 (2H, d, J = 10.0 Hz). 1.64 (2H, d, J = 13.2 Hz), 1.94-2.04 (5H, m). 2.88 (1H, s), 3.86 (2H, d, J = 10.0 Hz), 4.06 (1H, s), 5.70 (1H, s), 7.02 (1H, s), 7.89 (3H, s). 8.35 (2H, d, J = 8.4 Hz,). 8.43 (1H, s). |
| | | MS (ESI) m/z = 491 (M+H)⁺. |
| | | LC/MS t_{R} = 1.18 min. |

**[Table 2-2N]**

| Example No. | R | property data |
|---|---|---|
| Example 2-63 | 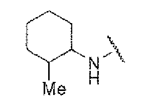 | MS (ESI) m/z = 503 (M+H)⁺. |
| | | LC/MS t_{R} = 1.66 min. |
| Example 2-64 | 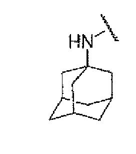 | 1H-NMR (400 MHz, DMSO-d₆) δ 0.59 (2H, s), 0.71 (2H, d, J = 4.4 Hz), 1.23 (2H, d, J = 11.6 Hz), 1.63 (2H, d, J = 11.6 Hz), 1.72 (6H, s), 1.93 (1H, s), 2.12 (10H, d, J = 11.6 Hz), 2.86 (1H, s), 3.06 (2H, s), 3.25-3.31 (2H, m), 3.86 (2H, d, J = 8.4 Hz), 5.68 (1H, s), 6.01 (1H, s), 6.86 (1H, s), 7.82 (1H, s), 7.86 (2H, d, J = 8.0 Hz), 8.31 (2H, d, J = 8.0 Hz), 8.49 (1H, s). |
| | | MS (ESI) m/z = 541 (M+H)⁺. |
| | | LC/MS t_{R} = 9.81 min. |
| Example 2-56 | 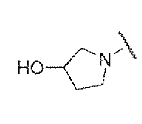 | 1H-NMR (400 MHz, DMSO-d₆ δ 0.60 (2H, s), 0.71 (2H, s), 1.26 (2H, s), 1.65 (2H, d, J = 10.4 Hz), 2.06-1.94 (3H, m), 2.51 (1H, s), 2.88 (1H. s), 3.54 (3H. s), 8.85 (2H, s), 4.42 (1H, s), 5.01 (1H, s), 5.63 (1H, s), 7.02 (1H, s), 7.89 (3H, s), 8.35 (2H, s), 8.42 (1H, s). |
| | | MS (ESI) m/z = 477 (M+H)⁺. |
| | | LC/MS t_{R} =1.11 min. |
| Example 2-66 | 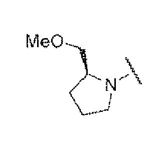 | 1H-NMR (400 MHz. DMSO-d₆) δ 0.60 (2H, s), 0.70 (2H, s), 1.26 (2H, d, J = 10.0 Hz), 1.64 (2H, d, J = 2.4 Hz), 1.97 (5H, s), 2.86 (1H, s), 3.23 (1H, d, J = 14.0 Hz), 3.32 (3H, s), 3.57 (2H, s), 3.86 (2H, d, J = 9.2 Hz), 4.21 (1H, s), 5.69 (1H, s), 7.08 (1H, s), 7.87-7.90 (3H, m), 8.34 (2H, d, J = 7.6 Hz), 8.45 (1H, s). |
| | | MS (ESI) m/z = 505 (M+H)⁺. |
| | | LC/MS t_{R} = 1.46 min. |
| Example 2-67 | 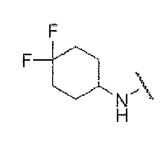 | 1H-NMR (400 MHz, DMSO-d₆) δ 0.60 (2H, s), 0.71 (2H, d, J = 5.2 Hz), 1.17-1.25 (3H, m), 1.63 (4H, d, J = 10.4 Hz), 1.98-2.11 (7H. m), 2.87 (1H, s), 3.10 (2H, s), 3.28 (2H, t, J = 11.2 Hz), 3.85 (3H, s), 5.62 (1H, s), 6.48 (1H, s), 6.99 (1H, s), 7.85 (1H. s), 7.90 (2H, d, J = 8.0 Hz), 8.30 (2H, d, J = 8.0 Hz), 8.45 (1H, s) |
| | | MS (ESI) m/z = 525 (M+H)⁺. |
| | | LC/MS t_{R} = 1.57 min. |

**[Table 2-2O]**

| Example No. | R | property data |
|---|---|---|
| Example 2-68 | 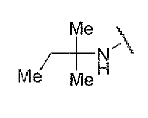 | 1H-NMR (400 MHz, DMSQ-d₆) δ 0.61 (2H, s), 0.71 (2H, s), 0.83 (3H, s), 1.22 (2H, q. J =11.2 Hz), 1.36 (6H, s), 1.62 (2H, d, J = 12.4 Hz), 1.87-1.98 (3H, m), 2.88 (1H, s), 3.07 (2H, s), 3.26 (2H, t, J = 11.2 Hz), 3.85 (2H, d, J = 10.4 Hz), 5.73 (1H. s), 5.95 (1H, s), 6.84 (1H, s), 7.79 (1H, s), 7.91 (2H, d, J = 7.2 Hz), 8.24 (2H, d, J = 7.2 Hz), 8.48 (1H, s). |
| | | MS (ESI) m/z = 477 (M+H)⁺. |
| | | LC/MS t_{R} = 1.54 min. |
| Example 2-69 | 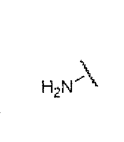 | 1H-NMR (400 MHz, OMSO-d₆) δ 0.60 (2H, s), 0.71 (2H, d, J = 5.2 Hz), 1.23-1.28 (2H, m), 1.63 (2H, d, J = 13.6 Hz), 1.95 (1H, s), 2.87 (1H, s), 3.11 (1H, s), 3.18 (1H, d, J = 4.8 Hz), 3.27 (2H. t, J = 11.2 Hz), 3.86 (2H, d, J = 11.2 Hz), 5.63 (1H. s), 5.81 (2H, s), 7.00 (1H, s), 7.81 (1H, s), 7.87 (2H, d, J = 8.4 Hz), 8.27 (2H, d, J = 8.4 Hz), 8.45 (1H, s). |
| | | MS (ESI) m/z = 407 (M+H)⁺. |
| | | LC/MS t_{R} = 0.94 min. |
| Examples 2-70 | 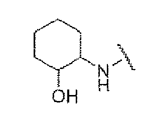 | 1H-NMR (400 MHz. DMSO-d₆) δ 0.61 (2H, s), 0.71 (2H, d, J =6.0 Hz), 1.18-1.27 (2H, m), 1.37 (2H, s), 1.54-1.78 (8H, m), 1.94 (1H, s), 2.87 (1H, s), 3.09 (2H, s), 3.28 (2H, t, J = 11.6 Hz), 3.75 (1H, s), 3.86 (2H, d, J = 11.6 Hz), 4.03 (1H, s), 4.61 (1H, s), 5.81 (1H, s), 6.07 (1H, d, J = 7.2 Hz), 6.91 (1H, s), 7.83 (1H, s), 7.88 (2H, d, J = 8.4 Hz), 8.3 (2H, d, J = 8.4 Hz), 8.46 (1H, s). |
| | | MS (ESI) m/z = 505 (M+H)⁺. |
| | | LC/MS t_{R} = 1.28 min. |
| Example 2-71 | 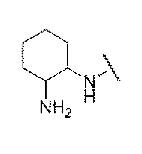 | 1H-NMR (400 MHz, DMSO-d₆) δ 0.60 (2H, s), 0.72 (2H, s), 0.85 (1H, s), 1.15-1.30 (8H, m), 1.64 (3H, d, J = 11.2 Hz), 1.72 (1H, s), 3.94 (1H, s), 2.27 (1H, s), 2.61 (1H, s), 2.87 (1H, s), 3.10 (2H, s), 3.86 (3H, d, J = 11.8 Hz), 5.64 (1H, s), 5.77 (1H. s), 6.17 (1H, s), 6.35 (1H, s), 6.94 (1H, s), 7.84 (1H, s), 7.88 (2H, d, J =8.0 Hz), 8.30-8.33 (2H, m), 8.46 (1H, s). |
| | | MS (ESI) m/z = 504 (M+H)⁺. |
| | | LC/MS t_{R} = 0.99 min. |

**[Table 2-2P]**

| Example No. | R | property data |
|---|---|---|
| Example 2-72 | 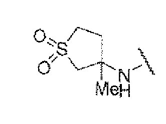 | 1H-NMR (400 MHz. DMSO-d₆) δ 0.60 (2H. s), 0.72 (2H, s), 1.24 (3H, d, J = 11.6 Hz), 1.63-1.66 (6H, m), 1.94 (1H, s), 2.21 (1H, s), 2.72 (1H, s), 2.86 (1H, s), 3.10 (2H, s), 3.87 (2H, d, J = 9.6 Hz), 4.09 (1H, d, J = 14.8 Hz), 5.64 (1H, s), 6.63 (1H, s), 7.1 (1H, s), 7.83 (1H, s), 7.9 (2H, d, J = 8.0 Hz), 8.15 (2H, d, J = 8.0 Hz), 8.47 (1H, s). |
| | | MS (ESI) m/z = 539 (M+H)⁺. |
| | | LC/MS t_{R} = 1.13 min. |
| Example 2-73 | 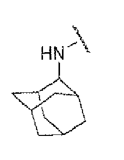 | 1H-NM_{R} (400 MHz, DMSO-d₆) δ 0.60 (2H, s), 0.72 (2H, s), 0.85 (1H, s), 1.24 (4H, s), 1.55 (2H, d, J = 12.4 Hz), 1.65 (1H, d, J = 13.2 Hz), 1.74 (1H. s), 1.90 (5H, s), 2.05-2.14 (3H, m), 2.86 (1H. s), 3.10 (2H, s), 3.86 (2H, s), 3.94 (1H, s), 5.84 (1H, s), 6.40 (1H, s), 8.89 (1H, s), 7.86 (3H, d, J = 8.0 Hz), 8.32 (2H, d, J = 8.0 Hz), 8.47 (1H. s). |
| | | MS (ESI) m/z = 541 (M+H)⁺. |
| | | LC/MS t_{R} = 1.88 min. |
| Example 2-74 | 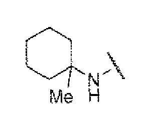 | 1H-NMR (400 MHz, DMSQ-d₆) δ 0.58 (2H, s), 0.69 (2H, d, J = 7.2 Hz), 1.19-1.29 (2H, m), 1.42 (10H, s), 1.63 (2H, d, J = 13.2 Hz), 1.94 (1H, s), 2.23 (2H, s), 2.85 (1H, s), 3.06 (2H, s), 3.85 (2H, d, J = 10.4 Hz), 5.79 (2H, d, J = 8.4 Hz), 6.84 (1H, s), 7.77 (1H, s), 7.86 (2H, d, J = 8.4 Hz), 8.22 (2H, d, J = 8.4 Hz), 8.44 (1H, s). |
| | | MS (ESI) m/z = 503 (M+H)⁺. |
| | | LC/MS t_{R} = 2.08 min. |
| Example 2-75 | 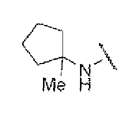 | 1H-NMR (400 MHz, DMSO-d₆) δ 0.56-0.59 (2H, m), 0.6S-0.70 (2H, m), 1.14-1.24 (2H, m), 1.50 (3H, s), 1.60-1.65 (7H, m), 1.93 (1H, s), 2.17 (2H, s), 2.81-2.88 (1H. m), 3.05 (2H. t, J = 6.4 Hz), 3.85 (2H, d, J = 11.2 Hz), 5.64 (1H, s), 6.17 (1H, s), 6.88 (1H, s), 7.79 (1H, s), 7.86 (2H, d, J = 8.8 Hz), 8.27 (2H, d, J = 8.8 Hz), 8.43 (1H. d, J =4.0 Hz). |
| | | MS (ESI) m/z = 489 (M+H)⁺. |
| Example 2-76 | 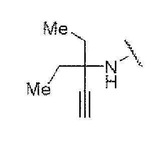 | 1H-NMR (400 MHz, DMSO-d₆) δ 0.61 (2H, s), 0.72 (2H, d, J = 6.0 Hz), 0.86 (1H, s), 1.24-1.28 (6H, m), 1.35 (1H, s), 2.88 (1H, s), 3.28 (2H, t, J = 11.2 Hz), 3.86 (2H, d, J = 9.4 Hz), 6.23 (1H, s), 7.69 (1H, s), 7.93 (2H, d, J = 8.4 Hz), 8.09 (1H, s), 8.21 (2H, d, J = 8.4 Hz), 8.48 (1H, s). |
| | | MS (ESI) m/z = 501 (M+H)⁺. |
| | | LC/MS t_{R} = 1.21 min. |

**[Table 2-2Q]**

| Example No. | R | property data |
|---|---|---|
| Example 2-77 | 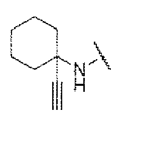 | 1H-NMR (300 MHz, DMSO-d₆) δ 0.60 (2H, s), 0.72 (2H, d, J = 5.6 Hz), 0.86 (1H, s), 1.24-1.28 (4H, m), 1.49-1.67 (9H, m), 1.89-1.92 (3H, m), 2.88 (1H, s), 3.28 (4H, t, J = 11.2 Hz), 3.86 (2H, d, J = 8.8 Hz), 6.26 (1H, s), 7.71 (1H, s), 7.93 (2H, d, J = 8.0 Hz), 8.09 (1H, s), 8.20 (2H, d, J = 8.4 Hz), 8.26 (1H, s), 8.48 (9H, s). |
| | | MS (ESI) m/z = 513 (M+H)⁺. |
| | | LC/MS t_{R} = 1.21 min. |
| Example 2-78 | 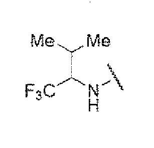 | 1H-NMR (300 MHz, DMSQ-d₆) δ 0.62 (2H, s), 0.72 (2H, d, J = 5.6 Hz). 1.05 (6H, t, J = 7.2 Hz), 1.25-1.23 (2H, m), 1.66 (2H, d, J = 13.2 Hz), 2.00 (1H, s), 2.21 (1H, s), 2.89 (1H, s), 3.14 (2H, s), 3.31 (2H, t, J = 11.6 Hz), 3.88 (2H, d, J = 10.4 Hz), 4.65 (1H, s), 5.92 (1H, s), 6.82 (1H, d, J = 9.6 Hz), 7.20 (1H, s), 7.89 (1H, s), 7.94 (2H, d, J = 8.0 Hz), 8.23 (2H, d, J = 8.0 Hz). 8.48 (1H. s). |
| | | MS (ESI) m/z = 531 (M+H)⁺. |
| | | LC/MS t_{R} = 1.70 min. |
| Example 2-79 | 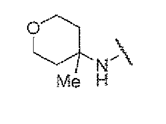 | 1H-NMR (400 MHz, DMSO-d₆) δ 0.60 (1H, s), 0.71 (1H, d, J = 5.6 Hz). 0.86 (0H, s), 0.94 (1H, d, J = 4.8 Hz), 1.09-1.28 (4H, m), 1.49 (3H, s), 1.61-1.68 (4H, m), 1.99 (1H, s), 2.23 (2H, d, J = 13.6 Hz), 2.87 (1H, s), 3.11 (2H, s), 3.31 (2H, t, J = 12.0 Hz), 3.62 (2H, s), 3.88 (2H, d, J = 7.6 Hz), 5.91 (1H, s), 6.25 (1H, s), 6.90 (1H, s), 7.91 (2H, d, J = 8.0 Hz), 7.99 (1H, s), 8.18 (2H, d, J = 8.0 Hz), 8.48 (1H, s). |
| | | MS (ESI) m/z = 505 (M+H)⁺. |
| | | LC/MS t_{R} = 1.26 min. |
| Example 2-80 | 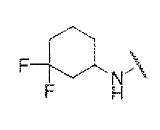 | 1H-NMR (300 MHz, DMSO-d₆) δ 0.61 (2H, s), 0.71 (2H, d, J =6.4 Hz), 1.18-1.28 (2H, m), 1.58-1.66 (4H, m), 1.75 (1H, s), 1.94-2.05 (2H, m), 2.88 (1H, s), 3.09 (2H, s), 3.20-3.31 (4H, m), 3.74-3.88 (4H, m), 4.01 (1H, d, J = 11.2 Hz), 5.73 (1H, s), 6.46 (1H, s), 7.09 (1H, s), 7.91 (3H, d, J = 8.8 Hz), 8.28 (2H, d, J = 8.4 Hz), 8.50 (1H, s), |
| | | MS (ESI) m/z = 525 (M+H)⁺. |
| | | LC/MS t_{R} = 1.50 min. |

**[Table 2-2R]**

| Example No. | R | property data |
|---|---|---|
| Example 2-81 | 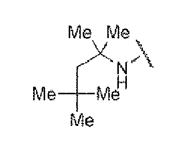 | 1H-NMR (400 MHz, DMSO-d₆) δ 0.60 (2H, s), 0.70 (2H, d, J = 5.6 Hz), 0.97 (10H, s), 1.24 (3H, s), 1.45 (6H, s), 1.63 (2H, d, J = 12.4 Hz), 1.97 (3H, s), 2.87 (3H, s), 3.06 (2H, s), 3.28 (2H, t, J = 11.6 Hz), 3.86 (2H, d, J = 9.6 Hz), 5.68 (1H, s), 5.99 (1H. s), 6.82 (1H. s), 7.77 (1H. s), 7.88 (2H, d, J = 8.4 Hz), 8.24 (2H, d, J = 8.0 Hz), 8.47 (1H, s). |
| | | MS (ESI) m/z = 519 (M+H)⁺. |
| | | LC/MS t_{R} = 1.82 min. |
| Example 2-82 | 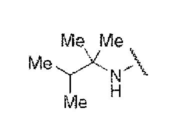 | 1H-NMR (400 MHz, DMSO-d₆) δ 0.60 (2H, s), 0.70 (2H, d, J = 6.0 Hz), 0.90 (6H, d, J = 7.2 Hz), 1.23 (6H, s), 1.64 (2H, d, J = 12.8 Hz), 1.97-2.01 (3H, m), 2.73 (1H, s), 2.86 (1H, s), 3.07 (2H, s), 3.27 (2H, d, J = 10.8 Hz), 3.86 (2H, d, J = 7.8 Hz), 5.74 (1H, s), 5.94 (1H, s), 6.84 (1H, s), 7.78 (1H, s), 7.88 (2H, d, J = 7.6 Hz), 8.23 (2H, d, J = 8.0 Hz), 8.46 (1H, s). |
| | | MS (ESI) m/z = 491 (M+H)⁺. |
| | | LC/MS t_{R} = 1.66 min. |
| Example 2-83 | 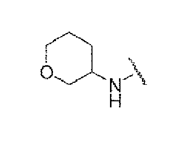 | 1H-NMR (400 MHz, DMSO-d₆) δ 0.61 (2H, s), 0.71 (2H, d, J = 6.4 Hz), 1.18-1.28 (2H. m), 1.68-1.66 (5H, m), 1.75 (1H, s), 1.95-2.05 (2H, m), 2.88 (1H. s), 3.09 (2H, s), 3.21-3.31 (2H, m), 3.74-3.79 (2H. m), 3.87 (2H, d, J = 10.4 Hz), 4.03 (2H. d, J = 9.2 Hz), 5.73 (1H, s), 6.46 (1H, s), 7.09 (1H. s), 7.91 (3H, d, J = 8.8 Hz), 8.28 (2H, d, J = 8.4 Hz), 8.50 (1H. s). |
| | | MS (ESI) m/z = 491 (M+H)⁺. |
| | | LC/MS t_{R} = 1.29 min. |
| Example 2-84 | 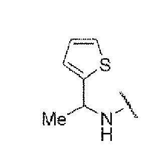 | 1H-NMR (300 MHz, DMSO-d₆) δ 0.60 (2H, s), 0.71 (2H, d, J = 5.2 Hz), 1.24 (2H, d, J = 6.4 Hz), 1.57-1.64 (4H, m), 1.94 (1H, s), 2.86 (1H, s), 3.10 (2H, s), 3.28 (1H, t, J = 11.6 Hz), 3.86 (2H, d, J = 10.0 Hz), 5.24 (1H, t, J = 6.4 Hz), 6.69 (1H, s), 6.96 (2H, d, J = 5.2 Hz), 7.03 (1H, t, J = 6.0 Hz), 7.08 (1H, s), 7.32 (1H, d, J = 4.4 Hz), 7.82 (1H, s), 7.86 (1H, d, J = 8.0 Hz), 8.19 (2H, d, J = 8.0 Hz), 8.44 (1H, s). |
| | | MS (ESI) m/z =517 (M+H)⁺. |
| | | LC/MS t_{R} = 1.53 min. |

**[Table 2-2S]**

| Example No. | R | property data |
|---|---|---|
| Example 2-85 | 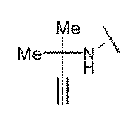 | 1H-NMR (300 MHz, DMSO-d₆) δ 0.63 (2H, s), 0.74 (2H, d, J = 7.2 Hz), 1.27-1.35 (2H, m), 1.45 (6H, s), 1.67 (2H, d, J = 10.4 Hz), 2.90 (1H. s), 3.29 (2H. d, J = 10.4 Hz), 3.88 (2H, d, J = 8.8 Hz), 6.26 (1H. s), 7.72 (1H, s), 7.96 (2H, d, J = 8.4 Hz), 8,1 (1H. s), 8.21 (2H, d, J = 8.4 Hz), 8.51 (1H, d, J = 4.0 Hz). |
| | | MS (ESI) m/z = 473 (M+H)⁺. |
| | | LC/MS t_{R} = 2.09 min, |
| Example 2-86 | 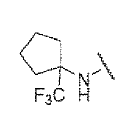 | 1H-NMR (300 MHz, DMSO-d₆) δ 0.60-0.61 (m, 2H), 0.67-0.73 (m, 2H), 1.22-1.26 (m, 2H), 1.65-1.69 (m, 6H), 1.96-2.06 (m, 3H), 2.85-2.88 (m, 1H), 3.09 (t, J = 6.4 Hz, 2H), 3.27 (d, J = 11.2 Hz, 2H), 3.87 (dd, J = 11.2. 2.6 Hz, 2H), 5.75 (s, 1H), 6.52 (s, 1H), 7.07 (t, J = 6.0 Hz, 1H), 7.83 (s, 1H), 7.88 (d, J = 8.6 Hz, 2H), 8.23 (d, J = 8.4 Hz, 2H), 8.44 (d, J = 4.2 Hz, 1H). |
| | | MS (ESI) m/z = 543 (M+H)⁺. |
| | | LC/MS t_{R}= 2.12 min. |

Example 2-87

### 4-(6-(sec-Butylamino)-8-((tetrahydro-2H-pyran-4-yl)methylamino)imidazo[1,2-b]pyridazin-3-yl)-N-cyclopropylbenzamide

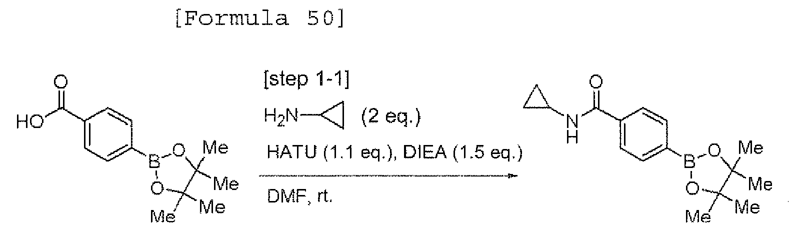

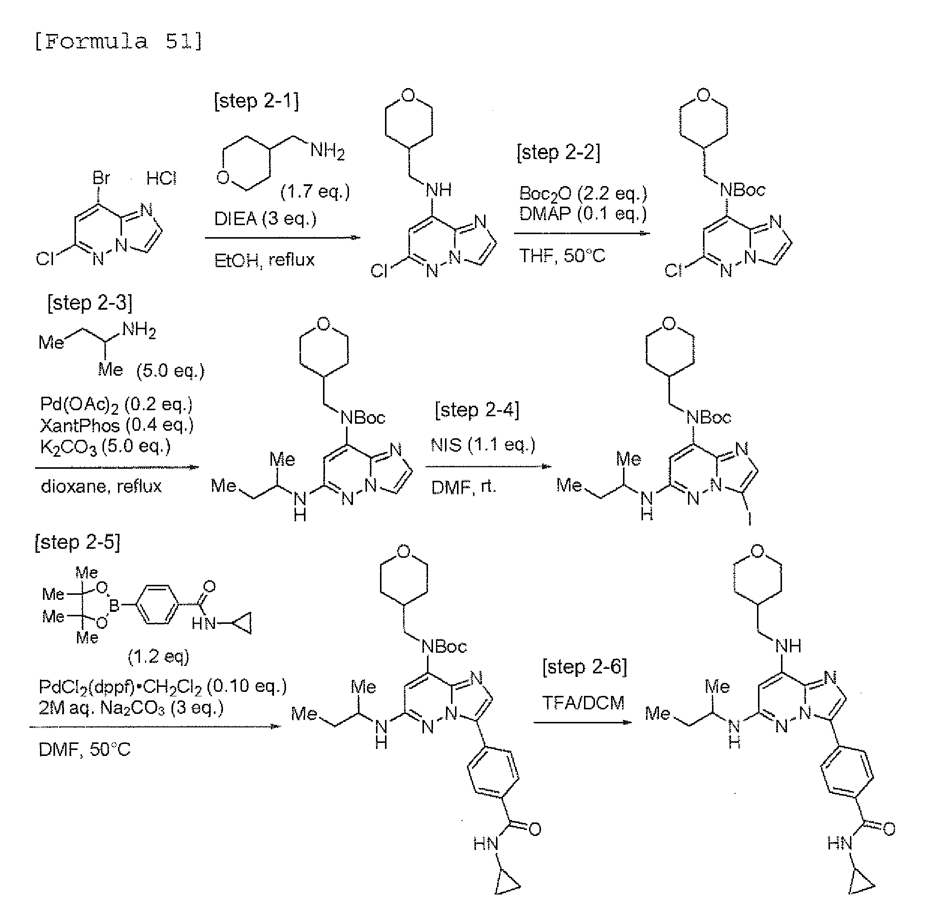

### Step 1-1: 4-(Cyclopropylcarbamoyl)phenylboronic acid pinacol ester

To a DMF (96 mL) solution of 4-carboxyphenylboronic acid pinacol ester (19.2 g, 77 mmol), cyclopropylamine (10.7 mL, 155 mmol) and triethylamine (11.8 mL, 116 mmol), HATU (32.4 mg, 85 mmol) was added and stirred at room temperature for one hour. The reaction solution was cooled to 0°C and water (96 mL) was added dropwise. The precipitated solid substance was obtained by filtration and washed with water to obtain the titled compound (13.7 g, 47.6 mmol, 62%) as a white solid substance.
1H-NMR (300 MHz, DMSO-d₆) δ 0.57-0.58 (m, 2H), 0.63-0.72 (m, 2H), 1.29 (s, 12H), 2.83-2.85 (m, 1H), 7.71 (d, J = 4.1 Hz, 2H), 7.81 (d, J = 7.7 Hz, 2H), 8.48 (d, J = 4.2 Hz, 1H).
MS (ESI) m/z = 288 (M+H)⁺.
LC/MS t_{R} = 1.72 min.

### Step 2-1: 6-Chloro-N-((tetrahydro-2H-pyran-4-yl)methyl)imidazo[1,2-b]pyridazine-8-amine

To an ethanol (400 mL) solution of 8-bromo-6-chloroimidazo[1,2-b]pyridazine hydrochloride (111 g, 414 mmol) and DIEA (217 mL, 1243 mmol), 4-aminomethyltetrahydropyran (76 g, 663 mmol) was added and stirred for 2 hours while heating under reflux. To the reaction solution, water and ethyl acetate were added to separate phases and the insoluble matter was removed by Celite filtration. Thereafter, the water phase was extracted with ethyl acetate and organic phases were combined, washed with water and saturated saline, and dried over magnesium sulfate. The organic phase was filtrated and then concentrated under reduced pressure to obtain the titled compound (73 g, 274 mmol, 66%) as a brown solid substance.
1H-NMR (300 MHz, DMSO-d₆) δ 1.13-1.30 (m, 2H), 1.54-1.65 (m, 2H), 1.82-2.00 (m, 1H), 3.20-3.28 (m, 4H), 3.80-3.84 (m, 2H), 6.23 (s, 1H), 7.49 (d, J = 1.2 Hz, 1H), 7.94-7.96 (m, 2H).
MS (ESI) m/z = 267 (M+H)⁺.
LC/MS t_{R} = 1.33 min.

### Step 2-2: tert-Butyl 6-chloroimidazo[1,2-blpyridazin-8-yl((tetrahydro-2H-pyran-4-yl)methyl)carbamate

To a THF (300 mL) solution of 6-chloro-N-((tetrahydro-2H-pyran-4-yl)methyl)imidazo[1,2-b]pyridazine-8-amine (73 g, 274 mmol) and BOC₂O (131 g, 602 mmol), DMAP (3.34 g, 27.4 mmol) was added at room temperature and stirred at 50°C for 2 hours. The reaction solution was diluted with saturated sodium bicarbonate water and THF was distilled away under reduced pressure and then ethyl acetate was added to separate phases. Thereafter, the water phase was extracted with ethyl acetate and organic phases were combined, washed with water and saturated saline, and dried over magnesium sulfate. The resultant solid substance was purified by medium-pressure silica gel chromatography (hexane/ethyl acetate; 10-60% ethyl acetate gradient) to obtain the titled compound (88.6 g, 242 mmol, 88%) as a colorless solid substance.
1H-NMR (300 MHz, DMSO-d₆) δ 1.10-1.15 (m, 2H), 1.35 (s, 10H), 1.53-1.56 (m, 2H), 1.67-1.69 (m, 1H), 3.15 (t, J = 10.8 Hz, 2H), 3.76 (dd, J = 11.3, 2.9 Hz, 2H), 3.93 (d, J = 7.3 Hz, 2H), 7.46 (s, 1H), 7.81 (br s, 1H), 8.32 (br s, 1H).
MS (ESI) m/z = 367 (M+H)⁺.
LC/MS t_{R} = 1.93 min.

### Step 2-3: tert-Butyl 6-(sec-butylamino)imidazo[1,2-b]pyridazin-8-yl((tetrahydro-2H-pyran-4-yl)methyl)carbamate

To a dioxane (300 mL) solution of tert-butyl 6-chloroimidazo[1,2-b]pyridazin-8-yl((tetrahydro-2H-pyran-4-yl)methyl)carbamate (20 g, 54.5 mmol), sec-butylamine (27.5 mL, 273 mmol), Xantphos (12.6 g, 21.8 mmol), and potassium carbonate (37.7 g, 273 mmol), Pd(OAc)₂ (2.49 g, 10.9 mmol) was added and stirred for one hour while heating under reflux. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 30-100% ethyl acetate gradient) to obtain the titled compound (12.5 g, 31.0 mmol, 57%) as a light yellow amorphous substance.
1H-NMR (300 MHz, DMSO-d₆) δ 0.90 (t, J = 7.4 Hz, 3H), 1.13-1.16 (m, 5H), 1.36 (s, 9H), 1.46-1.72 (m, 5H), 3.18 (t, J = 10.8 Hz, 2H), 3.67-3.81 (m, 5H), 6.56 (s, 1H), 6.66 (d, J = 7.6 Hz, 1H), 7.33 (d, J = 0.9 Hz, 1H), 7.77 (d, J = 0.9 Hz, 1H).
MS (ESI) m/z = 404 (M+H)⁺.
LC/MS t_{R} = 1.39 min.

### Step 2-4: tert-Butyl 6-(sec-butylamino)-3-iodoimidazo[1,2-b]pyridazin-8-yl((tetrahydro-2H-pyran-4-yl)methyl)carbamate

To a DMF (100 mL) solution, tert-butyl 6-(sec-butylamino)imidazo[1,2-b]pyridazin-8-yl((tetrahydro-2H-pyran-4-yl)methyl)carbamate (12.5 g, 31.0 mol), NIS (7.67 g, 34.1 mmol) was added and stirred at room temperature for 3 hours. To the reaction solution, a 10% aqueous NaHSO₃ solution and ethyl acetate were added and separate phases. Thereafter, the water phase was extracted with ethyl acetate and organic phases were combined and washed with a 10% aqueous potassium carbonate solution and saturated saline and dried over magnesium sulfate. The organic phase was filtrated and then concentrated under reduced pressure to obtain the titled compound (16.7 g, 31.5 mmol, >100%) as a light yellow amorphous substance.
1H-NMR (300 MHz, DMSO-d₆) δ 0.93 (t, J = 7.4 Hz, 3H), 1.19-1.21 (m, 5H), 1.35 (s, 9H), 1.49-1.65 (m, 5H), 3.17 (t, J = 11.0 Hz, 2H), 3.74-3.79 (m, 5H), 6.63 (s, 1H), 7.45 (s, 1H).
MS (ESI) m/z = 530 (M+H)⁺.
LC/MS t_{R} = 2.24 min.

### Step 2-5: tert-Butyl 6-(sec-butylamino)-3-(4-(cyclopropylcarbamoyl)phenyl)imidazo[1,2-b]pyridazin-8-yl((tetrahydro-2H-pyran-4-yl)methyl)carbamate

To a DMF (100 mL) solution of tert-butyl 6-(sec-butylamino)-3-iodoimidazo[1,2-b]pyridazin-8-yl((tetrahydro-2H-pyran-4-yl)methyl)carbamate (16.7 g, 31.5 mmol) and 4-(cyclopropylcarbamoyl)phenylboronic acid pinacol ester (10.9 g, 37.9 mmol) and a 2M aqueous sodium carbonate solution (47.3 mL, 95 mmol), PdCl₂(dppf)/CH₂Cl₂ (2.58 g, 3.15 mmol) was added and stirred at 50°C for 5 hours. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 40-100% ethyl acetate gradient) to obtain the titled compound (14.4 g, 25.6 mmol, 81%) as a brown amorphous substance.
1H-NMR (300 MHz, DMSO-d₆) δ 0.57-0.64 (m, 2H), 0.66-0.74 (m, 2H), 0.96 (t, J = 7.4 Hz, 3H), 1.15-1.26 (m, 5H), 1.37 (s, 9H), 1.49-1.77 (m, 5H), 2.81-2.90 (m, 1H), 3.19 (t, J = 11.1 Hz, 2H), 3.73-3.82 (m, 5H), 6.68 (s, 1H), 6.91 (d, J = 7.1 Hz, 1H), 7.91 (d, J = 8.6 Hz, 2H), 7.98 (s, 1H), 8.29 (d, J = 8.4 Hz, 2H), 8.45 (d, J = 4.0 Hz, 1H).
MS (ESI) m/z = 563 (M+H) +.
LC/MS t_{R} = 1.69 min.

### Step 2-6: Titled compound

To a dichloromethane (70 mL) solution of tert-butyl 6-(sec-butylamino)-3-(4-(cyclopropylcarbamoyl)phenyl)imidazo[1,2-b]pyridazin-8-yl((tetrahydro-2H-pyran-4-yl)methyl)carbamate (14.4 g, 25.6 mmol), TFA (30 mL) was added and stirred at room temperature for one hour. To the reaction solution, a 2 mol/L aqueous sodium hydroxide solution (200 mL) was added to neutralize and then ethyl acetate was added to separate phases. Thereafter, the water phase was extracted with ethyl acetate and organic phases were combined and washed with a 10% aqueous potassium carbonate solution and saturated saline, and dried over magnesium sulfate. The organic phase was filtrated and then concentrated under reduced pressure. The resultant residue was solidified by using methanol/water to obtain the titled compound (10.3 g, 22.2 mmol, 87%) as a white solid substance.
1H-NMR (400 MHz, DMSO-d₆) δ 0.59 (s, 2H), 0.70 (s, 2H), 0.93-0.97 (m, 2H), 1.18-1.24 (m, 6H), 1.45-1.52 (m, 1H), 1.63 (d, J = 13.2 Hz, 2H), 1.93 (s, 1H), 2.86 (s, 1H), 3.09 (s, 2H), 3.27 (t, J = 11.2 Hz, 2H), 3.74-3.76 (m, pH), 3.86 (d, J = 10.4 Hz, 2H), 5.63 (s, 1H), 6.24 (d, J = 6.0 Hz, 1H), 6.88 (s, 1H), 7.82 (s, 1H), 7.88 (d, J = 7.6 Hz, 2H), 8.31 (d, J = 8.4 Hz, 2H), 8.45 (s, 1H).
MS (ESI) m/z = 463 (M+H)⁺.
LC/MS t_{R} = 1.47 min.

Example 2-88

### N-Cyclopropyl-4-(8-((tetrahydro-2H-pyran-4-yl)methylamino)-6-(1,1,1-trifluoro-2-methylpropan-2-ylamino)imidazo[1,2-b]pyridazin-3-yl)benzamide

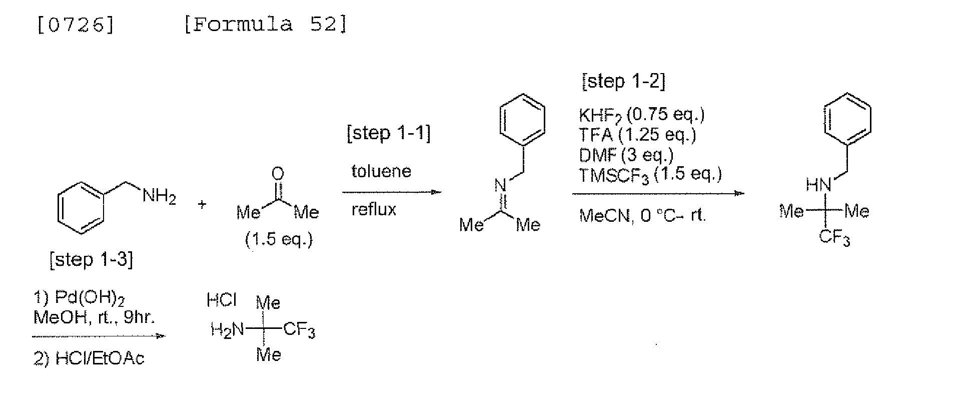

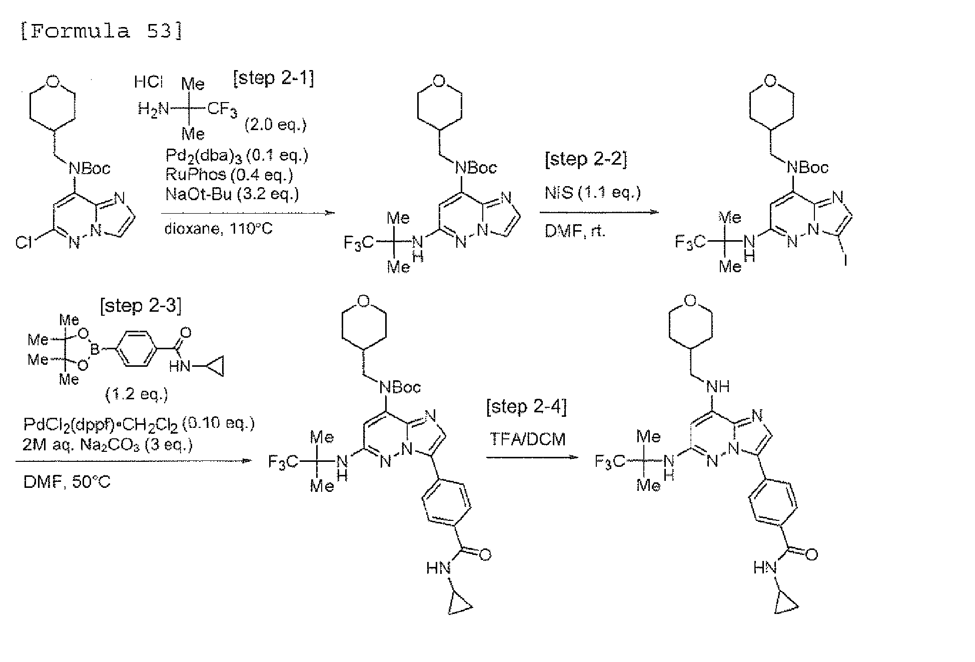

### Step 1-1: 1-phenyl-N-(propan-2-ylidene)methylamine

To a toluene (250 mL) solution of acetone (103 mL, 1400 mmol), benzylamine (51.0 mL, 467 mmol) was added and stirred for 7 hours while water was distilled away by use of Dean-Stark and while heating under reflux. The reaction solution was concentrated under reduced pressure to distill away toluene and acetone and thereafter, the resultant residue was purified by distillation to obtain the titled compound (47.1 g, 320 mmol, 69%, bp 49-52°C (0.8 mmHg)) as colorless liquid.
1H-NMR (300 MHz, CDCl₃) δ 1.94-1.95 (m, 3H), 2.08-2.10 (m, 3H), 4.45 (s, 2H), 7.23-7.26 (m, 1H), 7.30-7.36 (m, 4H).

### Step 1-2: N-Benzyl-1,1,1-trifluoro-2-methylpropane-2-amine

To an acetonitrile (140 mL) solution of 1-phenyl-N-(propan-2-ylidene)methylamine (20 g, 136 mmol) and DMF, (31.7 mL, 408 mmol), KHF₂ (7.96 g, 102 mmol) was added at 0°C and then TFA (13.1 mL, 170 mmol) and trifluoromethyl trimethylsilane (30.5 mL, 204 mmol) were added dropwise. After the reaction solution was stirred at room temperature for 4 hours, a saturated aqueous sodium carbonate solution was added to the reaction solution to neutralize it. After ethyl acetate was added to separate phases, the water phase was extracted with ethyl acetate. Organic phases were combined, washed with water and saturated saline, and dried over magnesium sulfate. The resultant residue was purified by distillation to obtain the titled compound (15.0 g, 69 mmol, 51%, bp 55-59°C (1.1 mmHg)) as colorless liquid.
1H-NMR (300 MHz, CDCl₃) δ 1.32-1.32 (m, 6H), 3.85 (s, 2H), 7.28-7.34 (m, 5H).
MS (ESI) m/z = 218 (M+H)⁺.
LC/MS t_{R} = 1.11 min.

### Step 1-3: 1,1,1-trifluoro-2-methylpropane-2-amine hydrochloride

To a dioxane (100 mL) solution of N-benzyl-1,1,1-trifluoro-2-methylpropane-2-amine (15.0 g, 69.0 mmol), Pd(OH)₂ (2.25 g, 3.20 mmol) was added and then stirred under a hydrogen atmosphere for 2 hours. The reaction solution was filtrated by Celite and 4 mol/L ethyl acetate solution (35 mL) of hydrochloric acid was added. After the solvent was concentrated under reduced pressure, the resultant residue was solidified by use of hexane/ethyl acetate to obtain the titled compound (4.40 g, 26.9 mmol, 39%) as a white solid substance. 1H-NMR (300 MHz, CDCl₃) δ 1.67 (s, 6H), 2.10 (br s, 2H).

### Step 2-1: tert-Butyl(tetrahydro-2H-pyran-4-yl)methyl(6-(1,1,1-trifluoro-2-methylpropan-2-ylamino)imidazo[1,2-b]pyridazin-8-yl)carbamate

After a dioxane (250 mL) solution of Pd₂(dba)₃ (6.24 g, 6.81 mmol) and RuPhos (12.7 g, 27.3 mmol) was stirred at room temperature for 5 minutes, tort-butyl 6-chloroimidazo[1,2-b]pyridazin-8-yl((tetrahydro-2H-pyran-4-yl)methyl)carbamate (25.0 g, 68.1 mmol), 1,1,1-trifluoro-2-methylpropane-2-amine hydrochloride (22.3 g, 136 mmol) and sodium-t-butoxide (21.0 g, 218 mmol) were added and stirred for 6 hours while heating under reflux. To the reaction solution, water and ethyl acetate were added to separate phases. Thereafter, the water phase was extracted with ethyl acetate. Organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 30-100% ethyl acetate gradient) to obtain the titled compound (22.1 g, 48.3 mmol, 71%) as a light brown amorphous substance.
1H-NMR (300 MHz, DMSO-d₆) δ 1.36 (s, 9H), 1.64 (s, 6H), 3.17 (t, J = 10.8 Hz, 2H), 3.74-3.83 (m, 4H), 6.74 (s, 1H), 6.90 (s, 1H), 7.39 (d, J = 1.1 Hz, 1H), 7.82 (d, J = 1.2 Hz, 1H).
MS (ESI) m/z = 1.55 (M+H)⁺.
LC/MS t_{R} = 458 min.

### Step 2-2: tert-Butyl 3-iodo-6-(1,1,1-trifluoro-2-methylpropan-2-ylamino)imidazo[1,2-b]pyridazin-8-yl((tetrahydro-2H-pyran-4-yl)methyl)carbamate

The titled compound was synthesized in accordance with the process of Example 2-87 (Step 2-4) (26.9 g, 46.1 mmol, 95%).
1H-NMR (300 MHz, DMSO-d₆) δ 1.35 (s, 9H) 1.71 (s, 6H), 3.17 (t, J = 10.8 Hz, 2H), 3.75-3.78 (m, 4H), 6.60 (s, 1H), 7.10 (s, 1H), 7.51 (s, 1H).
MS (ESI) m/z = 584 (M+H)⁺.
LC/MS t_{R} = 2.32 min.

### Step 2-3: tert-Butyl 3-(4-(cyclopropylcarbamoyl)phenyl)-6-(1,1,1-trifluoro-2-methylpropan-2-ylamino)imidazo[1,2-b]pyridazin-8-yl((tetrahydro-2H-pyran-4-yl)methyl)carbamate

The titled compound was synthesized in accordance with the process of Example 2-87 (Step 2-5) (26.8 g, 43.5 mmol, 94%). 1H-NMR (300 MHz, DMSO-d₆) δ 0.56-0.61 (m, 2H), 0.67-0.73 (m, 2H), 1.36-1.39 (m, 2H), 1.37 (s, 9H), 1.53-1.57 (m, 2H), 1.66 (s, 6H), 1.98 (s, 2H), 3.19 (t, J = 10.8 Hz, 2H), 3.74-3.84 (m, 4H), 6.85 (s, 1H), 7.10 (s, 1H), 7.91 (d, J = 8.7 Hz, 2H), 7.96 (s, 1H) 8.12 (d, J = 8.7 Hz, 2H), 8.49 (d, J = 4.3 Hz, 1H).
MS (ESI) m/z = 617 (M+H)⁺.
LC/MS t_{R} = 1.80 min.

### Step 2-4: Titled compound

The titled compound was synthesized in accordance with the process of Example 2-87 (Step 2-6) (17.5 g, 33.9 mmol, 78%).
1H-NMR (300 MHz, DMSO-d₆) δ 0.54-0.74 (m, 4H), 1.16-1.32 (m, 2H), 1.56-1-70 (m, 2H), 1.64 (s, 6H), 1.95 (m, 1H), 2.85 (m, 1H), 3.08 (t, J = 6.0 Hz, 2H), 3.22-3.34 (m, 2H), 3.81-3.92 (m, 2H), 5.80 (s, 1H), 6.47 (s, 1H), 7.11 (t, J = 6.0 Hz, 1H), 7.79 (s, 1H), 7.88 (d, J = 8.4 Hz, 2H), 8.15 (d, J = 8.4 Hz, 2H), 8.45 (d, J = 4.2 Hz, 1H).
MS (ESI) m/z = 517 (M+H)⁺.
LC/MS t_{R} = 1.55 min.

Example 2-89

### 4-(6-(Cyclohexylamino)-8-((tetrahydro-2H-pyran-4-yl)methoxy)imidazo[1,2-b] pyridazin-3-yl)-N-cyclopropylbenzamide

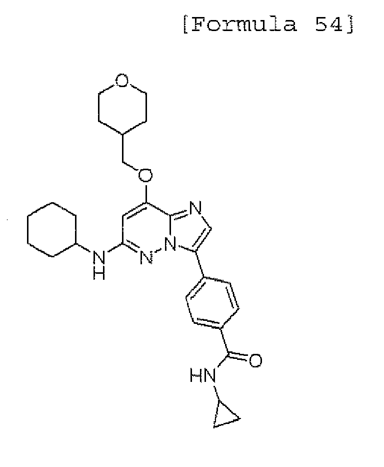

The titled compound was synthesized using (tetrahydro-2H-pyran-4-yl)methanol and sodium hydride as a base in Example 2-1 (Step 1) and in accordance with the process of Example 2-1 and 2-1.
1H-NMR (400 MHz, CDCl₃) δ 0.65-8.67 (m, 2H), 0.87-0.93 (m, 2H), 1.19-1.33 (m, 3H), 1.39-1.52 (m, 4H), 1.66-1.76 (m, 1H), 1.77-1.91 (m, 4H), 2.12-2.21 (m, 2H), 2.23-2.35 (m, 1H), 2.90-2.98 (m, 1H), 3.46 (t, J = 11.7 Hz, 2H), 3.69-3.80 (m, 1H), 3.97-4.05 (m, 4H), 4.14 (d, J = 6.6 Hz, 1H), 5.75 (s, 1H), 6.25 (s, 1H), 7.77-7.81 (m, 3H), 8.20 (d, J = 8.1 Hz, 2H).
MS (ESI) m/z = 490 (M+H)⁺.
LC/MS t_{R} = 1.58 min.

Example 2-90

### 4-(6-(2-Chlorophenoxy)-8-((tetrahydro-2H-pyran-4-yl)methylamino)imidazo[1,2-b]pyridazin-3-yl)-N-cyclopropylbenzamide

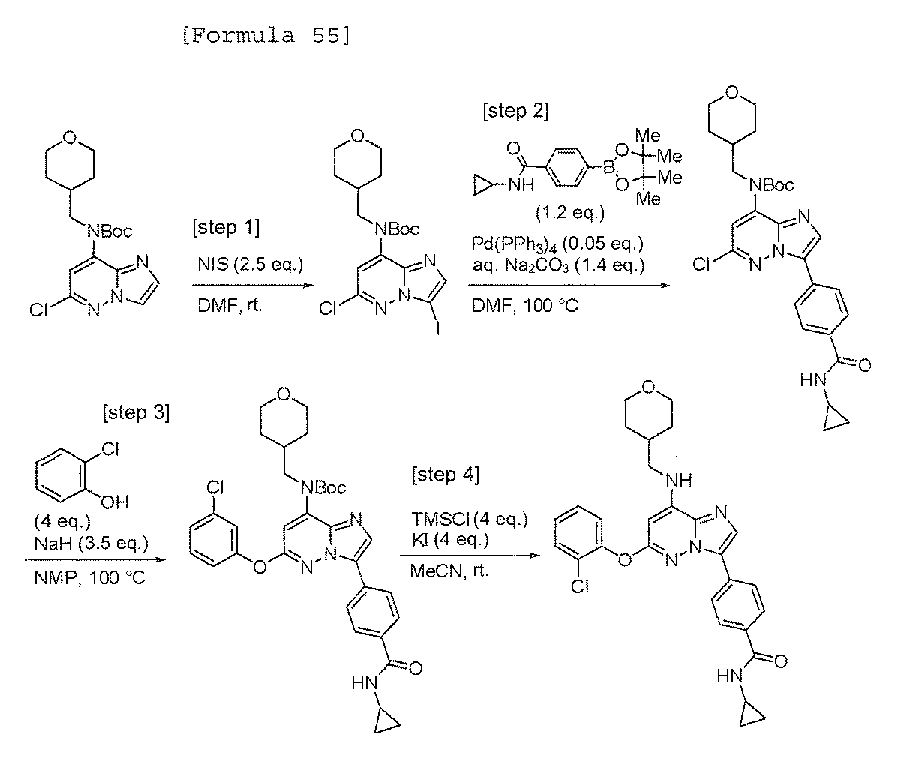

### Step 1: tert-Butyl 6-chloro-3-iodoimidazo[1,2-b]pyridazin-8-yl((tetrahydro-2H-pyran-4-yl)methyl)carbamate

To a DMF (10 mL) solution of tert-butyl 6-chloroimidazo[1,2-b]pyridazin-8-yl((tetrahydro-2H-pyran-4-yl)methyl)carbamate (1.25 g, 3.40 mol), NIS (1.92 g, 8.51 mmol) was added and stirred at room temperature for 17 hours. To the reaction solution, a 10% aqueous NaHSO₃ solution and ethyl acetate were added and separate phases. Thereafter, the water phase was extracted with ethyl acetate and organic phases were combined, washed with a 10% aqueous potassium carbonate solution and saturated saline, and dried over magnesium sulfate. The resultant residue was purified by medium-pressure silica gel chromatography (hexane/ethyl acetate; 20-50% ethyl acetate gradient) to obtain the titled compound (1.66 g, 3.36 mmol, 99%) as a yellow amorphous substance.
1H-NMR (300 MHz, DMSO-d₆) δ 1.01-1.20 (m, 2H), 1.33 (s, 9H), 1.46-1.57 (m, 2H), 1.58-1.75 (m, 1H), 3.13 (t, J = 10.9 Hz, 2H), 3.69-3.80 (m, 2H), 3.89 (d, J = 7.2 Hz, 2H), 7.53 (s, 1H), 7.92 (s, 1H).
MS (ESI) m/z = 493 (M+H)⁺.
LC/MS t_{R} = 2.50 min.

### Step 2: tert-Butyl 6-chloro-3- (4-(cyclopropylcarbamoyl)phenyl)imidazo[1,2-b]pyridazin-8-yl((tetrahydro-2H-pyran-4-yl)methyl)carbamate

To a DMF (40 mL) solution of tert-butyl 6-chloro-3-iodoimidazo[1,2-b]pyridazin-8-yl((tetrahydro-2H-pyran-4-yl)methyl)carbamate (4.03 g, 8.18 mmol), 4-(cyclopropyl carbamoyl)phenyl boronic acid pinacol ester (2.82 g, 9.81 mmol) and a 2M aqueous sodium carbonate solution (5.73 mL, 11.5 mmol), Pd(PPh₃)₄ (473 mg, 0.409 mmol) was added and stirred at 100°C for 23 hours. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 50-100% ethyl acetate gradient) to obtain the titled compound (1.54 g, 2.93 mmol, 36%) as yellow oil.
1H-NMR (300 MHz, CDCl₃) δ 0.64-0.72 (m, 2H), 0.90-0.96 (m, 2H), 1.26-1.41 (m, 2H), 1.52 (s, 9H), 1.56-1.67 (m, 2H), 1.74-1.89 (m, 1H), 2.92-3.02 (m, 1H), 3.26-3.38 (m, 2H), 3.95 (dd, J = 11.6, 2.7 Hz, 2H), 4.20 (d, J = 7.2 Hz, 2H), 6.36 (s, 1H), 7.18 (s, 1H), 7.90 (d, J = 6.0 Hz, 2H), 8.06 (s, 1H), 8.15 (d, J = 6.0 Hz, 2H).

### Step 3: tert-Butyl 6-(2-chlorophenoxy)-3-(4-(cyclopropylcarbamoyl)phenyl)imidazo[1,2-b]pyridazin-8-yl((tetrahydro-2H-pyran-4-yl)methyl)carbamate

To an NMP (5.0 mL) solution of 2-chlorophenol (293 g, 2.28 mmol) and 60% sodium hydride (300 mg, 2.00 mmol) was stirred at 40°C for 30 minutes and then tert-butyl 6-chloro-3-(4-(cyclopropylcarbamoyl)phenyl)imidazo[1,2-b]pyridazin-8-yl((tetrahydro-2H-pyran-4-yl)methyl)carbamate (300 mg, 0.570 mmol) was added and stirred at 100°C for 8 hours. To the reaction solution, a saturated aqueous ammonium chloride solution and ethyl acetate were added and separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 50-100% ethyl acetate gradient) to obtain the titled compound (282 mg, 0.456 mmol, 80%) as a white solid substance.
MS (ESI) m/z = 618 (M+H)⁺.
LC/MS t_{R} = 2.29 min.

### Step 4: Titled compound

To an acetonitrile (4.0 mL) solution of tert-butyl 6-(2-chlorophenoxy)-3-(4-(cyclopropylcarbamoyl)phenyl)imidazo[1,2-b]pyridazin-8-yl((tetrahydro-2H-pyran-4-yl)methyl)carbamate (282 mg, 0.456 mmol) and potassium iodide (303 mg, 1.83 mmol), chlorotrimethylsilane (0.233 mL, 1.83 mmol) was added and stirred at room temperature for 2 hours. To the reaction solution, saturated sodium bicarbonate water was added and a precipitated solid substance was obtained by filtration. The resultant residue was washed with hexane/ethyl acetate to obtain the titled compound (167 mg, 0.321 mmol, 71%) as a white solid substance.
1H-NMR (300 MHz, DMSO-d₆) δ 0.52-0.74 (m, 4H), 1.16-1.35 (m, 2H), 1.60-1.72 (m, 2H), 1.99 (m, 1H), 2.83 (m, 1H), 3.22-3.32 (m, 4H), 3.81-3.91 (m, 2H), 6.20 (s, 1H), 7.38 (m, 1H), 7.46-7.50 (m, 2H), 7.63 (d, J = 8.7 Hz, 2H), 7.65 (m, 1H), 7.80 (t, J = 6.0 Hz, 1H), 7.86 (d, J = 8.7 Hz, 2H), 8.01 (s, 1H), 8.40 (d, J = 3.9 Hz, 1H).
MS (ESI) m/z = 518 (M+H)⁺.
LC/MS t_{R} = 1.96 min.

[Table 2-3A]

**Table 2-3**

| | | |
|---|---|---|
| 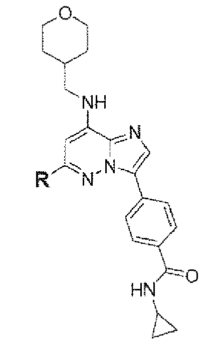 | | |

| Compounds described in Table 2-3 were synthesized in accordance with the process of Example 2-90 mentioned above. | | |
|---|---|---|
| Example No. | R | property data |
| Example 2-91 | 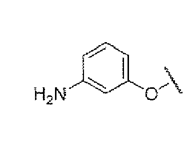 | 1H-NMR (300 MHz, DMSO-d₆) δ 0.52-0.74 (m, 4H), 1.15-1.32 (m, 2H), 1.56-1.68 (m, 2H), 1.95 (m, 1H), 2.83 (m, 1H), 3.15-3.32 (m, 4H), 3.78-3.90 (m, 2H), 5.25 (s, 2H), 6.01 (s, 1H), 6.32-6.47 (m, 3H), 7.06 (t, J = 8.1 Hz, 1H), 7.71 (t, J = 6.3 Hz, 1H), 7.75 (d, J = 8.7 Hz, 2H), 8.01(s, 1H), 8.07 (d, J = 8.7 Hz, 2H), 8.43 (d, J = 3.9 Hz, 1H). |
| | | MS (ESI) m/z = 499 (M+H)⁺. |
| | | LC/MS t_{R} = 1.47 min. |
| Example 2-92 | 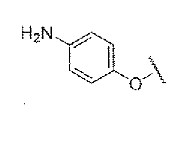 | 1H-NMR (300 MHz, DMSO-d₆) δ 0.52-0.74 (m, 4H), 1.16-1.33 (m, 2H), 1.57-1.68 (m, 2H), 1.95 (m, 1H), 2.83 (m, 1H), 3.17-3.32 (m, 4H), 3,80-3,90 (m, 2H), 5.06 (s, 2H), 5.97 (s, 1H), 6.63 (d, J = 9.0 Hz, 2H), 6.94 (d, J = 9.0 Hz, 2H), 7.64 (t, J = 6.0 Hz, 1H), 7.72 (d, J = 8.7 Hz, 2H), 8.00 (s, 1H), 8.05 (d, J = 8.7 Hz, 2H), 8.43 (d, J = 3.9 Hz, 1H). |
| | | MS (ESI) m/z = 499 (M+H)⁺. |
| | | LC/MS t_{R} = 1.23 min. |

**[Table 2-3B]**

| Example No. | R | property data |
|---|---|---|
| Example 2-93 | 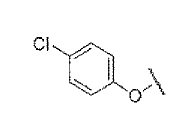 | 1H-NMR (300 MHz, DMSO-d₆) δ 0.53-0.75 (m, 4H), 1,16-1.34 (m, 2H), 1,58-1.71 (m, 2H), 1.99 (m, 1H), 2.83 (m, 1H), 3.18-3.32 (m, 4H), 3.80-3.90 (m, 2H), 6.13 (s, 1H), 7.34 (d, J = 9.0 Hz, 2H), 7.53 (d, J = 9.0 Hz, 2H), 7.72 (d, J = 8.7 Hz, 2H), 7.81 (t, J = 6.0 Hz, 1H), 7.97 (d, J = 8.7 Hz, 2H), 8.02 (s, 1H), 8.44 (d, J = 4.2 Hz, 1H). |
| | | MS (ESI) m/z =518 (M+H)⁺. |
| | | LC/MS t_{R} = 2.02 min. |
| Example 2-94 | 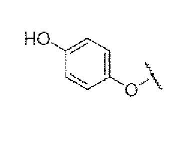 | 1H-NMR (300 MHz, DMSO-d₆) δ 0.54-0.75 (m, 4H), 1.15-1.34 (m, 2H), 1.58-1.70 (m, 2H), 1.96 (m, 1H), 2.84 (m, 1H), 3.17-3.34 (m, 4H), 3.80-3.90 (m, 2H), 6.02 (s, 1H), 6.83 (d, J = 8.7 Hz, 2H), 7.09 (d, J = 8.7 Hz, 2H), 7.67 (t, J = 6.0 Hz, 1H), 7.71 (d, J = 8.7 Hz, 2H), 8.00 (s, 1H), 8.01 (d, J = 8.7 Hz, 2H), 8.43 (d, J = 3.9 Nz, 1H), 9.45 (s, 1H). |
| | | MS (ESI) m/z = 500 (M+H)⁺, |
| | | LC/MS t_{R} = 1.55 min. |
| Example 2-95 | 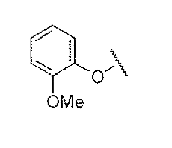 | 1H-NMR (300 MHz, DMSO-d₆) δ 0.52-0.74 (m, 4H), 1.16-1.34 (m, 2H), 1.60-1.72 (m, 2H), 1.99 (m, 1H), 2.83 (m, 1H), 3.20-3.30 (m, 4H), 3.73 (s, 3H), 3.82-3.92 (m, 2H), 6.09 (s, 1H), 7.03 (dt, J = 1.5 Hz, 7.8 Hz, 1H), 7.20-7.27 (m, 2H), 7.33 (m, 1H), 7.63 (d, J = 8.7 Hz, 2H), 7.67 (t, J = 6.3 Hz, 1H), 7.89 (d, J = 8.7 Hz, 2H), 7.99 (s, 1H), 8.41 (d, J = 4,2 Hz, 1H). |
| | | MS (ESI) m/z = 514 (M+H)⁺. |
| | | LC/MS t_{R} = 1.77 min. |
| Example 2-96 | 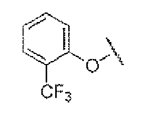 | 1H-NMR (300 MHz, DMSO-d₆) δ 0.52-0,74 (m, 4H), 1.17-1.35 (m, 2H), 1.58-1.73 (m, 2H), 1.99 (m, 1H), 2.83 (m, 1H), 3.18-3.32 (m, 4H), 3.80-3.91 (m, 2H), 6.19 (s, 1H), 7.55 (t, J = 7.8 Hz, 1H), 7.61 (t, J = 7.8 Hz, 1H), 7.65 (d, J = 8.7 Hz, 2H), 7.77-7.86 (m, 3H), 7.88 (d, J = 8.7 Hz, 2H), 8.02 (s, 1H), 8.40 (d, J = 4.2 Hz, 1H), |
| | | MS (ESI) m/z = 552 (M+H)⁺. |
| | | LC/MS t_{R} = 2.01 min. |

**[Table 2-3C]**

| Example No. | R | property data |
|---|---|---|
| Example 2-97 | 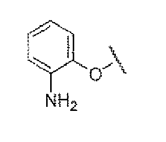 | 1H-MMR (300 MHz, DMSO-d₆) δ 0.54-0.73 (m, 4H), 1.16-1.34 (m, 2H), 1.58-1.70 (m, 2H), 1.99 (m, 1H), 2.83 (m, 1H), 3.14-3.34 (m, 4H), 3.80-3.92 (m, 2H), 4.96 (s, 2H), 6.06 (s, 1H), 6.62 (dt, J = 1.5 Hz, 7.8 Hz, 1H), 6.83 (dd. J = 1.5 Hz, 7.8 Hz, 1H), 6.98-7.09 (m, 2H), 7.66 (t, J = 6.3 Hz, 1H), 7.68 (d, J = 8.7 Hz, 2H), 8.00 (s, 1H), 8.02 (d, J = 8,7 Hz, 2H), 8.42 (d, J = 4.2 Hz, 1H). |
| | | MS (ESI) m/z = 499 (M+H)⁺. |
| | | LC/MS t_{R} = 1.89 min. |
| Example 2-98 | 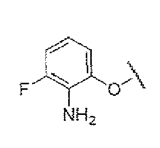 | 1H-NMR (300 MHz, DMSO-d₆) δ 0.54-0.75 (m, 4H), 1.16-1.34 (m, 2H), 1.60-1.70 (m, 2H), 1.99 (m, 1H), 2.83 (m, 1H), 3.20-3.32 (m, 4H), 3.82-3.92 (m, 2H), 5.02 (s, 2H), 6.1 (s, 1H), 6.62 (m, 1H), 6.84-7.07 (m, 2H), 7.68 (d, J = 8,7 Hz, 2H), 7.69 (m, 1H), 8.00 (d, J = 8.7 Hz, 2H), 8.01 (s, 1H), 8.43 (d, J = 4.2 Hz, 1H), |
| | | MS (ESI) m/z = 517 (M+H)⁺. |
| | | LC/MS t_{R} = 1.71 min, |
| Example 2-99 | 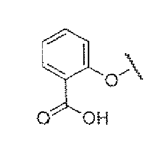 | 1H-NMR (300 MHz, DMSO-d₆) δ 0.53-0.74 (m, 4H), 1.16-1.38 (m, 2H), 1.58-1.72 (m, 2H), 1.96 (m, 1H), 2.82 (m, 1H), 3.18-3.32 (m, 4H), 3.82-3.92 (m, 2H), 6.13 (s, 1H), 7.35 (d, J = 7.5 Hz, 1H), 7.45 (m, 1H), 7.62 (d, J = 8.7 Hz, 2H), 7.64-7.72 (m, 2H), 7.86 (d, J = 8.7 Hz, 2H), 7.94 (s, 1H), 7.97 (m, 1H), 8.41 (d, J = 3.9 Hz, 1H), 12.75 (brs, 1H). |
| | | MS (ESI) m/z = 528 (M+H)⁺. |
| | | LC/MS t_{R} 1.51 min. |
| Example 2-100 | 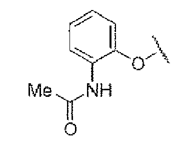 | 1H-NMR (300 MHz, DMSO-d₆) δ 0.54-0.74 (m, 4H), 1.16-1.35 (m, 2H), 1.60-1.71 (m, 2H), 1.99 (m, 1H), 2.01 (s, 3H), 2.83 (m, 1H), 3.20-3.34 (m, 4H), 3.81-3.91 (m, 2H), 6.10 (s, 1H), 7.15-7.32 (m, 3H), 7.67 (d, J = 8.4 Hz, 2H), 7.75 (t, J = 6.0 Hz, 1H), 7.94 (d, J = 8.4 Hz, 2H), 7.97 (m, 1H), 8.01 (s, 1H), 8,42 (d, J = 4.2 Hz, 1H), 9.43 (s, 1H). |
| | | MS (ESI) m/z = 541 (M+H)⁺, |
| | | LC/MS t_{R} = 1.50 min. |

**[Table 2-3D]**

| Example No. | R | property data |
|---|---|---|
| Example 2-101 | 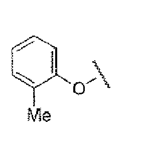 | 1H-NMR (400 MHz, DMSO-d₆) δ 0.59 (br s, 2H), 0.67-0.75 (m, 2H), 1.20-1.35 (m, 2H), 1.62-1.73 (m, 2H), 1.99 (s, 1H), 2.21 (s, 3H), 2.85 (br s, 1H), 3.22-3.34 (m, 4H), 3.88 (d, J = 9.3 Hz, 2H), 6.14 (s, 1H), 7.20-7.30 (m, 2H), 7.30-7.36 (m, 1H), 7.36-7.43 (m, 1H), 7.64-7.77 (m, 3H), 7.93 (d, J = 7.3 Hz, 2H), 8.04 (s, 1H), 8.44 (s, 1H). |
| | | MS (ESI) m/z = 498 (M+H)⁺, |
| | | LC/MS t_{R} = 1.98 min, |
| Example 2-102 | 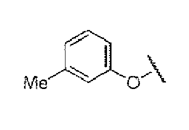 | 1H-NMR (400 MHz, DMSO-d₆) δ 0.56 (br s, 2H), 0.65-0.73 (m, 2H), 1.17-1.31 (m, 2H), 1.58-1.67 (m, 2H), 1,95 (br s, 1H), 2.35 (s, 3H), 2.83 (br s, 1H), 3.18-3.30 (m, 4H), 3.84 (d, J = 9.1 Hz, 2H), 6.06 (s, 1H), 7.03-7.16 (m, 3H), 7.34 (t, J = 7.6 Hz, 1H), 7.68-7.75 (m, 3H), 7.96-8.04 (m, 3H), 8.43 (s, 1H). |
| | | MS (ESI) m/z = (M+H)⁺. |
| | | LC/MS t_{R} = 1.59 min. |
| Example 2-103 | 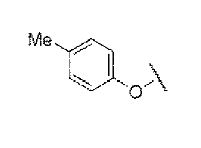 | 1H-NMR (400 MHz, DMSO-d₆) δ 0.56 (br s, 2H), 0.64-0.72 (m, 2H), 1.17-1.31 (m, 2H), 1.58-1.66 (m, 2H), 1.94 (br s, 1H), 2.35 (s, 3H), 2.83 (s, 1H), 3.19-3.30 (m, 4H), 3.84 (d, J = 10.1 Hz, 2H), 5.74 (s, 1H), 6.05 (s, 1H), 7.15 (d, J = 7.6 Hz, 2H), 7.26 (d, J = 7.6 Hz, 2H), 7.66-7.74 (m, 3H), 7.95-8.03 (m, 3H), 8.42 (s, 1H): |
| | | MS (ESI) m/z = 498 (M+H)⁺. |
| | | LC/MS t_{R} = 2.00 min. |
| Example 2-104 | 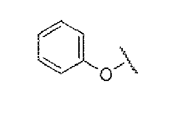 | 1H-NMR (400 MHz, COCl₃) δ 0.60-0.65 (m, 2H), 0.88 (q, J = 6.1 Hz, 2H), 1.38-1.49 (m, 2H), 1.17 (dd, J = 2.0. 13,7 Hz, 2H), 1.93-2.04 (m, 1H), 2.86-2.94 (m, 1H), 3.26 (t, J = 6.1 Hz, 2H), 3.42 (dt, J = 2.0, 11.7 Hz, 2H), 4.03 (dd, J = 11.7. 3.0 Hz, 2H), 5.88 (s, 2H), 6.16 (s, 1H), 7.22-7.31 (m, 3H), 7.44 (t, J = 7,6 Hz, 2H), 7.61 (d, J = 8.6 Hz, 2H), 7.77 (s, 1H), 7.92 (d, J = 8.6 Hz, 2H). |
| | | MS (ESI) m/z = 484 (M+H)⁺. |
| | | LC/MS t_{R} = 2.06 min. |

**[Table 2-3E]**

| Example No. | R | property data |
|---|---|---|
| Example 2-105 | 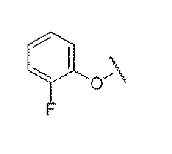 | 1H-NMR (400 MHz, CDCl₃) δ 0.60-0.65 (m, 2H), 0.88 (q, J = 6.6 Hz, 2H), 1.40-1.52 (m, 2H), 1.78 (d, J = 12.7 Hz, 2H), 1.96-2.06 (m, 1H), 2.86-2.94 (m, 1H), 3.28 (t, J = 6.1 Hz, 2H), 3.43 (t, J = 11.7 Hz, 2H), 4.00-4.07 (m, 2H), 5.89-5.94 (m, 1H), 5.96 (s, 1H), 6.16 (br s, 1H), 7.18-7.33 (m, 4H), 7.57 (d, J = 8.1 Hz, 2H), 7,75 (s, 1H), 7.84 (d, J = 8.1 Hz, 2H). |
| | | MS (ESI) m/z = 502 (M+H)⁺. |
| | | LC/MS t_{R} = 2.11 min. |
| Example 2-106 | 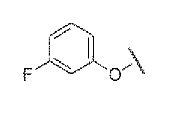 | 1H-NMR (400 MHz, CDCl₃) δ 0.60-0.66 (m, 2H), 0.89 (q, J = 6.4 Hz, 2H), 1,39-1.50 (m, 2H), 1.74-1.80 (m, 2H), 1.94-2.06 (m, 1H), 2.87-2.95 (m, 1H), 3.26 (t, J = 6.6 Hz, 2H), 3.38-3.46 (m, 2H), 4.03 (dd, J = 11,7, 3.5 Hz, 1H), 5.87 (s, 1H), 5.95 (t, J = 6.6 Hz, 1H), 6.19 (br s, 1H), 6.97-7.06 (m, 3H), 7.35-7.42 (m, 1H), 7.65 (d, J = 8.6 Hz, 2H), 7.78 (s, 1H), 7.93 (d, J = 8.6 Hz, 2H). |
| | | MS (ESI) m/z = 502 (M+H)⁺. |
| | | LC/MS t_{R} = 2.14 min. |
| Example 2-107 | 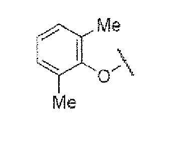 | 1H-NMR (400 MHz, CDCl₃) δ 0,59-0.65 (m, 2H), 0.88 (q, J = 6.4 Hz, 2H), 1.38-1.53 (m, 2H), 1.75-1.84 (m, 2H), 1.95-2.05 (m, 1H), 2.19 (s, 6H), 2.86-2.93 (m, 1H), 3.28 (t, J = 6.6 Hz, 2H), 3.39-3.47 (m, 2H), 4.00-4.07 (m, 2H), 5.82 (t, J = 5.6 Hz, 1H), 5.92 (s, 1 H), 6.14 (br s, 1H), 7.14 (s, 3H), 7.53 (d, J = 8.6 Hz, 2H), 7.77 (s, 1H), 7.82 (d, J = 8.6 Hz, 2H). |
| | | MS (ESI) m/z = 512 (M+H)⁺. |
| | | LC/MS t_{R} = 2.29 min. |
| Example 2-108 | 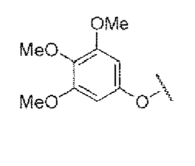 | 1H-NMR (400 MHz, CDCl₃) δ 0.62-0.67 (m, 2H), 0.88 (q, J = 6.6 Hz, 2H), 1.38-1.50 (m, 2H), 1.77 (d, J = 11.7 Hz, 2H), 1.93-2.03 (m, 1H), 2.85-2.94 (m, 1H), 3.26 (t, J = 6.6 Hz, 2H), 3.38-3.45 (m, 2H), 3.82 (s, 6H), 3.90 (s, 3H), 3.99-4.06 (m, 2H), 5.85 (s, 1H), 5.89 (t, J 5,1 Hz, 1H), 6.21 (br s, 1H), 6.52 (s, 2H), 7.66 (d, J = 8.6 Hz, 2H), 7.79 (s, 1H), 7.99 (d, J = 8.6 Hz, 2H). |
| | | MS (ESI) m/z = 574 (M+H)⁺. |
| | | LC/MS t_{R} = 1.96 min. |

**[Table 2-3F]**

| Example No. | R | property data |
|---|---|---|
| Example 2-109 | 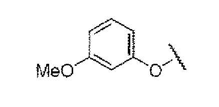 | 1H-NMR (400 MHz, CDCl₃) δ 0.59-0.66 (m, 2H), 0.85-0.92 (m, 2H), 1.37-1.49 (m, 2H), 1.73-1.79 (m, 2H), 1.92-2.03 (m, 1H), 2.86-2.95 (m, 1H), 3.25 (t, J = 6.6 Hz, 2H), 3.37-3.45 (m, 2H), 3.81 (s, 3H), 3.99-4.05 (m, 2H), 5.84-5.91 (m, 2H), 6.19 (br s, 1H), 6.79-6.86 (m, 3H), 7.33 (t, J = 8.1 Hz, 1H), 7.65 (d, J = 8.1 Hz, 2H), 7.78 (s, 1H), 7,97 (d, J = 8.1 Hz, 2H), |
| | | MS (ESI) m/z = 514 (M+H)⁺. |
| | | LC/MS t_{R} = 2.09 min. |
| Example 2-110 | 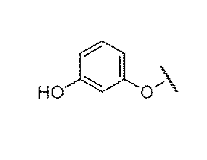 | 1H-NMR (300 MHz, DMSO-d₆) δ 0.57-0.58 (m, 2H), 0.68-0.69 (m, 2H), 1.22-1.28 (m, 2H), 1.63 (d, J = 11.0 Hz, 2H), 1.97-2.00 (m, 1H), 2.83-2.84 (m, 1H), 3.25-3.29 (m, 4H), 3.85 (dd, J = 11.3, 2.6 Hz, 2H), 6.06 (s, 1H), 6.62-6.70 (m, 3H), 7.24 (t, J = 8.1 Hz, 1 H), 7.73 (d, J = 8.2 Hz, 3H), 8.03 (d, J = 7.5 Hz, 3H), 8.44 (d, J = 4.1 Hz, 9H), 9.68 (s, 1H), |
| | | MS (ESI) m/z = 500 (M+H)⁺. |
| | | LC/MS t_{R} = 1,58 min. |
| Example 2-111 | 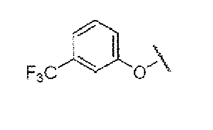 | 1H-NMR (300 MHz, DMSO-d₆) δ 0.55-0.57 (m, 2H), 0.66-0.72 (m, 2H), 1.23-1.32 (m, 2H), 1.65 (d, J = 11.3 Hz, 2H), 1.97-1.98 (m, 1H), 2.81-2.84 (m, 1H), 3.25-3.28 (m, 4H), 3.86 (dd, J = 11.1. 2.9 Hz, 2H), 6.17 (s, 1H), 7.61-7.85 (m, 7H), 7.92 (d, J = 8.4 Hz, 2H), 8.01 (s, 1H), 8.41 (d, J = 4.1 Hz, 1H), |
| | | MS (ESI) m/z = 552 (M+H)⁺, |
| | | LC/MS t_{R} = 2,10 min. |
| Example 2-112 | 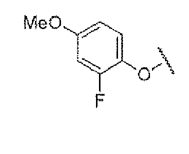 | 1H-NMR (300 MHz, DMSO-d₆) δ 0.56-0.68 (m, 2H), 0.67-0.70 (m, 2H), 1.24-1.28 (m, 2H), 1.66 (d, J = 10.8 Hz, 2H), 1.97-2.00 (m, 1H), 2.82-2.84 (m, 1H), 3.25-3.27 (m, 4H), 3.85-3.87 (m, 5H), 6.16 (s, 1H), 6.85-6.89 (m, 1H), 7.10 (dd, J = 12.5, 2.9 Hz, 1H), 7.37 (t, J = 9.1 Hz, 1H), 7.67 (d, J = 8.4 Hz, 2H), 7.78 (t, J = 6.1 Hz, 1H), 7.92 (d, J = 8.4 Hz, 2H), 8.01 (s, 1H), 8.43 (d, J = 4.1 Hz, 1H). |
| | | MS (ESI) m/z = 532 (M+H)⁺. |
| | | LC/MS t_{R} = 1.89 min. |

**[Table 2-3G]**

| Example No. | R | property data |
|---|---|---|
| Example 2-113 | 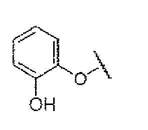 | 1H-NMR (300 MHz, DMSO-d₆) δ 0.56-0.59 (m, 2H), 0.66-0.72 (m, 2H), 1.24-1.28 (m, 2H), 1.66 (d, J = 12.9 Hz, 2H), 1.97-2.00 (m, 1H), 2.82-2.84 (m, 1H), 3.23-3.27 (m, 4H), 3.86 (dd, J = 11.3, 2.6 Hz, 2H), 6.07 (s, 1H), 6.88 (td, J = 7.6. 1.4 Hz, 1H), 7.02 (d, J = 7.6 Hz, 1H), 7.14-7.18 (m, 2H), 7.61-7.65 (m, 3H), 7.95-7.98 (m, 3H), 8.40 (d, J = 4.0 Hz, 1H), 9.56 (s, 1H). |
| | | MS (ESI) m/z = 500 (M+H)⁺. |
| | | LC/MS t_{R} = 1.61 min. |
| Example 2-114 | 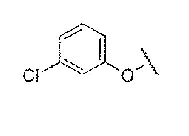 | 1H-NMR (300 MHz, DMSO-d₆) δ 0.56-0.60 (m, 2H), 0.69-0.71 (m, 2H), 1.24-1.29 (m, 2H), 1.66 (d, J = 12.6 Hz, 2H), 1.97-1.99 (m, 1H), 2.83-2.85 (m, 1 H), 3.28 (t, J = 10.7 Hz, 4H), 3.84-3.88 (m, 2H), 6.19 (s, 1H), 7.28-7.31 (m, 1H), 7.36-7.39 (m, 1H), 7.48-7.53 (m, 2H), 7.74 (d, J = 8.6 Hz, 2H), 7.82 (t, J = 6.0 Hz, 1H), 7.98 (d, J = 8.7 Hz, 2H), 8.09 (s, 1 H), 8,43 (d, J = 4.0 Hz, 1H). |
| | | MS (ESI) m/z = 518 (M+H)⁺. |
| | | LC/MS t_{R} = 2.03 min. |
| Example 2-115 | 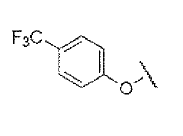 | 1H-NMR (300 MHz, DMSO-d₆) δ 0.55-0.57 (m, 2H), 0.66-0.72 (m, 2H), 1.23-1.31 (m, 2H), 1.65 (d, J = 12.4 Hz, 2H), 1.97-1.98 (m, 1H), 2.82-2.84 (m, 1H), 3.24-3.26 (m, 4H), 3.85 (dd, J = 11.4. 2.7 Hz, 2H), 6.18 (s, 1H), 7.50 (d, J = 8.4 Hz, 2H), 7.71 (d, J = 8.6 Hz, 2H), 7.84-7.86 (m, 3H), 7.95 (d, J = 8.6 Hz, 2H), 8.04 (s, 1H), 8.40 (d, J = 4.2 Hz, 1H). |
| | | MS (ESI) m/z = 552 (M+H)⁺. |
| | | LC/MS t_{R} = 2.10 min. |
| Example 2-116 | 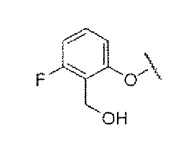 | 1H-NMR (300 MHz, DMSO-d₆) δ 0.54-0.59 (m, 2H), 0.66-0.72 (m, 2H), 1.22-1.30 (m, 2H), 1.64 (d, J = 11.6 Hz, 2H), 1.95-1.88 (m, 1H), 2.79-2.84 (m, 1H), 3.23-3.27 (m, 4H), 3.85 (dd, J = 11.0. 2.9 Hz, 2H), 4.55 (d, J = 5.8 Hz, 2H), 5.29 (t, J = 5.7 Hz, 1 H), 6.09 (s, 1H), 7,14-7.16 (m, 1H), 7.20-7.23 (m, 2H), 7.41 (t, J = 8.0 Hz, 1H), 7.69-7.77 (m, 3H), 7.98-8.00 (m, 3H), 8.41 (d, J 4,0 Hz, 1H). |
| | | MS (ESI) m/z = 514 (M+H)⁺. |
| | | LC/MS t_{R} = 1.53 min, |

**[Table 2-3H]**

| Example No. | R | property data |
|---|---|---|
| Example 2-117 | 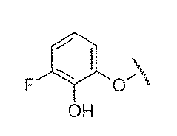 | 1H-NMR (300 MHz, DMSO-d₆) δ 0.57-0.58 (m, 2H), 0.66-0.72 (m, 2H), 1.26-1.30 (m, 2H), 1.66 (d, J = 12.6 Hz, 2H), 1.97-1.89 (m, 1H), 2.82-2.84 (m, 1H), 3.25-3.28 (m, 4H), 3.86 (dd, J = 11,1, 2.7 Hz, 2H), 6.11 (s, 1H), 6.89 (td, J = 8.2. 5.9 Hz, 1H), 7.05 (d, J = 8.2 Hz, 1H), 7.15-7.19 (m, 1H), 7.66-7.69 (m, 3H), 7.93 (d, J = 8.4 Hz, 2H), 8.00 (s, 1 H), 8.42 (d, J = 4.0 Hz, 1H), 9.80 (s, 1H). |
| | | MS (ESI) m/z = 518 (M+H)⁺. |
| | | LC/MS t_{R} = 1.64 min. |
| Example 2-118 | 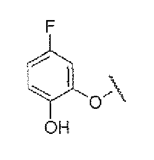 | 1H-NMR (300 MHz, DMSO-d₆) δ 0.57-0.58 (m, 2H), 0.68-0.69 (m, 2H), 1.26-1.28 (m, 2H), 1.66 (d, J = 12.8 Hz, 2H), 1.97-2.00 (m, 1H), 2.82-2.84 (m, 1H), 3.25-3.28 (m, 4H), 3.86 (dd, J = 11.3, 2.6 Hz, 2H), 6.09 (s, 1H), 6.99-7.03 (m, 2H), 7.16 (dd, J = 9.2. 2.5 Hz, 1H), 7.67 (d, J = 8.6 Hz, 3H), 7.95 (d, J = 8.4 Hz, 2H), 8.00 (s, 1H), 8.42 (d, J = 4.0 Hz, 1H), 9.56 (s, 1H). |
| | | MS (ESI) m/z = 518 (M+H)⁺. |
| | | LC/MS t_{R} = 1.65 min. |
| Example 2-119 | 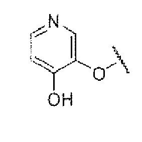 | 1H-NMR (300 MHz, DMSO-d₆) δ 0.55-0.61 (m, 2H), 0.63-0.73 (m, 2H), 1.26 (ddd, J = 24.5, 12.1, 4.2 Hz, 2H), 1.66 (d, J = 11.1 Hz, 2H), 1.94-2.01 (m, 1H), 2.81-2.86 (m, 1H), 3,25-3.28 (m, 4H), 3.86 (dd, J = 11.2. 2.9 Hz, 2H), 6.06 (s, 1H), 6.38 (s, 1H), 7.62 (t, J = 6.1 Hz, 1H), 7.70 (d, J = 8.4 Hz, 2H), 7.79 (d, J = 6.4 Hz, 1H), 7.96-8.00 (m, 4H), 8.13 (s, 1H), 8.42 (d, J = 4.2 Hz, 1H). |
| | | MS (ESI) m/z = 501 (M+H)⁺. |
| | | LC/MS t_{R} = 0.94 min. |
| Example 2-120 | 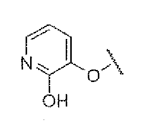 | 1H-NMR (300 MHz, DMSO-d₆) δ 0.57-0.58 (m, 2H), 0.66-0.73 (m, 2H), 1.26-1.30 (m, 2H), 1.66 (d, J = 12.4 Hz, 2H), 1.97-1.98 (m, 1H), 2.82-2.85 (m, 1H), 3.25-3.28 (m, 4H), 3.86 (dd, J = 11.3, 2.8 Hz, 2H), 6.09 (s, 1H), 6.27 (t, J = 6.8 Hz, 1H), 7.41-7.45 (m, 2H), 7.67-7.72 (m, 3H), 7.99 (t, J = 4.3 Hz, 3H), 8.42 (d, J = 4.2 Hz, 1H), 12.04 (s, 1H), |
| | | MS (ESI) m/z = 501 (M+H)⁺. |
| | | LC/MS t_{R} = 1.22 min. |

Example 2-121

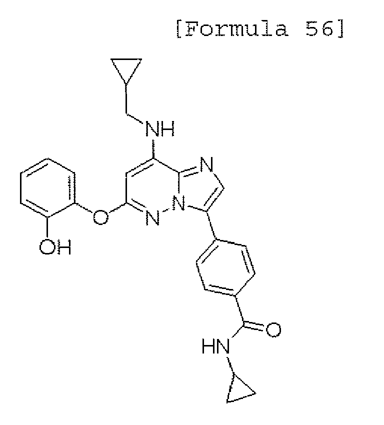

The titled compound was synthesized in accordance with the process of Example 2-90.
1H-NMR (300 MHz, DMSO-d₆) δ 0.31-0.33 (m, 2H), 0.47-0.53 (m, 2H), 0.55-0.58 (m, 2H), 0.66-0.72 (m, 2H), 1.18-1-21 (m, 1H), 2.82-2.84 (m, 1H), 3.24 (t, J = 6.2 Hz, 2H), 6.06 (s, 1H), 6.85-6.91 (m, 1H), 7.02 (d, J = 7.2 Hz, 1H), 7.16 (t, J = 7.4 Hz, 2H), 7.52 (t, J = 6.0 Hz, 1H), 7.65 (d, J = 8.6 Hz, 2H), 7.96 (d, J = 8.4 Hz, 2H), 8.01 (s, 1H), 8.41 (d, J = 4.0 Hz, 1H), 9.55 (s, 1H).
MS (ESI) m/z = 456 (M+H)⁺.
LC/MS t_{R} = 1.89 min.

[Table 2-4A]

**Table 2-4**

| | | |
|---|---|---|
| 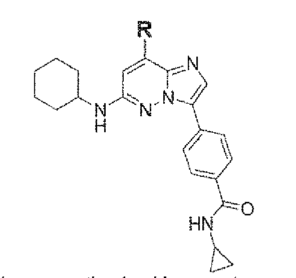 | | |

| Compounds described in Table 2-4 were synthesized in accordance with the process of Example 2-87 mentioned above. | | |
|---|---|---|
| Example No. | R | property data |
| Example 2-122 | 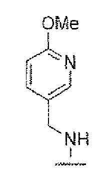 | 1H-NMR (400 MHz, DMSO-d₆) δ 0.58 (br s, 2H), 0.66-0,74 (m, 2H), 1.12-1.25 (m, 3H), 1.28-1.42 (m, 2H), 1.57-1.66 (m, 1H), 1.69-1.79 (m, 2H), 1.98-2.08 (m, 2H), 2.81-2.90 (m, 1H), 3.55 (br s, 1H), 3.81 (s, 3H), 4.36 (d, J = 5.3 Hz, 2H), 5.54 (s, 1H), 6.32 (d, J = 6.8 Hz, 1H), 6.79 (d, J = 8.3 Hz, 1H), 7.48-7.56 (m, 1H), 7.70 (d, J = 7.6Hz. 1H), 7.83-7.89 (m, 3H), 8.18 (s, 1H), 8.31 (d, J = 7.8 Hz, 2H), 8.43 (s, 1H). |
| | | MS (ESI) m/z = 512 (M+H)⁺, |
| | | LC/MS t_{R} = 1.70 min. |
| Example 2-123 | 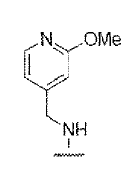 | 1H-NMR (400 MHz, DMSO-d₆) δ 0.60 (br s, 2H), 0.67-0.74 (m, 2H), 1.11-1.24 (m, 3H), 1.29-1.43 (m, 2H), 1.58-1.67 (m, 1H), 1.69-1.79 (m, 2H), 1.97-2.08 (m, 2H), 2.86 (s, 1H), 3.55 (br s, 1H), 3.82 (s, 3H), 4.43 (d, J = 5.1 Hz. 2H), 5.40 (s, 1H), 6.33 (d, J = 6.3 Hz, 1H), 6.72 (s, 1H), 6.96 (d, J = 4.5 Hz, 1H), 7.58-7.65 (m, 1H), 7.85-7.91 (m, 3H), 8.11 (d, J = 4.8 Hz, 1H), 8.33 (d, J = 8.3 Hz, 2H), 8.45 (s, 1H). |
| | | MS (ESI) m/z = 512 (M+H)⁺. |
| | | LC/MS t_{R} = 1.58 min. |

**[Table 2-4B]**

| Example No. | R | property data |
|---|---|---|
| Example 2-124 | 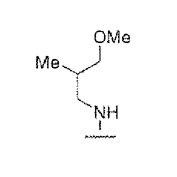 | 1H-NMR (400 MHz, DMSO-d₆) δ 0.59 (br s, 2H), 0.66-0.74 (m, 2H), 0.92 (d, J = 6.1 Hz, 3H), 1.13-1.30 (m, 3H), 1.31-1.45 (m, 2H), 1.61 (br s, 1H), 1.75 (br s, 2H), 2.00-2.19 (m, 3H), 2.85 (s, 1H), 2.97-3.21 (m, 2H), 3.21-3.30 (m, 5H), 3.60 (br s, 1H), 5.58 (s, 1H), 6.32 (d, J = 6.1 Hz, 1H), 6.81 (s, 1H), 7,80-7,92 (m, 3H), 8.32 (d, J = 8.1 Hz, 2H), 8.44 (s, 1H). |
| | | MS (ESI) m/z = 477 (M+H)⁺. |
| | | LC/MS t_{R} = 1.72 min. |
| Example 2-125 | 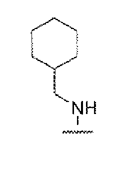 | 1H-NMR (400 MHz, DMSO-d₆) δ 0.57-0.61 (m, 2H), 0.68-0.73 (m, 2H), 0.91-0.99 (m, 2H), 1.16-1.28 (m, 6H), 1.35-1.44 (m, 2H), 1.62-1.80 (m, 9H), 2.06-2.09 (m, 2H), 2.82-2.89 (m, 1H), 3.03 (t, J = 6.34 Hz, 2H), 3.57-3.64 (m, 1H), 5.58 (s, 1H), 6.30 (d, J = 7.10 Hz, 1H), 6.83 (t, J = 5.83 Hz, 1H), 7.86 (d, J = 8.11 Hz, 2H), 8.32 (d, J = 8.62 Hz, 2H), 13.43 (d, J = 4.06 Hz, 1H). |
| | | MS (ESI) m/z = 487 (M+H)⁺. |
| | | LC/MS t_{R} = 2.29 min. |
| Example 2-126 | 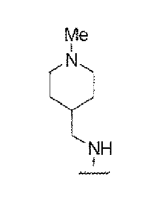 | 1H-NMR (400 MHz, DMSO-d₆) δ 0.57-0.61 (m, 2H), 0.68-0.73 (m, 2H), 1.19-1.28 (m, 5H), 1.35-1.41 (m, 2H), 1.63-1.68 (m, 4H), 1.75-1.84 (m, 4H), 2.05-2.09 (m, 2H), 2.14 (s, 3H), 2.76 (d, J = 10.65 Hz, 2H), 2.82-2.89 (m, 1H), 3.06 (t, J = 5.83 Hz, 2H), 3.56-3.65 (m, 1H), 5.59 (s, 1H), 6.29 (d, J = 7.10 Hz, 1H), 6.87 (t, J = 6.08 Hz, 1H), 7.82 (s, 1H), 7.86 (d, J = 8.62 Hz, 2H), 8.32 (d, J = 8.62 Hz, 2H), 8.43 (d, J = 4.06 Hz, 1H). |
| | | MS (ESI) m/z = 502 (M+H)⁺. |
| | | LC/MS t_{R} = 1.29 min. |

**[Table 2-4C]**

| Example No. | R | property data |
|---|---|---|
| Example 2-127 | 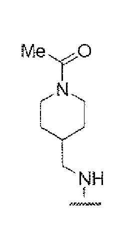 | 1H-NMR (400 MHz, DMSO-d₆) δ 0.63-0.67 (m, 2H), 0.90 (q, J = 6.42 Hz, 2H), 1.17-1.31 (m, 6H), 1.39-1.50 (m, 3H), 1.66-1.72 (m, 2H), 1.79-1.94 (m, 6H), 2,14-2.18 (m, 2H), 2.52-2.59 (m, 1H), 2,90-2.97 (m, 1H), 3.01-3.09 (m, 1H), 3.10-3.22 (m, 2H), 3.67-3.76 (m, 1H), 3.83-3.87 (m, 1H), 4.02 (d, J = 7.10 Hz, 1H), 4.66-4.70 (m, 1H), 5.35 (s, 1H), 5.60 (br s, 1H), 6.25 (s, 1H), 7.67 (s, 1H), 7.79 (d, J = 8.62 Hz, 2H), 8.21 (d, J = 8.11 Hz, 2H). |
| | | MS (ESI) m/z = 530 (M+H)⁺. |
| | | LC/MS t_{R} = 1.78 min. |
| Example 2-128 | 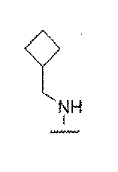 | 1H-NMR (400 MHz, DMSO-d₆) δ 0.57-0.63 (m, 2H), 0.67-0.75 (m, 2H), 1.15-1.30 (m, 4H), 1.33-1.48 (m, 2H), 1.60-1.92 (m, 8H), 1.98-2.12 (m, 5H), 2.65-2.75 (m, 1H), 2.82-2.91 (m, 1H), 3.18-3.26 (m, 2H), 3.56-3.68 (m, 1H), 5.58-5.62 (m, 1H), 6.28-6.35 (m, 1H), 6.76-6.82 (m, 1H), 7.81-7.90 (m, 3H), 8.30-8.36 (m, 2H), 8.42-8.47 (m, 1H). |
| | | MS (ESI) m/z = 459 (M+H)⁺. |
| | | LC/MS t_{R} = 2.06 min. |
| Example 2-129 | 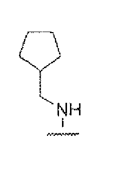 | 1H-NMR (400 MHz, DMSO-d₆) δ 0.57-0.61 (m, 2H), 0.68-0:73 (m, 2H), 1.17-1.33 (m, 6H), 1.33-1.46 (m, 2H), 1.46-1.82 (m, 10H), 2.07 (d, J = 10.14 Hz, 2H), 2.27-2.35 (m, 1H), 2.82-2.89 (m, 1H), 3,09 (t, J = 6.59 Hz, 2H), 3.56-3.67 (m, 1H), 5.61 (s, 1H), 6.31 (d, J = 7.10 Hz, 1H), 6.84 (t, J = 5.83 Hz, 1H), 7.83 (s, 1H), 7.87 (d, J = 8.62 Hz. 2H), 8.32 (d, J = 8.62 Hz, 2H), 8.44 (d, J = 4.06 Hz, 1H). |
| | | MS (ESI) m/z = 473 (M+H)⁺. |
| | | LC/MS t_{R} = 2.20 min. |

**[Table 2-4D]**

| Example No. | R | property data |
|---|---|---|
| Example 2-130 | 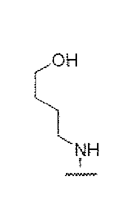 | 1H-NMR (400 MHz, DMSO-d₆) δ 0.57-0.61 (m, 2H), 0.68-0.73 (m, 2H), 1.18-1.29 (m, 3H), 1.33-1.46 (m, 2H), 1.46-1.84 (m, 2H), 1.60-1.70 (m, 3H), 1.73-1.82 (m, 2H), 2.43-2.12 (m, 2H), 2.86 (dq, J = 14,83, 3.72 Hz, 1H), 3.19 (q, J = 6.76 Hz, 2H), 3.42-3.46 (m, 2H), 3,56-3.67 (m, 1H), 4.44 (t, J = 5.07 Hz, 1H), 5.58 (s, 1H), 6.31 (d, J = 7.10 Hz, 1H), 6.81 (t, J = 5.83 Hz, 1H), 7.83 (s, 1H), 7.87 (d, J = 8.62 Hz, 2H), 8.32 (d, J = 8.62 Hz, 2H), 8.44 (d, J = 4.06 Hz, 1H). |
| | | MS (ESI) m/z = 463 (M+H)⁺. |
| | | LC/MS t_{R} = 1.56 min. |
| Example 2-131 | 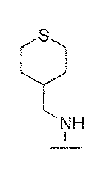 | 1H-NMR (400 MHz, CDCl₂) δ 0.63-0.67 (m, 2H), 0.90 (q, J = 6.42 Hz, 2H), 1.18-1.32 (m, 5H), 1.39-1.53 (m, 5H), 1.88-1.76 (m, 4H), 1.76-1.86 (m, 4H), 2.12-2.20 (m, 4H), 2.60-2.74 (m, 4H), 2.90-2.97 (m, 1H), 3.12 (t, J = 6.59 Hz, 2H), 3.66-3.77 (m, 1H), 4.01 (d, J = 7.60 Hz, 1H), 5.34 (s, 1H), 5.57 (br s, 1H), 6.24 (s, 1H), 7.66 (s, 1H), 7.79 (d, J = 8.62 Hz, 2H), 8.22 (d, J = 8.11 Hz, 2H). |
| | | MS (ESI) m/z = 505 (M+H)⁺. |
| | | LC/MS t_{R} = 2.70 min. |
| Example 2-132 | 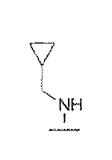 | 1H-NMR (400 MHz, CDCl₃) δ 0.31 (q, J = 5.07 Hz, 2H), 0.60-0.67 (m, 4H), 0.89 (q, J = 6.42 Hz. 2H), 1.13-1.31 (m, 5H), 1.39-1.50 (m, 2H), 1.67-1.72 (m, 1H), 1.78-1.83 (m, 2H), 2.14-2.18 (m, 2H), 2.90-2.97 (m, 1H), 3.09 (dd, J = 6.84, 5.32 Hz, 2H), 3.66-3.75 (m, 1H), 4.00 (d, J = 7.10 Hz, 1H), 5.34 (s, 1H), 5.61 (t, J = 5.07 Hz, 1H), 6.26 (s, 1H), 7.68 (s, 1H), 7.79 (d, J = 8.62 Hz, 2H), 8.23 (d, J = 8.11 Hz. 2H). |
| | | MS (ESI) m/z = 445 (M+H)⁺. |
| | | LC/MS t_{R} = 1.87 min. |

**[Table 2-4E]**

| Example No. | R | property data |
|---|---|---|
| Example 2-133 | 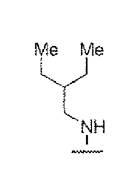 | 1H-NMR (400 MHz, CDCl₃) δ 0.63-0.67 (m, 2H), 0.87-0,95 (m, 8H), 1.18-1.31 (m, 4H), 1.39-1.49 (m, 6H), 1.57-1.72 (m, 4H), 1.78-1.83 (m, 2H), 2.15-2.19 (m, 2H), 2.90-2.97 (m, 1H), 3.15 (t, J = 6.08 Hz, 2H), 3.66-3.75 (m, 1H), 4.02 (d, J = 7.60 Hz, 1H), 5.36 (s, 1H), 5,47 (t, J = 5.58 Hz, 1H), 6.27 (s, 1H), 7.26 (s, 1H), 7.66 (s, 1H), 7.79 (d, J = 8.13 Hz. 2H), 8.22 (d, J = 8.62 Hz, 2H). |
| | | MS (ESI) m/z = 475 (M+H)⁺. |
| | | LC/MS t_{R} = 2.25 min. |
| Example 2-134 | 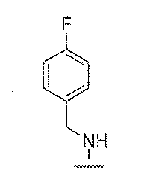 | 1H-NMR (400 MHz, DMSQ-d₆) δ 0.56-0.62 (m, 2H), 0.68-0.73 (m, 2H), 1.12-1.27 (m, 3H), 1.29-1.43 (m, 2H), 1.58-1.68 (m, 1H), 1.70-1.80 (m, 2H), 1.97-2.07 (m, 2H), 2.81-2.90 (m, 1H), 3.50-3.61 (m, 1H), 4.42 (d, J = 6.08 Hz. 2H), 5.47 (s, 1H), 6.30 (d, J = 6,59 Hz, 1H), 7.17 (t, J = 8.87 Hz, 2H), 7.38-7.41 (m, 2H), 7.54 (t, J = 6.34 Hz, 1H), 7.86 (t, J = 4.06 Hz, 3H), 8.32 (d, J = 8.62 Hz, 2H), 8.44 (d, J = 3.55 Hz, 1H). |
| | | MS (ESI) m/z = 499 (M+H)⁺. |
| | | LC/MS t_{R} = 2.08 min. |
| Example 2-135 | 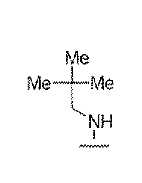 | 1H-NMR (400 MHz, CDCl₃) δ 0.65 (s, 2H), 0.85-0,92 (m, 6H), 1.20-1.33 (m, 9H), 1.38-1.51 (m, 9H),1.38-1.51 (m, 2H), 1.66-1.73 (m, 1H), 1.76-1.85 (m, 2H), 2.12-2.20 (m, 2H), 2.90-2.98 (m, 1H), 3.02 (d, J = 6.08 Hz, 2H), 3.65-3.76 (m, 1H), 3.95-4.02 (m, 1H), 5.39 (s, 1H), 5.53-5.59 (m, 1H), 6.24 (s, 1H), 7.67 (s, 1H), 7.79 (d, J = 8.11 Hz, 2H), 8.22 (d, J = 8.11 Hz, 2H). |
| | | MS (ESI) m/z = 461 (M+H)⁺. |
| | | LC/MS t_{R} = 2.08 min. |

**[Table 2-4F]**

| Example No. | R | property data |
|---|---|---|
| Example 2-136 | 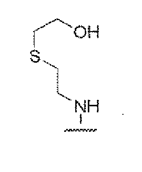 | 1H-NMR (400 MHz, CDCl₃) δ 0.62-0.69 (m, 2H), 0.85-0.93 (m, 2H), 1.17-1.32 (m, 4H), 1.38-1.50 (m, 3H), 1.65-1.74 (m, 1H), 1.75-1.85 (m, 2H), 2.12-2.20 (m, 2H), 2,80 (t, J = 5.58 Hz. 2H), 2.93 (t, J = 6.34 Hz, 3H), 3.43-3.48 (m, 2H), 3.65-3.76 (m, 1H), 3.86 (t, J = 5.32 Hz, 2H), 4.03 (d, J = 7.60 Hz, 1H), 5.37 (s, 1H), 6.25 (s, 1H), 6.53-6.55 (m, 1H), 7.65 (s, 1H), 7.79 (d, J = 8.11 Hz. 2H), 8.21 (d, J = 8.11 Hz, 2H). |
| | | MS (ESI) m/z = 495 (M+H)⁺. |
| | | LC/MS t_{R} = 1.61 min. |
| Example 2-137 | 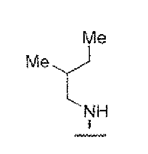 | 1H-NMR (400 MHz, CDCl₃) δ 0.63-(1.67 (m, 2H), 0.89 (q, J = 6.25 Hz, 2H), 0.93-0.98 (m, 3H), 1.02 (d, J = 6.59 Hz, 2H), 1.19-1.32 (m, 4H), 1.39-1.56 (m, 4H), 1.66-1.85 (m, 5H), 2.13-2.20 (m, 2H), 2.90-2.97 (m, 1H), 3.00-3.07 (m, 1H), 3.13-3.19 (m, 1H), 3.66-3.76 (m, 1H), 4.00 (d, J = 7.1 Hz, 1H), 5.35 (s, 1H), 5.52-5.60 (m, 1H), 6.25 (s, 1H), 7.67 (s, 1H), 7.79 (d, J = 8.62 Hz, 2H), 8,22 (d, J = 8.62 Hz, 2H). |
| | | MS (ESI) m/z = 461 (M+H)⁺. |
| | | LC/MS t_{R} = 2.12 min. |
| Example 2-138 | 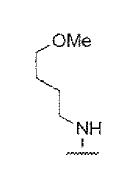 | 1H-NMR (400 MHz, CDCl₃) δ 0.63-0,67 (m, 2H), 0.89 (q, J = 6.42 Hz, 2H), 1.18-1.30 (m, 3H), 1.39-1.50 (m, 2H), 1.67-1.75 (m, 3H), 1.77-1.83 (m, 4H), 2.12-2.28 (m, 2H), 2.90-2.96 (m, 1H), 3.27 (q, J = 6.42 Hz, 2H), 3,36 (s, 3H), 3.43 (t, J = 6.08 Hz, 2H), 3.67-3.76 (m, 1H), 4.00 (d, J = 7.60 Hz, 1H), 5.36 (s, 1H), 5.53-5.59 (m, 1H), 6,24 (s, 1H), 7.66 (s, 1H), 7.78 (d, J = 8.62 Hz, 2H), 8.22 (d, J = 8.62 Hz. 2H). |
| | | MS (ESI) m/z = 477 (M+H)⁺. |
| | | LC/MS t_{R} = 1.79 min. |
| Example 2-139 | 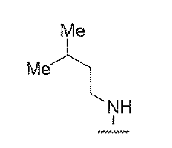 | 1H-NMR (400 MHz, CDCl₃) δ 0.65 (s, 2H), 0.84-0.93 (m, 3H), 0.93-1.00 (m, 6H), 1.17-1.32 (m, 5H), 1.37-1.52 (m, 3H), 1.65-1.84 (m, 5H), 2.12-2.21 (m, 2H), 2.89-2.97 (m, 1H), 3.21-3.29 (m, 2H), 3.66-3.77 (m, 1H), 3.97-4.04 (m, 1H), 5.36 (s, 1H), 5.43 (br s, 1H), 6.25 (br s, 1H), 7.66 (s, 1H), 7.79 (d, J = 7.60 Hz, 2H), 8.22 (d, J = 8.11 Hz, 2H). |
| | | MS (ESI) m/z = 461 (M+H)⁺. |
| | | LC/MS t_{R} = 2.14 min. |

**[Table 2-4G]**

| Example No. | R | property data |
|---|---|---|
| Example 2-140 | 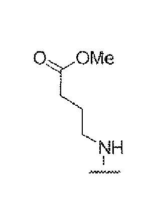 | 1H-NMR (400 MHz, CDCl₃) δ 0.63-0.67 (m, 2H), 0.87-0.92 (m, 2H), 1.04 (t, J = 7.35 Hz, 3H), 1.18-1.32 (m, 4H), 1.38-1.51 (m, 2H), 1.86-1.85 (m, 5H), 2.12-2.20 (m, 2H), 2.90-2.97 (m, 1H), 3.21 (q, J = 6.42 Hz, 2H), 3.67-3.76 (m, 1H), 4.00 (d, J = 7.60 Hz, 1H), 5.36 (s, 1H), 5.46-5.51 (m, 1H), 6.24 (s, 1H), 7.67 (s, 1H), 7.19 (d, J = 8.62 Hz, 2H), 8.23 (d, J = 8.62 Hz, 2H). |
| | | MS (ESI) m/z = 491 (M+H)⁺. |
| | | LC/MS t_{R} = 1.76 min. |
| Example 2-141 | 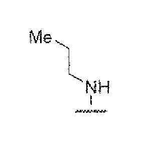 | 1H-NMR (400 MHz, CDCl₃) δ 0.63-0.67 (m, 2H), 0.87-0.92 (m, 2H), 1.04 (t, J = 7.35 Hz, 3H), 1.18-1.32 (m, 3H), 1.38-1.51 (m, 2H), 1.86-1.85 (m, 5H), 2.12-2.20 (m, 2H), 2.90-2.97 (m, 1H), 3.21 (q, J = 6.42 Hz, 2H), 3.67-3.76 (m, 1H), 4.00 (d, J = 7.60 Hz, 1H), 5.36 (s, 1H), 5.47-5.50 (m, 1H), 6.24 (s, 1H), 7.67 (s, 1H), 7.79 (d, J = 8.62 Hz, 2H), 8.23 (d, J = 8.62 Hz, 2H). |
| | | MS (ESI) m/z = 433 (M+H)⁺. |
| | | LC/MS t_{R} = 1.85 min. |
| Example 2-142 | 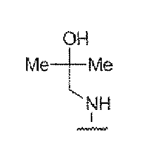 | 1H-NMR (400 MHz, CDCl₃) δ 0.63-0.67 (m, 2H), 0.87-0.92 (m, 4H), 0.99 (t, J = 7.60 Hz, 3H), 1.18-1.33 (m, 9H), 1.38-1.51 (m, 2H), 1.59-1.74 (m, 3H), 1.76-1.85 (m, 2H), 2.12-2.21 (m, 2H), 2.90-2.97 (m, 1H), 3.45-3.51 (m, 1H), 3.67-3.75 (m, 1H), 3.98 (d, J = 7.60 Hz, 1H), 5.34 (s, 2H), 6.24 (s, 1H), 7.66 (s, 1H), 7.79 (d, J = 8.11 Hz, 2H), 8.22 (d, J = 8.11 Hz, 2H). |
| | | MS (ESI) m/z = 463 (M+H)⁺. |
| | | LC/MS t_{R} = 1.59 min. |

**[Table 2-4H]**

| Example No. | R | property data |
|---|---|---|
| Example 2-143 | 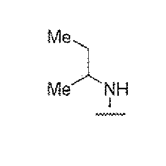 | 1H-NMR (400 MHz, CDCl₃) δ 0.63-0.67 (m, 2H), 0.87-0.92 (m, 2H), 1.17-1.32 (m, 5H), 1.36-1.47 (m, 8H), 1.65-1.73 (m, 1H), 1.74-1.85 (m, 2H), 2.11-2.19 (m, 2H), 2.90-2.98 (m, 2H), 3.23 (d, J = 6.08 Hz, 2H), 3.64-3.75 (m, 1H), 3.99 (d, J = 7.10 Hz, 1H), 4.12 (q, J = 7.10 Hz, 1H), 5.45 (s, 1H), 5.91-5.97 (m, 1H), 6.26 (s, 1H), 7.66 (s, 1H), 7.78 (d, J = 8.62 Hz, 2H), 8.21 (d, J = 8.62 Hz, 2H). |
| | | MS (ESI) m/z = 447 (M+H)⁺. |
| | | LC/MS t_{R} = 1.83 min. |
| Example 2-144 | 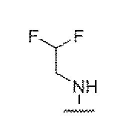 | 1H-NMR (400 MHz, CDCl₃) δ 0.63-0.67 (m, 2H), 0.87-0.92 (m, 2H), 1.18-1.32 (m, 3H), 1.40-1.50 (m, 2H), 1.67-1.72 (m, 1H), 1.78-1.83 (m, 2H), 2.12-2.20 (m, 2H), 2.91-2.97 (m, 1H), 3.62-3.77 (m, 3H), 4.06 (d, J = 7.10 Hz, 1H), 5.71 (t, J = 6.84 Hz, 1H), 5.82-6.12 (m, 1H), 6.24 (s, 1H), 7.69 (s, 1H), 7.79 (d, J = 8.11 Hz, 2H), 8.21 (d, J = 8.62 Hz, 2H). |
| | | MS (ESI) m/z = 455 (M+H)⁺. |
| | | LC/MS t_{R} = 1.83 min. |
| Example 2-145 | 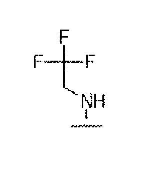 | 1H-NMR (400 MHz, CDCl₃) δ 0.63-0.67 (m, 2H), 0.90 (q, J = 6.25 Hz, 2H), 1.19-1.33 (m, 3H), 1.38-1.51 (m, 2H), 1.66-1.75 (m, 1H), 1.76-1.85 (m, 2H), 2.12-2.20 (m, 2H), 2.90-2.97 (m, 1H), 3.67-3.78 (m, 1H), 3.85-3.93 (m, 2H), 4.08 (d, J = 7.10 Hz, 1H), 5.51 (s, 1H), 5.81 (t, J =6.59 Hz, 1H), 6.25 (s, 1H), 7.70 (s, 1H), 7.80 (d, J = 8.11 Hz, 2H), 8.21 (d, J = 8.11 Hz, 2H). |
| | | MS (ESI) m/z = 473 (M+H)⁺. |
| | | LC/MS t_{R} = 2.03 min. |
| Example 2-146 | 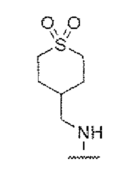 | 1H-NMR (300 MHz, DMSO-d₆) δ 0.56-0.63 (2H, m), 0.65-0.74 (2H, m), 1.18-1.46 (5H, m), 1.64-1.80 (5H, m), 1.96-2.09 (5H, m), 2.81-2.90 (1H, m), 3.03-3.17 (6H, m), 3.56-3.65 (1H, m), 5.61 (1H, s), 6.30 (1H. d, J = 7.0 Hz), 7.07 (1H, t, J = 6.2 Hz), 7.84-7.87 (3H, m), 8.31 (2H, d, J = 8.4 Hz), 8.44 (1H, d, J = 4.1 Hz). |
| | | MS (ESI) m/z = 537 (M+H)⁺. |
| | | LC/MS t_{R} = 1.60 min. |

**[Table 2-4I]**

| Example No. | R | property data |
|---|---|---|
| Example 2-147 | 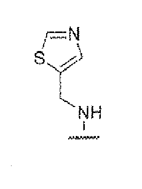 | 1H-NMR (300 MHz, DMSO-d₆) δ 0.56-0.61 (2H, m), 0.65-0.74 (2H, m), 1.15-1.44 (5H, m), 1.63-1.67 (1H, m), 1.75-1.79 (2H, m), 2.03-2.07 (2H, m), 2.81-2.89 (1H, m), 3.53-3.62 (1H, m), 4.67 (2H, d, J = 6.2 Hz), 5.64 (1H, s), 6.39 (1H, d, J = 6.9 Hz), 7.58 (1H, t, J = 6.2 Hz), 7.84-7.88 (4H, m), 8.30 (2H, d, J = 8.4 Hz), 8.44 (1H, d, J = 3.8 Hz), 8.98 (1H, s). |
| | | MS (ESI) m/z = 488 (M+H)⁺. |
| | | LC/MS t_{R} = 1.54 min. |
| Example 2-148 | 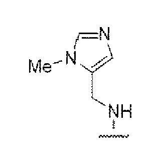 | 1H-NMR (300 MHz, DMSO-d₆) δ 0.57-0.63 (2H, m), 0.65-0.74 (2H, m), 1.17-1.45 (5H, m), 1.63-1.68 (1H, m), 1.76-1.80 (2H, m), 2.05-2.08 (2H, m), 2.81-2.90 (1H, m), 3.58-3.67 (4H, m), 4.41 (2H. d, J = 5.6 Hz), 5.68 (1H, s), 6.40 (1H, d, J = 7.2 Hz), 7.00 (1H, s), 7.38 (1H, t, J = 5.6 Hz), 7.66 (1H. s), 7.84-7.87 (3H, m), 8.31 (2H, d, J = 8.4 Hz), 8.44 (1H, d, J = 4.0 Hz). |
| | | MS (ESI) m/z = 485 (M+H)⁺. |
| | | LC/MS t_{R} = 1.17 min. |
| Example 2-149 | 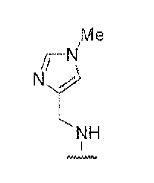 | 1H-NMR (300 MHz, DMSO-d₆) δ 0.56-0.62 (2H, m), 0.65-0.74 (2H, m), 1.16-1.45 (5H, m), 1.63-1.67 (1H, m), 1.75-1.79 (2H, m), 2.04-2.07 (2H, m), 2.81-2.89 (1H, m), 3.54-3.83 (4H, m), 4.26 (2H, d, J = 5.6 Hz), 5.62 (1H, s), 6.37 (1H, d, J = 7.0 Hz), 6.96-6.99 (2H, m), 7.52 (1H, s), 7.83-7.87 (3H, m), 8.32 (2H, d, J = 8.5 Hz), 8.44 (1H, d, J = 4.1 Hz). |
| | | MS (ESI) m/z = 485 (M+H)⁺. |
| | | LC/MS t_{R} = 1.21 min. |
| Example 2-150 | 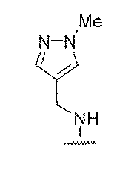 | 1H-NMR (300 MHz, DMSO-d₆) δ 0.56-0.62 (2H, m), 0.65-0.74 (2H, m), 1.16-1.45 (5H, m), 1.63-1.79 (3H, m), 2.04-2.08 (2H, m), 2.81-2.89 (1H, m), 3.54-3.63 (1H, m), 3.78 (3H, s), 4.24 (2H, d, J = 6.1 Hz), 5.60 (1H, s), 6.34 (1H, d, J = 7.0 Hz), 7.16 (1H, t, J = 6.1 Hz), 7.40 (1H, s), 7.59 (1H. s), 7.83-7.87 (3H, m), 8.31 (2H, d, J = 8.4 Hz), 8.44 (1H, d, J = 4.0 Hz). |
| | | MS (ESI) m/z = 485 (M+H)⁺. |
| | | LC/MS t_{R} = 1.47 min. |

**[Table 2-4J]**

| Example No. | R | property data |
|---|---|---|
| Example 2-151 | 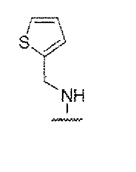 | 1H-NMR (300 MHz, DMSO-d₆) δ 0.56-0.61 (2H, m), 0.65-0.74 (2H, m), 1.15-1.26 (3H, m), 1.32-1.44 (2H, m), 1.63-1.66 (1H, m), 1.74-1.79 (2H, m), 2.03-2.06 (2H, m), 2.81-2.89 (1H, m), 3.53-3.62 (1H, m), 4.60 (2H, d, J = 6.0 Hz), 5.62 (1H, s), 6.38 (1H, d, J = 6.9 Hz), 6.98 (1H, dd, J = 5.0, 3.6 Hz), 7.07 (1H, d, J = 3.6 Hz), 7.39 (1H, d, J = 5.0 Hz), 7.53 (1H, t, J = 6.0 Hz), 7.84-7.87 (3H, m), 8.3 (2H, d, J = 8.4 Hz), 8.44 (1H, d, J = 4.1 Hz). |
| | | MS (ESI) m/z = 487 (M+H)⁺. |
| | | LC/MS t_{R} = 1.86 min. |
| Example 2-152 | 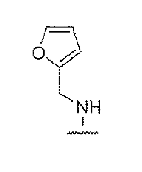 | 1H-NMR (300 MHz, DMSO-d₆) δ 0.56-0.62 (2H, m), 0.65-0.74 (2H, m), 1.16-1.29 (3H, m), 1.33-1.45 (2H, m), 1.63-1.67 (1H, m), 1.75-1.79 (2H, m), 2.03-2.08 (2H, m), 2.81-2.89 (1H, m), 3.53-3.63 (3H, m), 4.41 (2H, d, J = 6.0 Hz), 5.66 (1H, s), 6.33 (1H, d, J = 2.7 Hz), 6.38-6.41 (2H, m), 7.32 (1H. t, J = 6.0 Hz), 7.58-7.59 (1H, m), 7.84-7.81 (3H, m), 8.31 (2H, d, J = 8.5 Hz), 8.44 (1H, d, J = 4.0 Hz). |
| | | MS (CSI) m/z = 471 (M+H)⁺. |
| | | LC/MS t_{R} = 1.74 min. |
| Example 2-153 | 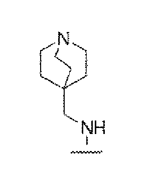 | 1H-NMR (300 MHz, DN(SO-d₆) δ 0.59-0.62 (2H, m), 0.65-0.74 (2H, m), 0.84-0.88 (1H, m), 1.18-1.49 (10H, m), 1.63-1.67 (1H, m), 1.75-1.80 (2H, m), 2.05-2.08 (2H, m), 2.84-2.89 (7H, m), 3.01 (2H, d, J = 6.0 Hz), 3.56-3.65 (1H, m), 5.68 (1H. s), 6.29 (1H, d, J = 6.9 Hz), 6.61 (1H, t, J = 6.0 Hz), 7.85-7.87 (3H, m), 8.32 (2H, d, J = 8.4 Hz), 8.45 (1H, d, J = 4.0 Hz). |
| | | MS (ESI) m/z = 514 (M+H)⁺. |
| | | LC/MS t_{R} = 1.25 min. |

**[Table 2-4K]**

| Example No. | R | property data |
|---|---|---|
| Example 2-154 | 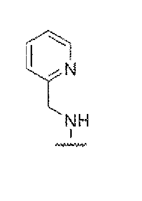 | 1H-NMR (300 MHz, DMSO-d₆) δ 0.59-0.62 (2H, m), 0.65-0.74 (2H, m), 1.12-1.43 (5H, m), 1.61-1.65 (1H, m), 1.73-1.77 (2H, m), 2.00-2.04 (2H, m), 2.81-2.89 (1H, m), 3.52-3.60 (1H, m), 4.53 (2H, d, J = 6.0 Hz), 5.46 (1H, s), 6.37 (1H, d, J = 7.0 Hz), 7.26-7.35 (2H, m), 7.53 (1H, t, J = 6.0 Hz), 7.76 (1H. t, J = 7.7 Hz), 7.85-7.88 (3H, m), 8.32 (2H. d, J = 8.1 Hz), 8.44 (1H, d, J = 4.1 Hz), 8.55 (1H, d, J = 4.9 Hz). |
| | | MS (ESI) m/z = 482 (M+H)⁺. |
| | | LC/MS t_{R} = 1.49 min. |
| Example 2-155 | 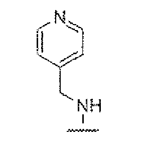 | 1H-NMR(300 MHz, DMSO-d₆) δ 0.57-0.61 (2H, m), 0.65-0.74 (2H, m), 1.11-1.21 (3H, m), 1.30-1.42 (2H, m), 1.61-1.65 (1H, m), 1.72-1.76 (2H, m), 1.99-2.03 (2H, m), 2.81-2.90 (1H, m), 3.50-3.60 (1H, m), 4.48 (2H, d, J = 6.1 Hz), 5.38 (1H. s), 6.31 (1H, d, J = 6.7 Hz), 7.33 (2H, d, J = 4.8 Hz), 7.63 (1H, t, J = 6.1 Hz), 7.85-7.88 (3H, m), 8.31 (2H, d, J = 8.1 Hz), 8.44 (1H, d, J = 3.8 Hz), 8.51 (2H, d, J = 4.8 Hz). |
| | | MS (ESI) m/z = 482 (M+H)⁺. |
| | | LC/MS t_{R} = 1.25 min. |

**[Table 2-4L]**

| Example No. | R | property data |
|---|---|---|
| Example 2-156 | 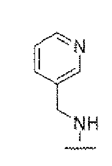 | 1H-NMR (300 MHz, DMSO-d₆) δ 0.56-0.61 (2H, m), 0.65-0.74 (2H, m), 11.13-1.23 (3H, m), 1.31-1.43 (2H, m), 1.62-1.65 (1H, m), 1.73-1.77 (2H, m), 2.01-2.04 (2H, m), 2.82-2.88 (1H, m), 3.51-3.59 (1H, m), 4.46 (2H, d, J = 6.0 Hz), 5.50 (1H, s), 6.33 (1H, d, J = 6.9 Hz), 7.36 (1H, dd, J = 7.9, 4.8 Hz), 7.62 (1H, t, J = 6.0 Hz), 7.75 (1H, d, J = 7.9 Hz), 7.84-7.87 (3H, m), 8.31 (2H, d, J = 8.4 Hz), 8.43-8.47 (2H, m), 8.60 (1H, s). |
| | | MS (ESI) m/z = 482 (M+H)⁺. |
| | | LC/MS t_{R} = 1.30 min, |
| Example 2-157 | 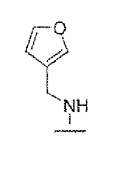 | 1H-NMR (300 MHz, DMSO-d₆) δ 0.56-0.61 (2H, m), 0.65-0.74 (2H. m), 1.16-1.45 (5H, m), 1.63-1.67 (1H, m), 1.75-1.79 (2H, m), 2.05-2.08 (2H, m), 2.81-2.89 (1H, m), 3.54-3.63 (1H. m), 4.25 (2H, d, J = 6.0 Hz), 5.60 (1H, s), 6.34 (1H, d, J = 7.0 Hz), 6.49 (1H, d, J = 1.2 Hz), 7.23 (1H, t, J = 6.0 Hz), 7.60 (2H, s), 7.84-7.87 (3H. m), 8.31 (2H, d, J = 8.4 Hz), 8.44 (1H, d, J = 3.7 Hz). |
| | | MS (ESI) m/z = 471 (M+H)⁺. |
| | | LC/MS t_{R} = 1.72 min. |
| Example 2-158 | 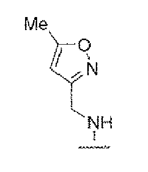 | 1H-NMR (300 MHz, DMSO-d₆) δ 0.58-0.64 (2H, m), 0.67-0.73 (2H, m), 1.21-1.43 (6H, m), 1.62-1.66 (1H, m), 1.74-1.78 (2H, m), 2.00-2.04 (2H, m), 2.39 (3H, s), 2.82-2.91 (1H, m), 4.52 (2H, d, J = 5.0 Hz), 5.91 (7H, s), 6.25 (1H, s), 7.89-7.94 (3H, m), 8.27 (2H, d, J = 7.3 Hz), 8.36 (1H, s), 8.56 (1H, d, J = 4.0 Hz). |
| | | MS (ESI) m/z = 486 (M+H)⁺. |
| | | LC/MS t_{R} = 1.68 min. |
| Example 2-159 | 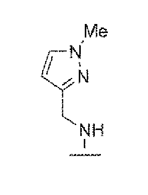 | 1H-NMR (300 MHz, DMSO-d₆) δ 0.56-0.61 (2H, m), 0.65-0.74 (2H, m), 1.16-1.44 (5H, m), 1.63-1.67 (1H, m), 1.75-1.79 (2H, m), 2.03-2.07 (2H, m), 2.81-2.89 (1H, m), 3.5 7-3.59 (1H, m), 3.79 (3H, s), 4.34 (2H, d, J = 5.9 Hz), 5.59 (1H, s), 6.15 (1H, s), 6.41 (1H, d, J = 7.0 Hz), 7.14 (1H, t, J = 5.9 Hz), 7.59 (1H, s), 7.84-7.87 (3H, m), 8.31 (2H, d, J = 8.2 Hz), 8.44 (1H, d, J = 4.0 Hz). |
| | | MS (ESI) m/z = 485 (M+H)⁺, |
| | | LC/MS t_{R} = 1.51 min. |

**[Table 2-4M]**

| Example No. | R | property data |
|---|---|---|
| Example 2-160 | 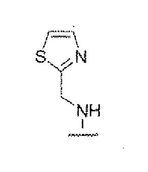 | 1H-NMR (300 MHz, DMSO-d₆) δ 0.56-0.61 (2H, m). 0.65-0.74 (2H, m), 1.13-1.43 (5H, m), 1.61-1.65 (1H, m), 1.73-1.77 (2H, m), 2.01-2.04 (2H, m), 2.81-2.89 (1H, m). 3.51-3.62 (1H, m), 4.73 (2H, d, J = 5.9 Hz). 5.56 (1H, s). 6.46 (1H, d, J = 7.2 Hz), 7.62 (1H, d, J = 3.2 Hz), 7.75-7.79 (2H, m), 7.85-7.88 (3H, m), 8.32 (2H, d, J = 7.8 Hz), 8.45 (1H, d, J = 3.8 Hz). |
| | | MS (ESI) m/z = 488 (M+H)⁺, |
| | | LC/MS t_{R} = 1.60 min. |
| Example 2-161 | 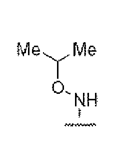 | 1H-NMR (300 MHz, DMSO-d₆) δ 0.57-0.63 (2H, m), 0.65-0.74 (2H, m), 1.24-1.46 (11H, m), 1.63-1.67 (1H, m), 1.76-1.81 (2H, m), 2.07-2.10 (2H, m), 2.81-2.90 (1H, m), 3.50-3.67 (1H, m), 3.97-4.09 (1H, m), 6.05 (1H, s), 6.62 (1H, d, J = 6.9 Hz), 7.85-7.88 (3H, m), 8.31 (2H, d, J = 8.5 Hz), 8.45 (1H, d, J = 4.1 Hz), 10.08 (1H, s). |
| | | MS (ESI) m/z = 449 (M+H)⁺. |
| | | LC/MS t_{R} = 1.81 min. |
| Example 2-162 | 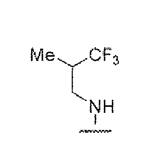 | 1H-NMR (300 MHz, DMSO-d₆) δ 0.56-0.63 (2H, m), 0.65-0.74 (2H, m), 1.12-1.48 (8H, m), 1.63-1.67 (1H, m), 1.76-1,80 (2H, m), 2.05-2.09 (2H, m), 2.81-2.88 (2H, m), 3.17-3.27 (1H, m), 3.47-3.64 (2H, m), 5.64 (1H, s), 6.42 (1H, d, J = 6.9 Hz). 7.10 (1H, t, J =6.1 Hz), 7.85-7.88 (3H, m), 8.31 (2H, d, J = 8.7 Hz), 8.45 (1H, d, J = 4.1 Hz). |
| | | MS (ESI) m/z = 501 (M+H)⁺. |
| | | LC/MS t_{R} = 1.95 min. |
| Example 2-163 | 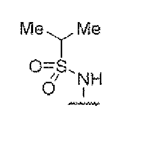 | 1H-NMR (300 MHz, DMSO-d₆) δ 0.57-0.63 (2H, m), 0.66-0.74 (2H, m), 1.20-1.46 (11H, m), 1.63-1.67 (1H, m), 1.76-1.80 (2H, m), 2.05-2.08 (2H, m), 2.81-2.90 (1H, m), 3.50-3.59 (2H, m), 6.68 (1H, s), 6.98 (1H, d, J = 6.7 Hz), 7.89 (2H, d, J = 8.4 Hz), 8.04 (1H, s), 8.31 (2H, d, J = 8.4 Hz), 8.48 (1H, d, J = 4.1 Hz). |
| | | MS (ESI) m/z = 497 (M+H)⁺. |
| | | LC/MS t_{R} = 1.88 min. |

**[Table 2-4N]**

| Example No. | R | property data |
|---|---|---|
| Example 2-164 | 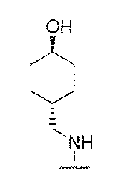 | 1H-NMR (300 MHz, DMSO-d₆) δ 0.58-0.62 (2H, m), 0.65-0.74 (2H, m), 0.91-1.42 (8H, m), 1.62-1.86 (7H, m), 2.06-2.09 (2H, m), 2.83-2.90 (1H, m), 2.99-3.08 (2H, m), 3.17 (2H, d, J = 5.3 Hz), 3.60-3.62 (1H, m), 4.09 (1H, q, J = 5.3 Hz), 4,48 (1H, d, J = 4.5 Hz), 5.57 (1H, s), 6.29 (1H, d, J = 7.1 Hz), 6.85 (1H, t, J = 5.9 Hz), 7,82 (1H, s), 7.86 (2H, d, J = 8.4 Hz), 8.32 (2H, d, J = 8.4 Hz). 8.43 (1H, d, J = 4.0 Hz). |
| | | MS (ESI) m/z = 503 (M+H)⁺. |
| | | LC/MS t_{R} = 1.53 min. |
| Example 2-165 | 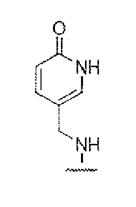 | 1H-NMR (300 MHz, DMSO-d₆) δ 0.56-0.62 (2H, m), 0.65-0.74 (2H, m), 1,15-1,44 (5H, m), 1.62-1.79 (3H, m), 2.04-2.07 (2H, m), 2.81-,2.89 (1H, m), 3.53-3.62 (1H, m), 4.14 (2H, d, J = 6.0 Hz), 5.57 (1H, s), 6.33 (2H, d, J = 9.2 Hz), 7.26 (1 H, d, J = 2.4 Hz). 7.38 (1H, t, J = 6.0 Hz), 7.48 (1H, dd, J = 9.2, 2.4 Hz), 7.84-7.87 (3H, m), 8.31 (2H, d, J = 8.5 Hz). 8.44 (1H, d, J = 4.1 Hz), 11.50 (1H, s). |
| | | MS (ESI) m/z = 498 (M+H)⁺. |
| | | LC/MS t_{R} = 1.36 min. |
| Example 2-166 | 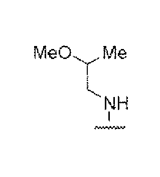 | 1H-NMR (300 MHz, DMSO-d₆) δ 0.56-0.63 (2H, m), 0.65-0.74 (2H, m), 1.13-1.46 (8H, m), 1,63-1.67 (1H, m), 1.76-1.80 (2H, m), 2.06-2.09 (2H, m), 2.81-2.90 (1H, m), 3.13-3.26 (2H, m), 3.30 (3H, s), 3,56-3.65 (2H, m), 5.63 (1H, s), 6.36 (1H, d, J = 7.2 Hz), 6.60 (1H, t, J = 5.8 Hz), 7.83-7.87 (3H, m), 8.32 (2H, d, J = 8.5 Hz), 8.45 (1H, d, J = 4.0 Hz). |
| | | MS (ESI) m/z = 463 (M+H)⁺. |
| | | LC/MS t_{R} = 1.61 min. |
| Example 2-167 | 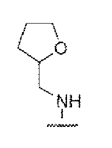 | 1H-NMR (300 MHz, DMSO-d₆) δ 0.56-0.62 (2H, m), 0.65-0.72 (2H, m), 1.17-1.42 (5H, m), 1.62-2.08 (9H, m), 2.83-2.88 (1H, m), 3.23 (2H, t, J = 5.9 Hz), 3.56-3.68 (2H, m), 3.76-3.83 (1H, m), 4.06-4.14 (1H, m), 5.67 (1H, s), 6.36 (1H, d, J = 6.9 Hz), 6.67 (1H, t, J = 6.0 Hz), 7.83-7.87 (3H, m), 8.32 (2H, d, J = 8.5 Hz), 8.45 (1H, d, J = 4.0 Hz). |
| | | MS (ESI) m/z = 475 (M+H)⁺. |
| | | LC/MS t_{R} = 1.63 min. |

**[Table 2-40]**

| Example No. | R | property data |
|---|---|---|
| Example 2-168 | 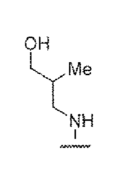 | 1H-NMR (300 MHz, DMSO-d₆) δ 0.59-0.62 (2H, m), 0.65-0.74 (2H, m), 0.91 (3H, d, J = 6.7 Hz), 1.17-1.46 (5H, m), 1.63-1.67 (1H, m), 1.76-1.80 (2H, m), 1,95-2.08 (3H, m), 2.81-2.90 (1H, m), 2.98-3,07 (1H, m), 3.14-3.23 (1H, m), 3.36-3.40 (2H, m), 3.56-3.65 (1H, m), 4.64 (1H, t, J = 4.7 Hz), 5.59 (1H, s), 6.33 (1H, d, J = 6.9 Hz), 6.85 (1H, t, J = 5.7 Hz), 7.82-7.87 (3H, m), 8.32 (2H, d, J = 8.4 Hz), 8.44 (1H, d, J = 3.8 Hz). |
| | | MS (ESI) m/z = 463 (M+H)⁺, |
| | | LC/MS t_{R} = 1.48 min. |
| Example 2-169 | 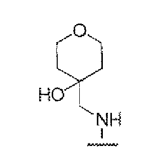 | 1H-NMR (300 MHz, DMSO-d₆) δ 0.56-0.63 (2H, m), 0.65-0.74 (2H, m), 1.21-1.66 (10H, m), 1.75-1.80 (2H, m), 2.05-2.08 (2H, m), 2.81-2.90 (1H, m), 3.17 (2H, d, J = 5.8 Hz), 3.61-3.64 (5H, m), 4.89 (1H, s), 5.70 (1H, s), 6.31-6.35 (2H, m), 7.85-7.88 (3H, m), 8.32 (2H, d, J = 8.7 Hz), 8.45 (1H, d, J = 4.1 Hz). |
| | | MS (ESI) m/z = 505 (M+H)⁺. |
| | | LC/MS t_{R} = 1.43 min, |

### Example 2-170

4-(6-(Cyclohexyloxy)-8-((tetrahydro-2H-pyran-4-yl)methylamino)imidazo[1,2-b]pyridazin-3-yl)-N-cyclopropylbenzamide

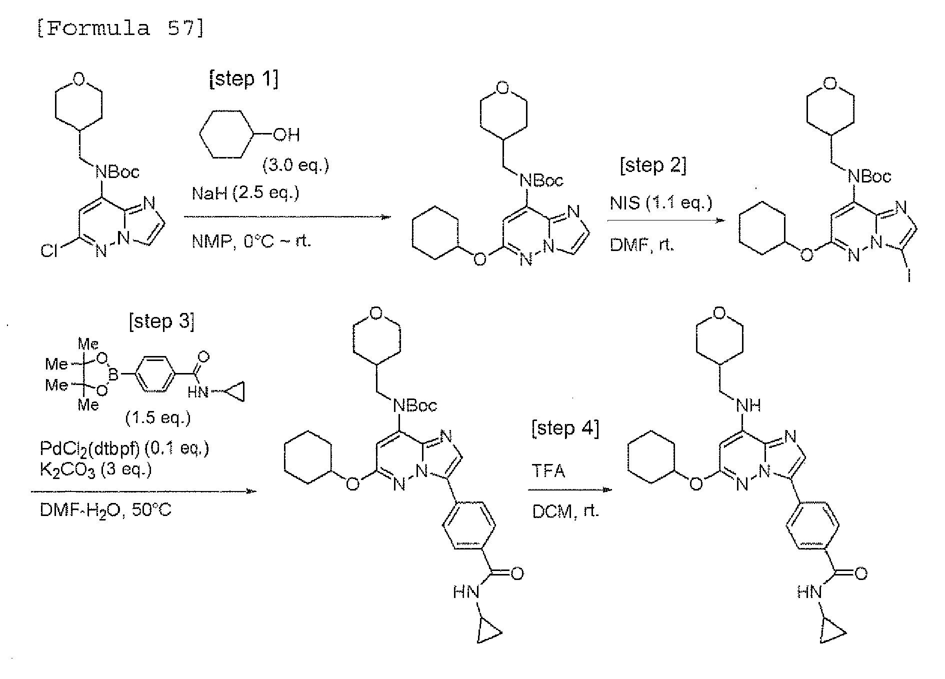

### Step 1: tert-Butyl 6-(cyclohexyloxy)imidazo[1,2-b]pyridazin-8-y1((tetrahydro-2H-pyran-4-yl)methyl)carbamate

After an NMP (3.0 mL) solution of cyclohexanol (0.259 mL, 2.45 mmol) and 60% sodium hydride (82 mg, 2.04 mmol) was stirred at 40°C for 30 minutes, tert-butyl 6-chloroimidazo[1,2-b]pyridazin-8-yl((tetrahydro-2H-pyran-4-yl)methyl)carbamate (300 mg, 0.818 mmol) was added and stirred at room temperature for one hour. To the reaction solution, a 10% aqueous citric acid solution and ethyl acetate were added and separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 20-75% ethyl acetate gradient) to obtain the titled compound (239 mg, 0.554 mmol, 68%) as a white amorphous substance.
MS (ESI) m/z = 431 (M+H)⁺.
LC/MS t_{R} = 2.35 min.

### Step 2: tert-Butyl 6-(cyclohexyloxy)-3-iodoimidazo[1,2-b]pyridazin-8-yl((tetrahydro-2H-pyran-4-yl)methyl)carbamate

The titled compound was synthesized in accordance with the process of Example 2-87 (Step 2-4) (268 mg, 0.481 mmol, 87%).
MS (ESI) m/z = 557 (M+H)⁺.
LC/MS t_{R} = 3.04 min.

### Step 3: tert-Butyl 6-(cyclohexyloxy)-3-(4-(cyclopropylcarbamoyl)phenyl)imidazo[1,2-b]pyridazin-8-yl((tetrahydro-2H-pyran-4-yl)methyl)carbamate

To a DMF/water (3.0 mL/0.3 mL) solution of tert-butyl 6-(cyclohexyloxy)-3-iodoimidazo[1,2-b]pyridazin-8-yl((tetrahydro-2H-pyran-4-yl)methyl)carbamate (267 mg, 0.480 mmol), 4-(cyclopropylcarbamoyl)phenylboronic acid pinacol ester (207 mg, 0.072 mmol) and potassium carbonate (199 mg, 1.44 mmol), PdCl₂(dtbpf) (15.6 mg, 0.024 mmol) was added and stirred at 50°C for 2 hours. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 10-30% ethyl acetate gradient) to obtain the titled compound (283 mg, 0.480 mmol, 100%) as a white amorphous substance.
MS (ESI) m/z = 590 (M+H)⁺.
LC/MS t_{R} = 2.62 min.

### Step 4: Titled compound

The titled compound was synthesized in accordance with the process of Example 2-87 (Step 2-6) (182 mg, 0.371 mmol, 77%).
1H-NMR (400 MHz, CDCl₃) δ 0.62-0.68 (m, 2H), 0.90 (q, J = 6.3 Hz, 2H), 1.23-2.18 (m, 15H), 2.90-2.98 (m, 1H), 3.20 (t, J = 6.6 Hz, 2H), 3.40 (t, J = 11.2 Hz, 2H), 4.01 (dd, J = 3.5, 11.7 Hz, 2H), 4.90-4.99 (m, 1H), 5.65 (s, 1H), 5.72 (t, J = 6.1 Hz, 1H), 6.26 (s, 1H), 7.73 (s, 1H), 7.81 (d, J = 8.1 Hz, 2H), 8.16 (d, J = 8.1 Hz, 2H).
MS (ESI) m/z = 490 (M+H)⁺.
LC/MS t_{R} = 2.30 min.

### Example 2-171

N-Cyclopropyl-4-(6-(1-methylcyclohexyloxy)-8-((tetrahydro-2H-pyran-4-yl)methylamino)imidazo[1,2-b]pyridazin-3-yl)benzamide

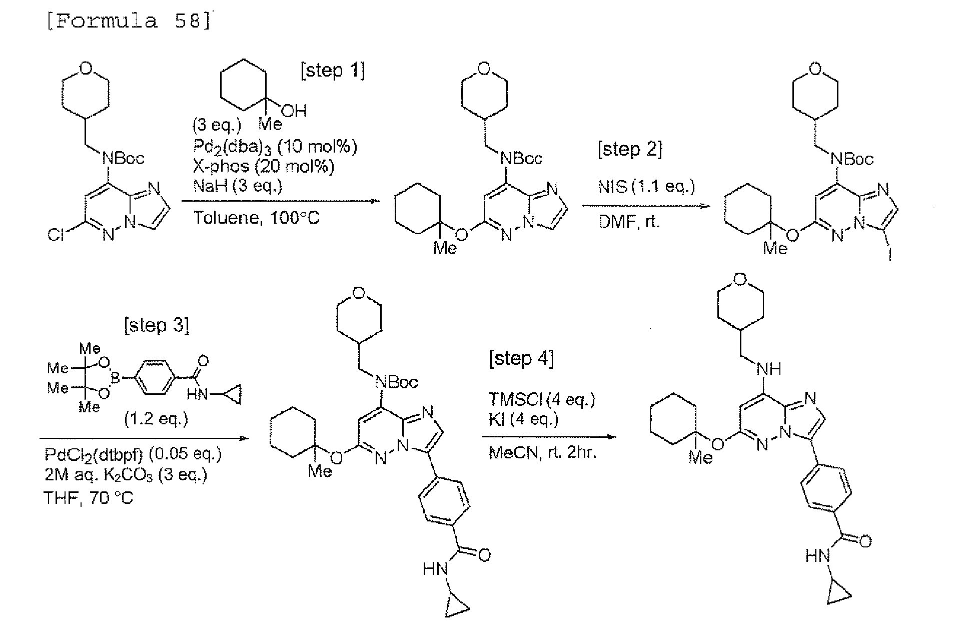

### Step 1: tert-Butyl 6-(1-methylcyclohexyloxy)imidazo[l,2-b]pyridazin-8-yl((tetrahydro-2H-pyran-4-yl)methyl)carbamate

fter a toluene (30 mL) solution of 1-methylcyclohexanol (747 mg, 6.54 mmol) and 60% sodium hydride (262 mg, 6.54 mmol) was stirred at 40°C for 30 minutes, tert-butyl 6-chloroimidazo[1,2-b]pyridazin-8-yl((tetrahydro-2H-pyran-4-yl)methyl)carbamate (800 mg, 2.18 mmol), Pd₂(dba)₃ (200 mg, 0.218 mmol) and X-Phos (208 mg, 0.436 mmol) were added and stirred at 100°C for 8 hours. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 10-50% ethyl acetate gradient) to obtain the titled compound (543 mg, 1.22 mmol, 56%) as a light brown amorphous substance.
1H-NMR (400 MHz, DMSO-d₆) δ 0.87-1.66 (m, 31H), 2.19-2.30 (m, 2H), 3.14 (t, J = 10.8 Hz, 2H), 3.75 (dd, J = 11.3, 2.4 Hz, 2H), 3.85 (d, J = 7.1 Hz, 2H), 6.77 (s, 1H), 7.54 (d, J = 1.2 Hz, 1H), 8.00 (d, J = 1.2 Hz, 1H).
MS (ESI) m/z = 445 (M+H)⁺.
LC/MS t_{R} = 2.51 min.

### Step 2: tert-Butyl 3-iodo-6-(1-methylcyclohexyloxy)imidazo[1,2-b]pyridazin-8-yl((tetrahydro-2H-pyran-4-yl)methyl)carbamate

The titled compound was synthesized in accordance with the process of Example 2-87 (Step 2-4) (699 mg, 1.23 mmol, 100%).
1H-NMR (400 MHz, DMSO-d₆) δ 1.12-1.70 (m, 29H), 2.35 (d, J = 12.8 Hz, 2H), 3.14 (t, J = 10.8 Hz, 2H), 3.70-3.85 (m, 4H), 6.87 (s, 1H), 7.67 (s, 1H)
MS (ESI) m/z = 571 (M+H)⁺.
LC/MS t_{R} = 3.12 min.

### Step 3: tert-Butyl 3-(4-(cyclopropylcarbamoyl)phenyl)-6-(1-methylcyclohexyloxy)imidazo[1,2-b]pyridazin-8-yl((tetrahydro-2H-pyran-4-yl)methyl)carbamate

The titled compound was synthesized in accordance with the process of Example 2-170 (Step 3) (599 mg, 0.992 mmol, 81%).
1H-NMR (400 MHz, DMSO-d₆) δ 0.56-0.65 (m, 2H), 0.67-0.76 (m, 2H), 1.11-1.80 (m, 26H), 2.26-2.39 (m, 2H), 2.83-2.93 (m, 1H), 3.17 (t, J = 10.8 Hz, 2H), 3.78 (dd, J = 11.2, 2.4 Hz, 2H), 3.87 (d, J = 7.2 Hz, 2H), 6.93 (s, 1H), 7.95 (d, J = 8.6 Hz, 2H), 8.12-8.18 (m, 3H), 8.50 (d, J = 4.2 Hz, 1H).
MS (ESI) m/z = 604 (M+H)⁺.
LC/MS t_{R} = 2.65 min.

### Step 4: Titled compound

The titled compound was synthesized in accordance with the process of Example 2-90 (Step 4) (113 mg, 0.225 mmol, 23%). 1H-NMR (300 MHz, DMSO-d₆) δ 0.59-0.60 (m, 2H), 0.70-0.71 (m, 2H), 1.18-1.31 (m, 4H), 1.53-1.63 (m, 11H), 1.97-2.01 (m, 1H), 2.29-2.33 (m, 2H), 2.86-2.88 (m, 1H), 3.18-3.25 (m, 4H), 3.85 (dd, J = 11.3, 2.6 Hz, 2H), 5.74 (s, 1H), 7.32 (t, J = 6.0 Hz, 1H), 7.91 (d, J = 8.4 Hz, 3H), 8.16 (d, J = 8.6 Hz, 2H), 8.46 (d, J = 4.2 Hz, 1H).
MS (ESI) m/z = 504 (M+H)⁺,
LC/MS t_{R} = 2.12 min.

[Table 2-5A]

**Table 2-5**

| | | |
|---|---|---|
| 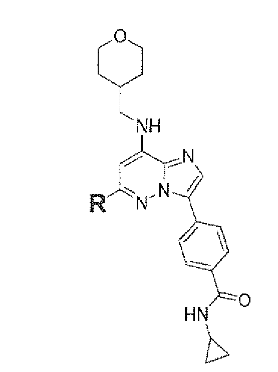 | | |

| Compounds described in Table 2-5 were synthesized in accordance with the process of Example 2-170 in the case where a primary or secondary alcohol is used, and synthesized in accordance with the process of Example 2-171 in the case where a tertiary alcohol is used. | | |
|---|---|---|
| Example No. | R | property data |
| Example 2-172 | 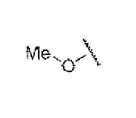 | 1H-NMR (400 MHz, CDCl₃) δ 0.61-0.67 (m, 2H), 0.90 (q, J = 6.4 Hz, 2H), 1.36-1.47 (m, 2H), 1.70-1.78 (m, 2H), 1.90-2.02 (m, 1H), 2,90-2,97 (m, 1H), 3,21 (t, J = 6.1 Hz, 2H), 3.36-3.44 (m, 2H), 3.98-4.04 (m, 5H), 5.87 (s, 1H), 5.75 (t, J = 6.1 Hz, 1H), 6.25 (s, 1H), 7.73 (s, 1H), 7.83 (d, J = 8.1 Hz, 2H), 8.17 (d, J = 8.1 Hz, 2H). |
| | | MS (ESI) m/z = 422 (M+H)⁺. |
| | | LC/MS t_{R} = 1.69 min. |
| Example 2-173 | 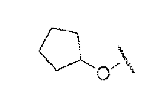 | 1H-NMR (400 MHz, CDCl₃) δ 0.62-0.67 (m, 2H), 0.87-0.93 (m, 2H), 1.35-1.47 (m, 2H), 1.60-2.08 (m, 11H), 2.90-2.97 (m, 1H), 3.19 (t, J = 6.6 Hz, 2H), 3.35-3.44 (m, 2H), 3.98-4.04 (m, 2H), 5.30-5.37 (m, 1H), 5.63 (s, 1H), 5.66-5.71 (m, 1H), 6.25 (s, 1H), 7.72 (s, 1H), 7.82 (d, J = 8.6 Hz, 2H), 8.17 (d, J = 8.6 Hz, 2H). |
| | | MS (ESI) m/z = 476 (M+H)⁺. |
| | | LC/MS t_{R} = 2.19 min. |

**[Table 2-5B]**

| Example No. | R | property data |
|---|---|---|
| Example 2-174 | 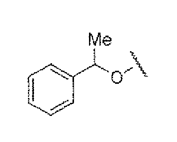 | 1H-NMR (400 MHz, CDCl₃) δ 0.64-0.69 (m, 2H), 0.91 (q, J = 6.4 Hz, 2H), 1.34-1.47 (m, 2H), 1.67 (d, J = 6.6 Hz, 3H), 1.71-1.78 (m, 2H), 2.91-3.00 (m, 1H), 3.21 (t, J = 6.6 Hz, 2H), 3.36-3.44 (m, 2H), 4.01 (dd, J = 11.4, 3.8 Hz, 2H), 5,66-5.72 (m, 1H), 5.75 (s, 1H), 5.98 (q, J = 6.6 Hz, 1H), 6.25 (s, 1H), 7.23-7.31 (m, 1H), 7.38 (t, J = 7.6 Hz, 2H), 7.44 (d, J = 7.6 Hz, 2H), 7.64 (s, 1H), 7.76 (d, J = 8,1 Hz, 2H), 7.87 (d, J = 8.1 Hz, 2H). |
| | | MS (ESI) m/z = 512 (M+H)⁺, |
| | | LC/MS t_{R} = 2.26 min. |
| Example 2-175 | 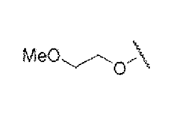 | 1H-NMR (400 MHz, CDCl₃) δ 0.61-0.67 (m, 2H), 0.90 (q, J = 6.4 Hz, 2H), 1.34-1.46 (m, 2H), 1.69-1.77 (m, 2H), 1.90-2.01 (m, 1H), 2.90-2.98 (m, 1H), 3.20 (t, J = 6.6 Hz, 2H), 3.35-3.43 (m, 2H), 3.47 (s, 3H), 3.77-3.81 (m, 2H), 3.97-4.04 (m, 2H), 4.47-4.51 (m, 2H), 5.76 (s, 2H), 6.24 (s, 1H), 7.72 (s, 1H), 7.82 (d, J = 8.6 Hz, 2H), 8.12 (d, J = 8.6 Hz, 2H). |
| | | MS (ESI) m/z = 466 (M+H)⁺. |
| | | LC/MS t_{R} = 1.74 min. |
| Example 2-176 | 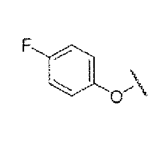 | 1H-NMR (400 MHz, CDCl₃) δ 0.61-0.67 (m, 2H), 0.89 (q, J = 6.4 Hz, 2H), 1.38-1.51 (m, 2H), 1.73-1.80 (m, 2H), 1.94-2.05 (m, 1H), 2.88-2.95 (m, 1H), 3.26 (t, J = 6.6 Hz, 2H), 3.38-3.46 (m, 2H), 3.99-4.07 (m, 2H), 5.87 (s, 2H), 6,18 (s, 1H), 7.10-7.16 (m, 2H), 7,19-7.24 (m, 2H), 7.63 (d, J = 8.1 Hz, 2H), 7.77 (s, 1H), 7.91 (d, J = 8.1 Hz, 2H). |
| | | MS (ESI) m/z = 502 (M+H)⁺. |
| | | LC/MS t_{R} = 2,13 min. |
| Example 2-177 | 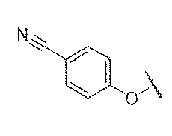 | 1H-NMR (300 MHz, DMSO-d₆) δ 0.54-0.59 (m, 2H), 0.66-0.72 (m, 2H), 1.20-1.31 (m, 2H), 1.64 (d, J = 13.2 Hz, 2H), 1.91-2.00 (m, 1H), 2.80-2.88 (m, 1H), 3.22-3.34 (m, 4H), 3.85 (dd, J = 3.0. 11.2 Hz, 2H), 6.19 (s, 1H), 7.50 (d, J = 8.6 Hz, 2H), 7.75 (d, J = 8.6 Hz, 2H), 7.88 (t, J = 6.6 Hz, 1H), 7.93-7.99 (m, 4H), 8.04 (s, 1H), 8.41 (d, J = 4.1 Hz, 1H). |
| | | MS (ESI) m/z = 509 (M+H)⁺. |
| | | LC/MS t_{R} = 2.07 min. |

**[Table 2-5C]**

| Example No. | R | property data |
|---|---|---|
| Example 2-178 | 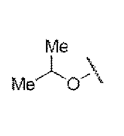 | 1H-NMR (400 MHz, CDCl₃) δ 0.61-0.68 (m, 2H), 0.90 (q, J = 6.4 Hz, 2H), 1.34-1.47 (m, 8H), 1.75 (d, J = 12.7 Hz, 2H), 1.90-2.03 (m, 1H), 2.90-2.97 (m, 1H), 3.20 (t, J = 6.4 Hz, 2H), 3.40 (t, J = 11.7 Hz, 2H), 4.01 (dd, J = 11.7, 4.1 Hz, 2H), 5.18-5.27 (m, 1H), 5.63 (s, 1H), 5.73 (t, J = 5.6 Hz, 1H), 6.26 (br s, 1H), 7.72 (s, 1H), 7.82 (d, J = 8.6 Hz, 2H), 8.14 (d, J = 8.6 Hz, 2H). |
| | | MS (ESI) m/z = 450 (M+H)⁺, |
| | | LC/MS t_{R} = 2.04 min. |
| Example 2-179 | 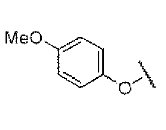 | 1H-NMR (300 MHz, DMSO-d₆) δ 0.54-0.59 (m, 2H), 0.66-0.72 (m, 2H), 1.19-1.31 (m, 2H), 1.64 (d, J = 11.2 Hz, 2H), 1,91-2.04 (m, 1H), 2.80-2.87 (m, 1H), 3.20-3.40 (m, 1H), 3.82 (s, 3H), 3.83-3.89 (m, 2H), 6.06 (s, 1H), 7.03 (d, J = 9.1 Hz, 2H), 7.23 (d, J = 9.1 Hz, 2H), 7.66-7.74 (m, 3H), 7.99 (d, J = 9.1 Hz, 3H), 8.43 (d, J = 4.1 Hz, 1H), |
| | | MS (ESI) m/z = 514 (M+H)⁺. |
| | | LC/MS t_{R} = 2.08 min. |
| Example 2-180 | 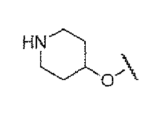 | 1H-NMR (400 MHz, CDCl₃) δ 0.63-0.69 (m, 2H), 0.90 (q, J = 6.4 Hz, 2H), 1.34-1.47 (m, 2H), 1.69-1.81 (m, 4H), 1.91-2.04 (m, 1H), 2.10-2.21 (m, 2H), 2.77-2.85 (m, 2H), 2.91-2.97 (m, 1H), 3.15-3.23 (m, 4H), 3.36-3.44 (m, 2H), 4.01 (dd, J = 3.5, 11.7 Hz, 2H), 5.01-5.10 (m, 1H), 5.67 (s, 1H), 5.73 (t, J = 6.1 Hz, 1H), 6.25 (br s, 1H), 7.73 (s, 1H), 7.81 (d, J = 8.6 Hz, 2H), 8.13 (d, J = 8.6 Hz, 2H), |
| | | MS (ESI) m/z = 491 (M+H)⁺. |
| | | LC/MS t_{R} 1.19 min. |
| Example 2-181 | 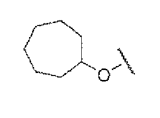 | 1H-NMR (400 MHz, CDCl₃) δ 0.63-0.69 (m, 2H), 0.90 (q, J = 6.4 Hz, 2H), 1.34-1.47 (m, 2H), 1.48-1.70 (m, 6H), 1.70-1.90 (m, 6H), 1.90-2.02 (m, 1H), 2.10-2.20 (m, 2H), 2.90-2.98 (m, 1H), 3.20 (t, J = 6.6 Hz, 2H), 3.36-3,44 (m, 2H), 4.01 (dd, J = 11.2, 4.1 Hz, 2H), 5.07-5.15 (m, 1H), 5.64 (s, 1H), 5.68 (t, J = 6.1 Hz, 1H), 6.25 (br s, 1H), 7.72 (s, 1H), 7.81 (d, J = 8.6 Hz, 2H), 8.16 (d, J = 8.6 Hz, 2H). |
| | | MS (ESI) m/z = 504 (M+H)⁺, |
| | | LC/MS t_{R} = 2.47 min. |

**[Table 2-5D]**

| Example No. | R | property data |
|---|---|---|
| Example 2-182 | 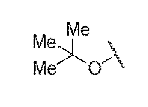 | 1H-NMR (400 MHz, CDCl₃) δ 0.63-0.68 (m, 2H), 0.90 (q, J = 6.4 Hz, 2H), 1.34-1.47 (m, 2H), 1.62 (s, 9H), 1.71-1.78 (m, 2H), 1.90-2.01 (m, 1H), 2.90-2.97 (m, 1H), 3.19 (t, J = 6.6 Hz, 2H), 3.36-3,44 (m, 2H), 4.01 (dd, J = 11.2. 3.5 Hz, 2H), 5.60 (s, 1H), 5.65 (t, J = 6.6 Hz, 1H), 6.26 (br s, 1H), 7.69 (s, 1H), 7.82 (d, J = 8.6 Hz, 2H), 8.08 (d, J = 8.6 Hz, 2H). |
| | | MS (EST) m/z = 464 (M+H)⁺. |
| | | LC/MS t_{R} = 2.04 min. |
| Example 2-183 | 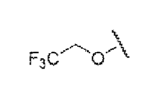 | 1H-NMR (300 MHz, DMSO-d₆) δ 0.57-0.62 (m, 2H), 0.67-0.72 (m, 2H), 1.21-1.29 (m, 2H), 1.63 (d, J = 11.4 Hz, 2H), 1.94-1.95 (m, 1H), 2.86-2.89 (m, 1H), 3.22-3.28 (m, 4H), 3.84 (dd, J = 11.3. 2.7 Hz, 2H), 5.01 (q, J = 9.0 Hz, 2H), 5.99 (s, 1H), 7.70 (t, J = 6.2 Hz, 1H), 7.93 (d, J = 8.4 Hz, 2H), 7.98 (s, 1H), 8.20 (d, J = 8.4 Hz, 2H), 8.48 (d, J = 4.3 Hz, 1H). |
| | | MS (ESI) m/z = 490 (M+H)⁺. |
| | | LC/MS t_{R} = 1.83 min. |
| Example 2-184 | 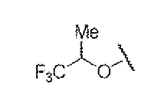 | 1H-NMR (300 MHz, DMSO-d₆) δ 0.58-0.60 (m, 2H), 0.69-0.71 (m, 2H), 1.20-1.26 (m, 2H), 1.55 (d, J = 6.4 Hz, 3H), 1.63 (d, J = 12.4 Hz, 2H), 1.94-1.99 (m, 1H), 2.86-2.89 (m, 1H), 3.21-3.25 (m, 4H), 3.84 (dd, J = 11.2, 2.7 Hz, 2H), 5.71-5.73 (m, 1H), 5.93 (s, 1H), 7.69 (t, J = 6.1 Hz, 1H), 7.93 (d, J = 8.5 Hz, 2H), 7.98 (s, 1H), 8.17 (d, J = 8.4 Hz, 2H), 8.47 (d, J = 4.3 Hz, 1H). |
| | | MS (ESI) m/z = 504 (M+H)⁺. |
| | | LC/MS t_{R} = 1.93 min. |
| Example 2-185 | 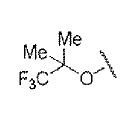 | 1H-NMR (300 MHz, DMSO-d₆) δ 0.60-0.61 (m, 2H), 0.68-0.74 (m, 2H), 1.2t-1.25 (m, 2H), 1.63 (d, J = 12.8 Hz, 2H), 1.79 (s, 6H), 1.93-1.99 (m, 2H), 2.86-2.89 (m, 1H), 3.21-3.25 (m, 4H), 3.84 (dd, J = 11.2, 2,7 Hz, 2H), 5.82 (s, 1H), 7.61 (t, J = 6.2 Hz, 1H), 7.94 (d, J = 8.2 Hz, 3H), 8.10 (d, J = 8.4 Hz, 2H), 8.48 (d, J = 4.2 Hz, 1H). |
| | | MS (ESI) m/z = 518 (M+H)⁺. |
| | | LC/MS t_{R} = 1.99 min. |

**[Table 2-5E]**

| Example No. | R | property data |
|---|---|---|
| Example 2-186 | 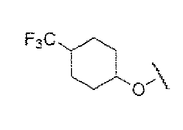 | 1H-NMR (300 MHz, DMSO-d₆) δ 0.59-0.61 (m, 2H), 0.68-0.74 (m, 2H), 1.20-1.27 (m, 2H), 1.51-1.61 (m, 5H), 1.95-2.00 (m, 3H), 2.33-2.44 (m, 3H), 2.84-2.87 (m, 1H), 3.18-3.24 (m, 4H), 3.84 (dd, J = 11.3, 2.4 Hz, 2H), 4.83-4.86 (m, 1H), 5.79 (s, 1H), 7.46 (t, J = 8.1 Hz, 1H), 7.90 (d, J = 8.7 Hz, 2H), 7.97 (s, 1H), 8.23 (d, J = 8.6 Hz, 2H), 8.48 (d, J = 4.0 Hz, 1H). |
| | | MS (ESI) m/z = 558 (M+H)⁺. |
| | | LC/MS t_{R} = 2.12 min. |
| Example 2-187 | 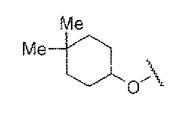 | 1H-NMR (300 MHz, DMSO-d₆) δ 0.59-0.61 (m, 2H), 0.70-0.72 (m, 2H), 0.98 (s, 6H), 1.21-1.35 (m, 4H), 1.50-1.52 (m, 2H), 1.61-1.71 (m, 4H), 1.98-2.01 (m, 3H), 2.86-2.87 (m, 1H), 3.18-3.24 (m, 4H), 3.84 (dd, J = 11.1, 2.7 Hz, 2H), 4.86-4.89 (m, 1H), 5.78 (s, 1H), 7.41 (t, J = 6.0 Hz, 1H), 7.90 (d, J = 8.6 Hz, 2H), 7.97 (s, 1H), 8.23 (d, J = 8.7 Hz, 2H), 8.46 (d, J = 4.0 Hz, 1H). |
| | | MS (ESI) m/z = 518 (M+H)⁺. |
| | | LC/MS t_{R} = 2.33 min. |
| Example 2-188 | 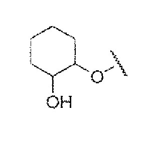 | 1H-NMR (300 MHz, DMSO-d₆) δ 0.59-0.61 (m, 2H), 0.68-0.75 (m, 2H), 1.20-1.35 (m, 6H), 1.64-1.69 (m, 4H), 1.92-1.99 (m, 2H), 2.28-2.31 (m, 1H), 2.85-2.88 (m, 1H), 3.16-3.27 (m, 4H), 3.57-3.81 (m, 1H), 3.85 (dd, J = 11.3, 2.6 Hz, 2H), 4.70 (d, J = 3.9 Hz, 1H), 4.86 (d, J = 4.9 Hz, 1H), 5.79 (s, 1H), 7.42 (t, J = 6.2 Hz, 1H), 7.91 (d, J = 8.6 Hz, 2H), 7.97 (s, 1H), 8.25 (d, J = 8.6 Hz, 2H), 8.47 (d, J = 4.2 Hz, 1H). |
| | | MS (ESI) m/z = 506 (M+H)⁺. |
| | | LC/MS t_{R} = 1.78 min. |
| Example 2-189 | 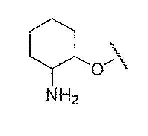 | 1H-NMR (300 MHz, DMSO-d₆) δ 0.58-0.62 (m, 2H), 0.67-0.72 (m, 2H), 1.24-1.32 (m, 4H), 1.53-1.77 (m, 6H), 1.94-1.95 (m, 1H), 2.10-2.13 (m, 1H), 2.85-2.88 (m, 1H), 3.16-3.34 (m, 8H), 3.83-3.85 (m, 2H), 4.99-5.00 (m, 1H), 5.84 (s, 1H), 7.45 (t, J = 8.0 Hz, 1H), 7.90 (d, J = 8.5 Hz, 2H), 7.97 (s, 1H), 8.22 (d, J = 8.0 Hz, 2H), 8.47 (d, J = 4.3 Hz, 1H). |
| | | MS (ESI) m/z = 505 (M+H)⁺. |
| | | LC/MS t_{R} = 1.26 min. |

**[Table 2-5F]**

| Example No. | R | property data |
|---|---|---|
| Example 2-190 | 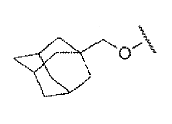 | 1H-NMR (300 MHz, DMSO-d₆) δ 0.60-0.61 (m, 2H), 0.70-0.71 (m, 2H), 1.24-1.26 (m, 2H), 1.62-1.76 (m, 14H), 1.98-2.02 (m, 4H), 2.86-2.87 (m, 1H), 3.18-3.27 (m, 4H), 3.84 (dd, J = 11.2, 2.9 Hz, 2H), 3.90 (s, 2H), 5.82 (s, 1H), 7.39 (t, J = 6.0 Hz, 1H), 7.93 (t, J = 8.2 Hz, 3H), 8.24 (d, J = 8.4 Hz, 2H), 8.45 (d, J = 4.2 Hz, 1H). |
| | | MS (ESI) m/z = 556 (M+H)⁺. |
| | | LC/MS t_{R} = 2.90 min. |
| Example 2-191 | 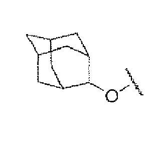 | 1H-NMR (300 MHz, DMSO-d₆) δ 0.59-0.60 (m, 2H), 0.70-0.71 (m, 2H), 1.24-1.26 (m, 2H), 1.59-1.64 (m, 4H), 1.76 (s, 2H), 1.88-1.92 (m, 7H), 2.07-2.11 (m, 2H), 2.30 (s, 2H), 2.85-2.86 (m, 1H), 3.18-3.27 (m, 4H), 3.85 (dd, J = 11.3, 2.8 Hz, 2H), 5.07 (s, 1H), 5.83 (s, 1H), 7.40 (t, J = 6.0 Hz, 1H), 7.90 (d, J = 8.6 Hz, 2H), 7.98 (s, 1H), 8.24 (d, J = 8.6 Hz, 2H), 8.46 (d, J = 4.2 Hz, 1H). |
| | | MS (ESI) m/z = 542 (M+H)⁺. |
| | | LC/MS t_{R} = 2.73 min. |
| Example 2-192 | 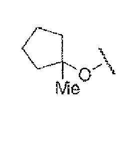 | 1H-NMR (300 MHz, DMSO-d₆) δ 0.60-0.61 (m, 2H), 0.70-0.71 (m, 2H), 1.18-1.24 (m, 4H), 1.61-1.99 (m, 10H), 2.26-2.30 (m, 2H), 2.86-2.88 (m, 1H), 3.15-3.26 (m, 4H), 3.84 (dd, J = 11.2, 2.9 Hz, 2H), 5.70 (s, 1H), 7.34 (t, J = 6.0 Hz, 1H), 7.92 (d, J = 7.9 Hz, 3H), 8.20 (d, J = 7.7 Hz, 2H), 8.46 (d, J = 3.9 Hz, 1H). |
| | | MS (ESI) m/z = 490 (M+H)⁺. |
| | | LC/MS t_{R} = 2.01 min. |
| Example 2-193 | 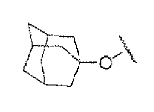 | 1H-NMR (300 MHz, DMSO-d₆) δ 0.60-0.61 (m, 2H), 0.68-0.74 (m, 2H), 1.20-1.28 (m, 2H), 1.63-1.68 (m, 8H), 1.91-1.94 (m, 1H), 2.22-2.26 (m, 9H), 2.84-2.87 (m, 1H), 3.17-3.25 (m, 4H), 3.84 (dd, J = 11.2, 2.7 Hz, 2H), 5.69 (s, 1H), 7.36 (t, J = 6.1 Hz, 1H), 7.90 (d, J = 8.4 Hz, 2H), 7.96 (s, 1H), 8.22 (d, J = 8.4 Hz, 2H), 8.49 (d, J = 4.0 Hz, 1H), |
| | | MS (ESI) m/z = 542 (M+H)⁺. |
| | | LC/MS t_{R} = 2.30 min. |

**[Table 2-5G]**

| Example No. | R | property data |
|---|---|---|
| Example 2-194 | 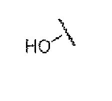 | 1H-NMR (300 MHz, DMSO-d₆) δ 0.57-0.61 (m, 2H), 0.69-0.71 (m, 2H), 1.21-1.25 (m, 2H), 1.63 (d, J = 11.4 Hz, 2H), 1.92-1.94 (m, 1H), 2.84-2.87 (m, 1H), 3.18-3.27 (m, 4H), 3.85 (dd; J = 11.2, 2.8 Hz, 2H), 5.71 (s, 1H), 7.38 (t, J = 5.9 Hz, 1H), 7.88-7.90 (m, 3H), 8.25 (d, J = 8.2 Hz, 2H), 8.45 (d, J = 4.0 Hz, 1H), 10.76 (s, 1H), |
| | | MS (ESI) m/z = 408 (M+H)⁺. |
| | | LC/MS t_{R} = 1.08 min. |
| Example 2-195 | 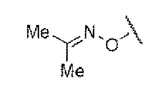 | 1H-NMR (300 MHz, DMSO-d₆) δ 0.57-0.61 (m, 2H), 0.69-0.71 (m, 2H), 1.21-1.25 (m, 2H), 1.63 (d, J = 11.4 Hz, 2H), 1.92-1.94 (m, 1H), 2.84-2.87 (m, 1H), 3.18-3.27 (m, 4H), 3.85 (dd, J = 11.2, 2.8 Hz, 2H), 5.71 (s, 1H), 7.38 (t, J = 5.9 Hz, 1H), 7.88-7.90 (m, 3H), 8.25 (d, J = 8.2 Hz, 2H), 8.45 (d, J = 4.0 Hz, 1H), 10.76 (s, 1H). |
| | | MS (ESI) m/z = 408 (M+H)⁺. |
| | | LC/MS t_{R} = 1.08 min. |
| Example 2-196 | 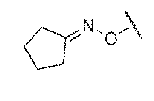 | 1H-NMR (300 MHz, DMSO-d₆) δ 0.57-0.61 (m, 2H), 0.69-0.71 (m, 2H), 1.21-1.25 (m, 2H), 1.63 (d, J = 11.4 Hz, 2H), 1.92-1.94 (m, 1H), 2.84-2.87 (m, 1H), 3.18-3.27 (m, 4H), 3.85 (dd, J = 11.2, 2.8 Hz, 2H), 5.71 (s, 1H), 7.38 (t, J = 5.9 Hz, 1H), 7.88-7.90 (m, 3H), 8.25 (d, J = 8.2 Hz, 2H), 8.45 (d, J = 4.0 Hz, 1H), 10.76 (s, 1H). |
| | | MS (ESI) m/z = 408 (M+H)⁺. |
| | | LC/MS t_{R} = 1.08 min. |
| Example 2-197 | 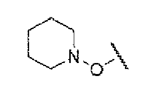 | 1H-NMR (300 MHz, DMSO-d₆) δ 0.57-0.61 (m, 2H), 0.69-0.71 (m, 2H), 1.21-1.25 (m, 2H), 1.63 (d, J = 11.4 Hz, 2H), 1.92-1.94 (m, 1H), 2.84-2.87 (m, 1H), 3.18-3.27 (m, 4H), 3.85 (dd, J = 11.2, 2.8 Hz, 2H), 5.71 (s, 1H), 7.38 (t, J = 5.9 Hz, 1H), 7.88-7.90 (m, 3H), 8.25 (d, J = 8.2 Hz, 2H), 8.45 (d, J = 4.0 Hz, 1H), 10.76 (s, 1H), |
| | | MS (ESI) m/z = 408 (M+H)⁺. |
| | | LC/M$ t_{R} = 1.08 min. |

**[Table 2-5H]**

| Example No. | R | property data |
|---|---|---|
| Example 2-198 | 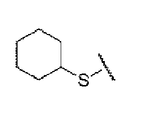 | 1H-NMR (300 MHz, DMSO-d₆) δ 0.63-0.69 (m, 2H), 0.90 (q, J = 6.4 Hz, 2H), 1.29-1.58 (m, 7H), 1.67-2.01 (m, 6H), 2.19-2.27 (m, 2H), 2.91-2.98 (m, 1H), 3.20 (t, J = 6.6 Hz, 2H), 3.40 (dt, J = 1.5, 11.7 Hz, 2H), 3.81-3.90 (m, 1H), 4.01 (dd, J = 4.1, 11.1 Hz, 2H), 5.75 (t, J = 6.1 Hz, 1H), 5.87 (s, 1H), 6.25 (s, 1H), 7.75 (s, 1H), 7.81 (d, J = 8.6 Hz, 2H), 8.16 (d, J = 8.6 Hz, 2H). |
| | | MS (ESI) m/z = 506 (M+H)⁺. |
| | | LC/MS t_{R} = 2.45 min. |

[Table 2-6A]

**Table 2-6**

| | | |
|---|---|---|
| 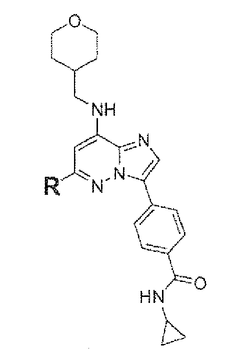 | | |

| Compounds described in Table 2-6 were synthesized in accordance with the processes of Example 2-1 and Example 2-4. | | |
|---|---|---|
| Example No. | R | property data |
| Example 2-199 | 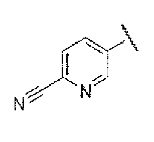 | 1H-NMR (400 MHz, DMSO-d₆) δ 0.59 (br s, 2H), 0.66-0.75 (m, 2H), 1.22-1.36 (m, 2H), 1.59-1.69 (m, 2H), 1.99 (s, 1H), 2.87 (s, 1H), 3.13-3.31 (m, 4H), 3.84 (d, J = 10.1 Hz, 2H), 6.85 (s, 1H), 7.80 (br s, 1H), 7.94 (d, J = 7.6 Hz, 2H), 8.10 (s, 1H), 8.17 (d, J = 7.3 Hz, 1H), 8.26 (d, J = 7.3 Hz, 2H), 8.47 (s, 1H), 8.66 (d, J = 7.3 Hz, 1H), 9.41 (s, 1H). |
| | | MS (ESI) m/z = 494 (M+H)⁺. |
| | | LC/MS t_{R} = 1.73 min. |

**[Table 2-6B]**

| Example No. | R | property data |
|---|---|---|
| Example 2-200 | 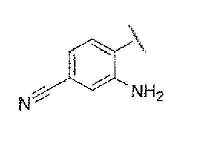 | 1H-NMR (400 MHz, DMSO-d₆) δ 0.54-0.64 (m, 2H), 0.67-0.77 (m, 2H), 1.19-1.37 (m, 2H), 1.61-1.72 (m, 2H, m), 1.89-2.05 (m, 1H), 2.80-2.91 (m, 1H), 3.85-3.88 (m, 2H), 6.58 (s, 1H), 7.05 (d, J = 7.97 Hz, 1H), 7.13 (s, 1H), 7.28-7.36 (m, 2H), 7.57-7.74 (m, 3H), 7.93 (d, J = 9.34 Hz, 2H), 8.16 (d, J = 7.97 Hz, 3H), 8.20 (s, 1H), 8.49 (d, J = 4.12 Hz, 1H). |
| | | MS (ESI) m/z = 508 (M+H)⁺. |
| | | LC/MS t_{R} = 1.91 min. |
| Example 2-201 | 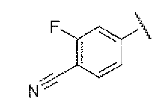 | MS (ESI) m/z = 494 (M+H)⁺. |
| | | LC/MS t_{R} = 1.73 min. |
| Example 2-202 | 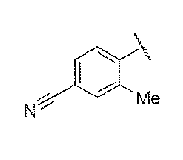 | 1H-NMR (400 MHz, DMSO-d₆) *δ* 0.55-0.66 (m, 2H), 0.81-0.93 (m, 2H), 1.33-1.53 (m, 2H), 1.63-1.83 (m, 2H), 1.86-2.02 (m, 1H), 2.45 (s, 3H), 2.83-2.97 (m, 1.0H), 3.26 (t, J = 6.59 Hz, 2H), 3.40 (td, J = 11.81, 1.65 Hz, 2H), 4.00 (dd, J = 11.81, 4.12 Hz, 2H), 5.97-6.13 (m, 2.0H), 6.18-6.32 (br s, 1.0H), 7.49-7.66 (m, 3.0H), 7.63-7.79 (d, J = 8.52 Hz, 2H), 7.88 (s, 1H), 8.12 (d, J = 8.52 Hz, 2H). |
| | | MS (ESI) m/z = 507 (M+H)⁺. |
| | | LC/MS t_{R} = 2.03 min, |
| Example 2-203 | 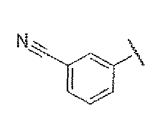 | 1H-NMR (400 MHz, DMSO-d₆) δ 0.60-0.71 (m, 2.0H), 0.85-0.95 (m, 2H), 1.39-1.56 (m, 2H), 1.75-1.86 (m, 2.0H), 1.94-2.09 (m, 1H), 2.88-2.99 (m, 1.0H), 3.28-3.38 (m, 2H), 3.38-3.48 (m, 2H), 4.03 (dd, J = 10.9, 3.85 Hz, 2H), 6.01-8.10 (m, 1.0H), 6.28-6.34 (m, 1H), 6.38 (s, 1H), 7.62 (t, J 7.88 Hz, 1H), 7.75 (d, J = 8.69 Hz, 1H), 7.83-7.90 (m, 2H), 8.17 (s, 1H, s), 8.21-8.23 (m, 2H). |
| | | MS (ESI) m/z = 493 (M+H)⁺. |
| | | LC/MS t_{R} = 2.04 min. |

**[Table 2-6C]**

| Example No. | R | property data |
|---|---|---|
| Example 2-204 | 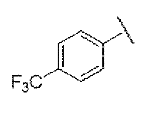 | 1H-NMR (400 MHz, DMSO-d₆) δ 0.57-0.69 (m, 2H), 0.80-0.96 (m, 2H), 1.37-1.54 (m, 2.0H), 1.74-1.85 (m, 2H), 1.90-2.09 (m, 1H), 2.85-2.99 (m, 1H), 3.28-3.47 (m, 4H), 4.02 (dd, J = 4.12, 11.54 Hz, 2H), 5.89-6.00 (m, 1H), 6.19-6.31 (m, 1H), 6.42 (s, 1H), 7.75 (d, J = 8.24 Hz, 2H), 7.86 (d, J = 6.87 Hz, 2H), 7.86 (s, 1H), 8.05 (d, J = 7.69 Hz, 2H), 8.22 (d, J = 8.52 Hz, 2H). |
| | | MS (ESI) m/z = 535 (M+H)⁺. |
| | | LC/MS t_{R} = 2.38 min. |
| Example 2-205 | 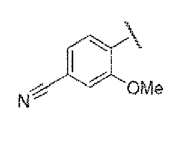 | 1H-NMR (400 MHz, DMSO-d₆) δ 0.57-0.66 (m, 1H), 0.83-0.92 (m, 1HH), 1.44 (ddd, J = 24.72, 12.36, 4.94 Hz, 2H), 1.71-1.81 (m, 2H). 1.90-2.06 (m, 1H), 2.87-2.95 (m, 1H), 3.26-3.28 (m, 2H), 3.37-3.41 (m, 2H), 3.89 (s, 3H), 4.01 (dd, J = 11.81, 4.12 Hz, 2H), 5.83-5.92 (m, 1H), 6.20-6.25 (m, 1H), 6.41 (s, 1H), 7.27 (d, J = 1.37 Hz, 1H), 7.39 (dd, J = 7.69, 1.37 Hz, 1H), 7.75 (d, J = 7.69 Hz, 1H), 7.81 (d, J = 8.24 Hz, 2H), 7.86 (s, 1H), 8.17 (d, J = 8.24 Hz, 2H). |
| | | MS (ESI) m/z = 523 (M+H)⁺. |
| | | LC/MS t_{R} = 1.98 min. |
| Example 2-206 | 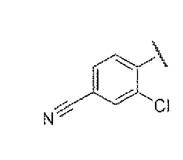 | 1H-NMR (400 MHz, DMSO-d₆) δ 0.56-0.65 (m, 2HH), 0.83-0.91 (m, 2H), 1.43 (ddd, J = 25.27, 12.36, 4.39 Hz, 2H), 1.70-1.81 (m, 2H), 1.89-2.06 (m, 1H), 2.85-2.94 (m, 1H), 3.28 (t, J = 6.59 Hz, 2H), 3.39 (dt, J = 1.65, 11.81 Hz, 2H), 4.00 (dd, J = 11.81, 3.57 Hz, 2H), 6.00 (t, J = 4.67 Hz, 1H), 6.19-6.28 (m, 2H), 7.64-7.74 (m, 2H), 7.76-7.79 (m, 1H), 7.80-7.84 (m, 2H), 7.87-7.90 (m, 1H), 8.14 (d, J = 8.52 Hz, 2H). |
| | | MS (ESI) m/z = 527 (M+H)⁺. |
| | | LC/MS t_{R} = 2.06 min. |
| Example 2-207 | 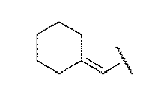 | 1H-NMR (400 MHz, DMSO-d₆) δ 0.61 (s, 2H), 0.72 (s, 2H), 1.16-1.29 (m, 2H), 1.64 (s, 7H), 2.28 (s, 2H), 2.78 (s, 2H), 2.87 (s, 1H), 3.26 (t, J = 12.4 Hz, 4H), 3.85 (d, J = 10.0 Hz, 3H), 6.16 (d, J = 19.6 Hz, 2H), 7.44 (s, 1H), 7.91 (d, J = 8.1 Hz, 2H), 8.06 (s, 1H), 8.28 (d, J = 8.1 Hz, 2H), 8.47 (s, 1H). |
| | | MS (ESI) m/z = 486 (M+H)⁺. |
| | | LC/MS t_{R} = 2.11 min. |

**[Table 2-6D]**

| Example No. | R | property data |
|---|---|---|
| Example 2-208 | 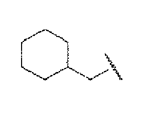 | 1H-NMR (400 MHz, DMSO-d₆) δ 0.60 (s, 2H), 0.72 (d, J = 5.6 Hz, 2H), 1.01-1.06 (m, 1H). 1.19-1.28 (m, 5H), 1.62-1.73 (m, 8H), 1.94 (s, 1H), 2.59 (d, J = 6.6 Hz, 2H), 2.88 (s, 1H), 3.23-3.29 (m, 5H), 3.85 (d, J = 8.3 Hz, 2H), 6.15 (s, 1H), 7.36 (s, 1H), 7.92 (d, J = 8.6 Hz, 2H), 8.02 (s, 1H). 8.29 (d, J = 8.0 Hz, 2H), 8.45 (s, 1H). |
| | | MS (ESI) m/z = 488 (M+H)⁺. |
| | | LC/MS t_{R} = 2.14 min. |
| Example 2-209 | 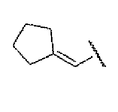 | 1H-NMR (400 MHz, DMSO-d₆) δ 0.61 (s, 2H), 0.72 (d, J = 5.4 Hz, 2H), 1.26 (d, J = 10.0 Hz, 2H), 1.63-1.68 (m, 4H), 1.77-1.80 (m, 2H), 1.96 (s, 1H), 2.78 (s, 2H), 2.87 (s, 1H), 3.24-3.30 (m, 4H), 3.86 (d, J = 10.8 Hz, 2H), 6.20 (s, 1H), 6.38 (s, 1H), 7.40 (s, 1H), 7.92 (d, J = 8.4 Hz, 2H), 8.03 (s, 1H), 8.29 (d, J = 8.4 Hz, 2H), 8.49 (s, 1H). |
| | | MS (ESI) m/z = 472 (M+H)⁺. |
| | | LC/MS t_{R} = 1.98 min. |
| Example 2-210 | 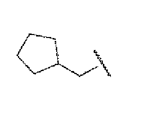 | 1H-NMR (400 MHz, DMSO-d₆) δ 0.61 (s, 2H), 0.71 (s, 2H), 1.28 (s, 4H), 1.52-1.75 (m, 8H), 1.95 (s, 1H), 2.33 (t, J = 6.8 Hz, 1H), 2.70 (d, J = 6.8 Hz, 2H), 2.89 (s, 1H), 3.24-3.30 (m, 3H), 3.86 (d, J = 11.6 Hz, 2H), 6.17 (s, 1H), 7.37 (s, 1H), 7.92 (d, J = 7.2 Hz, 2H), 8.03 (s, 1H), 8.29 (d, J = 3.2 Hz, 2H), 8.46 (s, 1H). |
| | | MS (ESI) m/z = 474 (M+H)⁺. |
| | | LC/MS t_{R} = 2.01 min. |
| Example 2-211 | 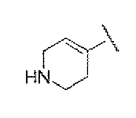 | 1H-NMR (400 MHz, DMSO-d₆) δ 0.60 (s, 2H), 0.72 (s, 2H), 1.24-1.33 (m, 2H), 1.65 (d, J = 10.0 Hz, 2H), 1.96 (s, 1H), 2.86 (s, 3H), 3.86 (s, 4H), 6.53 (s, 1H), 6.79 (s, 1H), 7.53 (s, 1H), 7.94 (d, J = 6.8 Hz, 2H), 8.09 (s, 1H), 8.27 (d, J = 6.8 Hz, 2H), 8.51 (s, 1H). |
| | | MS (ESI) m/z = 473 (M+H)⁺. |
| | | LC/MS t_{R} = 1.01 min. |
| Example 2-212 | 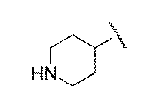 | 1H-NMR (400 MHz, DMSO-d₆) & 0.70 (s, 2H), 0.86 (s, 2H), 1.44-1.48 (m, 2H), 1.81-1.91 (m, 3H), 2.03 (s, 3H), 2.81-2.91 (m, 4H), 3.22 (d, J = 10.0 Hz, 1H), 3.48 (t, J = 10.0 Hz, 2H), 4.01 (d, J = 9.6 Hz, 2H), 6.21 (s, 1H), 7.93 (s, 3H), 8.29 (s, 2H), |
| | | MS (ESI) m/z = 475 (M+H)⁺. |
| | | LC/MS t_{R} = 0.90 min. |

**[Table 2-6E]**

| Example No. | R | property data |
|---|---|---|
| Example 2-213 | 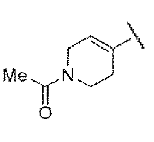 | 1H-NMR (400 MHz, DMSO-d₆) δ 0.60 (s, 2H), 0.71 (d, J = 4.4 Hz, 2H), 1.28 (d, J = 10.8 Hz, 2H), 1.65 (d, J = 11.6 Hz, 2H), 1.97 (br s, 1H), 2.09 (d, J = 2.8 Hz, 3H), 2.63 (s, 1H), 2.74 (s, 1H), 2.88 (s, 1H), 3.25-3.30 (m, 3H), 3.66 (s, 2H), 3.85 (d, J = 8.8 Hz, 2H), 4.21 (d, J = 17.6 Hz, 2H), 6.50 (s, 1H), 6.74 (d, J = 9.2 Hz, 1H), 7.40 (s, 1H), 7.94 (d, J = 7.6 Hz, 2H), 8.06 (s, 1H), 8.29 (d, J = 7.6 Hz, 2H), 8.47 (s, 1H). |
| | | MS (ESI) m/z = 515 (M+H)⁺. |
| | | LC/MS t_{R} = 1.35 min. |

Example 2-214

### 4-(7-Chloro-8-((tetrahydro-2H-pyran-4-yl)methylamino)-6-(1,1,1-trifluoro-2-methylpropan-2-ylamino)imidazo[1,2-b]pyridazin-3-yl)-N-cyclopropylbenzamide

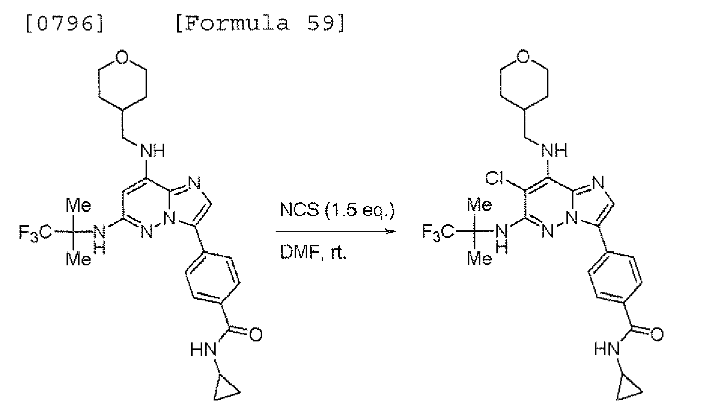

To a DMF (3.0 mL) solution of N-cyclopropyl-4-(8-((tetrahydro-2H-pyran-4-yl)methylamino)-6-(1,1,1-trifluoro-2-methylpropan-2-ylamino)imidazo[1,2-b]pyridazin-3-yl)benzamide (300 mg, 0.581 mol), NCS (116 mg, 0.871 mmol) was added and stirred at room temperature for one hour. To the reaction solution, water and ethyl acetate were added to separate phases. Thereafter, the water phase was extracted with ethyl acetate and organic phases were combined, washed with a 10% aqueous potassium carbonate solution and saturated saline, and dried over magnesium sulfate. The organic phase was filtrated and then concentrated under reduced pressure. The resultant residue was purified by medium-pressure silica gel chromatography (hexane/ethyl acetate; 30-100% ethyl acetate gradient) to obtain the titled compound (217 mg, 0.394 mmol, 68%) as a colorless solid substance.
1H-NMR (400 MHz, DMSO-d₆) δ 0.56-0.74 (m, 4H), 1.15-1.32 (m, 2H), 1.54-1.64 (m, 2H), 1.68 (s, 6H), 1.90 (m, 1H), 2.86 (m, 1H), 3.16-3.27 (m, 2H), 3.77-3.87 (m, 2H), 4.04 (t, J = 6.9 Hz, 2H), 5.31 (s, 1H), 6.94 (t, J = 6.3 Hz, 1H), 7.90 (d, J = 8.4 Hz, 2H), 7.74 (s, 1H), 8.10 (d, J = 8.4Hz, 2H), 8.49 (d, J = 4.2 Hz, 1H).
MS (ESI) m/z = 551 (M+H)⁺.
LC/MS t_{R} = 2.12 min.

### Example 2-215

N-Cyclopropyl 7-4-(7-flucro-8-((tetrahydro-2H-pyran-4-yl)methylamino)-6-(1,1,1-trifluoro-2-methylpropan-2-ylamino)imidazo[1,2-b]pyridazin-3-yl)benzamide

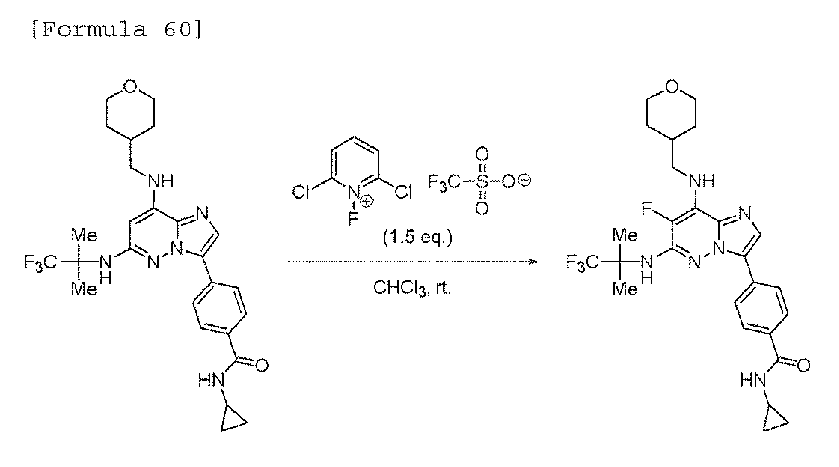

To a DMF (3.0 mL) solution of N-cyclopropyl-4-(8-((tetrahydro-2H-pyran-4-yl)methylamino)-6-(1,1,1-trifluoro-2-methylpropan-2-ylamino)imidazo[1,2-b]pyridazin-3-yl)benzamide (300 mg, 0.581 mol), N-fluoro-2,6-dichloropyridinium triflate (275 mg, 0.871 mmol) was added and stirred at room temperature for 3 hours. To the reaction solution, water and ethyl acetate were added to separate phases. Thereafter, the water phase was extracted with ethyl acetate and organic phases were combined, washed with a 10% aqueous potassium carbonate solution and saturated saline, dried over magnesium sulfate. The organic phase was filtrated and then concentrated under reduced pressure. The resultant residue was purified by medium-pressure silica gel chromatography (hexane/ethyl acetate; 30-100% ethyl acetate gradient) to obtain the titled compound (33 mg, 0.062 mmol, 11%) as a light brown solid substance.
1H-NMR (400 MHz, DMSO-d₆) δ 0.56-0.74 (m, 4H), 1.14-1.30 (m, 2H), 1.56-1.65 (m, 2H), 1.67 (s, 6H), 1.88 (m, 1H), 2.86 (m, 1H), 3.18-3.30 (m, 2H), 3.59 (t, J = 6.6 Hz, 2H), 3.79-8.88 (m, 2H), 6.12 (d, J = 2.4 Hz, 1H), 6.92 (m, 1H), 7.85 (s, 1H), 7.88 (d, J = 8.7 Hz, 2H), 8.11 (d, J = 8.7Hz, 2H), 8.47 (d, J = 4.2 Hz, 1H).
MS (ESI) m/z = 535 (M+H)⁺.
LC/MS t_{R} = 1.86 min.

[Table 2-7A]

**Table 2-7**

| | | | |
|---|---|---|---|
| 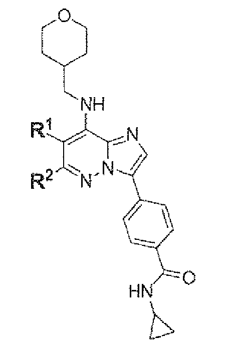 | | | |

| Compounds described in Table 2-7 were synthesized in accordance with the processes of Example 2-214 and Example 2-215. | | | |
|---|---|---|---|
| Example No. | R¹ | R² | property data |
| Example 2-216 | Cl | 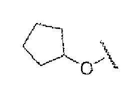 | 1H-NMR (400 MHz, DMSO-d₆) δ 0.57-0.61 (m, 2H), 0.68-0.73 (m, 2H), 1.21-1.29 (m, 2H), 1.55-1.69 (m, 4H), 1.69-1.80 (m, 2H), 1.82-1.96 (m, 3H), 1.67-2.08 (m, 2H), 2.84-2.90 (m, 1H), 3.18-3.26 (m, 2H), 3.83 (dd, J = 11.66, 2.53 Hz, 2H), 4.05 (t, J = 6.59 Hz, 2H), 5.30-5.34 (m, 1H), 7.02 (t, J = 6.59 Hz, 1H), 7.93 (d, J = 8.62 Hz, 2H), 8.23 (d, J = 8.62 Hz, 2H), 8.46 (d, J = 4.06 Hz, 1H). |
| | | | MS (ESI) m/z = 510 (M+H)⁺. |
| | | | LC/MS t_{R} = 2.58 min. |
| Example 2-217 | F | 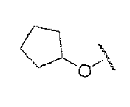 | 1H-NMR (400 MHz, DMSO-d₆) δ 0.57-0.61 (m, 2H), 0.68-0.73 (m, 2H), 1.21 (ddd, J = 24.71, 12.29, 4.18 Hz, 2H), 1.57-1.69 (m, 4H), 1.69-1.81 (m, 2H), 1.82-1.93 (m, 3H), 2.00-2.10 (m, 2H), 2.84-2.90 (m, 1H), 3.21-3.29 (m, 2H), 3.59 (t, J = 6.34 Hz, 2H), 3.82-3.85 (m, 2H), 5.32-5.36 (m, 1H), 7.10-7.16 (m, 1H), 7.92 (d, J = 8.62 Hz, 2H), 8.22 (d, J = 8.11 Hz, 2H), 8.45 (d, J = 4.06 Hz, 1H). |
| | | | MS (ESI) m/z = 494 (M+H)⁺. |
| | | | LC/MS t_{R} = 2.37 min. |

**[Table 2-7B]**

| Example No. | R¹ | R² | property data |
|---|---|---|---|
| Example 2-218 | Cl | 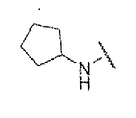 | 1H-NMR (400 MHz, DMSO-d₆) δ 0.57-0.61 (m, 2H), 0.68-0.73 (m, 2H), 1.18-1.28 (m, 2H), 1.53-1.76 (m, 9H), 1.83-1.93 (m, 1H), 1.99-2.09 (m, 2H), 2.83-2.89 (m, 1H), 3.18-3.25 (m, 2H), 3.81-3.84 (m, 2H), 4.02 (t, J = 6.84 Hz, 2H), 4.10-4.15 (m, 1H), 5.81 (d, J = 6.59 Hz, 1H), 6.66 (t, J = 6.84 Hz, 1H), 7.90 (d, J = 8.62 Hz, 2H), 8.30 (d, J = 8.62 Hz, 2H), 8.44 (d, J = 4.06 Hz, 1H). |
| | | | MS (ESI) m/z = 509 (M+H)⁺. |
| | | | LC/MS t_{R} = 2.39 min. |

[Table 2-8A]

**Table 2-8**

| | | | |
|---|---|---|---|
| 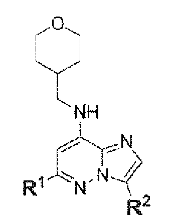 | | | |

| Compounds described in Table 2-8 were synthesized in accordance with the processes of Example 2-87 and Example 2-88. | | | |
|---|---|---|---|
| Example No. | R¹ | R² | property data |
| Example 2-219 | 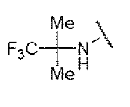 | 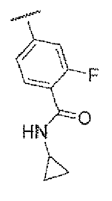 | 1H-NMR (300 MHz, DMSO-d₆) δ 0.52-0.74 (m, 4H), 1.15-1.32 (m, 2H), 1.56-1.70 (m, 2H), 1.64 (s, 6H), 1.95 (m, 1H), 2.85 (m, 1H), 3.08 (t, J = 6.6 Hz, 2H), 3.22-3.32 (m, 2H), 3.81-3.91 (m, 2H), 5.81 (s, 1H), 6.52 (s, 1H), 7.16 (t, J = 6.0 Hz, 1H), 7.59 (t, J = 8.1 Hz, 1H), 7.88 (s, 1H), 7.90 (m, 1H), 8.16 (dd, J = 1.5 Hz, 12.9 Hz, 1H), 8.32 (m, 1H). |
| | | | MS (ESI) m/z = 535 (M+H)⁺. |
| | | | LO/MS t_{R} = 1.75 min. |

**[Table 2-8B]**

| Example No. | R¹ | R² | property data |
|---|---|---|---|
| Example 2-220 | 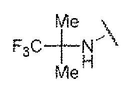 | 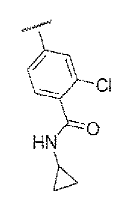 | 1H-NMR (300 MHz, DMSO-d₆) δ 0.50-0.75 (m, 4H), 1.15-1.32 (m, 2H), 1.56-1.72 (m, 2H), 1.65 (s, 6H), 1.95 (m, 1H), 2.83 (m, 1H), 3.08 (t, J = 6.6 Hz, 2H), 3.22-3.32 (m, 2H), 3.81-3.91 (m, 2H), 5.81 (s, 1H), 8.5 (s, 1H), 7.15 (t, J = 6.0 Hz, 1H), 7.42 (d, J = 8.1 Hz, 1H), 7.85 (s, 1H), 7.93 (dd, J = 1.8, Hz, 8.1 Hz, 1H), 8.43 (d, J = 1.8 Hz, 1H), 8.49 (d, J =4.5 Hz, 1H). |
| | | | MS (ESI) m/z = 551 (M+H)⁺. |
| | | | LC/MS t_{R} = 1.71 min. |
| Example 2-221 | 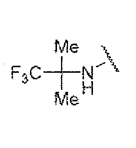 | 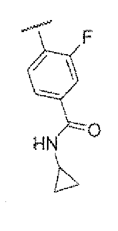 | 1H-NMR (300 MHz, DMSO-d₆) δ 0.56-0.76 (m, 4H), 1.15-1.32 (m, 2H), 1.54-1.68 (m, 2H), 1.59 (s, 6H), 1.95 (m, 1H), 2.86 (m, 1H), 3.09 (t, J = 6.6 Hz, 2H), 3.22-3.32 (m, 2H), 3.81-3.91 (m, 2H), 5.82 (s, 1H), 6.49 (s, 1H), 7.19 (t, J = 6.0 Hz, 1H), 7.65 (d, J = 4.2 Hz, 1H), 7.74 (s, 1H), 7.77 (dd, J = 1.5 Hz, 5.4 Hz, 1H), 8.39 (t, J = 8.4 Hz, 1H), 8.57 (d, J = 4.2 Hz, 1H). |
| | | | MS (ESI) m/z = 535 (M+H)⁺. |
| | | | LC/MS t_{R} = 1.66 min. |
| Example 2-222 | 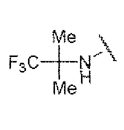 | 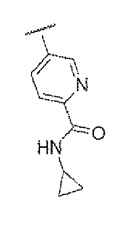 | 1H-NMR (300 MHz, DMSO-d₅) δ 0.70 (d, J = 5.7 Hz, 4H), 1.16-1.32 (m, 2H), 1.56-1.70 (m, 2H), 1.63 (s, 6H), 1.94 (m, 1H), 2.91 (m, 1H), 3.09 (t, J = 6.6 Hz, 2H), 3.22-3.34 (m, 2H), 3.80-3.92 (m, 2H), 5.83 (s, 1H), 6.52 (s, 1H), 7.20 (t, J = 6.0 Hz, 1H), 7.91 (s, 1H), 8.04 (d, J = 8.1 Hz, 1H), 7.59 (dd, J = 2.1 Hz, 8.1 Hz, 1H), 8.71 (d, J = 4.8 Hz, 1H), 9.21 (d, J = 2.1 Hz, 1H). |
| | | | MS (ESI) m/z = 518 (M+H)⁺. |
| | | | LC/MS t_{R} = 1.74 min. |

**[Table 2-8C]**

| Example No. | R¹ | R² | property data |
|---|---|---|---|
| Example 2-223 | 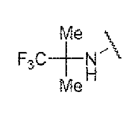 | 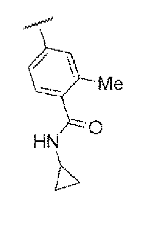 | 1H-NMR (400 MHz, DMSO-d₆) δ 0.55 (br s, 2H), 0.63-0.72 (m, 2H), 1.15-1.30 (m, 2H), 1.56-1.67 (m, 8H), 1.93 (br s, 1H), 2.35 (s, 3H), 2.77-2.88 (m, 1H), 3.03-3.12 (m, 2H), 3.27 (t, J = 11.1 Hz, 2H), 3.85 (d, J = 10.6 Hz, 2H), 5.80 (s, 1H), 6.47 (s, 1H), 7.01-7.10 (m, 1H), 7.33 (d, J = 8.1 Hz, 1H), 7.72-7.85 (m, 2H), 8.08 (s, 1H), 8.30 (d, J = 3.5 Hz, 1H). |
| | | | MS (ESI) m/z =518 (M+H)⁺. |
| | | | LC/MS t_{R} = 1.74 min. |
| Example 2-224 | 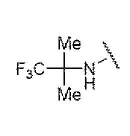 | 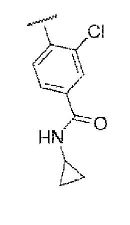 | 1H-NMR (400 MHz, DMSO-d₆) δ 0.60 (br s, 2H), 0.67-0.75 (m, 2H), 1.23-1.25 (m, 2H), 1.49 (s, 6H), 1.63 (d, J = 11.9 Hz, 2H), 1.86-2.01 (br m, 1H), 2.82-2.91 (m, 1H), 3.04-3.13 (m, 2H), 3.28 (t, J = 11.7 Hz, 2H), 3.81-3.91 (m, 2H), 5.81 (s, 1H), 6.42 (s, 1H), 7.13 (br s, 1H), 7.62 (s, 1H), 7.81-7.91 (m, 2H), 8.01 (d, J = 12.4 Hz, 1H), 8.62 (s, 1H). |
| | | | MS (ESI) m/z = 518 (M+H)⁺. |
| | | | LC/MS t_{R} = 1.74 min. |
| Example 2-225 | 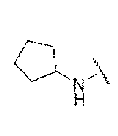 | 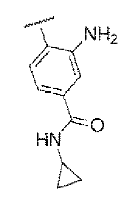 | 1H-NMR (400 MHz, DMSO-d₆) δ 0.56 (br s, 2H), 0.63-0.71 (m, 2H), 1.13-1.29 (m, 3H), 1.36-1.56 (m, 5H), 1.56-1.70 (m, 4H), 1.88 (br s, 3H), 2.84 (br s, 1H), 3.06-3.08 (br m, 2H), 3.25 (t, J = 11.5 Hz, 2H), 3.79-3.95 (m, 3H), 5.42 (s, 2H), 5.58 (s, 1H), 6.40 (d, J = 5.8 Hz, 1H), 6.94 (s, 1H), 7.07 (d, J = 7.8 Hz, 1H), 7.28 (s, 1H), 7.34 (s, 1H), 7.39 (d, J = 7.8 Hz, 1H), 8.31 (s, 1H). |
| | | | MS (ESI) m/z = 490 (M+H)⁺. |
| | | | LC/MS t_{R} = 1.31 min. |

**[Table 2-8D]**

| Example No. | R¹ | R² | property data |
|---|---|---|---|
| Example 2-226 | 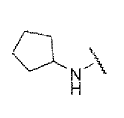 | 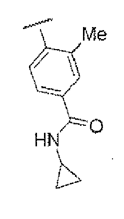 | 1H-NMR (400 MHz, DMSO-d₆) δ 0.60 (br s, 2H), 0.65-0.73 (m, 2H), 1.14-1.28 (m, 3H), 1.36-1.67 (m, 9H), 1.81 (br s, 2H), 1.82 (br s, 1H), 2.34 (s, 3H), 2.87 (br s, 1H), 3.00-3.10 (br m, 2H), 3.13-3.19 (br m, 2H), 3.25 (t, J =11.5 Hz, 2H), 3.77-3.89 (m, 3H), 4.15-4.17 (m, 1H). 5.59 (s, 1H), 6.27 (d, J = 5.1 Hz, 1H). 6.88 (s, 1H), 7.34 (s, 1H), 7.62-7.73 (m, 2H), 7.79 (s, 1H), 8.49 (s, 1H), |
| | | | MS (ESI) m/z = 489 (M+H)⁺. |
| | | | LC/MS t_{R} = 1.46 min. |
| Example 2-227 | 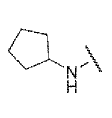 | 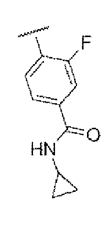 | 1H-NMR (300 MHz, OMSO-d₆) δ 0.59-0.62 (m, 2H), 0.71-0.72 (m, 2H), 1.21-1.25 (m, 2H), 1.58-1.65 (m, 8H), 1.94-1.98 (m, 3H), 2.86-2.88 (m, 1H), 3.09 (t, J = 6.5 Hz, 2H), 3.17 (d, J = 4.9 Hz, 1H), 3.25-3.27 (m, 1H), 3.86 (dd, J = 11.5, 2.6 Hz, 2H), 4.00-4.03 (m, 1H), 5.64 (s, 1H), 6.43 (d, J = 6.0 Hz, 1H), 7.00 (t, J = 6.0 Hz, 1H), 7.68-7.78 (m, 3H), 8.52 (d, J = 4.2 Hz, 1H), 8.81 (t, J = 8.1 Hz, 1H). |
| | | | MS (ESI) m/z = 493 (M+H)⁺. |
| | | | LC/MS t_{R} = 1.62 min. |
| Example 2-228 | 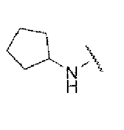 | 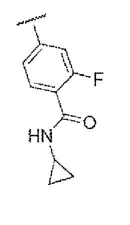 | 1H-NMR (300 MHz, DMSO-d₆) δ 0.56-0.58 (m, 2H), 0.69-0.71 (m, 2H), 1.20-1.28 (m, 2H), 1.60-1.67 (m, 8H), 1.97-2.04 (m, 3H), 2.84-2.85 (m, 1H), 3.08 (t, J = 6.5 Hz, 2H). 3.24-3.27 (m, 2H), 3.86 (dd, J =11.3, 2.6 Hz, 2H). 4.03-4.04 (m, 1H), 5.62 (s, 1H), 6.47 (d, J = 5.9 Hz, 1H), 6.97 (t, J = 6.1 Hz, 1H), 7.60 (t, J = 8.1 Hz, 1H), 7.90 (s, 1H), 8.02 (dd, J = 8.1, 1.4 Hz, 1H), 8.28 (dd, J = 4.3, 1.6 Hz, 1H), 8.39 (dd, J = 13.2. 1.4 Hz, 1H). |
| | | | MS (ESI) m/z = 493 (M+H)⁺. |
| | | | LC/MS t_{R} = 1.73 min. |

**[Table 2-8E]**

| Examph No. | R¹ | R² | property data |
|---|---|---|---|
| Example 2-229 | 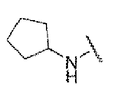 | 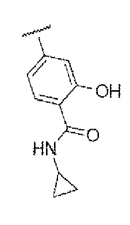 | 1H-NMR (300 MHz, DMSO-d₆) δ 0.61-0.66 (m, 2H), 0.69-0.78 (m, 2H), 1.18-1.27 (m, 2H), 1.54-1.75 (m, 8H), 1.95-2.00 (m, 3H), 2.87-2.89 (m, 1H), 3.08 (t, J = 6.5 Hz, 2H), 3.25-3.27 (m, 1H), 3.86 (dd, J = 11.2, 2.7 Hz, 2H), 4.03-4.07 (m, 2H), 5.60 (s, 1H), 6.41 (d, J = 5.9 Hz, 1H), 6.92 (t, J = 6.0 Hz, 1H), 7.70 (dd, J = 8.5, 1.6 Hz, 1H), 7.84 (t, J = 4.3 Hz, 2H), 7.94 (d, J = 1.7 Hz, 1H), 8.77 (s, 1H). 12.93 (s, 1H). |
| | | | MS (ESI) m/z = 491 (M+H)⁺. |
| | | | LC/MS t_{R} = 1.75 min. |
| Example 2-230 | 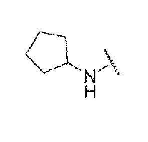 | 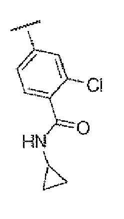 | 1H-NMR (300 MHz, DMSO-d₆) δ 0.53-0.54 (m, 2H), 0.69-0.70 (m, 2H), 1.21-1.23 (m, 2H), 1.58-1.66 (m, 8H), 2.03-2.06 (m, 3H), 2.82-2.83 (m, 1H). 3.08 (t, J = 6.5 Hz, 2H), 3.23-3.27 (m, 1H), 3.84-3.86 (m, 2H), 4.00-4.04 (m, 1H), 5.61 (s, 1H), 6.48 (d, J = 5.8 Hz, 1H), 6.99 (t, J = 6.0 Hz, 1H), 7.42 (d, J = 8.1 Hz, 1H), 7.87 (s, 1H), 8.06 (d, J = 8.1 Hz, 1H), 8.46 (d, J = 4.4 Hz, 1H), 8.66 (s, 1H). |
| | | | MS (ESI) m/z = 509 (M+H)⁺. |
| | | | LC/MS t_{R} = 1.67 min. |
| Example 2-231 | 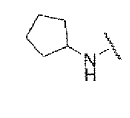 | 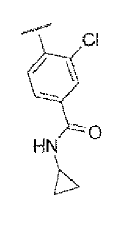 | 1H-NMR (300 MHz, DMSO-d₆) δ 0.60-0.61 (m, 2H), 0.71-0.72 (m, 2H), 1.21-1.23 (m, 2H), 1.45-1.52 (m, 4H), 1.61-1.65 (m, 4H), 1.88-1.95 (m, 4H), 2.86-2.90 (m, 1H), 3.08 (t, J = 6.5 Hz, 2H), 3.28 (t, J = 11.7 Hz, 2H), 3.85-3.87 (m, 3H), 5.59 (s, 1H), 6.31 (d, J =6.1 Hz, 1H). 6.97 (t, J = 6.0 Hz, 1H), 7.59 (s, 1H), 7.84 (dd, J = 8.2, 1.7 Hz, 1H), 8.01-8.05 (m, 2H), 8.60 (d, J = 4.3 Hz, 1H). |
| | | | MS (ESI) m/z = 509 (M+H)⁺. |
| | | | LC/MS t_{R} = 1.58 min. |

**[Table 2-8F]**

| Example No. | R¹ | R² | property data |
|---|---|---|---|
| Example 2-232 | 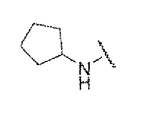 | 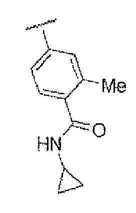 | 1H-NMR (300 MHz, OMSO-d₆) δ 0.53-0.54 (m, 2H), 0.67-0.69 (m, 2H), 1.21-1.24 (m, 2H), 1.57-1.68 (m, 8H), 2.00-2.02 (m, 3H), 2.37 (s, 3H), 2.82-2.84 (m, 1H), 3.08 (t, J = 6.5 Hz, 2H), 3.24-3.27 (m, 2H), 3.86 (dd, J = 11.3, 2.6 Hz, 2H), 4.00-4.03 (m, 1H), 5.58 (s, 1H), 6.38 (d, J = 5.7 Hz, 1H), 6.89 (t, J = 6.0 Hz, 1H), 7.32 (d, J = 8.1 Hz, 1H), 7.74 (s, 1H), 8.01 (dd, J = 8.1, 1.5 Hz, 1H), 8.20 (s, 1H), 8.26 (d, J = 4.4 Hz, 1H). |
| | | | MS (ESI) m/z = 489 (M+H)⁺. |
| | | | LC/MS t_{R} = 1.54 min. |
| Example 2-233 | 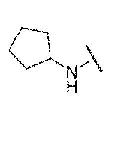 | 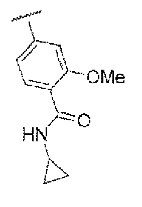 | 1H-NMR (300 MHz, DMSO-d₆) δ 0.55-0.58 (m, 2H), 0.69-0.71 (m, 2H), 1.21-1.23 (m, 2H), 1.52-1.68 (m, 8H), 1.95-1.99 (m, 3H), 2.84-2.85 (m, 1H), 3.08 (t, J = 6.5 Hz. 2H), 3.24-3.27 (m, 2H), 3.86 (dd, J = 11.6. 2.5 Hz. 2H), 3.94 (s, 3H), 4.12-4.15 (m, 1H), 5.60 (s, 1H), 6.42 (d, J = 6.5 Hz, 1H), 6.93 (t, J = 6.0 Hz, 1H), 7.72-7.77 (m, 2H), 7.84 (s, 1H), 8.06 (d, J = 4.4 Hz. 1H), 8.1 (d, J = 1.2 Hz, 1H). |
| | | | MS (ESI) m/z = 505 (M+H)⁺. |
| | | | LC/MS t_{R} = 1.66 min. |

### (Example 3)

### Example 3-1

4-(6-(Cyclopentyloxy)-8-(isobutylamino)-[1,2,4]triazolo[4,3-b]pyridazin-3-yl)-N-cyclopropylbenzamide

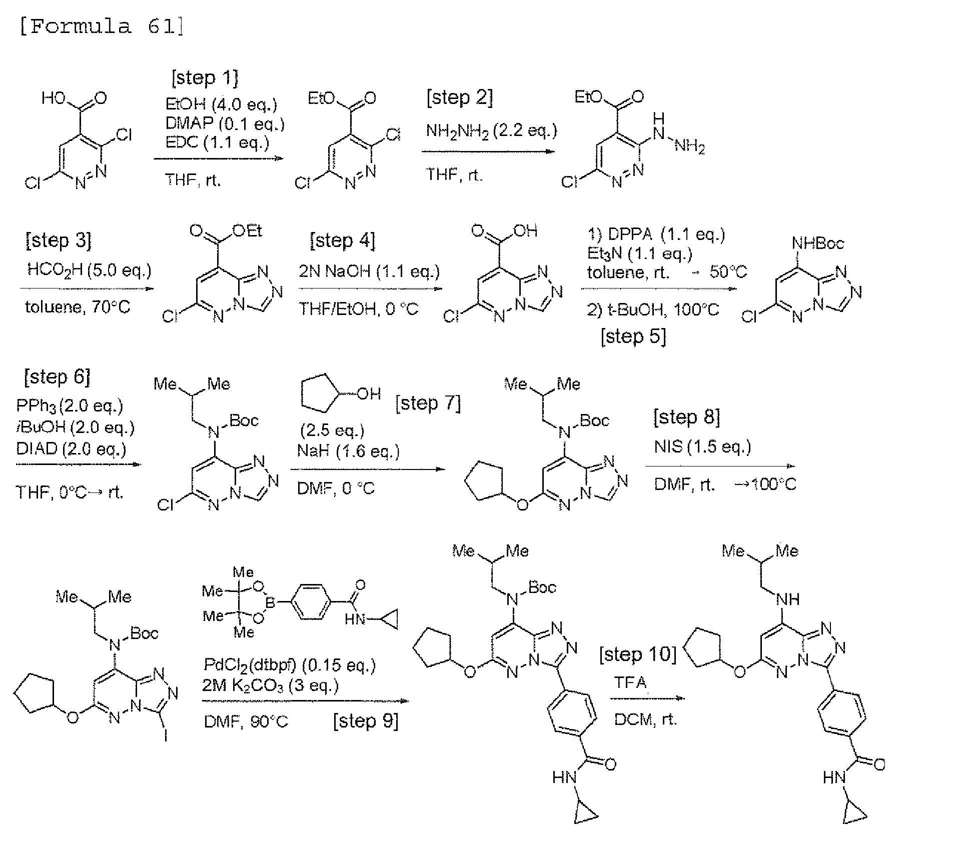

### Step 1: Ethyl 3,6-dichloropyridazine-4-carboxylate

To a THF (10 mL) solution of 3,6-dichloropyridazine-4-carboxylic acid (1.00 g, 5.18 mmol), EDC (1.09 g, 5.70 mmol) and DMAP (63 mg, 0.518 mmol), ethanol (1.21 mL, 20.7 mmol) was added and stirred at room temperature for one hour. To the reaction solution, water and ethyl acetate were added to separate phases. The water phase was extracted with ethyl acetate and organic phases were combined, washed with saturated sodium bicarbonate water and saturated saline, and dried over magnesium sulfate. The organic phase was filtrated and then concentrated under reduced pressure. The resultant residue was purified by medium-pressure silica gel chromatography (hexane/ethyl acetate: 0-50% ethyl acetate gradient) to obtain the titled compound (1.14 g, 5.14 mmol, 99%) as colorless oil.
1H-NMR (400 MHz, CDCl₃) δ 1.44 (t, J = 7.1 Hz, 3H), 4.48 (q, J = 7.1 Hz, 2H), 7.85 (s, 1H).
MS (ESI) m/z = 221 (M+H)⁺.
LC/MS t_{R} = 1.68 min.

### Step 2: Ethyl 6-chloro-3-hydrazinyl pyridazine-4-carboxylate

To a THF (1.0 mL) solution of ethyl 3,6-dichloropyridazine-4-carboxylate (568 mg, 2.57 mmol), a hydrazine hydrate (0.275 mL, 5.65 mmol) was added and stirred at room temperature for one hour. To the reaction solution, water and ethyl acetate were added to separate phases. The water phase was extracted with ethyl acetate. Organic phases were combined, washed with saturated sodium bicarbonate water and saturated saline, and dried over magnesium sulfate. The organic phase was filtrated and then concentrated under reduced pressure. The resultant residue was purified by medium-pressure silica gel chromatography (hexane/ethyl acetate: 0-100% ethyl acetate gradient) to obtain the titled compound (197 mg, 0.908 mmol, 35%) as an orange solid substance.
1H-NMR (400 MHz, CDCl₃) δ 1.41 (t, J = 7.1 Hz, 3H), 4.20 (s, 2H), 4.41 (q, J = 7.1 Hz, 2H), 7.75 (s, 1H), 8.44 (s, 1H).
MS (ESI) m/z = 217 (M+H)⁺.
LC/MS t_{R} = 0.84 min.

### Step 3: Ethyl 6-chloro-[1,2,4]triazolo[4,3-b]pyridazine-8-carboxylate

To a toluene (20 mL) solution of ethyl 6-chloro-3-hydrazinylpyridazine-4-carboxylate (1.99 g, 9.19 mmol), formic acid (1.76 mL, 45.9 mmol) was added and stirred at 70°C for 2 hours. The reaction solution was concentrated under reduced pressure to obtain the titled compound (2.10 g, 9.25 mmol, 100%) as a skin-color solid substance.
1H-NMR (400 MHz, CDCl₃) δ 1.38 (t, J = 7.1 Hz, 3H), 4.47 (q, J = 7.1 Hz, 2H), 7.89 (s, 1H), 9.79 (s, 1H).
MS (ESI) m/z = 227 (M+H)⁺.
LC/MS t_{R} = 1.06 main.

### Step 4: 6-Chloro-[1,2,4]triazolo[4,3-b]pyridazine-8-carboxylic acid.

To a THF (60 mL)/ethanol (20 mL) solution of ethyl 6-chloro-[1,2,4]triazolo[4,3-b]pyridazine-8-carboxylate (2.08 g, 9.18 mmol), a 2 mol/L aqueous sodium hydroxide solution (4.82 mL, 9.64 mmol) was added at 0°C and stirred at the same temperature for 30 minutes. To the reaction solution, a 2 mol/L aqueous hydrochloric acid solution (5.0 mL) was added to neutralize and concentrated under reduced pressure. To the resultant residue, water was added and the resultant solid substance was obtained by filtration, washed with water and THF to obtain the titled compound (1.75 g, 8.80 mmol, 96%) as a white solid substance.
1H-NMR (400 MHz, DMSO-d₆) δ 7.80 (s, 1H), 9.76 (s, 1H).
LC/MS t_{R} = 0.58 min.

### Step 5: tert-Butyl 6-chloro-[1,2,4]triazolo[4,3-blpyridazin-8-ylcarbamate

To a toluene (1.0 mL) solution of 6-chloro-[1,2,4]triazolo[4,3-b]pyridazine-8-carboxylic acid (50 mg, 0.252 mmol) and triethylamine (42 µL, 0.302 mmol), DPPA (65 µL, 0.302 mmol) was added, stirred at 50°C for 2 hours and cooled to room temperature. To this, t-butanol (0.5 mL) was added and further stirred at 100°C for 2 hours. To the reaction solution, water and ethyl acetate were added to separate phases. Thereafter, the water phase was extracted with ethyl acetate and organic phases were combined, washed with saturated sodium bicarbonate water and saturated saline, and dried over magnesium sulfate. The organic phase was filtrated and then concentrated under reduced pressure. The resultant residue was purified by medium-pressure silica gel chromatography (hexane/ethyl acetate: 0-100% ethyl acetate gradient) to obtain the titled compound (26.2 mg, 0.097 mmol, 39%) as a white solid substance.
1H-NMR (400 MHz, DMSO-d₆) δ 1.52 (s, 9H), 7.62 (s, 1H), 9.60 (s, 1H), 10.91 (s, 1H).
MS (ESI) m/z = 270 (M+H)⁺.
LC/MS t_{R} = 1.85 min.

### Step 6: tert-Butyl 6-chloro-[1,2,4]triazolo[4,3-b] pyridazin-8-yl(isobutyl)carbamate

To a THF (2.0 mL) solution of tert-butyl 6-chloro-[1,2,4]triazolo[4,3-b]pyridazin-8-ylcarbamate (72.3 mg, 0.268 mmol), triphenylphosphine (141 mg, 0.536 mmol) and isobutyl alcohol (39.7 mg, 0.536 mmol), diisopropyl azodicarboxylate (DIAD) (0.282 mL, 0.536 mmol) was added at 0°C and stirred at room temperature for 2 hours. The reaction solution was concentrated under reduced pressure and the resultant residue was purified by medium-pressure silica gel chromatography (hexane/ethyl acetate: 0-25% ethyl acetate gradient) to obtain the titled compound (98.9 mg, 0.304 mmol, >100%) as colorless oil.
1H-NMR (400MHz, CDCl₃) δ 0.88 (d, J = 6.6 Hz, 6H), 1.54 (s, 9H), 1.77-1.84 (m, 1H), 4.35 (d, J = 7.6 Hz, 2H), 7.37 (s, 1H), 9.00 (s, 1H).
MS (ESI) m/z = 326 (M+H)⁺.
LC/MS t_{R} = 2.23 min.

### Step 7: tert-Butyl 6-(cyclopentyloxy)-[1,2,4]triazolo[4,3-b]pyridazin-8-yl(isobutyl)carbamate

To a DMF (3.0 mL) solution of cyclopentyl alcohol (55 mg, 0.623 mmol), a 60% sodium hydride (17 mg, 0.411 mmol) was added at 0°C and stirred for 10 minutes. To this, tert-butyl 6-chloro-[1,2,4]triazolo[4,3-b]pyridazin-8-yl(isobutyl)carbamate (95 mg, 0.257 mmol, calculated assuring that purity was 88%) was added at the same temperature (0°C). The mixture was further stirred at 0°C for 2 hours. To the reaction solution, water and ethyl acetate were added to separate phases. Thereafter, the water phase was extracted with ethyl acetate and organic phases were combined, washed with water and saturated saline, and dried over magnesium sulfate. The reaction solution was concentrated under reduced pressure and the resultant residue was purified by medium-pressure silica gel chromatography (hexane/ethyl acetate: 0-25% ethyl acetate gradient) to obtain the titled compound (59.3 mg, 0.158 mmol, 62%) as colorless oil.
1H-NMR (400 MHz, CDCl₃) δ 0.88 (d, J = 5.6 Hz, 6H), 1.50 (s, 9H), 1.59-2.07 (m, 9H), 4.14 (d, J = 5.6 Hz, 2H), 6.82 (s, 1H), 8.83 (s, 1H).
MS (ESI) m/z = 376 (M+H)⁺.
LC/MS t_{R} = 2.68 min.

### Step 8: tert-Butyl 6-(cyclopentyloxy)-3-iodo-[1,2,4]triazolo[4,3-b]pyridazin-8-yl(isobutyl)carbamate

To a DMF (1.0 mL) solution of tert-butyl 6-(cyclopentyloxy)-[1,2,4]triazolo[4,3-b]pyridazin-8-yl(isobutyl)carbamate (57 mg, 0.152 mol), NIS (51 mg, 0.228 mmol) was added and stirred at 90°C for 2 hours. To the reaction solution, a 10% aqueous NaHSO₃ solution and ethyl acetate were added and separate phases. Thereafter, the water phase was extracted with ethyl acetate and organic phases were combined and washed with a 10% aqueous potassium carbonate solution and saturated saline, and dried over magnesium sulfate. The organic phase was filtrated and then concentrated under reduced pressure. The resultant residue was purified by medium-pressure silica gel chromatography (hexane/ethyl acetate: 0-25% ethyl acetate gradient) to obtain the titled compound (25 mg, 0.050 mmol, 33%) as a white amorphous substance.
MS (ESI) m/z = 502 (M+H)⁺.
LC/MS t_{R} = 2.68 min.

### Step 9: tert-Butyl 6-(cyclopentyloxy)-3-(4-(cyclopropylcarbamoyl)phenyl)-[1,2,4]triazolo[4,3-b]pyridazin-8-yl(isobutyl)carbamate

To a DMF/water (1.0 mL/0.1 mL) solution of tert-butyl 6-(cyclopentyloxy)-3-iodo-[1,2,4]triazolo[4,3-b]pyridazin-8-yl(isobutyl)carbamate (38 mg, 0.067 mmol), 4-(cyclopropylcarbamoyl)phenylboronic acid pinacol ester (29 mg, 0.101 mmol) and potassium carbonate (28 mg, 0.202 mmol), PdCl₂(dtbpf) (6.6 mg, 10.1 umol) was added and stirred at 90°C for 2 hours. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate; 0-100% ethyl acetate gradient) to obtain the titled compound (22 mg, 0.041 mmol, 61%) as a white amorphous substance.
1H-NMR (400 MHz, CDCl₃) δ 0.63-0.70 (m, 2H), 0.87-0.93 (m, 8H), 1.51 (s, 9H), 1.66-2.11 (m, 9H), 2.91-2.99 (m, 1H), 4.17 (d, J = 7.6 Hz, 2H), 5.34-5.40 (m, 1H), 6.30 (s, 1H), 6.87 (s, 1H), 7.91 (d, J = 8.1 Hz, 2H), 8.59 (d, J = 8.1 Hz, 2H).
MS (ESI) m/z = 535 (M+H)⁺.
LC/MS t_{R} = 2.77 min.

### Step 10: Titled compound

To a dichloromethane (1.0 mL) solution of tert-butyl 6-(cyclopentyloxy)-3-(4-(cyclopropylcarbamoyl)phenyl)-[1,2,4]triazolo[4,3-b]pyridazin-8-yl(isobutyl)carbamate (20 mg, 0.037 mmol), TFA (0.5 mL) was added and stirred at room temperature for one hour. To the reaction solution, a 2 mol/L aqueous potassium carbonate solution was added to neutralize and thereafter, ethyl acetate was added to separate phases. Thereafter, the water phase was extracted with ethyl acetate and organic phases were combined and washed with a 10% aqueous potassium carbonate solution and saturated saline, and dried over magnesium sulfate. The organic phase was filtrated and then concentrated under reduced pressure to obtain the titled compound (9.2 mg, 0.021 mmol, 57%) as a white solid substance.
1H-NMR (400 MHz, DMSO-d6) δ 0.58-0.63 (m, 2H), 0.70-0.74 (m, 2H), 0.93 (d, J = 6.6 Hz, 6H), 1.58-1.68 (m, 2H), 1.68-1.78 (m, 2H), 1.80-1.90 (m, 2H), 1.96-2.09 (m, 3H), 2.86-2.93 (m, 1H), 3.11-3.18 (m, 2H), 5.30-5.35 (m, 1H), 5.76 (d, J = 3.0 Hz, 1H), 8.02 (d, J = 8.6 Hz, 3H), 8.52 (d, J = 8.6 Hz, 2H), 8.56 (d, J = 4.1 Hz, 1H).
MS (ESI) m/z = 435 (M+H)⁺.
LC/MS t_{R} = 2.52 min.

### Example 3-2

4-(6-(Cyclopentyloxy)-8-((tetrahydro-2H-pyran-4-yl)methylamino)-[1,2,4]triazolo[4,3-b]pyridazin-3-yl)-N-cyclopropylbenzamide

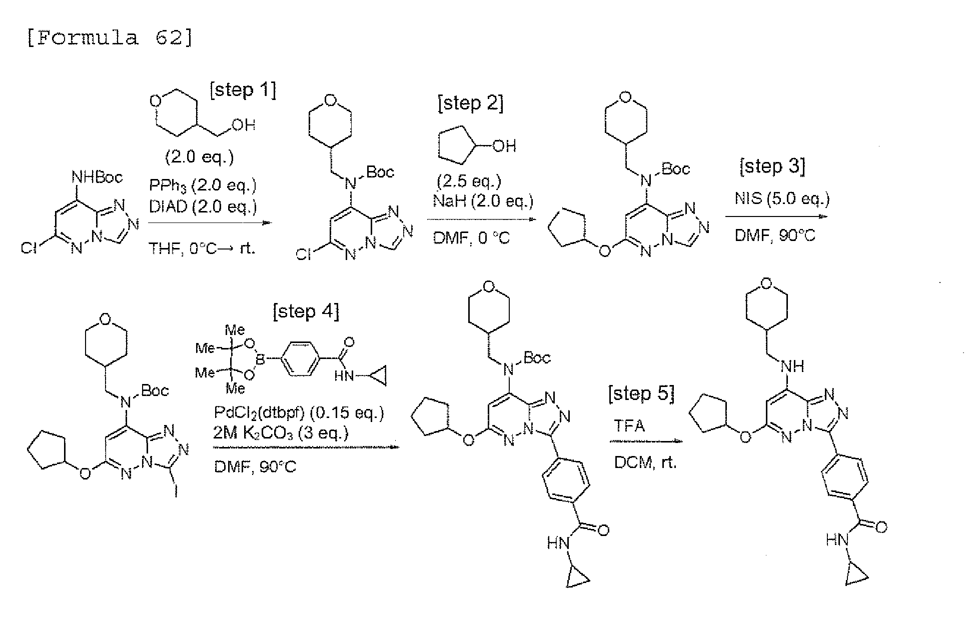

### Step 1: tert-Butyl 6-chloro-[1,2,4]triazolo[4,3-b]pyridazin-8-yl((tetrahydro-2H-pyran-4-yl)methyl)carbamate

The titled compound was synthesized in accordance with the process of Example 3-1 (Step 6) (312 mg, 0.848 mmol, 76%; colorless oil).
1H-NMR (400 MHz, CDCl₃) δ 1.24-1.39 (m, 2H), 1.53-1.59 (m, 11H), 1.70-1.83 (m, 1H), 3.27 (t, J = 11.7 Hz, 2H), 3.89-3.95 (m, 2H), 4.45 (d, J = 7.1 Hz, 2H), 7.37 (s, 1H), 9.01 (s, 1H). MS (ESI) m/z = 368 (M+H)⁺.
LC/MS t_{R} = 1.87 min.

### Step 2: tert-Butyl 6-(cyclopentyloxy)-[1,2,4]triazolo[4,3-b]pyridazin-8-yl((tetrahydro-2H-pyran-4-yl)methyl)carbamate

The titled compound was synthesized in accordance with the process of Example 3-1 (Step 7) (312 mg, 0.848 mmol, 76%; colorless oil).
1H-NMR (400 MHz, CDCl₃) δ 1.26-1.36 (m, 2H), 1.50 (s, 9H), 1.60-2.05 (m, 11H), 3.28 (td, J = 11.7, 1.9 Hz, 2H), 3.92 (dd, J = 11.4, 2.8 Hz, 2H), 4.24 (d, J = 7.1 Hz, 2H), 5.30-5.36 (m, 1H), 6.80 (s, 1H), 8.84 (s, 1H).
MS (ESI) m/z = 418 (M+H)⁺.
LC/MS t_{R} = 2.32 min.

### Step 3: tert-Butyl 6-(cyclopentyloxy)-3-iodo-[1,2,4]triazolo[4,3-b]pyridazin-8-yl((tetrahydro-2H-pyran-4-yl)methyl)carbamate

The titled compound was synthesized in accordance with the process of Example 3-1 (Step 8) (245 mg, 0.451 mmol, 108%; colorless oil).
1H-NMR (400 MHz, CDCl₃) δ 1.24-1.36 (m, 2H), 1.49 (s, 9H), 1.55-2.14 (m, 11H), 3.27 (td, J = 11.8, 1.9 Hz, 2H), 3.91 (dd, J = 11.9, 2.8 Hz, 2H), 4.20 (d, J = 7.6 Hz, 2H), 5.39-5.44 (m, 1H), 6.86 (s, 1H).
MS (ESI) m/z = 544 (M+H)⁺.
LC/MS t_{R} = 2.62 min.

### Step 4: tert-Butyl 6-(cyclopentyloxy)-3-(4-(cyclopropylcarbamoyl)phenyl)-[1,2,4]triazolo[4,3-b]pyridazin-8-yl((tetrahydro-2H-pyran-4-yl)methyl)carbamate

The titled compound was synthesized in accordance with the process of Example 3-1 (Step 9) (172 mg, 0.299 mmol, 71%; white amorphous substance).
1H-NMR (400 MHz, CDCl₃) δ 0.63 (dt, J = 14.4, 6.2 Hz, 2H), 0.68-0.75 (m, 2H), 1.11-1.24 (m, 2H), 1.38 (s, 9H), 1.53-1.81 (m, 7H), 1.87-1.97 (m, 2H), 2.04-2.11 (m, 2H), 2.86-2.92 (m, 1H), 3.17 (t, J = 10.9 Hz, 2H), 3.78 (d, J = 9.1 Hz, 2H), 3.92 (d, J = 7.6 Hz, 2H), 5.37-5.42 (m, 1H), 7.14 (s, 1H), 8.05 (d, J = 8.5 Hz, 2H), 8.50 (d, J = 8.6 Hz, 2H), 8.58 (d, J = 4.1 Hz, 1H).
MS (ESI) m/z = 577 (M+H)⁺.
LC/MS t_{R} = 2.45 min.

### Step 5: Titled compound

The titled compound was synthesized in accordance with the process of Example 3-1 (Step 10) (131 mg, 0.274 mmol, 93%; white solid substance).
1H-NMR (400 MHz, DMSO-d6) δ 0.57-0.63 (m, 2H), 0.70-0.74 (m, 2H), 1.18-1.30 (m, 2H), 1.58-2.09 (m, 11H), 2.85-2.93 (m, 1H), 3.17-3.30 (m, 4H), 3.85 (d, J = 9.1 Hz, 2H), 5.31-5.34 (m, 1H), 5.83 (s, 1H), 7.99-8.07 (m, 3H), 8.52 (d, J = 8.6 Hz, 2H), 8.56 (d, J = 4.1 Hz, 1H).
MS (ESI) m/z = 477 (M+H)⁺.
LC/MS t_{R} = 2.16 min.

### Example 3-3

4-(6-(Cyclopentylamino)-8-((tetrahydro-2H-pyran-4-yl)methylamino)-[1,2,4]triazolo[4,3-b]pyridazin-3-yl)-N-cyclopropylbenzamide

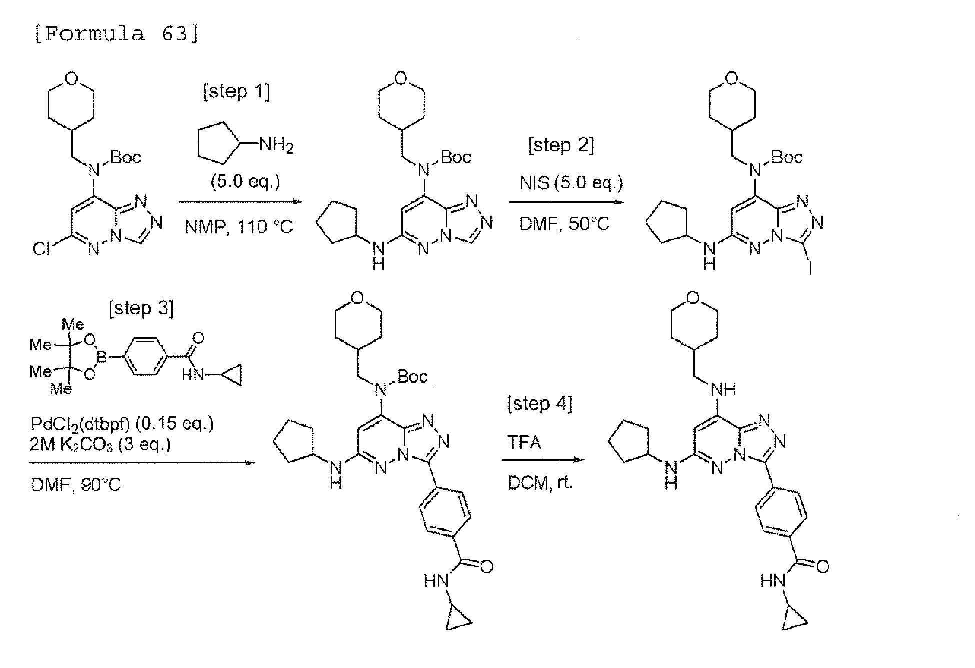

### Step 1: tert-Butyl 6-(cyclopentylamino)-[1,2,4]triazolo[4,3-b]pyridazin-8-yl((tetrahydro-2H-pyran-4-yl)methyl)carbamate

To an NMP (5.0 mL) solution of tert-butyl 6-chloro-[1,2,4]triazolo[4,3-b]pyridazin-8-yl((tetrahydro-2H-pyran-4-yl)methyl)carbamate (795 mg, 2.16 mmol), cyclopentylamine (0.639 mL, 6.48 mmol) was added and stirred at 110°C for 2 hours. To the reaction solution, water and ethyl acetate were added to separate phases. The water phase was extracted with ethyl acetate and organic phases were combined, washed with saturated sodium bicarbonate water and saturated saline, and dried over magnesium sulfate. The organic phase was filtrated and then concentrated under reduced pressure. The resultant residue was purified by medium-pressure silica gel chromatography (hexane/ethyl acetate: 0-100% ethyl acetate gradient) to obtain the titled compound (531 mg, 1.28 mmol, 59%) as a white amorphous substance.
1H-NMR (400 MHz, CDCl₃) δ 1.26-1.37 (m, 2H), 1.43-1.80 (m, 18H), 2.06-2.16 (m, 2H), 3.28 (t, J = 10.9 Hz, 2H), 3.88-3.95 (m, 2H), 4.09-4.18 (m, 1H), 4.24 (d, J = 7.1 Hz, 2H), 4.42 (d, J = 6.6 Hz, 1H), 6.60 (s, 1H), 8.75 (s, 1H).
MS (ESI) m/z = 417 (M+H)⁺.
LC/MS t_{R} = 1.94 min.

### Step 2: tert-Butyl 6-(cyclopentylamino)-3-iodo-[1,2,4]triazolo[4,3-b]pyridazin-8-yl((tetrahydro-2H-pyran-4-yl)methyl)carbamate

The titled compound was synthesized in accordance with the process of Example 3-1 (Step 8) (110 mg, 0.203 mmol, 85%; white amorphous substance).
1H-NMR (400 MHz, CDCl₃) δ 1.28-1.35 (m, 2H), 1.47-1.78 (m, 18H), 2.11-2.21 (m, 2H), 3.27 (t, J = 11.7 Hz, 2H), 3.88-3.95 (m, 2H), 4.09-4.15 (m, 1H), 4.15-4.23 (m, 2H), 4.68 (d, J = 6.1 Hz, 1H), 6.68 (s, 1H).
MS (ESI) m/z = 543 (M+H)⁺.
LC/MS t_{R} = 2.30 min.

### Step 3: tert-Butyl 6-(cyclopentylamino)-3-(4-(cyclopropylcarbamoyl)phenyl)-[1,2,4]triazolo[4,3-b]pyridazin-8-yl((tetrahydro-2H-pyran-4-yl)methyl)carbamate

The titled compound was synthesized in accordance with the process of Example 3-1 (Step 9) (90 mg, 0.157 mmol, 81%; white amorphous substance).
1H-NMR (400 MHz, CDCl₃) δ 0.63-0.69 (m, 2H), 0.87-0.94 (m, 2H), 1.26-1.37 (m, 2H), 1.49-1.82 (m, 18H), 2.08-2.20 (m, 2H), 2.91-2.99 (m, 1H), 3.28 (t, J = 11.4 Hz, 2H), 3.92 (d, J = 9.6 Hz, 2H), 4.14-4.28 (m, 3H), 4.64 (d, J = 5.6 Hz, 1H), 5.30 (s, 1H), 6.34 (s, 1H), 6.66 (s, 1H), 7.89 (d, J = 8.1 Hz, 2H), 8.65 (d, J = 8.1 Hz, 2H).
MS (ESI) m/z = 576 (M+H)⁺.
LC/MS t_{R} = 2.13 min.

### Step 4: Titled compound

The titled compound was synthesized in accordance with the process of Example 3-1 (Step 10) (131 mg, 0.274 mmol, 93%; white amorphous substance).
1H-NMR (400 MHz, DMSO-d₆) δ 0.58-0.63 (m, 2H), 0.69-0.74 (m, 2H), 1.19-1.28 (m, 2H), 1.51-1.76 (m, 9H), 1.93-2.04 (m, 3H), 2.85-2.92 (m, 1H), 3.11 (t, J = 6.3 Hz, 2H), 3.27 (d, J = 11.7 Hz, 2H), 3.83-3.90 (m, 2H), 4.02-4.11 (m, 1H), 5.64 (s, 1H), 6.76 (d, J = 6.1 Hz, 1H), 7.51 (t, J = 5.8 Hz, 1H), 7.98 (d, J = 8.6 Hz, 2H), 8.51 (d, J = 4.6 Hz, 1H), 8.60 (d, J = 8.6 Hz, 2H).
MS (ESI) m/z = 476 (M+H)⁺.
LC/MS t_{R} = 1.93 min.

### Example 3-4

4-(5-(cyclopentylamino)-7-((tetrahydro-2H-pyran-4-yl)methylamino)isoxazolo[4,5-d]pyrimidin-3-yl)-N-cyclopropylbenzamide

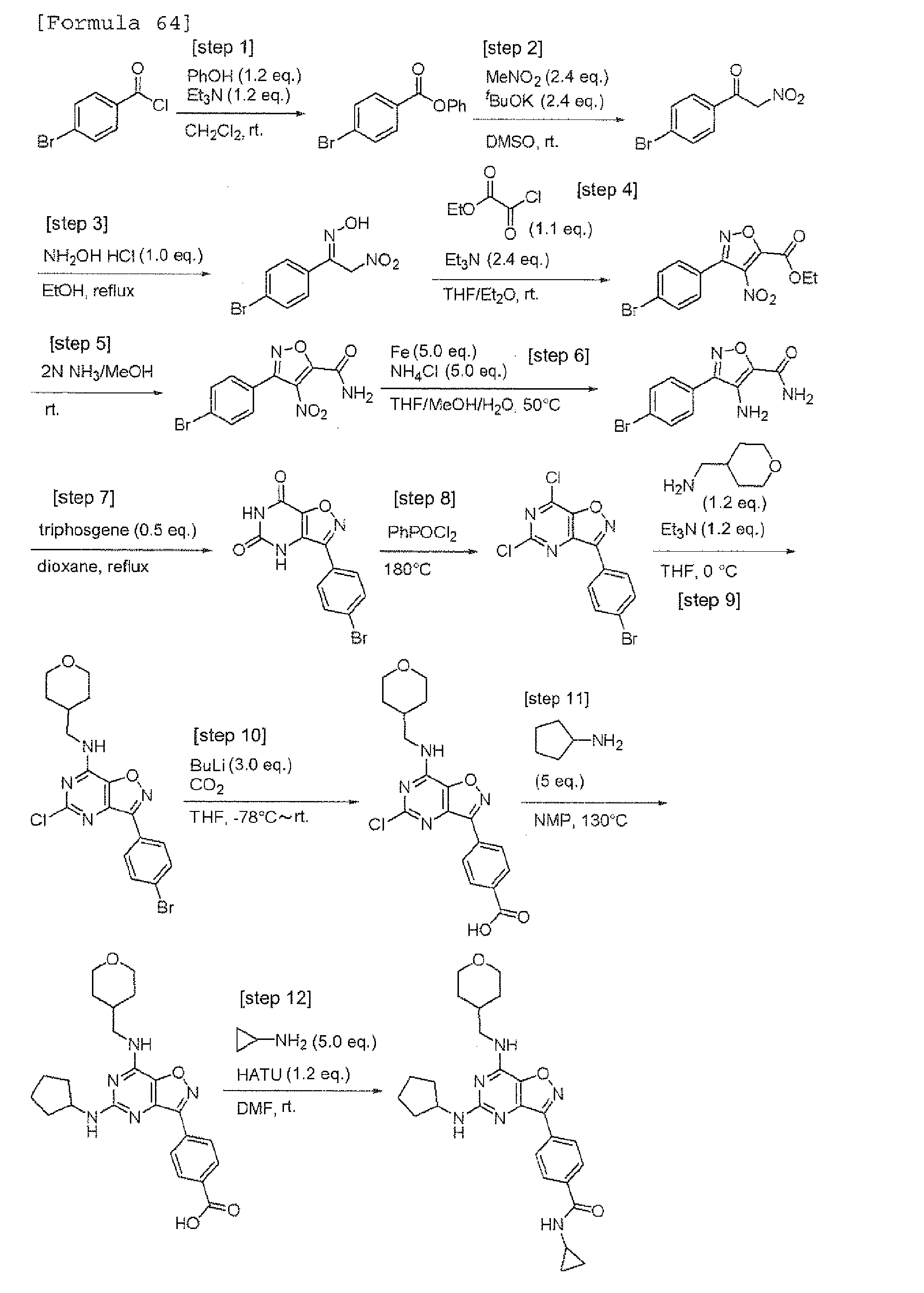

### Step 1: Phenyl 4-bromobenzoate

To a dichloromethane (250 mL) solution of 4-bromobenzoyl chloride (25 g, 114 mmol) and phenol (12.9 g, 137 mmol), triethylamine (19 mL, 137 mmol) was added at 0°C and stirred at room temperature for 2 hours. To the reaction solution, a 2 mol/L aqueous hydrochloric acid solution was added to separate phases. The water phase was extracted with dichloromethane and organic phases were combined, washed with saturated sodium bicarbonate water and saturated saline and dried over magnesium sulfate. The organic phase was filtrated and then concentrated under reduced pressure to obtain the titled compound (29.2 g, 92%) as a white solid substance. 1H-NMR (300 MHz, CDCl₃) δ 7.18-7.24 (m, 2H), 7.24-7.32 (m, 1H), 7.40-7.48 (m, 2H), 7.66 (dt, J = 8.9, 2.1 Hz, 2H), 8.06 (dt, J = 8.9, 2.1 Hz, 2H).

### Step 2: 1-(4-Bromophenyl)-2-nitroethanone

To a DMSO (290 mL) solution of potassium-t-butoxide (35.2 g, 314 mmol), nitromethane (17.0 mL, 314 mmol) was added at room temperature and stirred for one hour and then phenyl 4-bromobenzoate (25 g, 114 mmol) was added. The reaction solution was further stirred at room temperature for 2 hours. The reaction solution was poured to ice water and a 2 mol/L aqueous hydrochloric acid solution was added to neutralize. The resultant solid substance was filtrated to obtain the titled compound (36.2 g, 142%; including water) as a white solid substance.
1H-NMR (300 MHz, DMSO-d6) δ 6.51 (s, 2H), 7.79-7.88 (m, 4H).

### Step 3: 1-(4-Bromophenyl)-2-nitroethanone oxime

To an ethanol (240 mL) solution of 1-(4-bromophenyl)-2-nitroethanone (25.5 g, 104 mmol), hydroxylamine hydrochloride (7.26 g, 104 mmol) was added at room temperature and stirred for 4 hours while heating under reflux. The reaction solution was concentrated under reduced pressure and then saturated sodium bicarbonate water and dichloromethane were added to separate phases. The water phase was extracted with dichloromethane and organic phases were combined, washed with saturated sodium bicarbonate water and saturated saline, and dried over magnesium sulfate. The organic phase was filtrated and then concentrated under reduced pressure to obtain the titled compound (24.2 g, 89%) as a light yellow substance. 1H-NMR (300 MHz, DMSO-d6) δ 5.82 (s, 2H), 7.61-7.72 (m, 4H), 12.40 (s, 1H).

### Step 4: Ethyl 3-(4-bromophenyl)-4-nitroisoxazole-5-carboxylate

To a THF (240 mL) solution of 1-(4-bromophenyl)-2-nitroethanone oxime (24 g, 93 mmol) and DIEA (38.8 mL, 222 mmol), ethyl 2-chloro-2-oxoacetate (11.4 mL, 102 mmol) was added at 0°C and stirred at the same temperature for 2 hours. To the reaction solution, saturated sodium bicarbonate water and ethyl acetate were added to separate phases. The water phase was extracted with ethyl acetate and organic phases were combined, washed with saturated sodium bicarbonate water and saturated saline, and dried over magnesium sulfate. The organic phase was filtrated and then concentrated under reduced pressure. The resultant residue was purified by medium-pressure silica gel chromatography (hexane/ethyl acetate: 0-25% ethyl acetate gradient) to obtain the titled compound (8.99 g, 28%) as yellow oil.
1H-NMR (300 MHz, CDCl₃) δ 1.44 (t, J = 7.1 Hz, 3H), 4.53 (q, J = 7.1 Hz, 2H), 7.53 (dt, J = 8.9, 2.1 Hz, 2H), 7.66 (dt, J = 8.9, 2.1 Hz, 2H).

### Step 5: 3-(4-Bromophenyl)-4-nitroisoxazole-5-carboxamide

To ethyl 3-(4-bromophenyl)-4-nitroisoxazole-5-carboxylate (5.54 g, 16.2 mmol), a 2 mol/l ammonia methanol solution (25 mL) was added at room temperature and stirred for one hour. The reaction solution was concentrated under reduced pressure to obtain the titled compound (4.61 g, 91%) as a light yellow substance.
1H-NMR (300 MHz, DMSO-d6) δ 7.65 (d, J = 8.6 Hz, 2H), 7.79 (d, J = 8.6 Hz, 2H), 8.63 (s, 1H), 8.77 (s, 1H).

### Step 6: 4-Amino-3-(4-bromophenyl)isoxazole-5-carboxamide

To a THF (3 mL)/methanol (3 mL)/water (1 mL) solution of 3-(4-bromophenyl)-4-nitroisoxazole-5-carboxamide (320 mg, 1.03 mmol) and ammonium chloride (274 mg, 5.13 mmol), iron powder (286 mg, 5.13 mmol) was added and stirred at 50°C for 2 hours. The reaction solution was filtrated by Celite to remove insoluble matter and water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (hexane/ethyl acetate: 0-70% ethyl acetate gradient) to obtain the titled compound (56.2 mg, 19%) as a light orange solid substance.
1H-NMR (300 MHz, DMSO-d6) δ 5.27 (s, 1H), 7.69-7.78 (m, 4H), 7.95 (br s, 2H).

### Step 7: 3-(4-Bromophenyl)isoxazolo[4,5-d]pyrimidine-5,7(4H, 6H)-dione

To a dioxane (15 mL) solution of 4-amino,-3-(4-bromophenyl)isoxazole-5-carboxamide (1.50 g, 5.32 mmol), triphosgene (0.789 g, 2.66 mmol) was added at room temperature and stirred for one hour while heating under reflux. The reaction solution was concentrated under reduced pressure and then diluted with hexane, the resultant solid substance was filtrated to obtain the titled compound (1.55 g, 95%) as a light orange solid substance.
1H-NMR (300 MHz, DMSO-d6) δ 7.80 (br s, 4H), 11.66 (s, 1H), 11.75 (s, 1H).

### Step 8: 3-(4-Bromophenyl)-5,7-dichloroisoxazolo[4,5-d] pyrimidine

3-(4-Bromophenyl)isoxazolo[4,5-d] pyrimidine-5,7(4H, 6H)-dione (1.50 g, 4.87 mmol) and phenylphosphonic dichloride (15 mL, 106 mmol) were stirred at 180°C for 2 hours. The reaction solution was purified by medium-pressure silica gel chromatography (hexane/ethyl acetate: 0-20% ethyl acetate gradient) to obtain the titled compound (1.46 g, 87%) as a light yellow substance.
1H-NMR (300 MHz, DMSO-d6) δ 7.96 (d, J = 8.4 Hz, 1H), 8.28 (d, J = 8.4 Hz, 1H).

### Step 9: 3-(4-Bromophenyl)-5-chloro-N-((tetrahydro-2H-pyran-4-yl)methyl)isoxazolo[4,5-d]pyrimidine-7-amine

To a THF (15 mL) solution of 3-(4-bromophenyl)-5,7-dichloroisoxazolo[4,5-d]pyrimidine (1.46 g, 4.23 mmol) and triethylamine (0.704 mL, 5.08 mmol), (tetrahydro-2H-pyran-4-yl)methaneamine (585 mg, 5.08 mmol) was added at 0°C and stirred at the same temperature for 30 minutes. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure to obtain the titled compound (1.82 g, 100%) as a light orange solid substance.
1H-NMR (300 MHz, DMSO-d6) δ 1.16-1.36 (m, 2H), 1.62-1.72 (m, 2H), 3.23-3.36 (m, 2H), 3.43 (d, J = 6.7 Hz, 2H), 3.83-3.93 (m, 2H), 7.88 (d, J = 8.6 Hz, 2H), 8.24 (d, J = 8.6 Hz, 2H), 9.30 (s, 1H).

### Step 10: 4-(5-Chloro-7-((tetrahydro-2H-pyran-4-yl)methylamino)isoxazolo[4,5-d]pyrimidin-3-yl)benzoic acid

To a THF (5 mL) solution of 3-(4-bromophenyl)-5-chloro-N-((tetrahydro-2H-pyran-4-yl)methyl)isoxazolo[4,5-d]pyrimidine-7-amine (500 mg, 1.18 mmol), 2.76 mol/L n-butyl lithium/hexane solution (1.28 mL) was added at -78°C and stirred at the same temperature for one hour. To the reaction solution, dry ice was added at -78°C and further stirred for one hour. To the reaction solution, water and ethyl acetate were added to separate phases. Thereafter, the water phase was diluted with a 2 mol/L aqueous hydrochloric acid solution and extracted with ethyl acetate. Organic phases were combined, washed with water and saturated saline, and dried over magnesium sulfate. The organic phase was filtrated and then concentrated under reduced pressure to obtain the titled compound (120 mg, 26%) as an orange solid substance.
1H-NMR (300 MHz, DMSO-d6) δ 1.17-1.34 (m, 2H), 1.61-1.71 (m, 2H), 3.25-3.43 (m, 4H), 3.81-3.90 (m, 2H), 8.17 (d, J = 8.6 Hz, 2H), 8.39 (d, J = 8.6 Hz, 2H), 9.30 (s, 1H).

### Step 11: 4-(5-(Cyclopentylamino)-7-((tetrahydro-2H-pyran-4-yl)methylamino)isoxazolo[4,5-d]pyrimidin-3-yl)benzoic acid

To an NMP (1 mL) solution of 4-(5-chloro-7-((tetrahydro-2H-pyran-4-yl)methylamino)isoxazolo[4,5-d]pyrimidin-3-yl)benzoic acid (120 mg, 0.309 mmol), cyclopentylamine (0.152 mL, 1.543 mmol) was added and stirred at 120°C for 6 hours. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure to obtain the titled compound (45.4 mg, 34%) as a brown solid substance.
MS (ESI) m/z = 438 (M+H)⁺.

### Step 12: Titled compound

To a DMF (1 mL) solution of 4-(5-(cyclopentylamino)-7-((tetrahydro-2H-pyran-4-yl)methylamino)isoxazolo[4,5-d] pyrimidin-3-yl) benzoic acid (40 mg, 0.091 mmol) and cyclopropylamine (26.1 mg, 0.457 mmol), HATU (41.7 mg, 0.110 mmol) was added and stirred at room temperature for one hour. To the reaction solution, water and ethyl acetate were added to separate phases. The water phase was extracted with ethyl acetate and organic phases were combined, washed with saturated sodium bicarbonate water and saturated saline, and dried over magnesium sulfate. The organic phase was filtrated and then concentrated under reduced pressure. The resultant residue was purified by reverse-phase preparatory liquid chromatography (C18 column; water/acetonitrile/0.1% formic acid; 10-100% acetonitrile gradient) to obtain the titled compound (25.3 mg, 58%) as an orange solid substance.
1H-NMR (300 MHz, DMSO-d6) δ 0.55-0.64 (m, 2H), 0.66-0.76 (m, 2H), 1.13-2.03 (m, 17H), 2.83-2.93 (m, 1H), 3.15-3.41 (m, 4H), 3.78-3.92 (m, 2H), 4.12-4.24 (m, 1H), 6.73 (d, J = 6.2 Hz, 1H) 7.98 (d, J = 8.4 Hz, 2H), 8.45 (d, J = 8.4 Hz, 2H), 8.56 (d, J = 4.0 Hz, 1H).

### Example 3-5

4-(5-(cyclopentyloxy)-7-((tetrahydro-2H-pyran-4-yl)methylamino)isoxazolo[4,5-d]pyrimidin-3-yl)-N-cyclopropylbenzamide

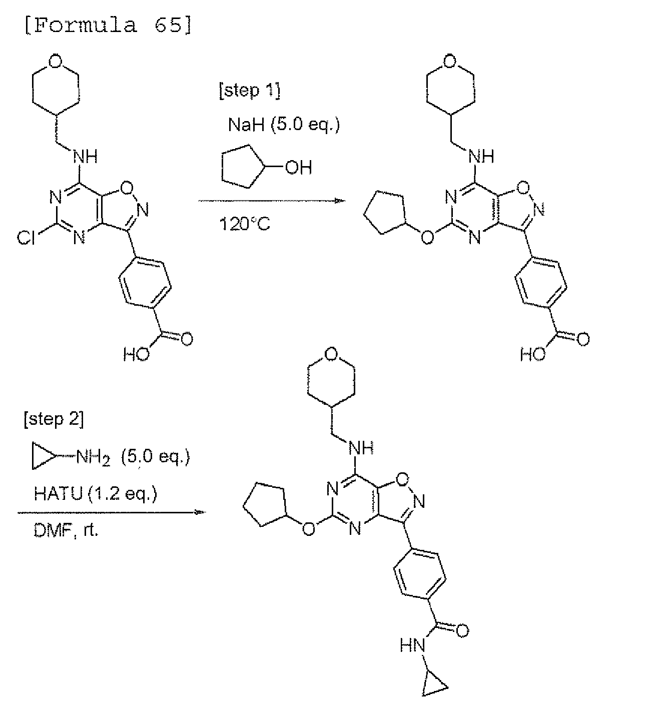

### Step 1: 4-(5-(Cyclopentyloxy)-7-((tetrahydro-2H-pyran-4-yl)methylamino)isoxazolo[4,5-d]pyrimidin-3-yl)benzoic acid

To a cyclopentanol (0.5 mL) solution of 4-(5-chloro-7-((tetrahydro-2H-pyran-4-yl)methylamino)isoxazolo[4,5-d]pyrimidin-3-yl)benzolc acid (120 mg, 0.309 mmol), a 60% sodium hydride (26 mg, 0.64 mmol) was added and stirred at 120°C for 3 hours. To the reaction solution, water and ethyl acetate were added to separate phases. Thereafter, the water phase was extracted with ethyl acetate and organic phases were combined, washed with water and saturated saline, and dried over magnesium sulfate. The organic phase was filtrated and then concentrated under reduced pressure. The resultant residue was purified by medium-pressure silica gel chromatography (chloroform /methanol: 0-10% methanol, gradient) to obtain the titled compound (17.6 mg, 31%) as a yellow solid substance.
MS (ESI) m/z = 439 (M+H)⁺.

### Step 2: Titled compound

The titled compound was synthesized in accordance with the process of Example 3-4 (Step 12) (2.2 mg, 12%; light yellow substance).
1H-NMR (300 MHz, DMSO-d6) δ 0.56-0.63 (m, 1H), 0.67-0.75 (m, 2H), 1.14-1.30 (m, 2H), 1.59-2.04 (m, 11H), 2.85-2.90 (m, 1H), 3.21-3.28 (m, 2H), 3.35-3.42 (m, 2H), 3.81-3.89 (m, 2H), 5.30-5.39 (m, 1H), 8.01 (d, J = 8.6 Hz, 1H), 8.40 (d, J = 8.6 Hz, 2H), 8.58 (br s, 1H), 8.76 (br s, 1H).
MS (ESI) m/z = 478 (M+H)⁺.

### (Example 4) The results of TTK assay and A549 assay

Representative results of TTK assay and A549 assay will be shown below. In the tables, IC₅₀ is represented by the unit of µM.

**[Table 3-1]**

| Example | TTK IC₅₀ (µM) | A549 IC₅₀ (µM) | Example | TTK IC₅₀ (µM) | A549 IC₅₀ (µM) |
|---|---|---|---|---|---|
| 1-1 | 0.0347 | 1.0 < IC₅₀ ≤ 10 | 1-155 | 0.0265 | 1.0 < IC₅₀ ≤ 10 |
| 1-36 | 0.0394 | 1.0 < IC₅₀ ≤ 10 | 1-156 | 0.0158 | 0.5 < IC₅₀ ≤ 1.0 |
| 1-37 | 0.0456 | 1.0 < IC₅₀ ≤ 10 | 1-157 | 0.0106 | 0.5 < IC₅₀ ≤ 1.0 |
| 1-43 | 0.0388 | 1.0 < IC₅₀ ≤ 10 | 1-158 | 0.0131 | 0.5 < IC₅₀ ≤ 1.0 |
| 1-97 | 0.0492 | 1.0 < IC₅₀ ≤ 10 | 1-159 | 0.0228 | 1.0 < IC₅₀ ≤ 10 |
| 1-98 | 0.0085 | 0.05 < IC₅₀ ≤ 0.5 | 1-160 | 0.0383 | 1.0 < IC₅₀ ≤ 10 |
| 1-101 | 0.0113 | 0.5 < IC₅₀ ≤ 1.0 | 1-161 | 0.0221 | 1.0 < IC₅₀ ≤ 10 |
| 1-103 | 0.0096 | 0.5 < IC₅₀ ≤ 1.0 | 1-162 | 0.0264 | 1.0 < IC₅₀ ≤ 10 |
| 1-105 | 0.0106 | 0.5 < IC₅₀ ≤ 1.0 | 1-163 | 0.0296 | 0.5 < IC₅₀ ≤ 1.0 |
| 1-106 | 0.0405 | 0.5 < IC₅₀ ≤ 1.0 | 1-166 | 0.0206 | 1.0 < IC₅₀ ≤ 10 |
| 1-107 | 0.0242 | 0.5 < IC₅₀ ≤ 1.0 | 1-167 | 0.0134 | 0.5 < IC₅₀ ≤ 1.0 |
| 1-108 | 0.0493 | 1.0 < IC₅₀ ≤ 10 | 1-168 | 0.0236 | 0.5 < IC₅₀ ≤ 1.0 |
| 1-110 | 0.0125 | 0.5 < IC₅₀ ≤ 1.0 | 1-169 | 0.0473 | 1.0 < IC₅₀ ≤ 10 |
| 1-111 | 0.0107 | 0.05 < IC₅₀ ≤ 0.5 | 1-170 | 0.0309 | 1.0 < IC₅₀ ≤ 10 |
| 1-114 | 0.0124 | 0.05 < IC₅₀ ≤ 0.5 | 1-175 | 0.0085 | 0.05 < IC₅₀ ≤ 0.5 |
| 1-115 | 0.0207 | 1.0 < IC₅₀ ≤ 10 | 1-177 | 0.0091 | 0.05 < IC₅₀ ≤ 0.5 |
| 1-116 | 0.0166 | 0.5 < IC₅₀ ≤ 1.0 | 1-178 | 0.0067 | 0.05 < IC₅₀ ≤ 0.5 |
| 1-117 | 0.0322 | 1.0 <IC₅₀ ≤ 10 | 1-179 | 0.0114 | 0.05 < IC₅₀ ≤ 0.5 |
| 1-118 | 0.0248 | 0.5 < IC₅₀ ≤ 1.0 | 1-212 | 0.0072 | 0.05 < IC₅₀ ≤ 0.5 |
| 1-119 | 0.0157 | 0.5 < IC₅₀ ≤ 1.0 | 1-213 | 0.0161 | 0.05 < IC₅₀ ≤ 0.5 |
| 1-120 | 0.0303 | 1.0 < IC₅₀ ≤ 10 | 1-214 | 0.0067 | 0.05 < IC₅₀ ≤ 0.5 |
| 1-121 | 0.0201 | 0.5 < IC₅₀ ≤ 1.0 | 1-216 | 0.0204 | 0.05 < IC₅₀ ≤ 0.5 |
| 1-122 | 0.0146 | 1.0 <IC₅₀ ≤ 10 | 1-217 | 0.0489 | 1.0 < IC₅₀ ≤ 10 |
| 1-123 | 0.0115 | 0.05 < IC₅₀ ≤ 0.5 | 1-218 | 0.0144 | 0.05 < IC₅₀ ≤ 0.5 |
| 1-124 | 0.0105 | 0.5 < IC₅₀ ≤ 1.0 | 1-219 | 0.0095 | 0.05 < IC₅₀ ≤ 0.5 |
| 1-125 | 0.0252 | 1.0 < IC₅₀ ≤ 10 | 1-220 | 0.0196 | 0.05 < IC₅₀ ≤ 0.5 |
| 1-127 | 0.0259 | 1.0 < IC₅₀ ≤ 10 | 1-221 | 0.0095 | 0.05 < IC₅₀ ≤ 0.5 |
| 1-138 | 0.0198 | 1.0 < IC₅₀ ≤ 10 | 1-222 | 0.0168 | 0.05 < IC₅₀ ≤ 0.5 |
| 1-139 | 0.0216 | 0.5 < IC₅₀ ≤ 1.0 | 1-224 | 0.0183 | 0.5 < IC₅₀ ≤ 1.0 |
| 1-140 | 0.0122 | 0.05 < IC₅₀ ≤ 0.5 | 1-226 | 0.0171 | 0.5 < IC₅₀ ≤ 1.0 |
| 1-141 | 0.0127 | 1.0 < IC₅₀ ≤ 10 | 1-228 | 0.0205 | 0.5 < IC₅₀ ≤ 1.0 |
| 1-143 | 0.0134 | 0.5 < IC₅₀ ≤ 1.0 | 1-230 | 0.0145 | 1.0 < IC₅₀ ≤ 10 |
| 1-144 | 0.0442 | 1.0 < IC₅₀ ≤ 10 | 1-233 | 0.0202 | 0.5 < IC₅₀ ≤ 1.0 |
| 1-147 | 0.0463 | 1.0 < IC₅₀ ≤ 10 | 1-234 | 0.0193 | 0.05 < IC₅₀ ≤ 0.5 |
| 1-148 | 0.0324 | 0.5 < IC₅₀ ≤ 1.0 | 1-235 | 0.0128 | 0.05 < IC₅₀ ≤ 0.5 |
| 1-149 | 0.0267 | 1.0 < IC₅₀ ≤ 10 | 1-236 | 0.0083 | 0.05 < IC₅₀ ≤ 0.5 |
| 1-151 | 0.0065 | 0.05 < IC₅₀ ≤ 0.5 | 1-237 | 0.0399 | 1.0 < IC₅₀ ≤ 10 |
| 1-153 | 0.0322 | 1.0 < IC₅₀ ≤ 10 | 1-238 | 0.0143 | 0.05 < IC₅₀ ≤ 0.5 |
| 1-154 | 0.0242 | 1.0 < IC₅₀ ≤ 10 | 1-239 | 0.0048 | 1.0 < IC₅₀ ≤ 10 |

**[Table 3-2]**

| Example | TTK IC₅₀ (µM) | A549 IC₅₀ (µM) | Example | TTK IC₅₀ (µM) | A549 IC₅₀ (µM) |
|---|---|---|---|---|---|
| 1-243 | 0.0283 | 0.5 < IC₅₀ ≤ 1.0 | 2-35 | 0.0193 | 1.0 < IC₅₀ ≤ 10 |
| 1-244 | 0.0134 | 0.05 < IC₅₀ ≤ 0.5 | 2-36 | 0.0442 | 1.0 < IC₅₀ ≤ 10 |
| 1-245 | 0.0041 | 0.05 < IC₅₀ 0.5 | 2-37 | 0.0027 | IC₅₀ ≤ 0.05 |
| 1-246 | 0.0064 | 0.05 < IC₅₀ ≤ 0.5 | 2-38 | 0.0045 | IC₅₀ ≤ 0.05 |
| 1-247 | 0.0294 | 1.0 < IC₅₀ ≤ 10 | 2-39 | 0.022 | 0.05 < IC₅₀ ≤ 0.5 |
| 1-248 | 0.0235 | 1.0 < IC₅₀ ≤ 10 | 2-40 | 0.0075 | 0.05 < IC₅₀ ≤ 0,5 |
| 1-249 | 0.0233 | 0.5 < IC₅₀ ≤ 1.0 | 2-41 | 0.0101 | 0.05 < IC₅₀ ≤ 0.5 |
| 1-250 | 0.0231 | 1,0 < IC₅₀ ≤ 10 | 2-42 | 0.013 | 0.05 < IC₅₀ ≤ 0.5 |
| 2-2 | 0.0118 | 0.05 < IC₅₀ ≤ 0.5 | 2-43 | 0.0125 | 0.5 < IC₅₀ ≤ 1.0 |
| 2-4 | 0.0045 | 1.0 <IC₅₀ ≤ 10 | 2-44 | 0.0061 | IC₅₀ ≤ 0.05 |
| 2-5 | 0.0042 | IC₅₀ ≤ 0.05 | 2-46 | 0.0064 | 1.0 < IC₅₀ ≤ 10 |
| 2-6 | 0.0119 | 0.05 < IC₅₀ ≤ 0.5 | 2-47 | 0.0031 | 0.05 < IC₅₀ ≤ 0.5 |
| 2-7 | 0.0091 | 0.5 < IC₅₀ ≤ 1.0 | 2-48 | 0.0139 | 1.0 < IC₅₀ ≤ 10 |
| 2-8 | 0.0182 | 0.5 < IC₅₀ ≤ 1.0 | 2-49 | 0.0178 | 0.5 < IC₅₀ ≤ 1.0 |
| 2-9 | 0.0197 | 0.5 < IC₅₀ ≤ 1.0 | 2-50 | 0.0086 | 0.05 < IC₅₀ ≤ 0.5 |
| 2-10 | 0.0082 | IC₅₀ ≤ 0.05 | 2-51 | 0.0134 | 0.05 < IC₅₀ ≤ 0.5 |
| 2-11 | 0.0053 | IC₅₀ ≤ 0.05 | 2-52 | 0.0178 | 0.05 < IC50 ≤ 0.5 |
| 2-12 | 0.0084 | 0.05 < IC₅₀ ≤ 0.5 | 2-53 | 0.0259 | 1.0 < IC₅₀ ≤ 10 |
| 2-13 | 0.0059 | 0.05 < IC₅₀ 0.5 | 2-54 | 0.0074 | 0.05 < IC₅₀ ≤ 0.5 |
| 2-14 | 0.0093 | 0.05 < IC₅₀ ≤ 0.5 | 2-55 | 0.0049 | 0.05 < IC₅₀ ≤ 0.5 |
| 2-15 | 0.0257 | 0.05 < IC₅₀ ≤ 0.5 | 2-56 | 0.0044 | 0.05 < IC₅₀ ≤ 0.5 |
| 2-16 | 0,0048 | 0.05 < IC₅₀ ≤ 0.5 | 2-57 | 0.0045 | 0.05 < IC₅₀ ≤ 0.5 |
| 2-17 | 0.0031 | IC₅₀ ≤ 0.05 | 2-58 | 0.0055 | 0.05 < IC₅₀ ≤ 0,5 |
| 2-18 | 0.0184 | 0.05 < IC₅₀ 0.5 | 2-59 | 0.0052 | 0.05 < IC₅₀ ≤ 0.5 |
| 2-19 | 0.006 | 0.05 < IC₅₀ ≤ 0.5 | 2-60 | 0.0046 | IC₅₀ ≤ 0.05 |
| 2-20 | 0.0036 | 0.05 < IC₅₀ ≤ 0.5 | 2-61 | 0.0051 | IC₅₀ ≤ 0.05 |
| 2-21 | 0.007 | 0.05 < IC₅₀ ≤ 0.5 | 2-62 | 0.0087 | 0.05 < IC₅₀ ≤ 0.5 |
| 2-22 | 0.0062 | 0.05 < IC₅₀ ≤ 0.5 | 2-63 | 0.0029 | IC₅₀ ≤ 0.05 |
| 2-23 | 0.0088 | 0.05 < IC₅₀ ≤ 0.5 | 2-64 | 0.0019 | IC₅₀ ≤ 0.05 |
| 2-24 | 0.0055 | 0.05 < IC₅₀ ≤ 0.5 | 2-65 | 0.0294 | 1.0 < IC₅₀ ≤ 10 |
| 2-25 | 0.0056 | 0.05 < IC₅₀ ≤ 0.5 | 2-66 | 0.0045 | 0.05 < IC₅₀ ≤ 0.5 |
| 2-26 | 0.0089 | 0.05 < IC₅₀ ≤ 0.5 | 2-67 | 0.0037 | 0.05 < IC₅₀ ≤ 0.5 |
| 2-27 | 0.006 | 0.05 < IC₅₀ ≤ 0.5 | 2-68 | 0.0029 | IC₅₀ ≤ 0.05 |
| 2-28 | 0,0037 | 0.05 < IC₅₀ ≤ 0.5 | 2-70 | 0.0045 | 0.05 < IC₅₀ ≤ 0.5 |
| 2-29 | 0.0062 | 0.05 < IC₅₀ ≤ 0.5 | 2-71 | 0.0094 | 1.0 < IC₅₀ ≤ 10 |
| 2-30 | 0.0119 | 0.05 <IC₅₀ ≤ 0.5 | 2-72 | 0.0039 | 0.05 < IC₅₀ ≤ 0.5 |
| 2-32 | 0.0039 | 0.05 < IG₅₀ ≤ 0.5 | 2-73 | 0.0035 | 0.05 < IC₅₀ ≤ 0.5 |
| 2-33 | 0.0054 | 0.5 < IC₅₀ ≤ 1.0 | 2-74 | 0.0026 | IC₅₀ ≤ 0.05 |
| 2-34 | 0.0037 | 0.05 < IC₅₀ ≤ 0.5 | 2-75 | 0.0036 | IC₅₀ ≤ 0.05 |

**[Table 3-3]**

| Example | TTK IC₅₀ (µM) | A549 IC₅₀ (µM) | Example | TTK IC₅₀ (µM) | A549 IC₅₀ (µM) |
|---|---|---|---|---|---|
| 2-76 | 0.0189 | 0.5 < IC₅₀ ≤ 1.0 | 2-127 | 0.005 | 0.05 < IC₅₀ ≤ 0.5 |
| 2-77 | 0.0213 | 0.5 < IC₅₀ ≤ 1.0 | 2-128 | 0.0074 | 0.05 < IC₅₀ ≤ 0.5 |
| 2-78 | 0.0035 | IC₅₀ ≤ 0.05 | 2-129 | 0.0067 | 0.05 < IC₅₀ ≤ 0.5 |
| 2-79 | 0.0059 | 0.05 < IC₅₀ ≤ 0.5 | 2-130 | 0.0097 | 0.05 < IC₅₀ ≤ 0.5 |
| 2-80 | 0.004 | IC₅₀ ≤ 0.05 | 2-131 | 0.0055 | 0.05 < IC₅₀ ≤ 0.5 |
| 2-81 | 0.004 | IC₅₀ ≤ 0.05 | 2-132 | 0.0068 | 0.05 < IC₅₀ ≤ 0.5 |
| 2-82 | 0.006 | IC₅₀ 0.05 | 2-133 | 0.0068 | 0.05 < IC₅₀ ≤ 0.5 |
| 2-83 | 0.0052 | 0.05 < IC₅₀ ≤ 0.5 | 2-134 | 0.0066 | 0.05 < IC₅₀ ≤ 0.5 |
| 2-84 | 0.0075 | 0.05 < IC₅₀ ≤ 0.5 | 2-135 | 0.0096 | 0.05 < IC₅₀ ≤ 0.5 |
| 2-85 | 0.004 | 1.0 < IC₅₀ ≤ 10 | 2-136 | 0.0073 | 0.05 < IC₅₀ ≤ 0.5 |
| 2-87 | 0.0062 | 0.05 < IC₅₀ ≤ 0.5 | 2-137 | 0.0073 | 0.05 < IC₅₀ ≤ 0.5 |
| 2-88 | 0.0022 | IC₅₀ ≤ 0.05 | 2-138 | 0.0055 | 0.05 < IC₅₀ ≤ 0.5 |
| 2-89 | 0.005 | 0.05 < IC₅₀ ≤ 0.5 | 2-139 | 0.0105 | 0.05 < IC₅₀ ≤ 0.5 |
| 2-90 | 0.0027 | 0.05 < IC₅₀ ≤ 0.5 | 2-140 | 0.0136 | 0.5 < IC₅₀ ≤ 1.0 |
| 2-91 | 0.0023 | IC₅₀ ≤ 0.05 | 2-141 | 0.007 | 0.05 < IC₅₀ ≤ 0.5 |
| 2-92 | 0.004 | 0.05 < IC₅₀ ≤ 0.5 | 2-142 | 0.013 | 0.05 < IC₅₀ ≤ 0.5 |
| 2-93 | 0.0033 | 0.05 < IC₅₀ ≤ 0.5 | 2-143 | 0.0198 | 0.5 < IC₅₀ ≤ 1.0 |
| 2-95 | 0.0025 | 0.05 < IC₅₀ ≤ 0.5 | 2-144 | 0.0035 | 0.05 < IC₅₀ ≤ 0.5 |
| 2-96 | 0.0075 | 0.05 < IC₅₀ ≤ 0.5 | 2-145 | 0.003 | 0.05 < IC₅₀ 0.5 |
| 2-97 | 0.0033 | IC₅₀ ≤ 0.05 | 2-146 | 0.0038 | 0.5 < IC₅₀ ≤ 1.0 |
| 2-98 | 0.0024 | IC₅₀ ≤ 0.05 | 2-147 | 0.0029 | 0.05 < IC₅₀ ≤ 0.5 |
| 2-101 | 0.0025 | 0.05 < IC₅₀ ≤ 0.5 | 2-148 | 0.0039 | 0.5 < IC₅₀ ≤ 1.0 |
| 2-102 | 0.003 | 0.05 < IC₅₀ ≤ 0.5 | 2-149 | 0.0054 | 0.05 < IC₅₀ ≤ 0.5 |
| 2-103 | 0.0044 | 0.05 < IC₅₀ ≤ 0.5 | 2-150 | 0.0038 | 0.05 < IC₅₀ ≤ 0.5 |
| 2-104 | 0.0068 | IC₅₀ ≤ 0.05 | 2-151 | 0.0031 | 0.05 < IC₅₀ ≤ 0.5 |
| 2-105 | 0.0066 | IC₅₀ ≤ 0.05 | 2-152 | 0.0036 | 0.05 < IC₅₀ ≤ 0.5 |
| 2-106 | 0.0064 | IC₅₀ ≤ 0.05 | 2-153 | 0.0075 | 0.05 < IC₅₀ ≤ 0.5 |
| 2-107 | 0.0344 | 0.05 < IC₅₀ ≤ 0.5 | 2-154 | 0.005 | 0.05 < IC₅₀ ≤ 0.5 |
| 2-109 | 0.0033 | 0.05 < IC₅₀ ≤ 0.5 | 2-155 | 0.0041 | 0.05 < IC₅₀ ≤ 0.5 |
| 2-110 | 0.0029 | 0.05 < IC₅₀ ≤ 0.5 | 2-156 | 0.0044 | 0.05 < IC₅₀ ≤ 0.5 |
| 2-111 | 0.0038 | 0.05 < IC₅₀ ≤ 0.5 | 2-157 | 0.0034 | 0.05 < IC₅₀ ≤ 0.5 |
| 2-112 | 0.0027 | IC₅₀ ≤ 0.05 | 2-158 | 0.0032 | 0.05 < IC₅₀ ≤ 0.5 |
| 2-113 | 0.0026 | IC₅₀ ≤ 0.05 | 2-159 | 0.0261 | 0.05 < IC₅₀ ≤ 0.5 |
| 2-114 | 0.0025 | 0.05 < IC₅₀ ≤ 0.5 | 2-160 | 0.0031 | 0.05 < IC₅₀ ≤ 0.5 |
| 2-116 | 0.0036 | IC₅₀ ≤ 0.05 | 2-161 | 0.0056 | 0.5 < IC₅₀ ≤ 1.0 |
| 2-117 | 0.0029 | IC₅₀ ≤ 0.05 | 2-162 | 0.0036 | 0.05 < IC₅₀ ≤ 0.5 |
| 2-118 | 0.0022 | IC₅₀ ≤ 0.05 | 2-164 | 0.0033 | 0.05 < IC₅₀ ≤ 0.5 |
| 2-125 | 0.0086 | 0.05 < IC₅₀ ≤ 0.5 | 2-165 | 0.0056 | 1.0 < IC₅₀ ≤ 10 |
| 2-126 | 0.0236 | 0.05 < IC₅₀ ≤ 0.5 | 2-166 | 0.0045 | 0.05 < IC₅₀ ≤ 0.5 |

**[Table 3-4]**

| Examples | TTK IC₅₀ (µM) | A549 IC₅₀ (µM) | Example | TTK IC₅₀ (µM) | A549 IC₅₀ (µM) |
|---|---|---|---|---|---|
| 2-167 | 0.0039 | 0,05 < IC₅₀ ≤ 0.5 | 2-210 | 0.0203 | 1.0 < IC₅₀ ≤ 10 |
| 2-168 | 0.0033 | 0.05 < IC₅₀ ≤ 0.5 | 2-211 | 0.0083 | 0.05 < IC₅₀ ≤ 0.5 |
| 2-169 | 0.0072 | 0.05 < IC₅₀ ≤ 0.5 | 2-212 | 0.0231 | 1.0 < IC₅₀ ≤ 10 |
| 2-170 | 0.0075 | 0.05 < IC₅₀ ≤ 0.5 | 2-213 | 0.012 | 0.05 < IC₅₀ ≤ 0.5 |
| 2-171 | 0.0035 | 0.05 < IC₅₀ ≤ 0.5 | 2-216 | 0.0027 | 0.05 < IC₅₀ ≤ 0.5 |
| 2-172 | 0.0203 | 0.5 < IC₅₀ ≤ 1.0 | 2-217 | 0.0139 | 1.0 < IC₅₀ ≤ 10 |
| 2-173 | 0.0054 | 0.05 < IC₅₀ ≤ 0.5 | 2-218 | 0.0126 | 0.5 < IC₅₀ ≤ 1.0 |
| 2-174 | 0.0141 | 0.05 < IC₅₀ ≤ 0.5 | 2-219 | 0.0043 | IC₅₀ ≤ 0.05 |
| 2-175 | 0.0114 | 0.05 < IC₅₀ ≤ 0.5 | 2-220 | 0.0039 | IC₅₀ ≤ 0.05 |
| 2-176 | 0.0056 | 0.05 < IC₅₀ ≤ 0.5 | 2-225 | 0.0097 | 0.05 < IC₅₀ ≤ 0.5 |
| 2-177 | 0.0143 | 0.05 < IC₅₀ ≤ 0.5 | 2-226 | 0.0069 | 0.05 < IC₅₀ ≤ 0.5 |
| 2-178 | 0.0071 | 0.05 < IC₅₀ ≤ 0.5 | 2-227 | 0.0035 | 0.05 < IC₅₀ ≤ 0.5 |
| 2-179 | 0.0037 | IC₅₀ ≤ 0.05 | 2-228 | 0.0036 | 0.05 < IC₅₀ ≤ 0.5 |
| 2-180 | 0.0122 | 1.0 < IC₅₀ ≤ 10 | 2-229 | 0.0044 | 0.05 < IC₅₀ ≤ 0.5 |
| 2-181 | 0.0102 | 0.05 < IC₅₀ ≤ 0.5 | 2-230 | 0.0043 | IC₅₀ ≤ 0.05 |
| 2-182 | 0.0084 | 0.05 < IC₅₀ ≤ 0.5 | 2-231 | 0.0057 | 0.05 < IC₅₀ ≤ 0.5 |
| 2-183 | 0.0057 | 0.05 < IC₅₀ ≤ 0.5 | 2-232 | 0.0028 | 0.05 < IC₅₀ ≤ 0.5 |
| 2-184 | 0.0056 | 0.05 < IC₅₀ ≤ 0.5 | 2-233 | 0.0057 | 0.5 < IC₅₀ ≤ 1.0 |
| 2-185 | 0.0027 | 0.05 < IC₅₀ ≤ 0.5 | 3-3 | 0.145 | 1.0 < IC₅₀ ≤ 10 |
| 2-186 | 0.0068 | 0.5 < IC₅₀ ≤ 1.0 | 3-4 | 0.527 | |
| 2-187 | 0.0078 | 0.5 < IC₅₀ ≤ 1.0 | | | |
| 2-188 | 0.0053 | 0.05 < IC₅₀ ≤ 0.5 | | | |
| 2-189 | 0.0086 | 0.5 < IC₅₀ ≤ 1.0 | | | |
| 2-190 | 0.0161 | 1.0 < IC₅₀ ≤ 10 | | | |
| 2-191 | 0.0039 | 0.05 < IC₅₀ ≤ 0.5 | | | |
| 2-192 | 0.0021 | 0.05 < IC₅₀ ≤ 0.5 | | | |
| 2-193 | 0.006 | 0.05 < IC₅₀ ≤ 0.5 | | | |
| 2-195 | 0.0051 | 0.05 < IC₅₀ ≤ 0.5 | | | |
| 2-199 | 0.0074 | 0.05 < IC₅₀ ≤ 0.5 | | | |
| 2-200 | 0.0072 | IC₅₀ ≤ 0.05 | | | |
| 2-201 | 0.02 | 0.05 < IC₅₀ ≤ 0.5 | | | |
| 2-202 | 0.0035 | 0.05 < IC₅₀ ≤ 0.5 | | | |
| 2-203 | 0.0047 | 0.05 < IC₅₀ ≤ 0.5 | | | |
| 2-204 | 0.0044 | 0.5 < IC₅₀ ≤ 1.0 | | | |
| 2-205 | 0.0045 | IC₅₀ ≤0.05 | | | |
| 2-206 | 0.0028 | 0.05 <IC₅₀ ≤ 0.5 | | | |
| 2-207 | 0.0057 | 0.5 < IC₅₀ ≤ 1.0 | | | |
| 2-208 | 0.0071 | 0.05 < IC₅₀ ≤ 0.5 | | | |
| 2-209 | 0.0082 | 0.05 < IC₅₀ ≤ 0.5 | | | |

### (Example 5) CYP3A4 fluorescent MBI test

The CYP3A4 fluorescent MBI test is a test for checking the extent of CYP3A4 inhibition of a compound by a metabolic reaction. CYP3A4 expressed by *Escherichia coli* was used as an enzyme. When benzene is removed from 7-benzyloxytrifluoromethyl coumarin (BFC) by the action of CYP3A4 enzyme, a fluorescence-emitting metabolite, 7-hydroxytrifluoromethyl coumarin (HFC) generates. The test was performed based on this reaction as an indicator.

The reaction conditions are as follows: substrate, 5.6 µmol/L 7-BFC; prereaction time, 0 or 30 minutes; reaction time, 15 minutes; reaction temperature, 25°C (room temperature); the content of CYP3A4 (enzyme expressed by *Escherichia coli*), 62.5 pmol/mL at prereaction time, 6.25 pmol/mL (diluted 10 fold) at reaction time; test drug concentration, 0.625, 1.25, 2.50, 5.00, 10.0, 20.0 µmol/L (6 samples).

To a 96-well plate, a pre-reaction solution, which contained an enzyme and a test drug solution in K-Pi buffer (pH 7.4) in accordance with the aforementioned composition for pre-reaction, was added. A part thereof was transferred to another 96-well plate so as to be diluted 1/10 fold with a substrate and K-Pi buffer, and then, NADPH as a coenzyme was added to initiate an indicator reaction (no pre-reaction). After the reaction was performed for a predetermined time, acetonitrile and 0.5 mol/L Tris (trishydroxyamiriomethane) were added in a ratio of 4: 1 to terminate the reaction. Furthermore, also to the remaining pre-reaction solution, NADPH was added to initiate the pre-reaction (pre-reaction was performed). After the pre-reaction was performed for a predetermined time, a part thereof was transferred to another plate so as to be diluted 1/10 fold with a substrate and the K-Pi buffer and the indicator reaction was initiated. After the reaction was performed for a predetermined time, acetonitrile and 0.5 mol/L Tris (trishydroxyaminomethane) was added in a ratio of 4: 1 to terminate the reaction. The plates in which respective indicator reactions were performed were measured for fluorescence, that is, fluorescence value of 7-HFC as a metabolite, by a fluorescent plate reader (Ex = 420 nm, Em = 535 nm).

DMSO, which is a solvent having a drug dissolved therein, was solely added to a reaction system. This was used as a control (100%) The residual activity (%) of each of the samples, in which a test drug solution was added in different concentrations, was calculated. IC₅₀ was calculated by inverse estimation using the concentration and suppression ratio and based on a logistic model. If the difference between IC₅₀ values was 5 µM or more, (+) was given, If the difference was 3 µM or less, (-) was given.

### (Example 6) CYP inhibition test

Using a commercially available pooled human liver microsome, how significantly production of a metabolite of a typical substrate is inhibited by a test compound was evaluated, based on typical substrate metabolic reactions of human major CYP5 molecular species, (CYPIA2, 2C9, 2C19, 2D6, 3A4), as indicators, which include O-deethylation of 7-ethoxyresorufin (CYP1A2), methyl-hydroxylation of tolbutamide (CYP2C9), 4'-hydroxidation of mephenytoin (CYP2C19), 0-demethylation (CYP2D6) of dextromethorphan and hydroxylation of terfenadine (CYP3A4).

The reaction conditions are as follows: substrates: 0.5 µmol/L ethoxyresorufin (CYPIA2), 100 µmol/L tolbutamide (CYP2C9), 50 µmol/L S-mephenytoin (CYP2C19), 5 µmol/L dextromethorphan (CYP2D6), 1 µmol/L terfenadine (CYP3A4); reaction time, 15 minutes; reaction temperature, 37°C; enzyme, pooled human liver microsome 0.2 mg protein/mL; test drug concentrations, 1.0, 5.0, 10, 20 µmol/L (4 samples).

To a 96-well plate, a reaction solution, which contains each of 5 types of substrates, human liver microsome, a test substance in a 50 mM Hepes buffer in accordance with the aforementioned composition, was added, and then, a coenzyme, i.e., NADPH, was added to initiate a reference metabolic reaction. After the reaction was performed at 37°C for 15 minutes, a solution containing methanol/acetonitrile = 1/1 (v/v) was added to terminate the reaction. After the resultant reaction solution was centrifuged at 3000 rpm for 15 minutes, resorufin (metabolite of CYP1A2) in the centrifugation supernatant was quantified by a fluorescent multi-label counter, and tolbutamide hydroxide (metabolite of CYP2C9), mephenytoin 4' hydroxide (metabolite of CYP2C19), dextrorphane (metabolite of CYP2D6) and terfenadine alcohol (metabolite of CYP3A4 metabolite) were quantified by LC/MS/MS.

DMSO, which is a solvent having a drug dissolved therein, was added to the reaction system. This was used as a control (100%). The residual activities (%) of the samples to which a test drug solution was added in different concentrations were calculated. IC₅₀ was calculated by inverse estimation using the concentration and suppression ratio and based on a logistic model.

### (Example 7) FAT test

Twenty µL of Rat's salmonella *typhimurium* (TA98 strain, TA100 strain) cryopreserved was inoculated in 10 mL-liquid nutrition medium (2.5% Oxoid nutrient broth No. 2) and pre-cultured with shaking at 37°C for 10 hours. A TA98-strain bacterial suspension (9 mL) was centrifuged (2000 × g, 10 minutes) to remove the culture solution. The bacterial cells were suspended in 9 mL of Micro F buffer (K2HPO4: 3.5 g/L, KH2PO4: 1 g/L, (NH4)2SO4: 1g/L, trisodium citrate dihydrate: 0.25 g/L, MgSO₄·7H₂0: 0.1 g/L) and added to 110 mL of Exposure medium (MicroF buffer containing biotin: 8 µg/mL, histidine: 0.2 µg/mL, glucose: 8 mg/mL). TA100 strain bacterial suspension (3.16 mL) was added to Exposure medium (120 mL) to prepare a test bacterial suspension. Each (12 µL) of the solutions: a test substance-containing DMSO solution (serially diluted 8 times in a common ratio of 2 from a maximum dose of 50 mg/mL); DMSO serving as a negative control; positive controls serving in metabolism deactivation conditions, i.e., a 50 µg/mL 4-nitroquinoline-1-oxide DMSO solution (for TA98 stain), and a 0.25 µg/mL 2-(2-furyl)-3-(5-nitro-2-furyl)acrylamide DMSO solution (for TA100 strain); and positive controls serving in metabolism activation conditions, i.e., a 40 µg/mL 2-aminoanthracene DMSO solution (for TA98 strain) and a 20 µg/mL 2-aminoanthracene DMSO solution (for TA100 strain), was mixed with a test bacterial suspension (588 µL) (in metabolism activation conditions, a solution mixture of a test bacterial suspension (498 µL) and S9 mix (90 µL)) and cultured with shaking at 37°C for 90 minutes. A bacterial suspension (460 µL) exposed to a test substance was mixed in 2300 µL of Indicator medium (MicroF buffer containing biotin: 8 µg/mL, histidine: 0.2 µg/mL, glucose: 8 mg/mL, bromocresol purple: 37.5 µg/mL) and an aliquot (50 µL) was taken and dispensed to each microplate (48 wells/dose) and subjected to stationary culture performed at 37°C for 3 days. Since a well containing a bacterial cell, which acquired proliferation potency by spontaneous mutation of a gene for an amino acid (histidine) synthesis enzyme, changes color from purple to yellow due to pH change, the number of wells of proliferated bacterial cells changed into yellow in the 48 wells per dose was counted. Evaluation was made in comparison with the negative control group.

### (Example 8) Solubility test

The solubility of a compound was determined under 1% DMSO addition conditions. A 10 mM compound solution was prepared using DMSO. The compound solution (6 µL) was added to 594 µL of artificial intestinal juice (pH 6.8), (which was prepared by adding a 0.2 mol/L NaOH test solution (118 mL) to a 0.2 mol/L potassium dihydrogen phosphate test solution (250 mL) and adding water to obtain 1000 mL). After allowed to stand still at 25°C for 16 hours, the solution mixture was filtrated by aspiration. The filtrate was diluted two fold with methanol/water = 1/1. The concentration of the compound in the filtrate was measured by the absolute calibration curve method using HPLC or LC/MS/MS.

### (Example 9) Metabolic stability test

Compounds of interest each were reacted with a commercially available pooled human liver microsome for a predetermined time. A residual rate was calculated by comparison between samples where a reaction occurred and samples where no reaction occurred. In this manner, the degree of the compound metabolized by the liver was evaluated.

In a human liver microsome (0.5 mg protein/mL)-containing buffer (0.2 mL) (50 mmol/L tris-HCl pH7.4, 150 mmol/L potassium chloride, 10 mmol/L magnesium chloride), a reaction was performed in the presence of 1 mmol/L NADPH at 37°C for 0 minute or 30 minutes (oxidative reaction). After complexion of the reaction, the reaction solution (50 µL) was added to methanol/acetonitrile =1/1 (v/v) solution (100 µL), mixed and centrifuged at 3000 rpm for 15 minutes. The test compound in the centrifugation supernatant was quantified by LC/MS/MS. The residual amount of test compound after complexion of the reaction was calculated by regarding the amount of compound at the time (0 minute) of the reaction as 100%.

### (Example 10) hERG test

To evaluate risk of extending the QT interval of cardiac electrogram, using HEK293 cells in which a human ether-a-go-go related gene (hERG) chancel was expressed, action on delayed rectifier K+ current (IKr which plays an important role in ventricular depolarization process, was investigated.

Using an automatic patch clump system (PatchXpress 7000A, Axon Instruments Inc.), cells were held at a membrane potential of -80 mV by a whole-cell patch clump method, and then, a depolarization stimulation of + 50 mV was applied for 2 seconds, and further a repolarization stimulation of -50 mV was applied for 2 seconds. IKr induced at this time was recorded. After the veneration current became stable, an extracellular fluid (NaCl: 137 mmol/L, KCl: 4 mmol/L, CaCl₂·2H₂O: 1.8 mmol/L, MgCl₂.6H₂O: 1 mmol/L, glucose: 10 mmol/L, HEPES (4-(2-hydroxyethyl)-1-piperazineethane-sulfonic acid) : 10 mmol/L, pH-=7.4), in which a test substance was dissolved in a desired concentration (1.0 µM), was applied to the cells under room temperature conditions for 10 minutes. The IKr obtained was analyzed by use of analysis software (DataXpress ver. 1, Molecular Devices Corporation) to obtain an absolute maximum tail current value based on the current value at the membrane potential at which the cells were maintained. Furthermore, an inhibition rate of the maximum tail current before the test substance was applied was calculated and compared to that of a group of cells applied to a medium (0.1% dimethylsulfoxide solution) to evaluate the effect of the test substance on IKr.

### (Example 11: Preparation Example 1 Tablet)

Tables having the following composition are produced by a customary method.

| | |
|---|---|
| Compound of the present invention | 100 mg |
| Lactose | 60 mg |
| Potato starch | 30 mg |
| Polyvinyl alcohol | 2 mg |
| Magnesium stearate | 1 mg |
| Tar pigment | trace amount. |

### (Example 12: Preparation Example 2 Powder)

Power having the following composition is produced by a customary method.

| | |
|---|---|
| Compound of the present invention | 150 mg |
| Lactose | 280 mg. |

### (Example 13: Preparation Example 3 Syrup)

Syrup having the following composition is produced by a customary method.

| | |
|---|---|
| Compound of the present invention | 100 mg |
| Purified white sugar | 40 g |
| Ethyl p-hydroxybenzoate | 40 mg |
| Propyl p-hydroxybenzoate | 10 mg |
| Chocolate flavor | 0.1cc |

To this, water is added to obtain a total volume of 100 cc.

In the foregoing, the present invention has been described by way of the preferred embodiment of the present invention; however, the present invention should not be construed to be limited to the embodiment. It is understood that the range of the present invention should be interpreted based on the scope of the claims. It is understood that those skilled in the art can carry out the equivalent range from the description of preferred embodiments specifically disclosed in the present invention and based on the description of the present invention and common technical knowledge. It is understood that the contents per se of patents, publications of Japanese Patent Application and literatures cited in the specification are specifically described in the specification as well as should be incorporated herein by reference.

The present application was based on Patent Application No. 2009-178452 filed in Japan and the content thereof should be incorporated herein in its entirety.

### INDUSTRIAL APPLICABILITY

The present invention provides a medicament for treating a TTK kinase dependent disease, a compound to be used in the medicament, a pharmaceutically acceptable salt thereof or a solvate thereof. The compound of the present invention exerts an excellent TTK kinase inhibitory action as described in Examples mentioned above.

## Claims

1. A compound represented by Formula (I): 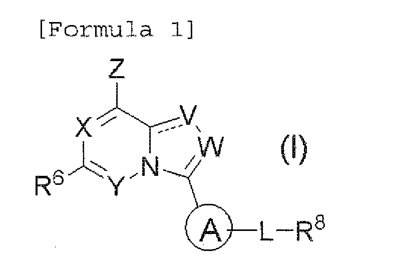 wherein
X, Y, V and W are any one of the following (X, Y, V, W) combinations:
(-N=, =CR¹-, =N-, -CR⁷=), (-CR²=, -N-, =N-, -CR⁷=), (-N=, =N-, =N-, -CR⁷=), (-N=, =CR¹-, =N-, -N=) and (-N=, =CR¹-, -O-, -N=),
R¹ and R² are each independently hydrogen, a halogen, a hydroxy, a cyano, a nitro, a carboxy, a substituted or unsubstituted alkyl, a substituted or unsubstituted alkenyl or a substituted unsubstituted alkynyl,
Z is a group represented by Formula: -NR³R⁴ or a group represented by Formula: -OR⁵,
R³ is hydrogen or a substituted or unsubstituted alkyl,
R⁴ and R⁵ are each independently hydrogen, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted alkoxy or a substituted or unsubstituted alkylsulfonyl,
R⁶ is hydrogen, a halogen, a hydroxy, a cyano, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted amino, a substituted or unsubstituted acyl, a substituted or unsubstituted alkoxy, a substituted or unsubstituted aryloxy, a substituted or unsubstituted heteroaryloxy, a substituted or unsubstituted cycloalkyloxy, a substituted or unsubstituted heterocyclyloxy, a substituted or unsubstituted carbamoyl, a group represented by Formula: -SO₂-R', a group represented by Formula: -SO-R', a group represented by Formula: -SR', a group represented by Formula: -O-N=C(R")₂ or a group represented by Formula: -O-N(R")₂ where two R" are each independently hydrogen, a substituted or unsubstituted alkyl, or two R" may be taken together with an adjacent carbon atom or nitrogen atom to form a substituted or unsubstituted nonaromatic, hydrocarbon ring or nonaromatic heterocyclic ring,
R' is hydrogen, a substituted or unsubstituted alkyl, a substituted or unsubstituted amino, a substituted or unsubstituted aryl, a substituted or unsubstituted cycloalkyl, a substituted or unsubstituted heteroaryl or a substituted or unsubstituted heterocyclyl,
R⁷ is hydrogen, a halogen, a hydroxy, a cyano, a nitro, a carboxy, a substituted or unsubstituted alkyl, a substituted or unsubstituted alkenyl or a substituted or unsubstituted alkynyl,
A is a substituted or unsubstituted aromatic hydrocarbon ring, a substituted or unsubstituted aromatic heterocyclic ring, a substituted or unsubstituted nonaromatic, hydrocarbon ring or a substituted or unstibstituted nonaromatic heterocyclic ring,
L is a single bond, -C(=O)-NR^{A}-, -NR^{B}-C(=O)-, -S(O)ₙ-NR^{C}-, -NR^{D}-S(O)ₙ-, a substituted or unsubstituted alkylene, a substituted or unsubstituted alkenylene or a substituted or unsubstituted alkynylene,
R⁸ is hydrogen, a halogen, a hydroxy, a cyano, a carboxy, a substituted or unsubstituted alkoxy, a substituted or unsubstituted alkyl, a substituted or unsubstituted alkenyl, a substituted or unsubstituted alkynyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl or a substituted or unsubstituted amino,
R^{A}, R^{B}, R^{C}, R^{D} are each independently hydrogen, a substituted or unsubstituted alkyl, or
R⁸ and R^{A}, or R⁸ and R^{C} may be taken together with an adjacent nitrogen atom to form a substituted or unsubstituted nitrogen-containing heterocyclic ring,
n is an integer of 1 or 2,
when (X, Y, V, W) is (-N=, =CR¹-, =N-, -CR⁷=) and Z is a group represented by Formula: -NR³R⁴, L is -C(=O)-NR^{A}-;
with the proviso that when (X, Y, V, W) is (-N=, =CR¹-, =N-, -CR⁷=) and Z is a group represented by Formula: -OR⁵, R⁶ is not halogen,
when (X, Y, V, W) is (-N=, =CR¹-, =N-, -CR⁷=), Z is a group represented by Formula: -NR³R⁴ and L is -C(=O)-NR^{A}-, R⁸ is not an alkyl substituted with an amino, a hydroxy, a pyridyl or a heterocyclyl, or hydrogen, and R⁸ and R^{A} are not taken together with an adjacent nitrogen atom to form a substituted or unsubstituted nitrogen-containing heterocyclic ring;
with the proviso that the following compounds are excluded: 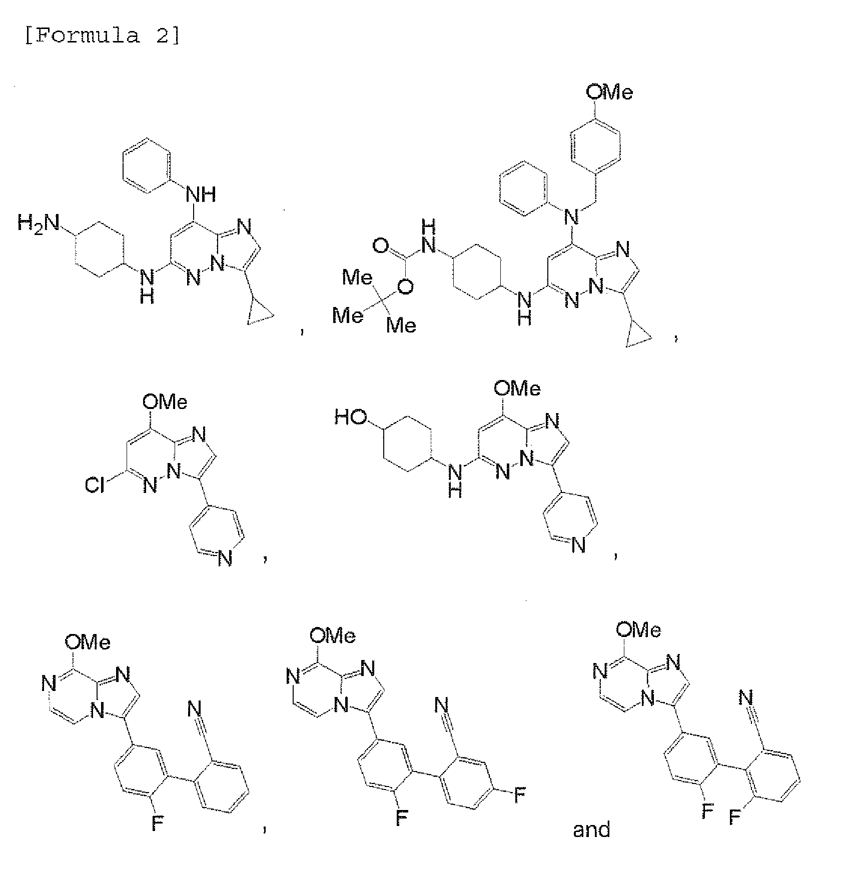
; a pharmaceutically acceptable salt thereof or a solvate thereof.

2. The compound according to claim 1, wherein (X, Y, V, W) is (-N=, =CR¹-, =N-, -CR⁷=), (-CR²=, =N-, =N-, -CR⁷=), (-N=, =CR¹-, =N-, -N=) or (-N=, =CR¹-, -O-, -N=) where R¹, R² and R⁷ are the same as defined in claim 1; a pharmaceutically acceptable salt thereof or a solvate thereof.

3. The compound according to claim 1 or 2, wherein (X, Y, V, W) is (-N=, =CH-, =N-, -CR⁷=), (-CH=, =N-, =N-, -CR⁷=), (-N=, =CH-, =N-, -N-) or (-N=, =CH-, -O-, -N=) where R⁷ is the same as defined in claim 1; a pharmaceutically acceptable salt thereof or a solvate thereof.

4. The compound according to claim 1 or 2, wherein Z is a group represented by Formula: -NR³R⁴ where R³ and R⁴ are the same as defined in claim 1; a pharmaceutically acceptable salt thereof or a solvate thereof.

5. The compound according to claim 1 or 2, wherein Z is a group represented by Formula: -NHR⁴ where R⁴ is the same as defined in claim 1; a pharmaceutically acceptable salt thereof or a solvate thereof.

6. The compound according to claim 4, wherein R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl; a pharmaceutically acceptable salt thereof or a solvate thereof.

7. The compound according to claim 1 or 2, wherein Z is a group represented by Formula: -OR⁵ where R⁵ is the same as defined in claim 1; a pharmaceutically acceptable salt thereof or a solvate thereof.

8. The compound according to claim 1 or 2, wherein R⁵ is a substituted or unsubstituted alkyl or a substituted or unsubstituted heterocyclylalkyl; a pharmaceutically acceptable salt thereof or a solvate thereof.

9. The compound according to claim 1 or 2, wherein A is a substituted or unsubstituted aromatic hydrocarbon ring or a substituted or unsubstituted aromatic heterocyclic ring; a pharmaceutically acceptable salt thereof or a solvate thereof.

10. The compound according to claim 1 or 2, wherein L is-C(=O)-NR^{A}-, -NR^{B}-C(=O)- or -S(O)ₙ-NR^{C}- where R^{A}, R^{B}, R^{C} and n are the same as defined in claim 1; a pharmaceutically acceptable salt thereof or a solvate thereof.

11. The compound according to claim 1 or 2, wherein R⁸ is a substituted or unsubstituted alkyl, a substituted or unsubstituted cycloalkyl or a substituted or unsubstituted aryl; a pharmaceutically acceptable salt thereof or a solvate thereof.

12. The compound according to claim 1 or 2, wherein L is - C(=O)-NH-, and R⁸ is a substituted or unsubstituted cycloalkyl; a pharmaceutically acceptable salt thereof or a solvate thereof.

13. The compound according to claim 1 or 2, wherein R⁶ is hydrogen, a halogen, a substituted or unsubstituted alkyl, a substituted or unsubstituted alkenyl, a substituted or unsubstituted cycloalkyl, a substituted or unsubstituted cycloalkenyl, a substituted or unsubstituted aryl, a substituted or unsubstituted heteroaryl, a substituted or unsubstituted heterocyclyl, a substituted or unsubstituted alkoxy, a substituted or unsubstituted aryloxy, a substituted or unsubstituted heteroaryloxy, a substituted or unsubstituted cycloalkyloxy, a substituted or unsubstituted heterocyclyloxy, a substituted or unsubstituted acyl or a substituted or unsubstituted amino,; a pharmaceutically acceptable salt thereof or a solvate thereof.

14. The compound according to claim 1 or 2, wherein R⁷ is hydrogen; a pharmaceutically acceptable salt thereof or a solvate thereof.

15. The compound according to claim 1 wherein
(X, Y, V, W) is (-N=, =CR¹-, =N-, -CR⁷=) where R¹ and R⁷ are the same as defined in claim 1,
Z is a group represented by Formula: -NHR⁴,
R⁴ is an alkyl substituted with a substituted or unsubstituted aryl, a substituted or unsubstituted cycloalkyl, a substituted or unsubstituted cycloalkenyl, a substituted or unsubstituted heteroaryl or a substituted or unsubstituted heterocyclyl; or R⁴ is an unsubstituted alkyl; A is a substituted or unsubstituted aromatic hydrocarbon ring,
L is -C(=O)-NH-, and
R⁸ is a substituted or unsubstituted cycloalkyl; a pharmaceutically acceptable salt thereof or a solvate thereof.

16. The compound according to claim 1 wherein
(X, Y, V, W) is (-CR²=, =N-, =N-, -CR⁷=) where R² and R⁷ are the same as defined in claim 1, and
A is a substituted or unsubstituted aromatic hydrocarbon ring; a pharmaceutically acceptable salt thereof or a solvate thereof.

17. A pharmaceutical composition containing the compound according to any one of claims 1 to 16, a pharmaceutically acceptable salt thereof or a solvate thereof.

18. The pharmaceutical composition according to claim 17, as a TTK inhibitory agent.
